# EUROPEAN PATENT SPECIFICATION

(11) **EP 1 597 578 B1**
(45) Date of publication and mention of the grant of the patent: **20.12.2006**
(21) Application number: 04715425.7
(22) Date of filing: 27.02.2004
(51) Int. Cl.: G01N 33/53, C12Q 1/68

(54) **STANDARD FOR IMMUNOHISTOCHEMISTRY, IMMUNOCYTOCHEMISTRY AND MOLECULAR CYTOGENETICS.**
STANDARD FÜR IMMUNHISTOCHEMIE, IMMUNZYTOLOGIE UND MOLEKULARE ZYTOGENETIKA
ETALON POUR IMMUNOHISTOCHIMIE, IMMUNOCYTOCHIMIE ET CYTOGENETIQUE MOLECULAIRE

(30) Priority: 27.02.2003 GB 0304515; 03.03.2003 US 451589 P
(43) Date of publication of application: 23.11.2005
(73) Proprietor: Dako Denmark A/S, 2600 Glostrup (DK)
(72) Inventor: Winther, Lars, DK-2600 Glostrup (DK)
(74) Representative: Khoo, Chong-Yee
(86) International application number: PCT/IB2004/001173
(87) International publication number: WO 2004/077057

(56) References cited:
- EP-A- 0 345 953
- WO-A-01/55346
- US-A- 5 187 099
- US-A- 5 541 059
- US-A- 5 610 022
- US-A- 5 846 749
- US-B1- 6 281 004
- US-B1- 6 509 196

## Description

### FIELD

This invention pertains to the fields of cytology and histology. In particular, the invention is related to the fields of immunohistochemistry and molecular cytogenetics, in particular, the provision of standards for gauging the presence or amount of detectable entities in cells, tissues and organs.

### BACKGROUND

Histological and cytological techniques have been used to analyse biopsies and other tissue samples, as an aid to medical diagnosis. Cytology is the study of the structure of all normal and abnormal components of cells and the changes, movements, and transformations of such components. Cells are studied directly in the living state or are killed (fixed) and prepared by for example embedding, sectioning, or staining for investigation in bright field or electron microscopes.

One well-known cytology procedure is the Papanicolaou test medical procedure used to detect cancer of the uterine cervix. A scraping, brushing, or smear, is taken from the surface of the vagina or cervix and is prepared on a slide and stained for microscopic examination and cytological analysis. The appearance of the cells determines whether they are normal, suspicious, or cancerous.

Histology is the study of groups of specialised cells called tissues that are found in most multi-cellular plants and animals. Histological investigation includes study of tissue death and regeneration and the reaction of tissue to injury or invading organisms. Because normal tissue has a characteristic appearance, histologic examination is often utilised to identify diseased tissue. Immunohistochemistry, IHC, and *in situ* hybridisation, ISH, analysis are useful tools in histological diagnosis and the study of tissue morphology.

Both immunohistochemistry (IHC) and *in situ* hybridisation (ISH), seek to detect a detectable entity in a sample by using specific binding agents capable of binding to the detectable entity. In immunohistochemistry (IHC), the specific binding agent comprises an antibody, and the detectable entity comprises a polypeptide, protein, or epitope comprised therein. In *in situ* hybridisation (ISH), the detectable entity comprises a nucleic acid (including DNA and RNA) in the sample, and the specific binding agent comprises a probe such as a nucleic acid probe. The increasing availability of such antibodies and probes may help in differential diagnosis of diseased and normal tissue. In situ hybridisation and immunochemistry methods are described in detail in Harlow and Lane (Antibodies: A Laboratory Manual).

IHC and ISH techniques require a series of treatment steps conducted on a tissue section mounted on a glass slide or other planar support to highlight by selective staining certain morphological indicators of disease states.

Thus, for example in IHC, a sample is taken from an individual, fixed and exposed to antibodies against the antigen of interest. Further processing steps, for example, antigen retrieval, exposure to secondary antibodies (usually coupled to a suitable enzyme), washing, and to chromogenic enzyme substrates, etc may be necessary to reveal the pattern of antigen binding. There are in general two categories of histological materials: (a) preparations, comprising fresh tissues and/or cells, which generally are not fixed with aldehyde-based fixatives, and (b) fixed and embedded tissue specimens, often archive material.

In ISH, a sample is taken from an individual, fixed and exposed to a probe against the nucleic acid of interest. The detectable entity typically comprises a detectable nucleic acid, such as DNA and RNA, including messenger RNA. Detection of DNA/RNA levels indicates the level of expression of a particular gene, and hence may be used to detect a condition (such as a disease condition) of a cell, tissue, organ or organism. The detectable entity being a nucleic acid is typically denatured to expose binding sites. The probe is typically a double or single stranded nucleic acid, such as a DNA or RNA, and is labelled using radioactive labels such as ³¹P, ³³P or ³²S, or non-radioactively, using labels such as digoxigenin, or fluorescent labels, a great many of which are known in the art.

Many methods of fixing and embedding tissue specimens are known, for example, alcohol fixation. However, the most widely used fixing/embedding technique employs formalin-fixation and subsequent paraffin embedding, FFPE. A "typical" FFPE IHC staining procedure may involve the steps of: cutting and trimming tissue, fixation, dehydration, paraffin infiltration, cutting in thin sections, mounting onto glass slides, baking, deparaffination, rehydration, antigen retrieval, blocking steps, applying primary antibody, washing, applying secondary antibody - enzyme conjugate, washing, applying enzyme chromogen substrate, washing, counter staining, cover slipping and microscope examination. Similar steps take place in ISH. The amount of the relevant antigen or other detectable entity such as a nucleic acid detected by such techniques is then assessed to determine whether it is above a certain pre-determined minimum threshold, and therefore diagnostically relevant. Suitable treatment may then be planned for the individual if necessary.

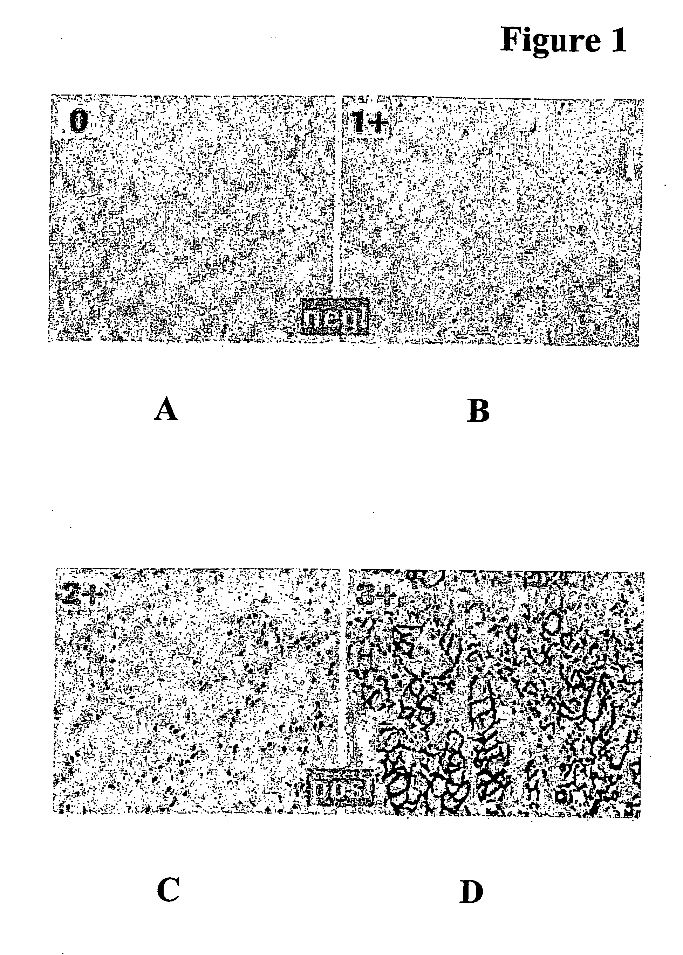

An example of immunohistochemical staining for diagnosis is shown in Figure 1, where tissues are stained for the breast cancer antigen HER2. Tissues which not express the antigen are not stained substantially by anti-HER2 antibody (Figure 1A), while those which do express the protein are stained to a substantial degree by anti-HER2 antibody (Figure 1D).

However, a major problem in such IHC and ISH techniques arises from the necessity of making an accurate determination of whether cells in a tissue being examined express the antigen or detectable entity such as a nucleic acid at a diagnostically significant level or not (i.e., whether the cells are "positive" or "negative" for expression of an antigen). There is a general lack of standardisation of laboratory techniques, leading to the requirement for a subjective judgement of the results. Even small procedural differences can influence the final staining outcome, and the final interpretation of the staining of the same cell population may not be exactly the same from laboratory to laboratory. Even when internal controls (including positive or negative controls, or both) are included in the procedures, variations in the pre-treatment and staining protocols between different workers will cause variations in the internal control. Other analytic sources of error include the quality and quantity of reagents, efficiency of antigen-retrieval and differences in instrumentation.

These problems hinder the assessment of new, alternative, prognostic factors, resulting in contradictory results for most of the prognostic factors in relation to their prognostic value.

The necessity for grading and standardisation has been addressed in the prior art in a number of ways. For example, a diagnostic kit may contain photomicrographs of different sets of cells at various levels of staining. The kit contains an indication that a particular set of cells represents the cut-off or threshold point, with cells matching or exceeding that staining being "positive", with those cells having less staining being "negative". More sophisticated kits may contain actual samples of tissues or cells, which are already stained, on control slides. For example, a kit may include several reference slides with sections of FFPE breast carcinoma cell lines that represent different levels of a breast specific protein expression. An example of such a grading system for the HER2 antigen is shown in Figure 1, where reference staining levels of 0 or 1+ are considered negative (Figures 1A and 1B respectively), while those of 2+ or 3+ are considered positive for that antigen (Figures 1C and 1D respectively).

Written descriptions relating to the pattern or distribution of staining may be included to aid the analysis. For example, Score 0 (negative): No staining is observed, or membrane staining is observed in less than 10 % of tumour cells. Score 1+ (negative): A faint or barely perceptible membrane staining is detected in more than 10 % of the tumour cells. The cells are only stained in part of the membrane. Score 2+ (weakly positive): a weak to moderate complete membrane staining is observed in more than 10% of the tumour cells. Score 3+ (strongly positive): a strong complete membrane staining is observed in more than 10 % of the tumour cells.

However, although it is possible with such kits to establish the reference levels of staining, there exists considerable difficulty in establishing a consistent quality of staining of the samples themselves. This arises from a variety of different factors, including inhomogeneous tissue material, the laborious and complex nature of the procedures, variability in reagent quality (including antibody/probe affinity and specificity), and the subjective nature of the interpretation carried out by the practitioner. Furthermore, other sources of variability in sample staining include the conditions under which tissue samples are collected, processed and stored, variability in epitope retrieval procedures, and enzyme catalysed chromogen precipitation.

As an attempt to solve these problems, it is known in the prior art to include reference sets of unstained tissues or cells with different levels of expression of the relevant antigen (or nucleic acid) with a diagnostic kit. The cells making up the references may comprise biopsy samples (i.e., tissue samples) from known diseased and un-diseased individuals, or individuals which express antigen or relevant detectable nucleic acid at higher than normal levels but are not clinically diseased. Furthermore, tissue culture cells, which may be transfected with expression vectors to enable them to express the antigen or detectable nucleic acid at various levels, may also be used as reference standards. The reference sets comprise slides with fixed and formalin embedded cells, but are otherwise unstained, and which are embedded in paraffin. The slides are then processed in parallel with the sample to reveal the level and pattern of antigen (or detectable nucleic acid) staining. Finally, the sample is compared to the reference set to determine whether protein or nucleic acid expression levels are diagnostically significant.

Examples of cell lines currently used as reference cells in cytochemistry include the HER2 positive cell line SK-BR-3, the estrogen receptor, ER, positive and progesterone receptor, PR, negative and p53 positive HCC70 cell line, the PR positive and ER negative HCC2218 cell line, the Epidermal Growth Factor Receptor, (EGFR) positive NCI-H23 cell line, the prostate specific antigen (PSA) and androgen receptor positive MDA PCA 2b cell line, and the cytokeratin 19 and the p53 positive, HCC38 cell line. Many human and non-human cell lines are obtainable through various organizations.

However, a problem exists with such reference standards in that it is necessary to specifically identify tissues and cells which express antigen or detectable nucleic acid at the various grading levels, and to obtain them for use. Where tissue culture cells are used, it is necessary to first clone the gene in question, then design and construct appropriate expression vectors. These vectors are then required to be transfected into the cells, and the expression of the gene to be regulated at the right level. As the cell lines are grown continuously and in many laboratories, the level of protein expression can change over time, and may not be have the same protein or mRNA expression from laboratory to laboratory. Both transient and stable transfected cell lines are labile during growth, resulting in changing expression of targets and consequently changing staining level and patterns.

Furthermore, there are issues with the need to include potentially hazardous biological material with the diagnostic kits. Regulatory and ethical problems are associated with the use of material of human origin; such human material is not easy to obtain in large amount from single sources, and may need to be pooled, leading to further variability.

Other techniques known include the use of reference dots made of polymer gels, which are attached to glass slides. The polymer material contains relevant epitopes to be stained. The quality control devices described in WO 00/62064 and Sompuram et al. Clin. Chem., 48 (3), p. 410, 2002 employ surrogate analytic targets, comprising synthetic peptides which resemble the 3D conformation of the epitope to which an antibody binds, which are applied to and coupled to a top surface of a glass slide.

EP1158047 describes describes a DNA microarray or DNA chip comprising a high density planar two-dimensional alignment with immobilised nucleic acids. EP 1158047 describes a method of production of such a microarray by immobilising the nucleic acids on fibres, producing a fibre alignment, and producing a "slice" from the fibre alignment. The microarrays may be used for hybridisation to detect substances in samples, and specifically for genome analysis. In other words, the nucleic acids immobilised on the fibres are used as probes, and EP1158047 is not concerned with the quantities of the nucleic acids immobilised on the fibre. Specifically, EP1158047 does not disclose or suggest that it is possible to use the microarrays to establish fixed or predetermined quantities of the nucleic acids immobilised on the fibres, or signal levels derived from such, for the purposes of grading or standarisation.

EP 0345953 discloses a test/control procedure and material to facilitate standardisation of immunostaining techniques and the assessment of their results. Pellets of an absorbent gel such as agar gel are caused to adsorb individual specific concentrations of an antigen of interest. The adsorbed antigens are confined to the individual pellets as by fixation or by enclosure in a diffusion-inhibiting barrier, and the pellets are installed in individual wells in a block of the gel in a manner to become integrated therein. The block may then be subjected to the same preparative routines as a tissue sample, sectioned and mounted like the sample, and then subjected to immunostaining by the same routine as the sample sections to provide a valid basis for assessment of the stained sample sections by comparison with the stained gel block sections. These reference samples do however not resemble the shape and size of a cell.

### SUMMARY

According to a first aspect of the present invention, we provide a method comprising: (a) providing a reference standard or a planar section thereof, the reference standard comprising (i) a support medium; and (ii) a quantity of a detectable entity supported by the support medium; in which the detectable entity has an elongate path the cross-sectional profile of which has a size between 0.5 µm to 100 µm; in which a predetermined amount of the detectable entity is present in a defined region in a cross section of the reference standard; (b) obtaining a first reference signal indicative of the presence or quantity of detectable entity in the reference standard, planar section thereof, or the defined region; (c) providing a biological sample and obtaining a second signal indicative of the presence or quantity of detectable entity in the biological sample, or a component thereof; and (d) comparing the reference signal obtained in (b) against the second signal obtained in (c).

There is provided, according to a second aspect of the present invention, a method of obtaining an indication of the presence, quantity or concentration of a detectable entity in a biological sample according to the first aspect of the invention, in which the reference signal is compared to the second signal to determine the presence, quantity or concentration of the detectable entity in the sample.

Preferably, the reference standard or planar section thereof is subjected to the same one or more steps or conditions, preferably substantially all, as the biological sample.

Preferably, the reference standard or planar section thereof is processed through one or more, preferably all, of the following steps: mounting onto a slide, baking, deparaffination, rehydration, antigen retrieval, blocking, exposure to antibody, exposure to primary antibody, exposure to nucleic acid probe, washing, exposure to secondary antibody-enzyme conjugate, exposure to enzyme substrate, exposure to chromogen substrate and counter staining.

Preferably, the detectable entity is selected from the group consisting of: a hapten, a biologically active molecule, an antigen, an epitope, a protein, a polypeptide, a peptide, an antibody, a nucleic acid, a virus, a virus-like particle, a nucleotide, a ribonucleotide, a deoxyribonucleotide, a modified deoxyribonucleotide, a heteroduplex, a nanoparticle, a synthetic analogue of a nucleotide, a synthetic analogue of a ribonucleotide, a modified nucleotide, a modified ribonucleotide, an amino acid, an amino acid analogue, a modified amino acid, a modified amino acid analogue, a steroid, a proteoglycan, a lipid, a carbohydrate, a dye, a diagnostically relevant target, preferably selected from the group consisting of: an antigen, an epitope, a peptide, a polypeptide, a protein, a nucleic acid, or two or more or a plurality of any of the above, or mixtures, fusions, combinations or conjugates of one or more of the above.

Preferably, the detectable entity comprises any one or more of HER2, ER, PR, p16, Ki-67 and EGFR protein, and nucleic acids encoding such.

Preferably, the method further comprises providing a second reference standard or a planar section thereof, obtaining a second reference signal indicative of the quantity of detectable entity in the second reference standard, planar section thereof or the defined region; and comparing the second signal obtained in (c) against the first reference signal and the second reference signal.

Preferably, the presence and/or quantity of the detectable entity is revealable by a binding agent, preferably a labelled binding agent, preferably selected from the group consisting of: an antibody, preferably an antibody capable of specific binding to the detectable entity, a nucleic acid such as a DNA or an RNA, preferably a nucleic acid capable of specific binding to the detectable entity, a protein nucleic acid (PNA), a dye, a special stain, Haematoxylin-Eosin (H & E), Gomori methenamine silver stain (GMS), Periodic Acid-Schiff (PAS) stain, Trichrome Blue, Masson's Trichrome, Prussian Blue, Giemsa, Diff-Quik, Reticulum, Congo Red, Alcian Blue, Steiner, AFB, PAP, Gram, Mucicarmine, Verhoeff-van Gieson, Elastic, Carbol Fuchsin and Golgi's stains.

Preferably, the detectable signal is selected from the group consisting of: radiation, optical density, reflectance, radioactivity, fluorescence, enzymatic activity.

Preferably, (a) the reference standard is in the shape of a rectangular box, and the detectable entity is in the form of a substantially linear rod disposed along or substantially parallel to a long axis of the reference standard; or (b) the defined region is present in at least one other cross section of the reference standard, preferably comprising a similar amount of detectable entity; or (c) the detectable entity has a substantially uniform distribution along the elongate path; or (d) the detectable entity is present at substantially the same area, quantity or concentration in all transverse planar sections of the reference standard; or any combination of the above.

Preferably, the support medium comprises an embedding medium, in which the detectable entity is embedded, the embedding medium preferably being selected from the group consisting of: ice, wax, paraffin, acrylic resin, methacrylate resin, epoxy, Epon, Araldite, Lowicryl, K4M and LR White and Durcupan.

Preferably, the detectable entity is attached, preferably chemically coupled, to an elongate fibre, preferably selected from the group consisting of: a polyamide fibre, a cellulose fibre, a polycarbamate fibre, a silk fibre, a polyester fibre, a Nylon fibre, a Rayon fibre and blends thereof.

Preferably, the detectable entity is substantially uniform at both core and surface portions of the fibre. Alternatively, or in addition, the detectable entity at surface portions of the fibre is present in greater amount than at core portions, preferably in which core portions of the fibre comprise substantially no detectable entity.

Preferably, the detectable entity is formed in an elongate channel in the embedding medium. Preferably, the fibre or channel has a uniform cross sectional area across substantially its entire length, preferably in which the fibre or channel has a diameter of between about 0.5µm to 25µm, preferably between 1.5µm to 15µm.

Preferably, the reference standard comprises: (a) two or more linear fibres or channels in substantially parallel orientation, preferably in a bundle; or (b) two or more different detectable entities, each of which is disposed in or on an individual fibre or channel; or (c) two or more fibres or channels each comprising the same detectable entity, preferably comprising different amounts of detectable entity on each; or any combination of the above.

Preferably, a planar section of the reference standard comprises a plurality of areas on which are presented the detectable entity at different density.

Preferably, a planar section of the reference standard comprises a first area comprising the detectable entity substantially at a diagnostically significant density, preferably further comprising a control comprising a fibre or channel which comprises substantially no detectable entity.

We provide, according to a third aspect of the present invention, a method according to the first or second aspect of the invention for obtaining an indication useful in the assessment of the likelihood of disease or condition in an individual, in which the presence of the detectable entity, or the presence of a predetermined quantity of the detectable entity, in the biological sample is indicative of the likelihood of a disease or a condition in the biological sample or an individual from which the sample is taken.

Preferably, the biological sample comprises a cell, tissue or organ, preferably a cell, tissue or organ of an organism suspected of suffering a disease or condition.

As a fourth aspect of the present invention, there is provided a method of providing and indication useful in the diagnosis of a disease or a condition in an individual, the method comprising comparing the amount of a detectable entity in a biological sample from the individual or component thereof with a reference standard, in a method as described herein; in which the individual is indicated as likely to be suffering from or susceptible to the disease or condition if the amount of detectable entity in the biological sample or component is similar to or greater than that in the reference standard.

The present invention, in a fifth aspect, provides a method of assessing the effectiveness or success of a procedure, the method comprising the steps of: (a) providing a reference standard or a planar section thereof, the reference standard comprising: (i) a support medium; and (ii) a quantity of a detectable entity supported by the support medium; in which the detectable entity has an elongate path the cross-sectional profile of which has a size between 0.5 µm to 100 µm; in which a predetermined amount of the detectable entity is present in a defined region in a cross section of the reference standard; and in which a detectable property of the detectable entity is changed as a result of the procedure; (b) conducting the procedure on the reference standard; and (c) detecting a change in the detectable property of the detectable entity.

Preferably, such a method is for assessing the effectiveness or success of a procedure carried out on a sample, in which the procedure is conducted on the reference standard or planar section substantially in parallel with the sample.

Preferably, a detectable property of the detectable entity is changed as a result of a successful procedure, which change in the detectable property of the detectable entity is detected to establish that the procedure is successful.

Alternatively, or in addition, a detectable property of the detectable entity is changed as a result of an unsuccessful procedure, which change in the detectable property of the detectable entity is detected to establish that the procedure is not successful.

Preferably, the procedure is selected from the group consisting of: an *in situ* hybridisation procedure, an immunohistochemical procedure, deparaffination, antigen retrieval, blocking, endogenous biotin blocking, endogenous enzyme blocking, a washing step, incubation with revealing agent such as a primary antibody, incubation with secondary visualisation components, chromogen staining, staining information acquisition and analysis.

The procedure may be an antigen retrieval procedure, and in which the detectable property of the detectable entity comprises the masking or unmasking of one or more epitopes. Preferably, the detectable entity in the reference standard is modified to mask one or more epitopes, some or all of which are unmasked in an antigen retrieval procedure which is successful.

The procedure may be a deparaffination procedure, and in which the detectable property of the detectable entity comprises the presence or quantity of detectable entity in the reference standard following the deparaffination procedure. Preferably, the detectable entity in the reference standard is soluble in the deparaffination medium, and in which at least a portion, preferably all, of the detectable entity is removed following a successful deparaffination procedure.

In preferred embodiments, the effectiveness or success of a two or more procedures are assessed preferably in parallel.

In a sixth aspect of the present invention, there is provided use of a reference standard for validating a procedure, in which the reference standard comprises (i) a support medium; and (ii) a quantity of a detectable entity supported by the support medium; in which the detectable entity has an elongate path the cross-sectional profile of which has a size between 0.5 µm to 100 µm; in which a predetermined amount of the detectable entity is present in a defined region in a cross section of the reference standard, and in which a detectable property of the detectable entity is changed as a result of the procedure.

Preferably, the reference standard is used as an antigen retrieval validation standard, a deparaffination standard, a blocking validation standard, a washing validation standard, a primary antibody validation standard, a secondary antibody validation standard, a calibration standard, or a diagnostic standard.

According to a seventh aspect of the present invention, we provide a kit comprising a reference standard or a planar section thereof together with instructions for use in a method as described herein, in which the reference standard comprises (i) a support medium; and (ii) a quantity of a detectable entity supported by the support medium; in which the detectable entity has an elongate path the cross-sectional profile of which has a size between 0.5 µm to 100 µm; in which a predetermined amount of the detectable entity is present in a defined region in a cross section of the reference standard.

Preferably, the kit further comprises a binding agent capable of specific binding to the detectable entity, which binding agent generates a signal indicative of the presence or quantity of detectable entity in the reference standard, planar section thereof, or the defined region.

We provide, according to an eighth aspect of the invention, a diagnostic kit for detecting a diagnostically relevant presence or amount of a detectable entity in a biological sample, the diagnostic kit comprising a kit according to the eighth aspect of the invention.

Preferably, the kit or diagnostic kit includes a therapeutic agent capable of treating or alleviating at least one of the symptoms of a disease or condition in an individual, preferably comprising an antibody against the detectable entity.

There is provided, in accordance with a ninth aspect of the present invention, a set of two or more planar sections each derived from a reference standard, which reference standard comprises (i) a support medium; and (ii) a quantity of a detectable entity supported by the support medium; in which the detectable entity has an elongate path the cross-sectional profile of which has a size between 0.5 µm to 100 µm; in which a predetermined amount of the detectable entity is present in a defined region in a cross section of the reference standard, in which at least two planar sections in the set comprise a different amount of detectable entity.

As a tenth aspect of the invention, we provide a set of two or more planar sections each comprising a defined region comprising detectable entity, in which the quantity of the detectable entity in the defined region is different between at least two of the planar sections in the set, each planar section being derived from a reference standard comprising (i) a support medium; and (ii) a quantity of a detectable entity supported by the support medium; in which the detectable entity has an elongate path.

The reference standard described here may conveniently be referred to as a "HistoFiber".

### BRIEF DESCRIPTION OF THE FIGURES

Embodiments of the invention are now described in detail with reference by way of example to the accompanying drawings, in which:
Figure 1 shows a typical grading or scoring system of antigen staining for assessment of protein expression. Human breast tissue is stained for HER2 antigen with anti-HER2 antibody using HercepTest™, Dakocytomation code No. K 5204. Figure 1A: Score 0 (negative). No staining is observed, or membrane staining is observed in less than 10% of tumour cells; Figure 1B: Score 1+ (negative). A faint or barely perceptible membrane staining is detected in more than 10% of the tumour cells. The cells are only stained in part of the membrane; Figure 1C: Score 2+ (weakly positive). A weak to moderate complete membrane staining is observed in more than 10 % of the tumour cells; Figure 1D: Score 3+ (strongly positive). A strong complete membrane staining is observed in more than 10 % of the tumour cells.

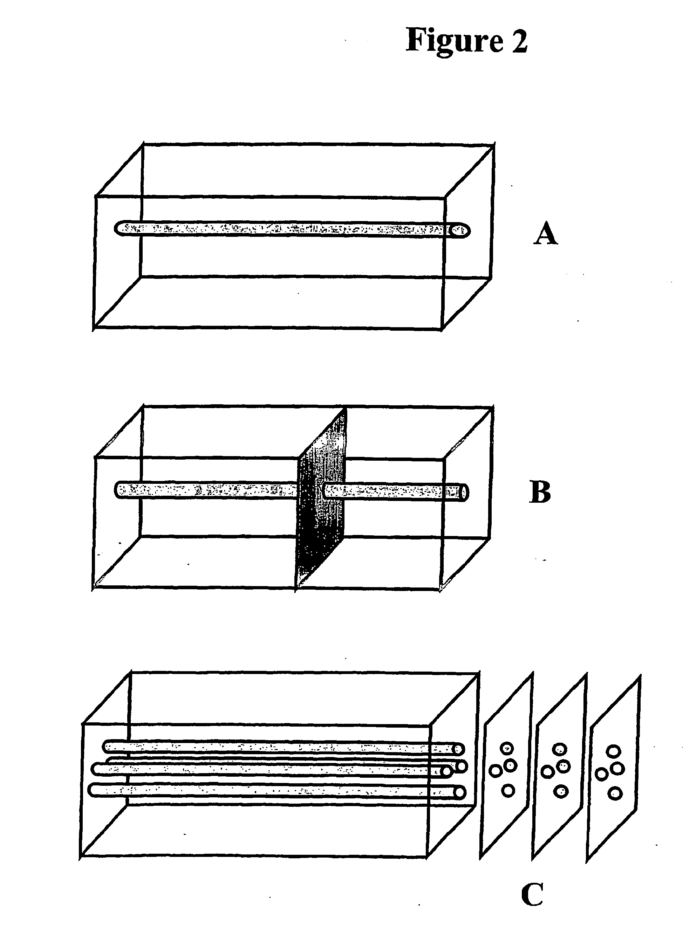

Figure 2 shows an embodiment of the reference standard described here. In this embodiment, a thin and homogeneous fibre is modified chemically with diagnostically relevant targets, and embedded in paraffin blocks. Multiple fibres are placed in bundles perpendicular to the cutting direction, and cut into thin slices containing uniform reference dots. The slices may be IHC or ISH stained as any other "tissue".
Figure 2A shows a block in which detectable entity such as a protein is maintained in an elongate path (for example, in the shape of a rod) and embedded in an embedding medium. Figure 2B shows a cross section taken through the block. Figure 2C shows an embodiment having multiple paths with different detectable entities, or comprising different amounts of the same detectable entity. Planar slices are taken of the block and comprise detectable entity as "dots".

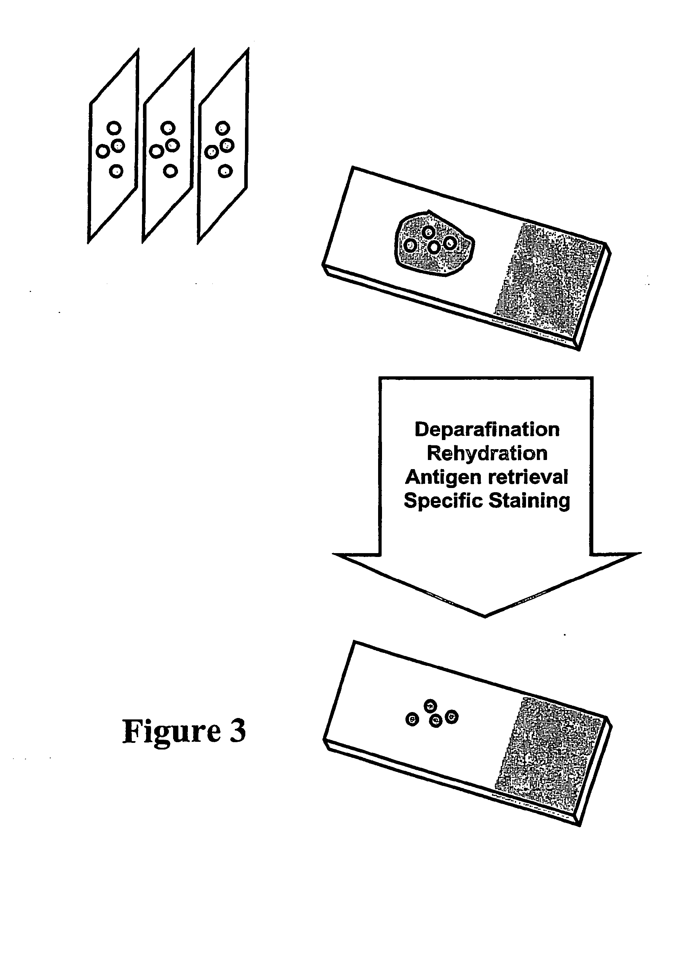

Figure 3 shows an example of how the reference standard (for example, the planar slices generated in Figure 2 above) may be processed, preferably in parallel with a biological sample which has been fixed, embedded and sliced. The planar slices are mounted on glass microscope slides, and the paraffin-embedding medium removed. The slide with a planar slice mounted on it is then rehydrated and may be subjected to standard antigen retrieval techniques. The slide is then stained using specific antibodies to reveal the presence and distribution of the antigen. Counterstaining and secondary staining, optionally with chromogenic substrate where the secondary stain is enzyme linked, may also be carried out.

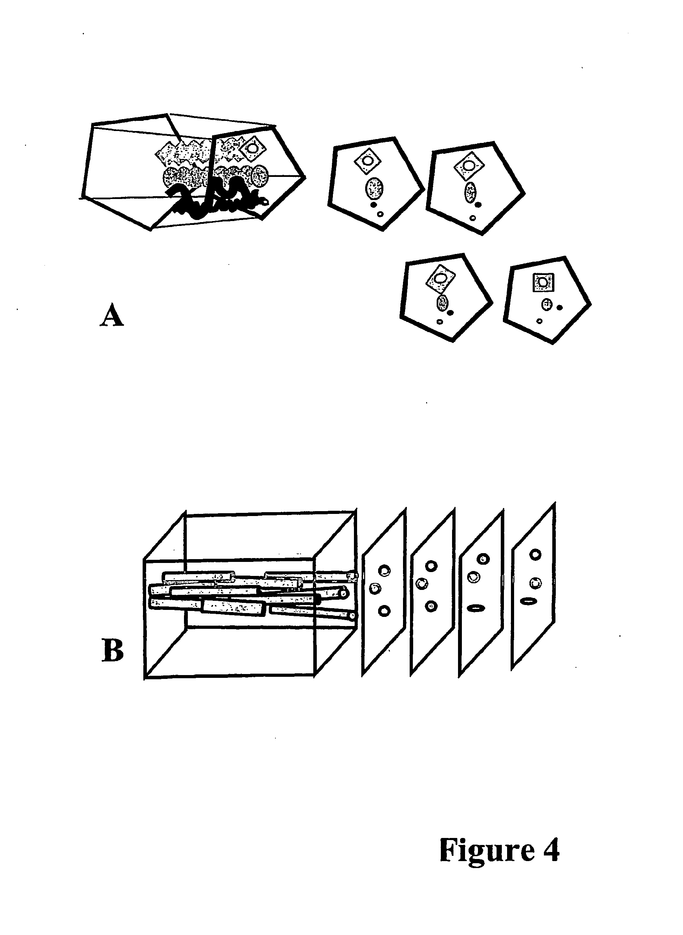

Figure 4 shows several embodiments of the reference standard described here. The reference standard may take-various shapes, and the elongate shape of the detectable entity may take different forms (Figure 4A). Bundles of detectable entities, at least one or some of which are in elongate shape, some or all of which may be shorter than the supporting medium, may be supported in the supporting medium (Figure 4B). The detectable entity may have varying diameter and shape, have at least portions which are curly, wavy or not aligned perfectly perpendicular to the cutting direction or with each other.

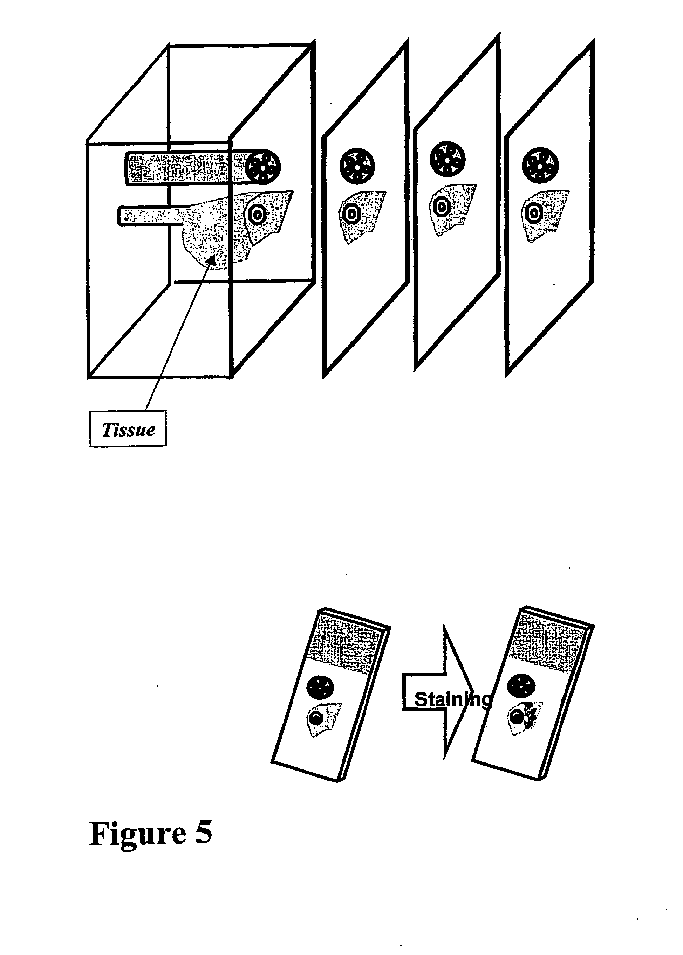

Figure 5 shows an embodiment of the reference standard described here, in which a sample comprising cells, tissues, etc is supported in the supporting medium together with the detectable entity. For example, a tissue sample and the detectable entity may be embedded in the same medium. Slices or sections of the reference standard comprise a detectable amount of the detectable entity, together with the tissue, etc.

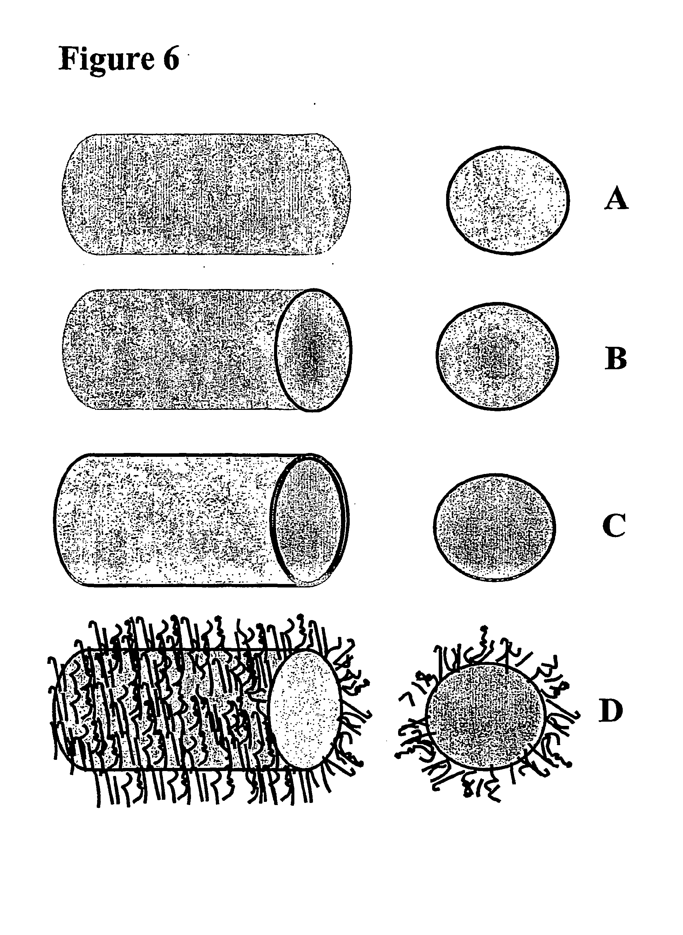

Figure 6 shows several embodiments of the reference standard described here, in which the detectable entity is coupled to a fibre, which is treated in various ways to enable various degrees penetration of the entity in the fibre, and hence modulate the degree of staining. Light gray areas represent areas which are stained, while dark gray areas represent areas which are not stained, or which are partially stained.
Figure 6A shows an embodiment in which the fibre has been allowed to swell during or before coupling, so that the detectable entity is coupled to substantially all portions in a cross section of the fibre. Exposure to a relevant antibody results in homogenous staining (i.e., staining at both core and peripheral regions of the fibre).
Figure 6B shows an embodiment in which the fibre has been allowed to partially swell during or before coupling, by for example controlling the amount or concentration of water relative to organic solvent, and/or the time of exposure to water. Antibody staining is non-homogenous, and is concentrated on the peripheral or surface regions of the fibre, with limited internal staining.
Figure 6C shows an embodiment in which the fibre is not substantially swollen during coupling. The detectable entity is only able to access and couple to the peripheral or surface portions of the fibre. No coupling takes place in the internal or core portion of the fibre, resulting in staining which is substantially restricted to the surface (dense surface staining, no internal staining).
Figure 6D shows an embodiment in which the fibre is not swollen but is pre-treated before coupling to introduce an intermediary coupling means (in this example, polymer chains). The polymer chains are activated, such that the detectable entity is only coupled to the polymer chains, but not to the fibre itself. The resulting staining pattern comprises a fluffy surface staining, with substantially no staining in internal or core portions of the fibre.

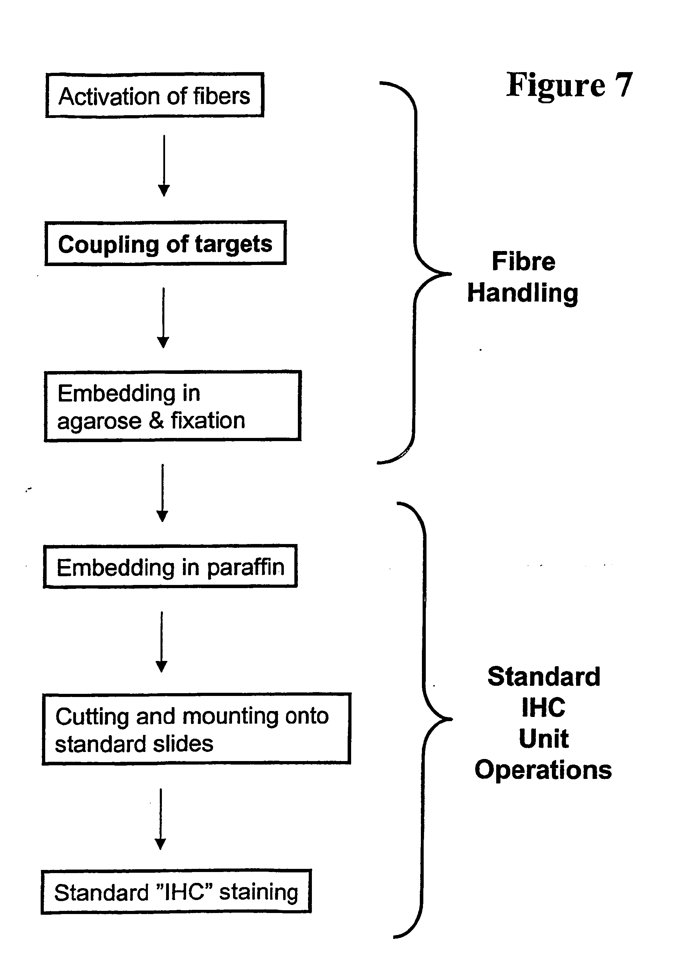

Figure 7 shows a flow chart of a method of producing and handling an embodiment of the reference described here. "Fibre Handling": fibres are activated, targets comprising detectable entities are coupled onto the fibre, and the resulting fibre is embedded in agarose and fixed. "Standard IHC Unit Operations": the fibres are embedded in paraffin, and cut into slices. The slices are mounted onto glass microscope slides, and subject to standard immunohistochemical (IHC) staining procedures.

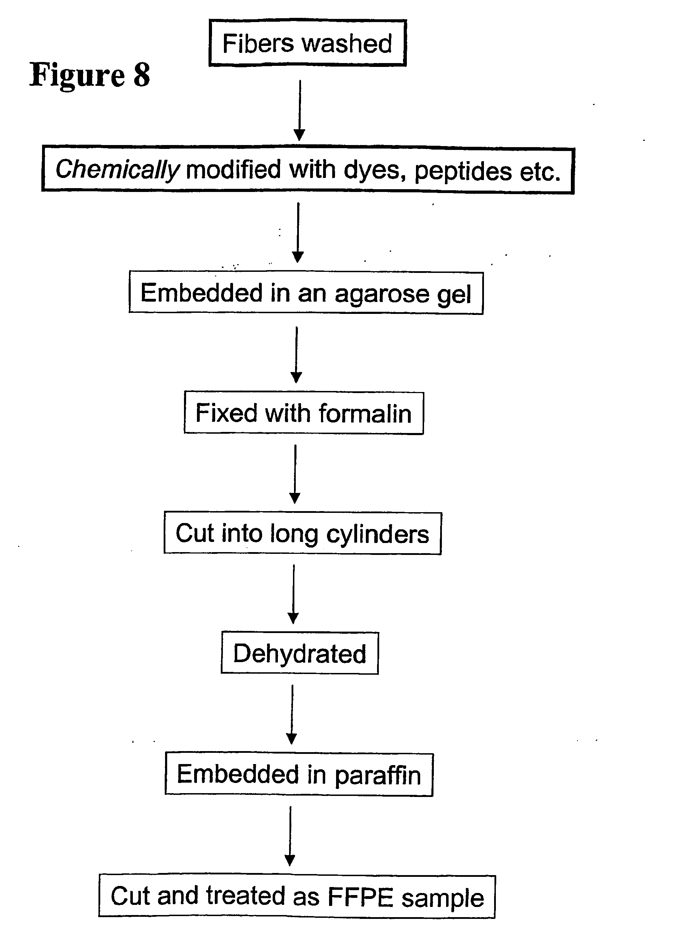

Figure 8 shows a flow chart of a method of producing and handling an embodiment of the reference standard described here. Fibres are washed, then chemically modified with a suitable detectable entity such as a peptide or dye. The modified fibres are optionally embedded in agarose gel, then fixed with formalin. The agarose blocks are then cut into long cylinders, dehydrated and embedded in paraffin. Slices are cut and treated as with other FFPE samples.

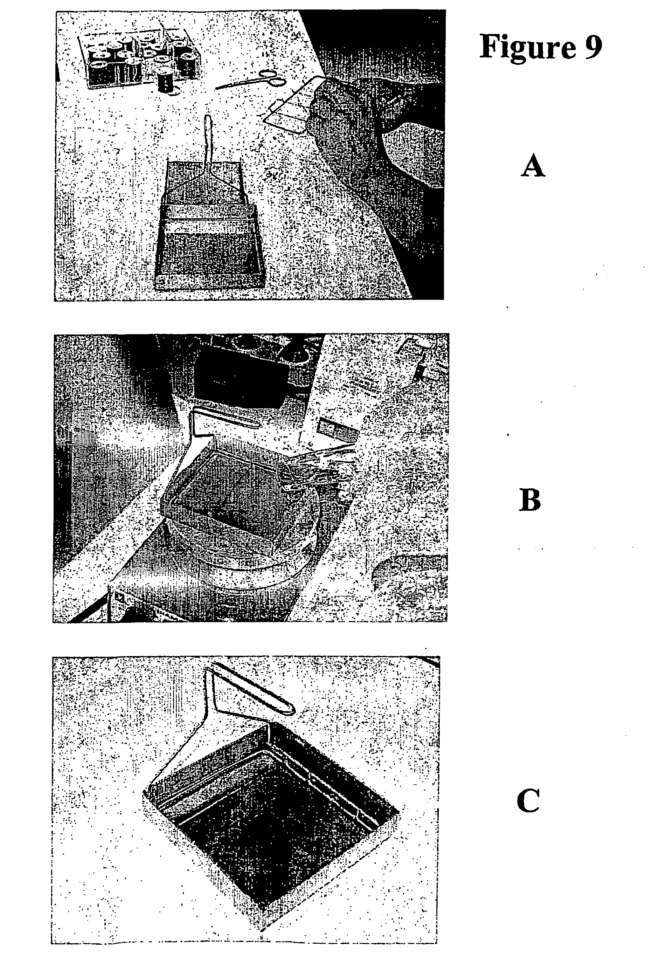

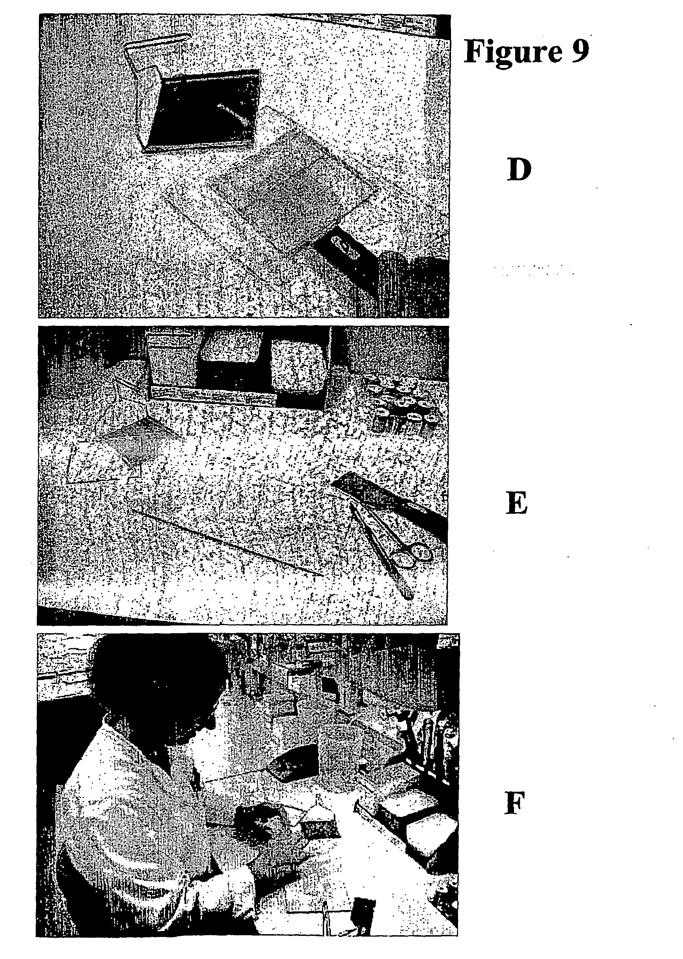

Figures 9 are photographs of the various steps in the formation of an embodiment of the reference standard described here. Fibres embedded in gel are cut in bars and trimmed, before being placed in histo capsules for fixation - followed by dehydration, paraffin infiltration, cutting, baking, mounting on slides, antigen retrieval, blocking, staining, cover slipping-etc. "Histo capsules" refer to commonly used small containers of plastic, for example as used in labs and manufactured by Sekura (Japan).
Figure 9A: fibres are strung on a frame and tensioned. Figure 9B: the frame is placed in a container, and molten agarose poured over the frame. Figure 9C: the agarose is allowed to set. Figure 9D: the set agarose is removed. Figure 9E: strips of agarose containing individual fibres are cut from the agarose block. Figure 9F: the agarose blocks containing individual fibres are embedded in paraffin.

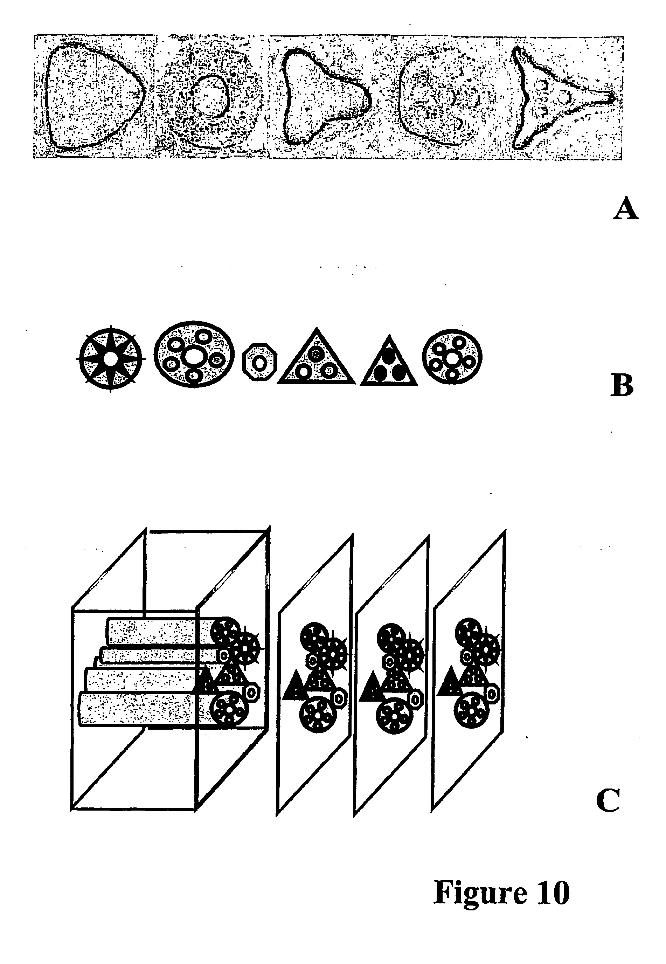

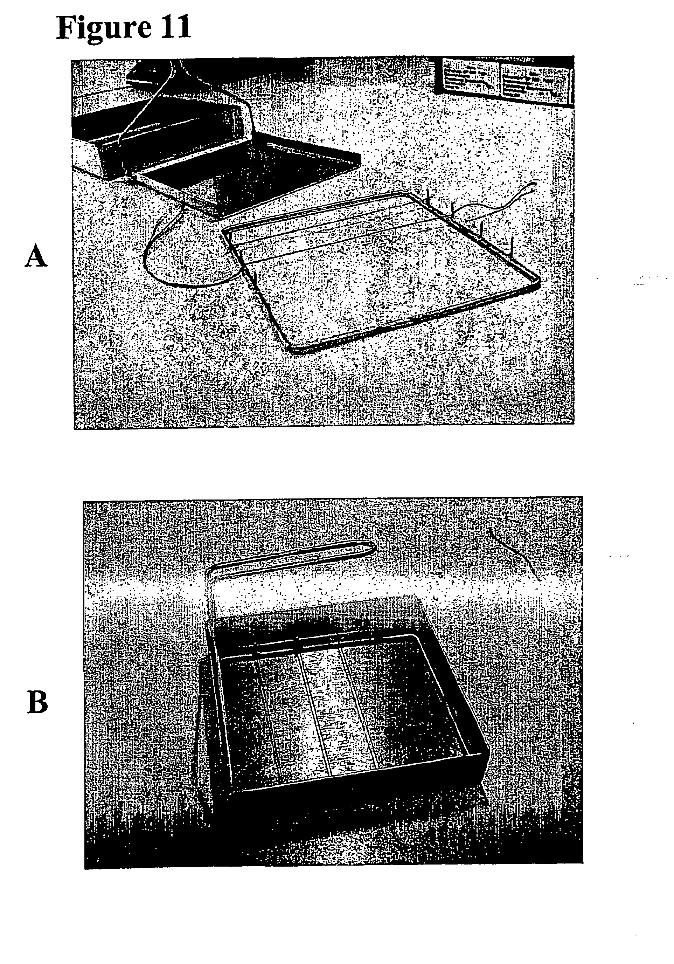

Figure 10 shows embodiments of the reference standard described here comprising fibres, illustrating various shapes and structures of the fibres. For example, the fibre may comprise internal structures, which may be simple or complex. Figure 10A shows photomicrographs of several commercially available fibres. Figure 10B shows examples of fibres with complex structures, including hollow fibres, fibres embedded in other fibres, and fibres containing material of other nature. Figure 10C is a schematic illustration of the cutting of embedded bundles of various fibres and combinations of fibres, resulting in a reference system with complex morphology and patterns.

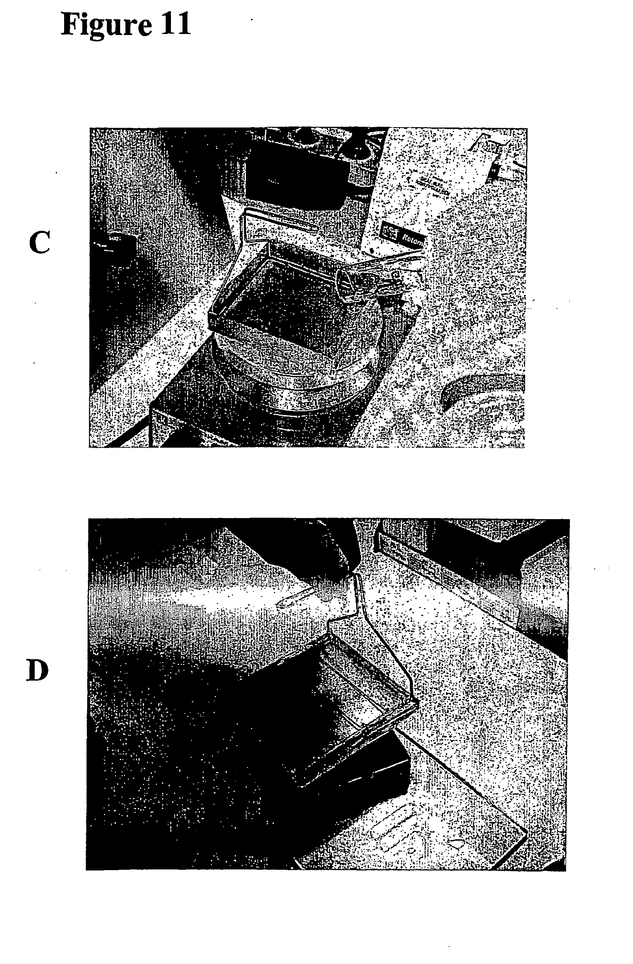

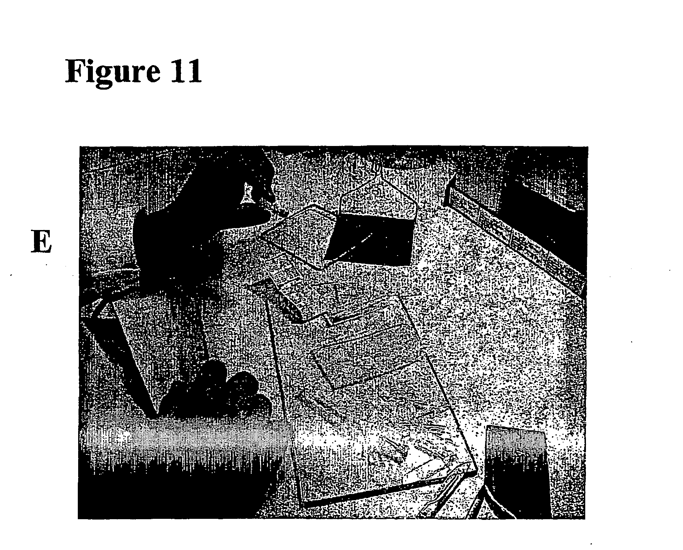

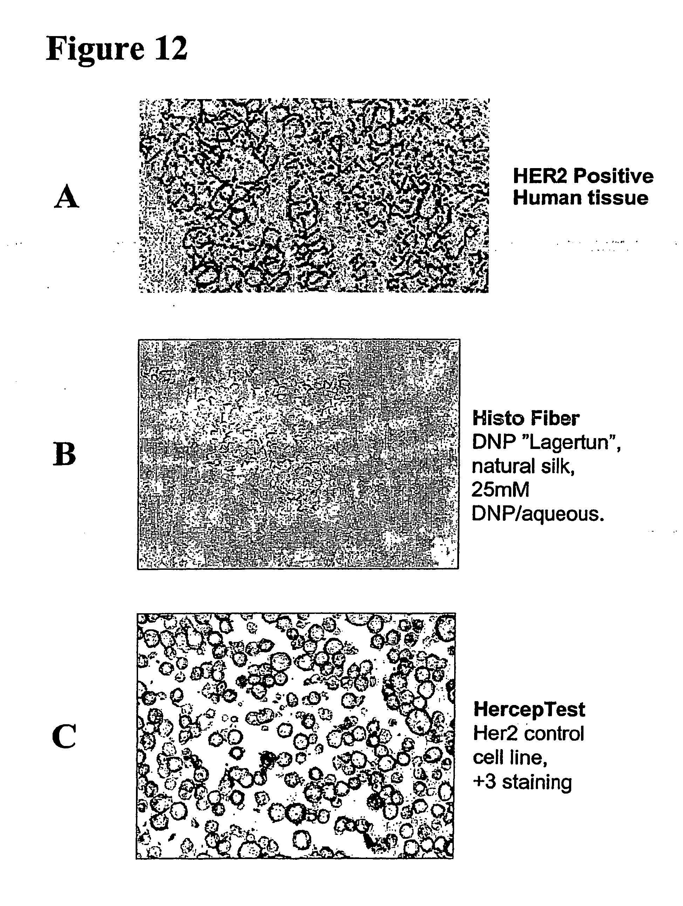

Figures 11A to 11E show results from Example 2. Figure 11A: Fibres mounted on frame, with basket device and container in the background. Figure 11B: Fibres mounted on frame and placed in container, with basket device to help lift the embedded fibres out at a later step. Figure 11C: Warm agarose is poured in the container containing fibres mounted on the frame. Figure 11D: The fibres mounted on the frame and embedded in solidified gel are gently taken out of the container. Figure 12E: The gel-embedded fibres are cut from the steel frame using a surgical knife and further cut into small rectangular pieces. The fibres are embedded in the centre of the agarose gel pieces.
Figure 12 shows a comparison of the reference system described here with a control cell line and a positively stained tissue sample. Figure 12A: HER2 positive human tissue stained with anti-HER2 antibody using HercepTest™ (DakoCytomation code No. K5204). Figure 12B: Lagertun, natural silk modified with 25 mM DNP and coupled in aqueous solvent. Figure 12C: HER2 control cell line stained with anti-HER2 antibody using HercepTest™ (DakoCytomation code No. K5204), corresponding to a 3+ staining score. Figures are not at the same optical magnification.

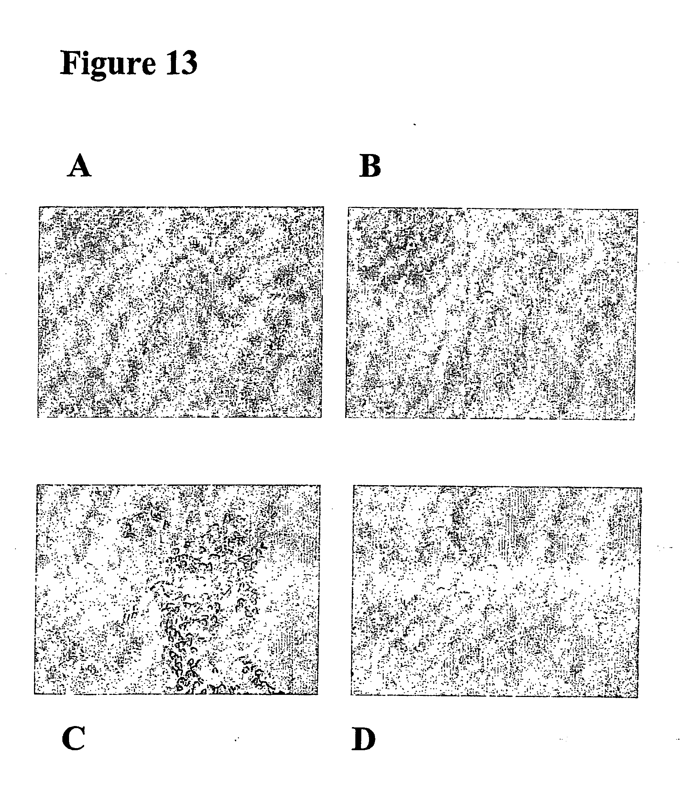

Figure 13 shows results from Example 3. Figure 13A: Unifloss, Figure 13B: LP floss and Figure 13C: Lagertun fibres coupled with 25 mM F-DNP in aqueous solution, and Figure 13D: Unmodified Unifloss fibres as negative control. All treated as FFPE samples and stained with rabbit F(ab') anti DNP-HRP/DAB+ (DakoCytomation code Nos. P 5102 and K 3467/3468). 10x magnification, no counterstaining.

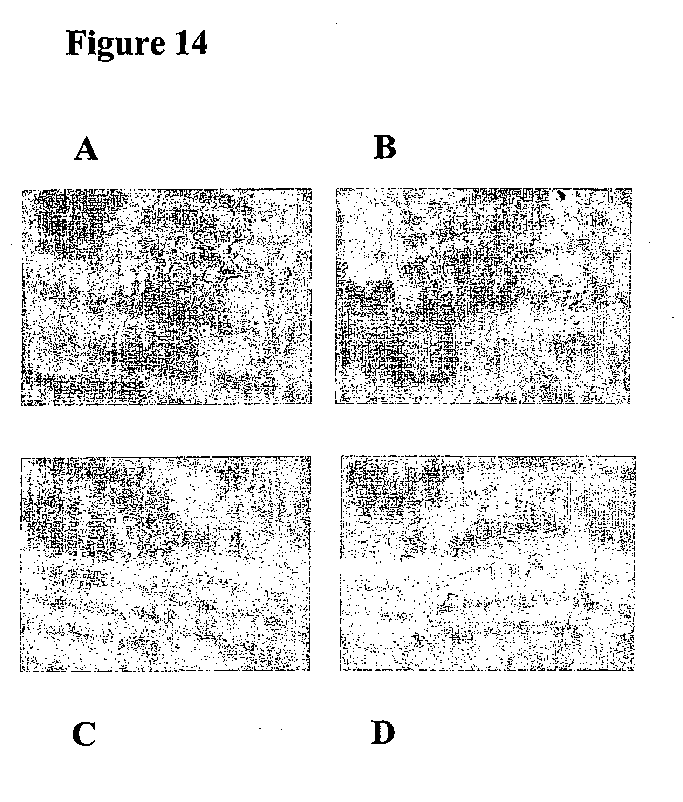

Figure 14 shows results from Example 3. Figure 14A: Unifloss, Figure 14B: LP floss and Figure 14C: Lagertun fibres coupled with 10 mM FITC in toluene, and Figure 14D: Unmodified Unifloss fibres as negative control. All treated as FFPE samples and stained with rabbitF (ab') anti FITC-HRP/DAB+ (DakoCytomation code Nos. P 5100 and K 3467/3468). 10x magnification, no counterstaining.

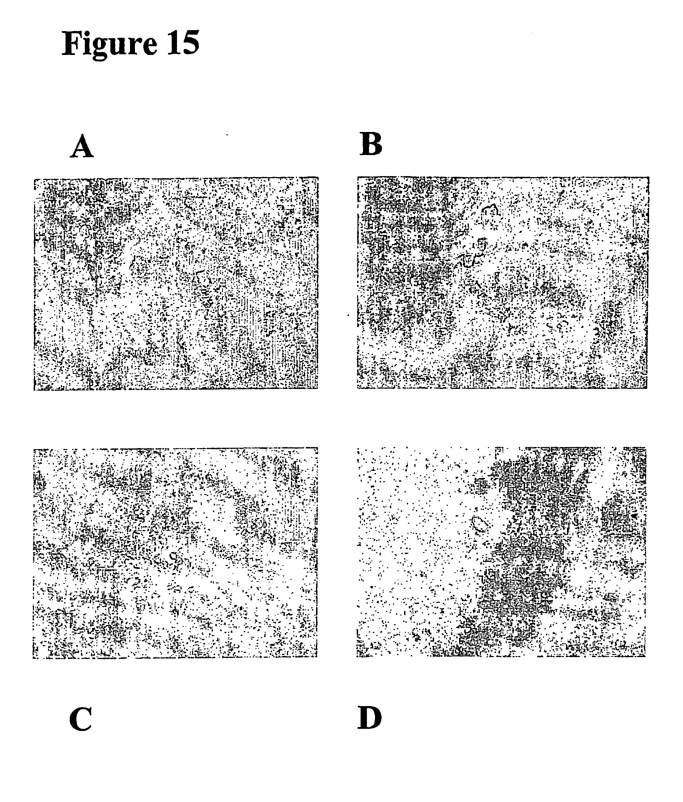

Figure 15 shows results from Example 4. Figure 15A: Unifloss, Figure 15B: LP floss and Figure 15C: Lagertun fibres coupled with 10 mM FITC in toluene, Figure 15D: Unmodified Unifloss as negative control. All treated as FFPE samples and stained with rabbitF (ab') anti FITC-HRP/EnVision+/DAB+ (DakoCytomation code Nos. P 5100 and K 4010/4011). 10x magnification. No counterstaining.

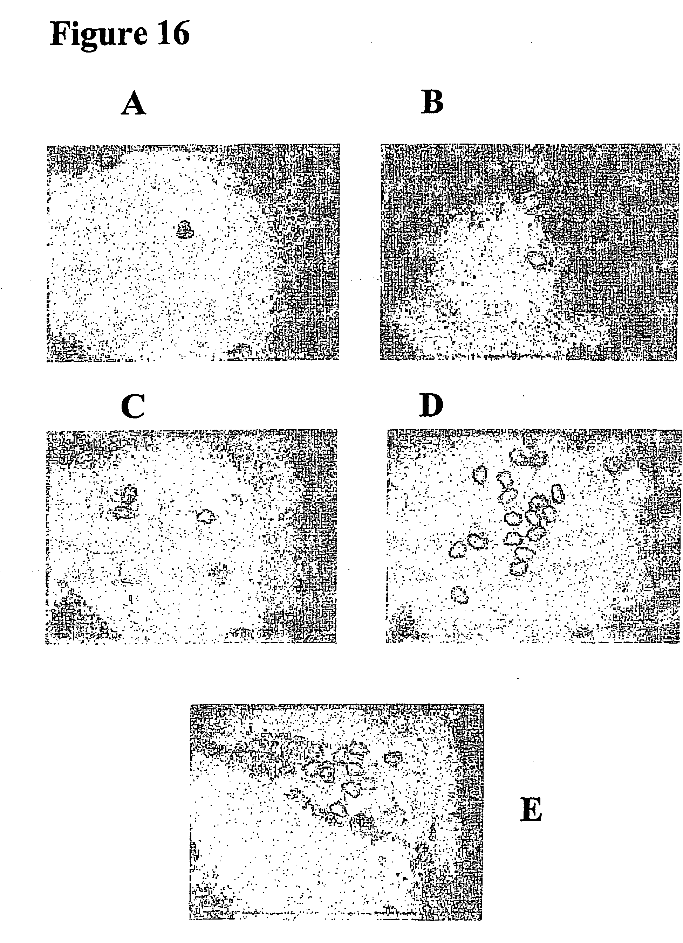

Figure 16 shows results from Example 4. Unifloss fibres are coupled with Sanger's reagent (F-DNP) at different conditions. Figure 16A: Coupled with 5 mM F-DNP in aqueous solvent, Figure 16B: Coupled with 25 mM F-DNP in aqueous solvent, Figure 16C: Coupled with 5 mM F-DNP in toluene, Figure 16D: Coupled with 10 mM F-DNP in toluene and Figure 16E: unmodified fibres. All treated as FFPE samples and stained with rabbit F(ab') anti DNP-HRP/EnVision+/DAB+ (DakoCytomation code Nos. P 5102 and K 4010/4011). Pictures are taken at 10x magnification.

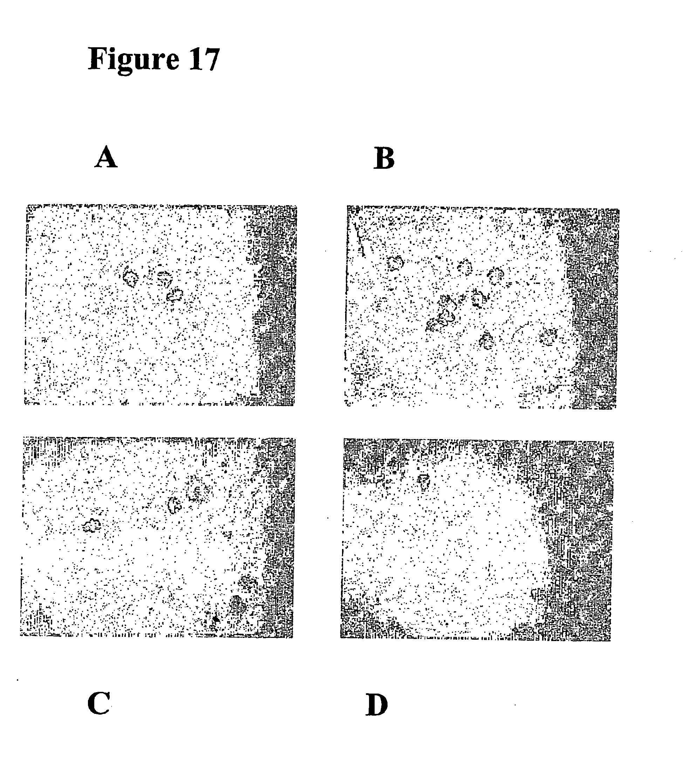

Figure 17 shows results from Example 4. LP Floss fibres are coupled with Sanger's reagent (F-DNP) at different conditions. Figure 17A: Coupled with 5 mM F-DNP in aqueous solvent, Figure 17B: Coupled with 25 mM F-DNP in aqueous solvent, Figure 17C: Coupled with 5 mM F-DNP in toluene, and Figure 17D: Coupled with 10 mM F-DNP in toluene. All treated as FFPE samples and stained with rabbit F(ab') anti DNP-HRP/EnVision+/DAB+ (DakoCytomation code Nos. P 5102 and K 4010/4011). Pictures are taken at 10x magnification.

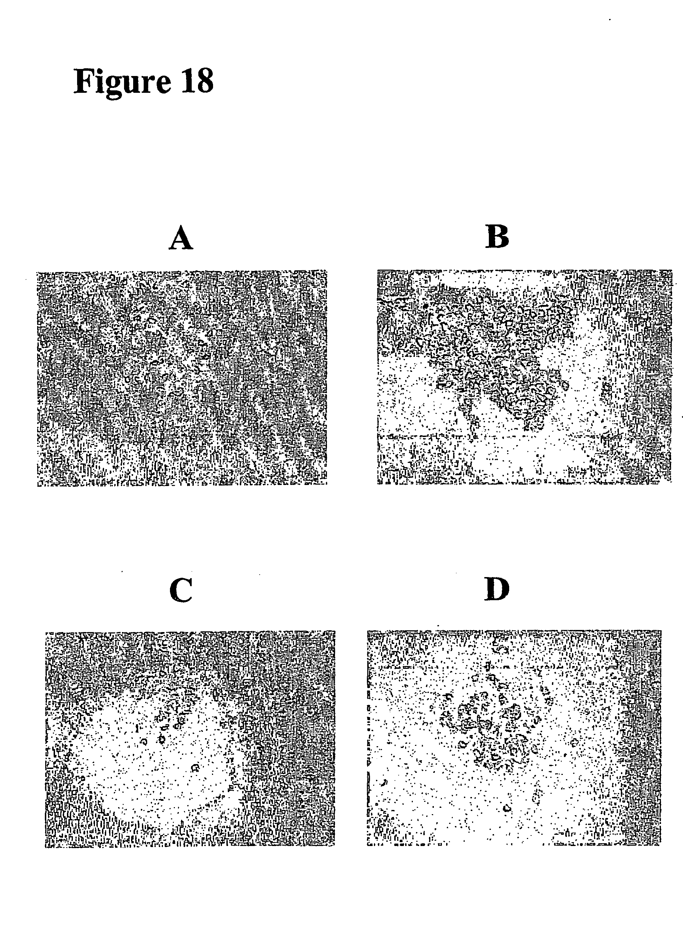

Figure 18 shows results from Example 4. Lagertun silk fibres are coupled with Sanger's reagent (F-DNP) at different conditions. Figure 18A: Coupled with 5 mM F-DNP in aqueous solvent, Figure 18B: Coupled with 25 mM F-DNP in aqueous solvent, Figure 18C: Coupled with 5 mM F-DNP in toluene, and Figure 18D: Coupled with 10 mM F-DNP in toluene. All treated as FFPE samples and stained with rabbit F(ab') anti DNP-HRP/EnVision+/DAB+ (DakoCytomation code Nos. P 5102 and K 4010/4011). Pictures are taken at 10x magnification.

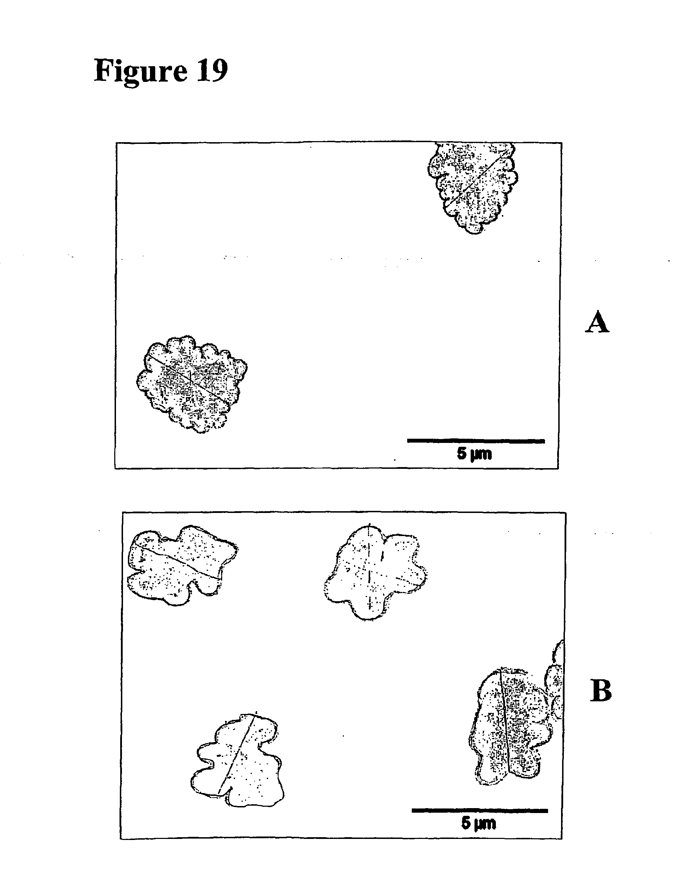

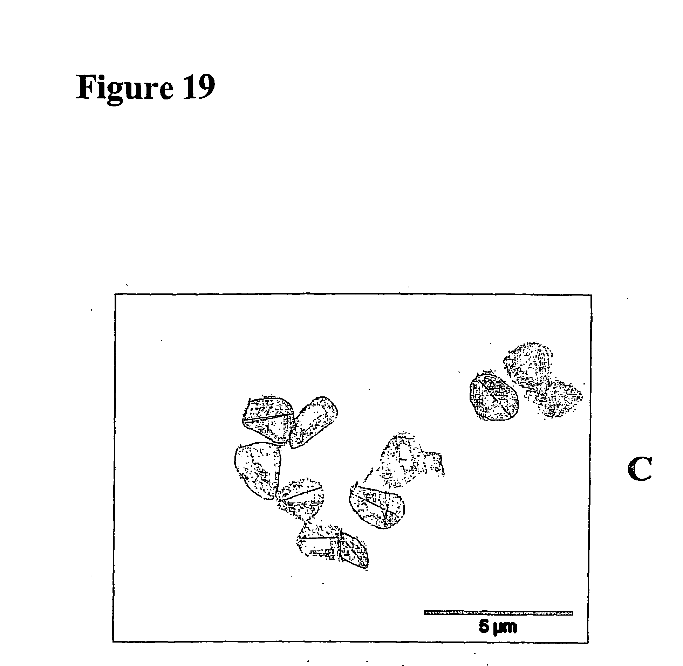

Figure 19 shows results from Example 5. Measurement of the average diameter of anti DNP-HRP/Envision+/DAB+ (DakoCytomation code Nos. P 5102 and K 4010/4411) stained Figure 19A:Unifloss, 3.3 µm Figure 19B: LP Floss 3.5 µm and Figure 19C: Lagertun fibres 1.6 µm using the Olympus DP-soft software. A scale bar is shown at the lower right corner.

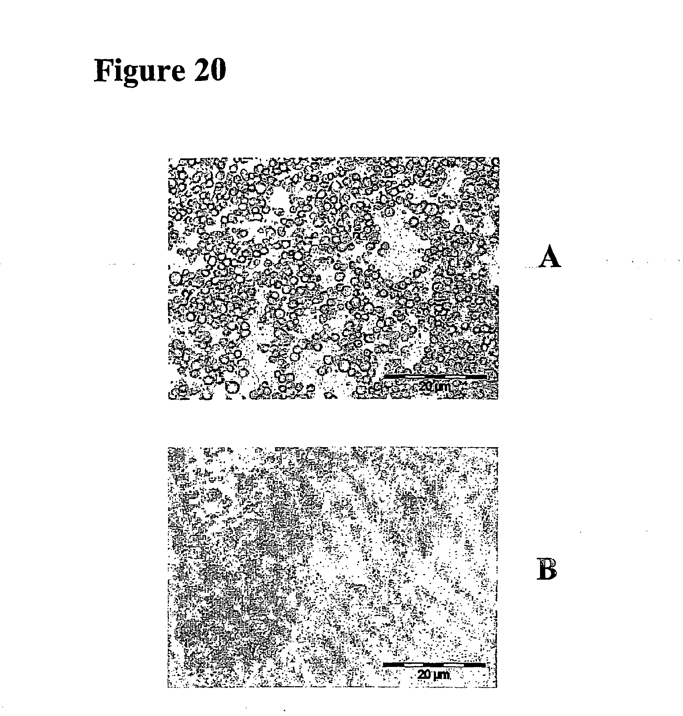

Figure 20 shows results from Example 5. HercepTest™ stained HER2 reference cells (DakoCytomation, code No. K 5204,anti HER2/Envision/HRP/DAB staining, according to instructions supplied with the kit), with Taken at 10x magnification. The 3+ cell line Figure 20A: giving strong membrane staining (3+) and no cytoplasmic staining. The staining is homogenous and the preparation contains few dead cells and some cell debris. Figure 20B: The HER2 negative control cell line with no visible staining.

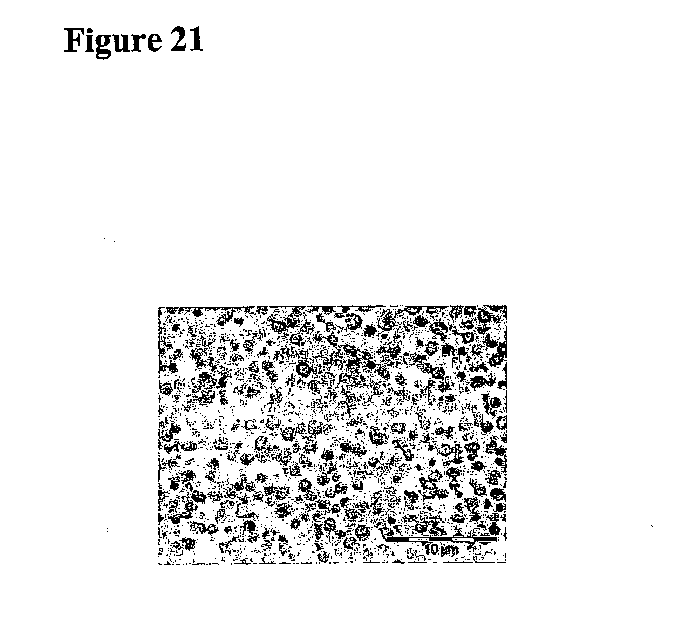

Figure 21 shows results from Example 5. EGFR stained and haematoxylin counterstained EGFR reference cells (DakoCytomation, code No. K 1492, according to instructions supplied with the kit using anti EGFR/Envision/HRP/DAB). Taken at 20x magnification, resulting in strong membrane staining (3+) and some weak cytoplasmic staining (1+). The staining is homogenous and the preparation contains some dead cells and some cell debris.

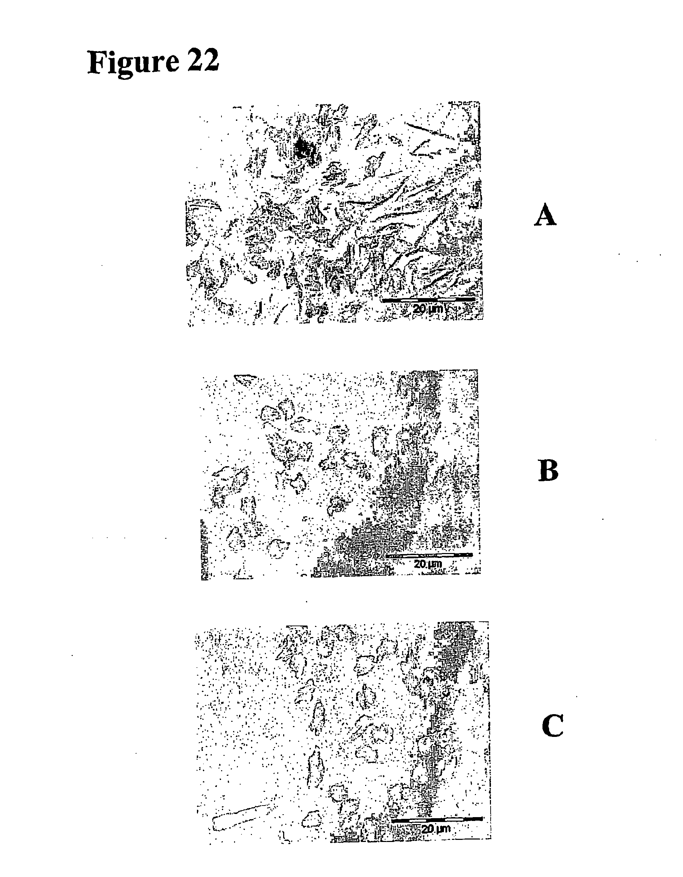

Figure 22 shows results from Example 6. Picture of Procion Red modified LP floss fibres mounted on slides. Figure 22A: Before the deparaffination, Figure 22B: after deparaffination and before the epitope retrieval step (Dakocytomation code No. S 1700) and Figure 22C: after deparaffination and epitope retrieval, taken at 10x magnification. The bar to the bottom right indicates the length scale. Epitope retrieval was conducted using citrate pH 6.0.

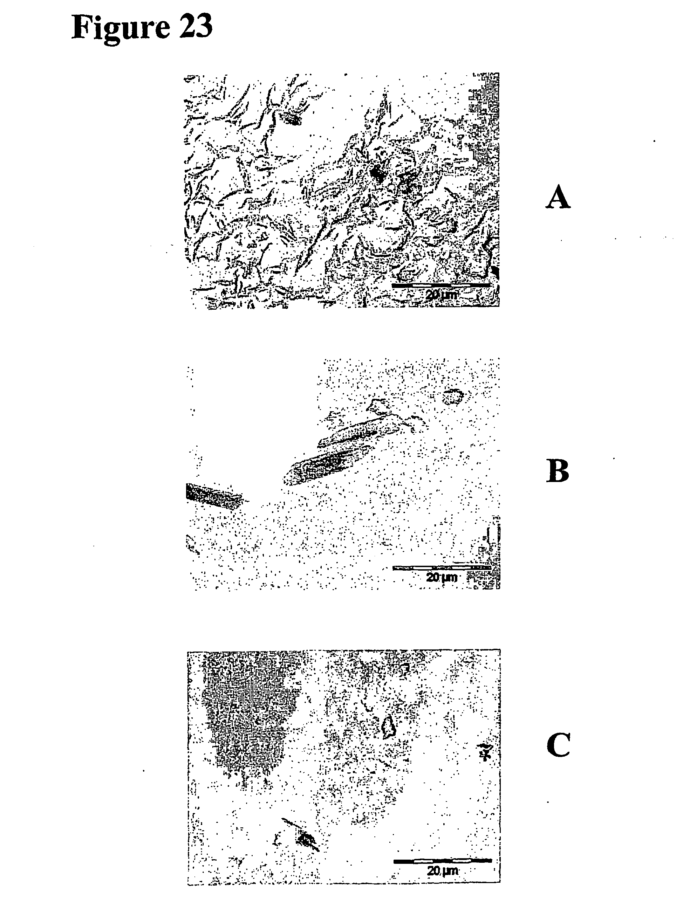

Figure 23 shows results from Example 6. Picture of Procion Red modified Unifloss fibres mounted on slides. Figure 23A: Before the deparaffination, Figure 23B: after deparaffination and before the epitope retrieval step(DakoCytomation code Nos. P 5102 and K 4010/4011) and Figure 23C: after deparaffination epitope retrieval, taken at 10x magnification. The bar to the bottom right indicates the length scale. Epitope retrieval was conducted using citrate pH 6.0.

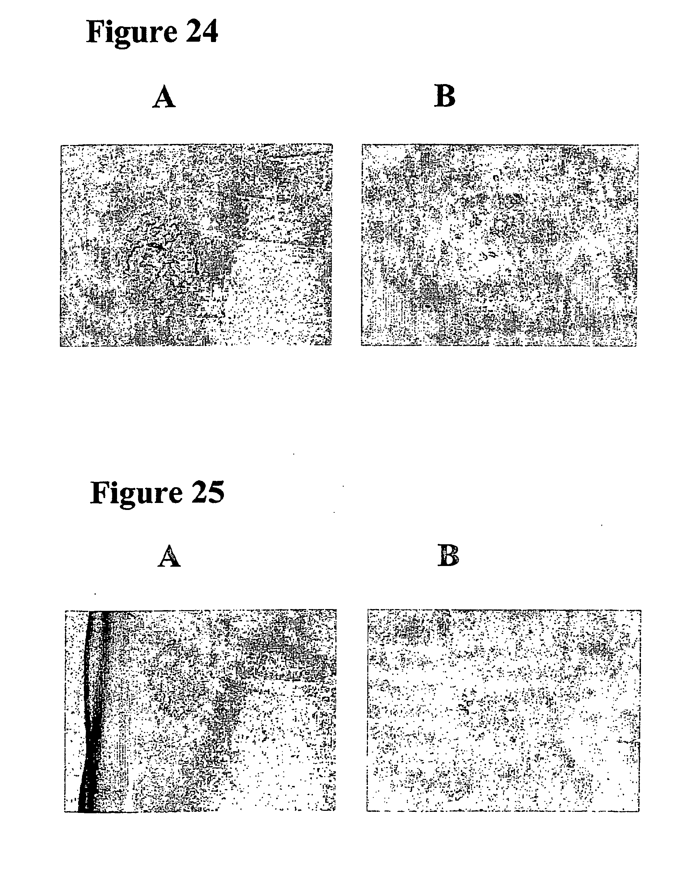

Figure 24 shows results from Example 7. Pictures of Unifloss fibres embedded in 2% agarose with 0.25% PEI (poly-ethylenimine) mounted on ChemMate™ Capillary Gap Microscope Slides (DakoCytomation code Nos. S 2024). Figure 24A: Before and Figure 24B: After deparaffination and epitope retrieval (Dakocytomation code No. S1700), taken at 10x magnification.
Figure 25 shows results from Example 7. Pictures of LP floss fibres embedded in 2% agarose with 0.25% PEI, mounted on Poly-L-Lysine Slides Figure 25A: before and Figure 25B: after deparaffination and epitope retrieval using high pH buffer, pH 9.9 (Dakocytomation code No. S3308), taken at 10x magnification.

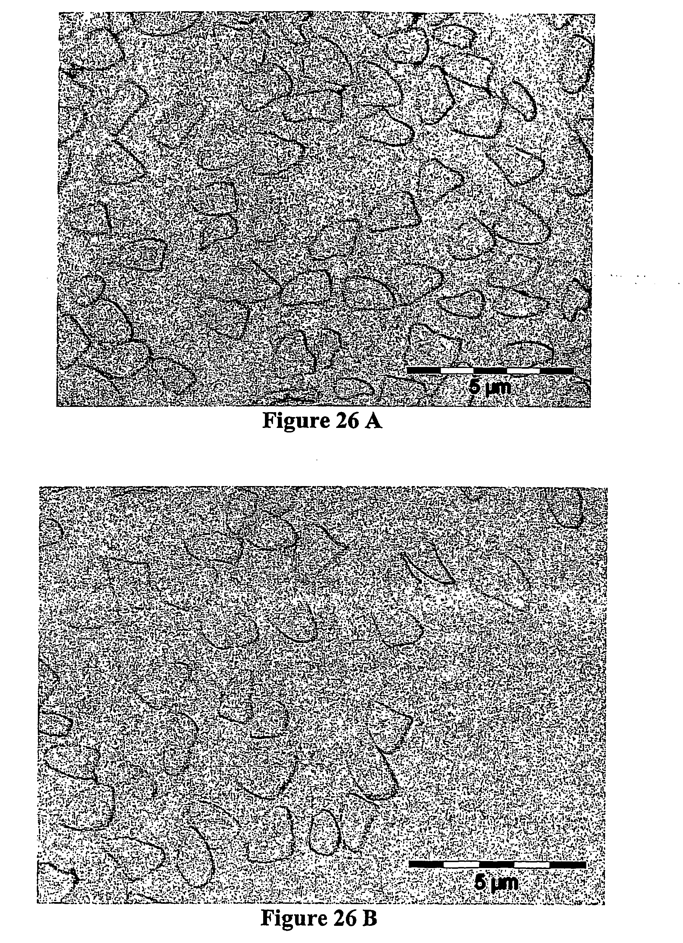

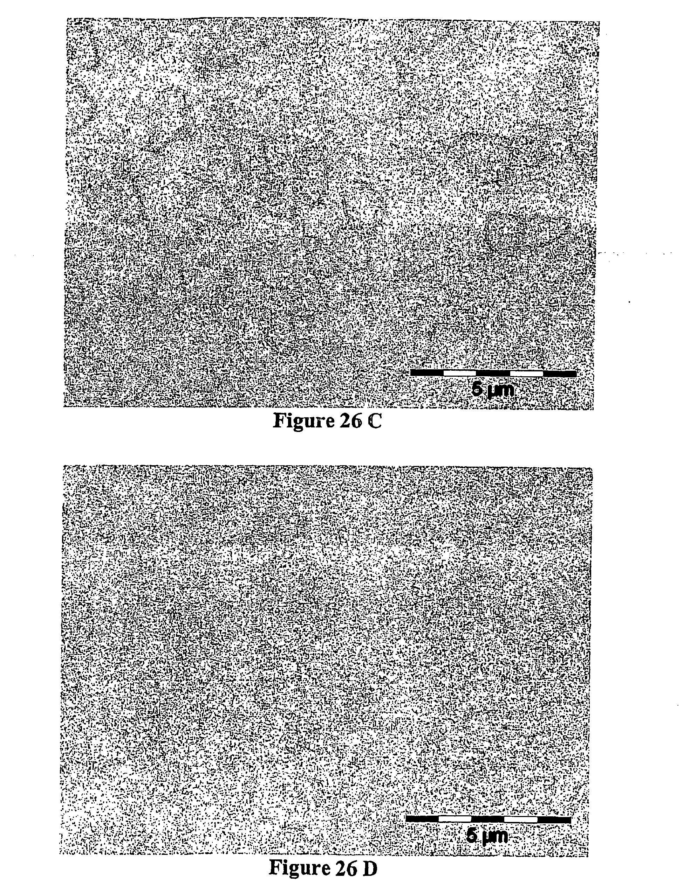

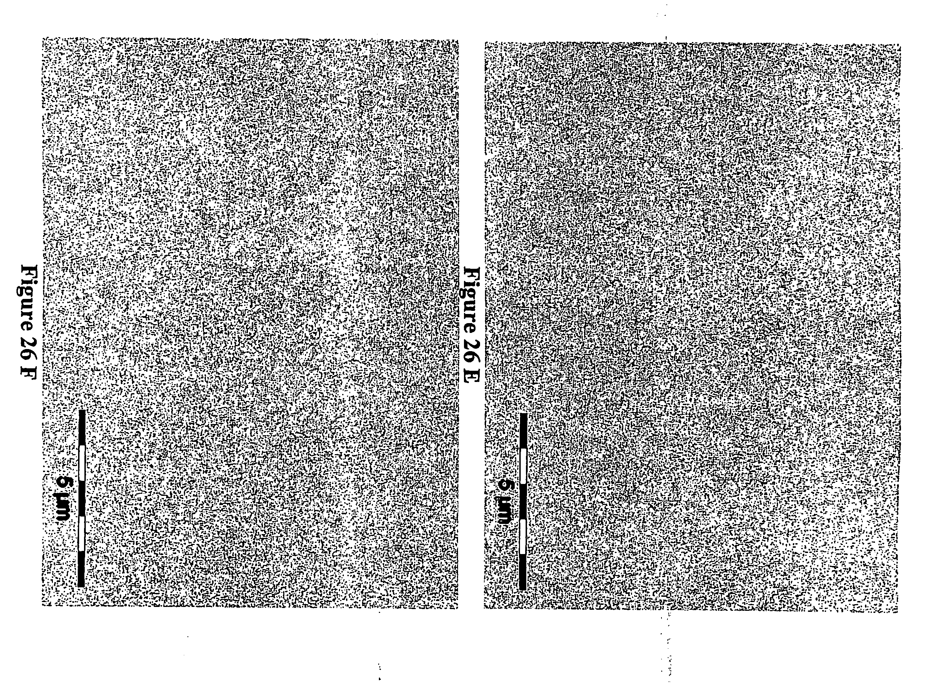

Figure 26 shows results from Example 8, taken at 40-time magnification with no counterstaining, showing the DAB staining of the Lagertun fibres:
Figure 26A: The CDI activated fibres coupled with rabbit IgG and stained with rabbit Envision HRP/DAB. Figure 26B:The CDI activated fibres coupled with rabbit IgG and antigen retrieved and stained with rabbit Envision HRP/DAB. Figure 26C: The CDI activated fibres coupled with biotinylated rabbit IgG and stained with Streptavidine HRP/DAB. Figure 26D: Native fibres, treated without CDI, with rabbit IgG and stained with Envision HRP/DAB. Figure 26E: Native fibres stained with Envision HRP/DAB. Figure 26F: The CDI activated fibres coupled with rabbit IgG, AR treated and stained with anti mouse Envision HRP/DAB

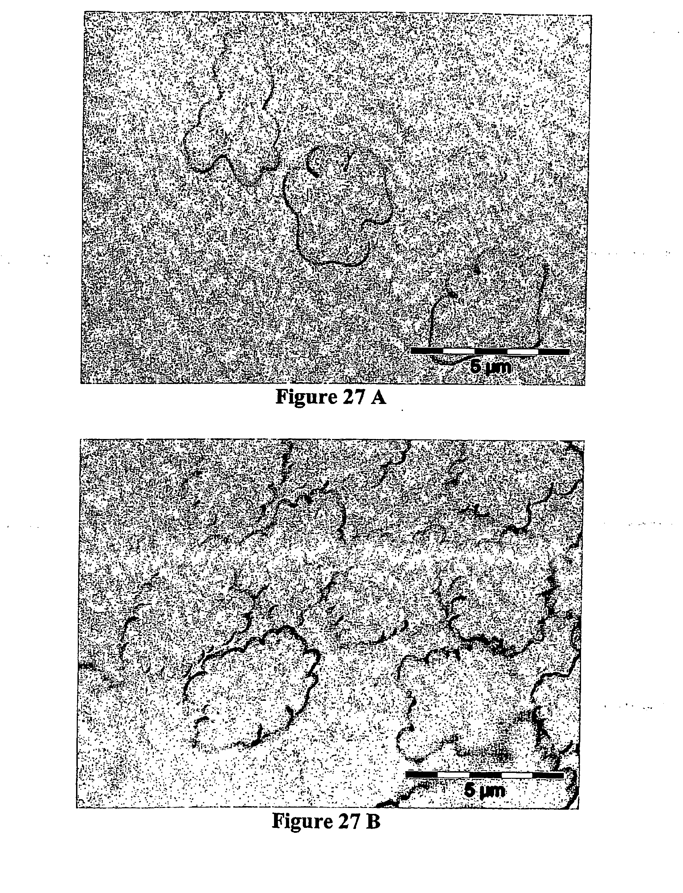

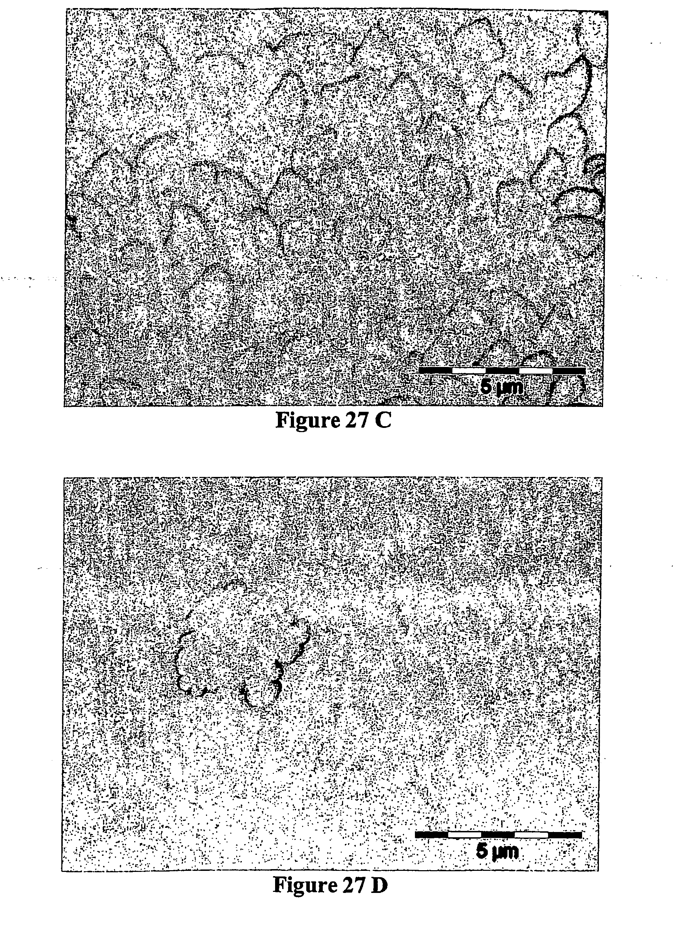

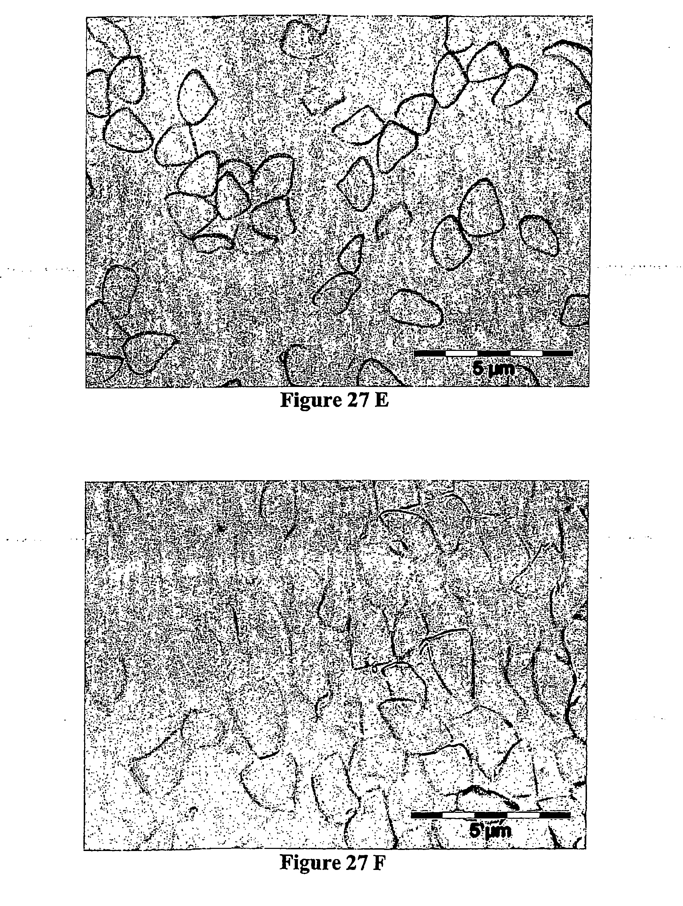

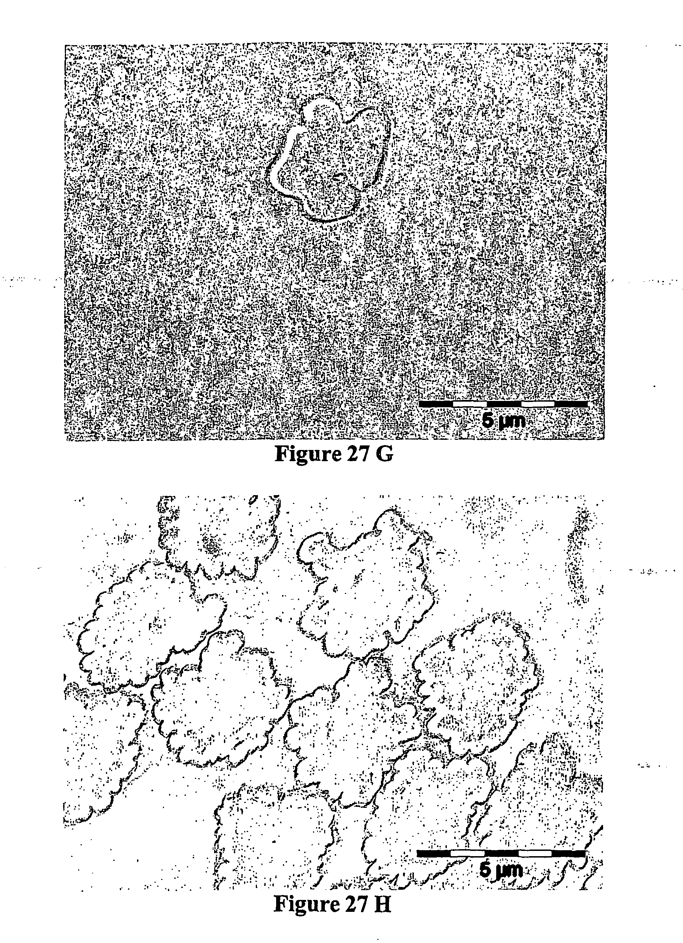

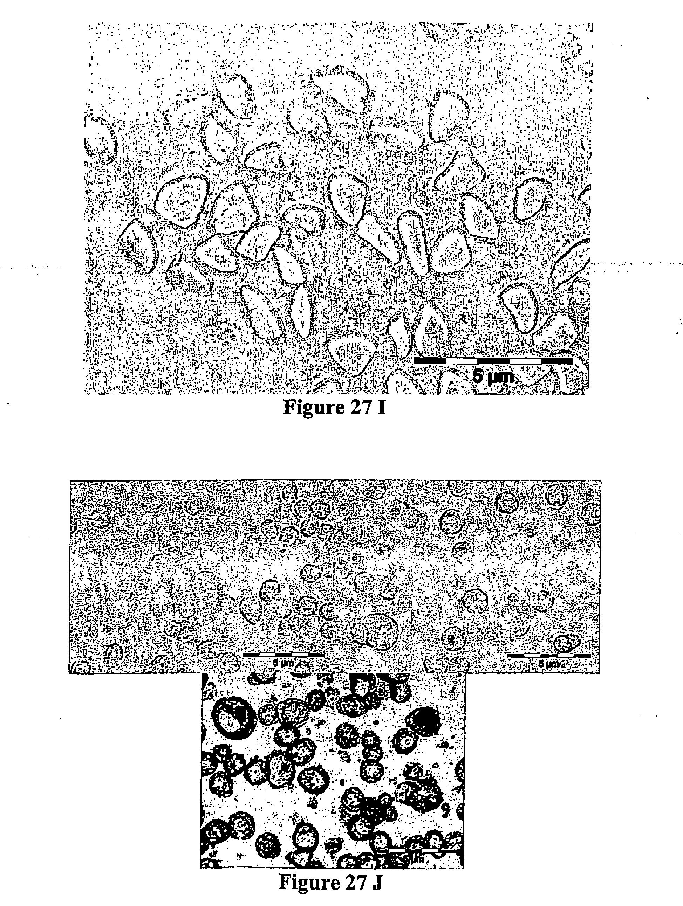

Figure 27 shows results from Example 9, taken at 40-time magnification with no counterstaining, showing the DakoCytomation HercepTest™ staining of various fibres:
Figure 27A HER2 modified LP floss, Figure 27B: HER2 modified Uni floss, Figure 27C: HER2 modified Lagertun fibres, Figure 27D: HER2 modified Uni floss including Antigen Retrieval, and Figure 27E: HER2 modified Lagertun including Antigen Retrieval. Figure 27 F: Negative coupling control using non-activated Lagertun fibres. Figure 27G: Negative primary antibody control of HER2 modified LP floss. Figure 27H: Negative primary antibody control of HER2 modified Uni floss, and Figure 27I: Negative primary antibody control of HER2 modified Lagertun fibres

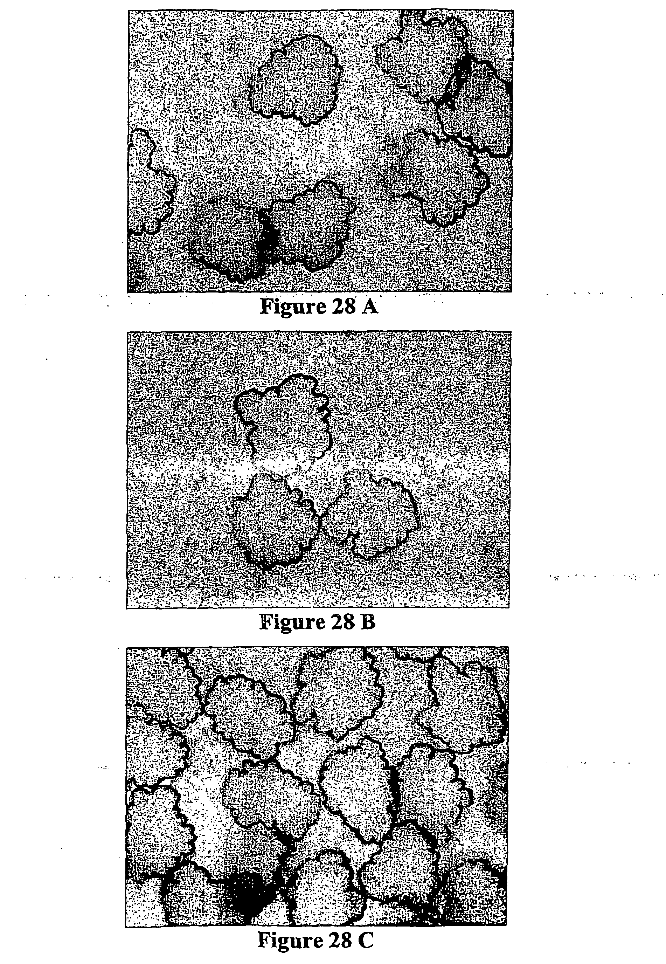

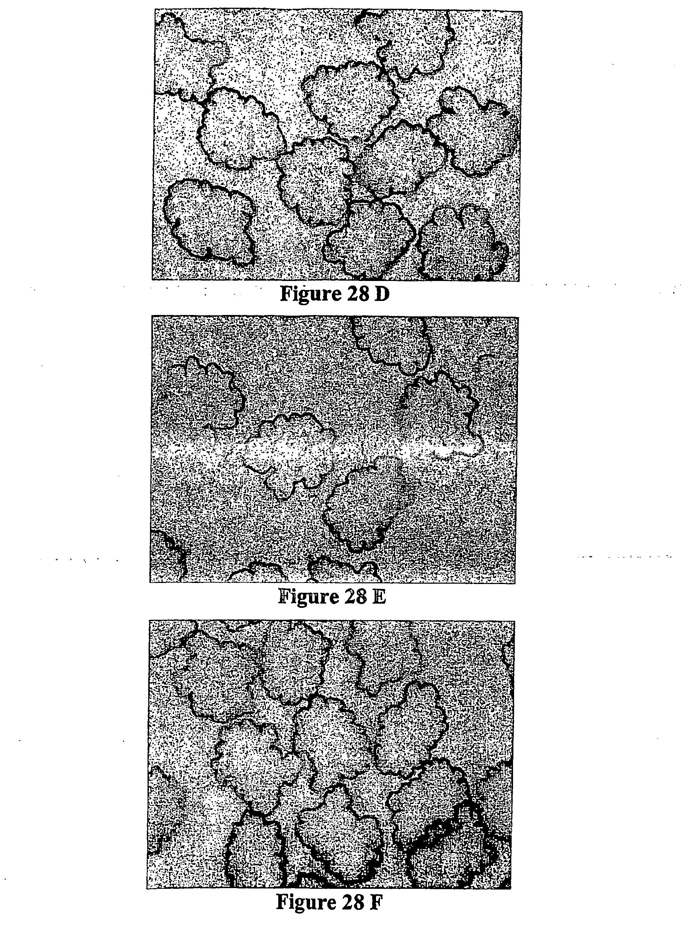

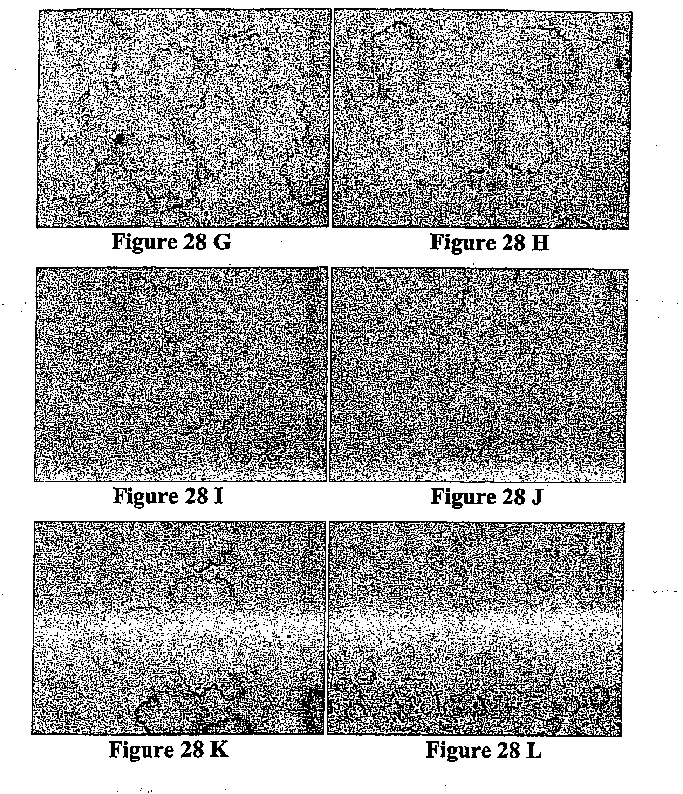

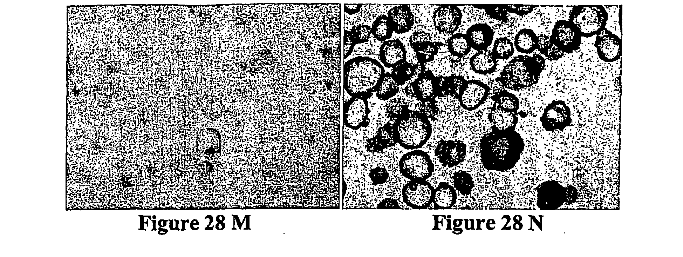

Figure 28 shows the HER2 staining results from Example 10, taken at 40-time magnification with no counterstaining, showing the DakoCytomation HercepTest™ staining of various fibres with graded modifications:
Figure 28 A, B, C, D, E and F is photomicrographs of the HER2 stained slides prepared using 0,20; 0,19; 0,15; 0,10; 0,05 and 0,01 g/l HER2 peptide, respectively. Figure 28 G is the coupling control that is, the slide with no HER2 peptide and only 0,20 g/l p16. Figure 28 H is the coupling control prepared without addition of GMBS or peptides. : Figure 28 I is the primary antibody negative control using nonsense rabbit antibody (DakoCytomation K5205). Figure 28 J is the native unmodified fibre control. Figure 28 K is the secondary visualization negative control using anti mouse IgG Envision HRP (DakoCytomation K4006). Figure 28 L, M and N are the stained reference cells included in the Herceptest ™ with 0+, 1+ and 3+ cells, respectively.

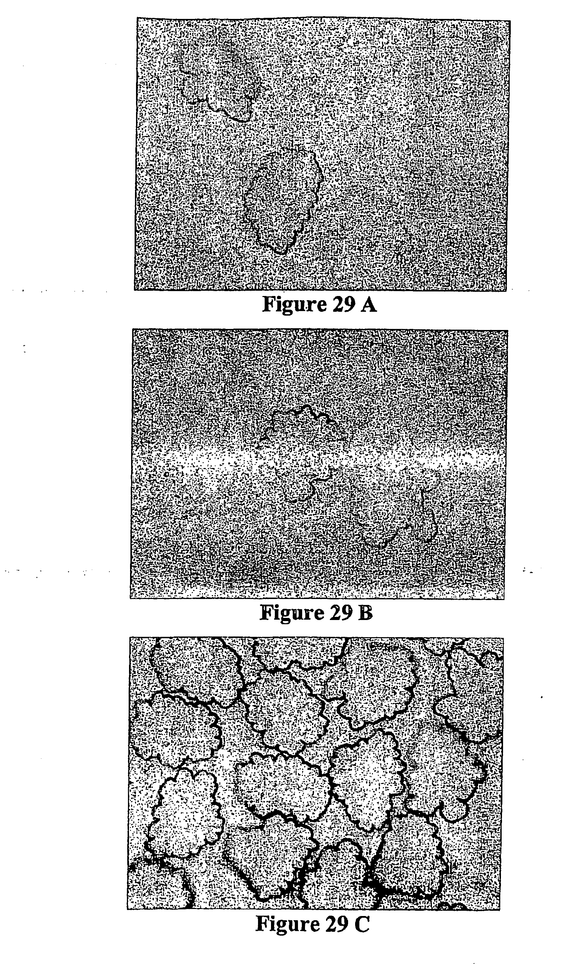

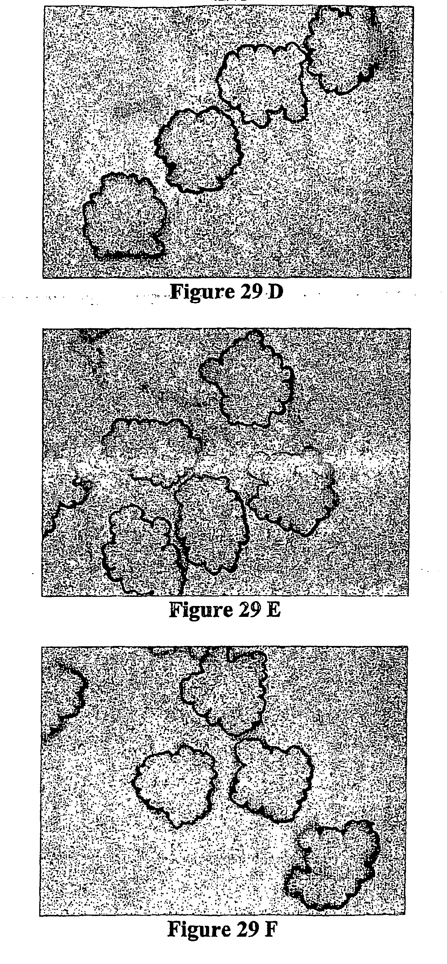

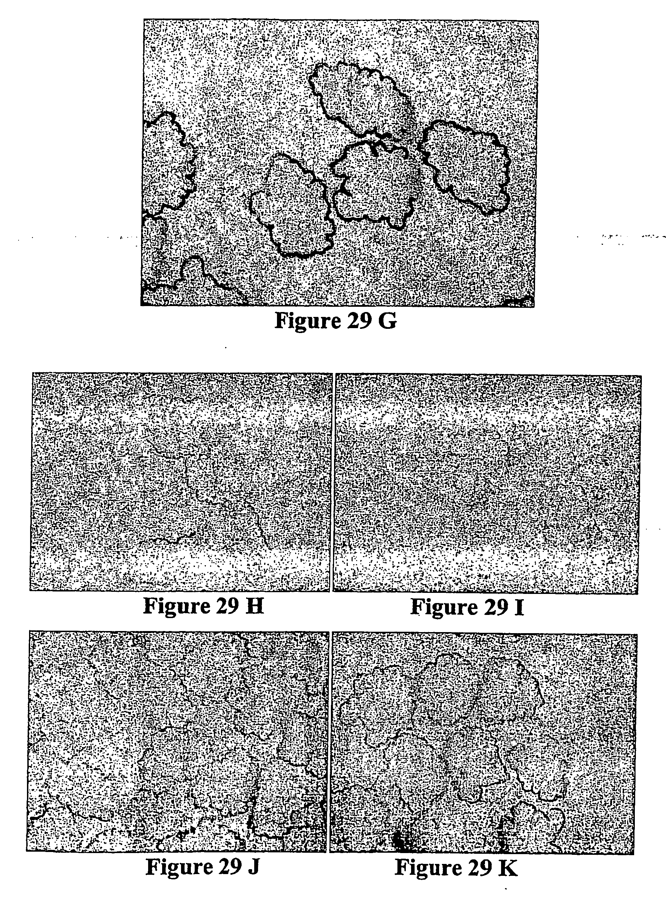

Figure 29 shows the p 16 staining results from Example 10, taken at 40-time magnification with no counterstaining, showing the DakoCytomation CINtec ™ p16-INK4 Histology Kit staining of various fibres with graded modifications:
Figure 29 A is the coupling control with no p16 peptide and only 0,20 g/l HER2. Figure 29 B, C, D, E, F and G is photomicrographs of the p 16 stained slides prepared using 0,01; 0,05; 0,10; 0,15; 0,19; and 0,20 g/l p16 peptide, respectively. Figure 29 H is a coupling control prepared without addition of GMBS. Figure 29 I is the primary antibody negative control using nonsense rabbit antibody (part of the CINtec ™ p16-INK4 Histology Kit). Figure 29 J is the native unmodified fibre control. Figure 29 K is the secondary visualization negative control using anti rabbit IgG Envision HRP (DakoCytomation 4003).

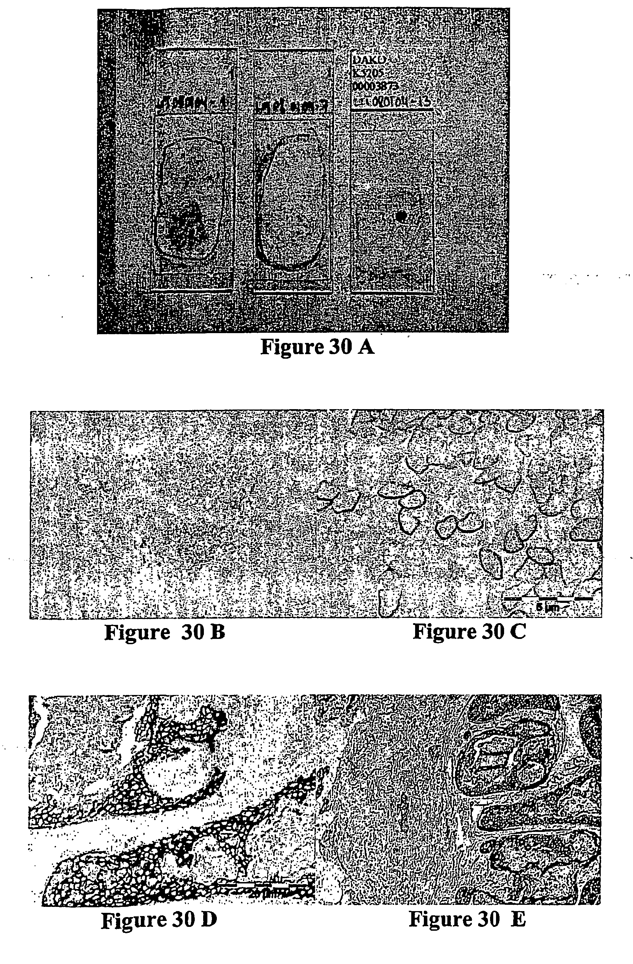

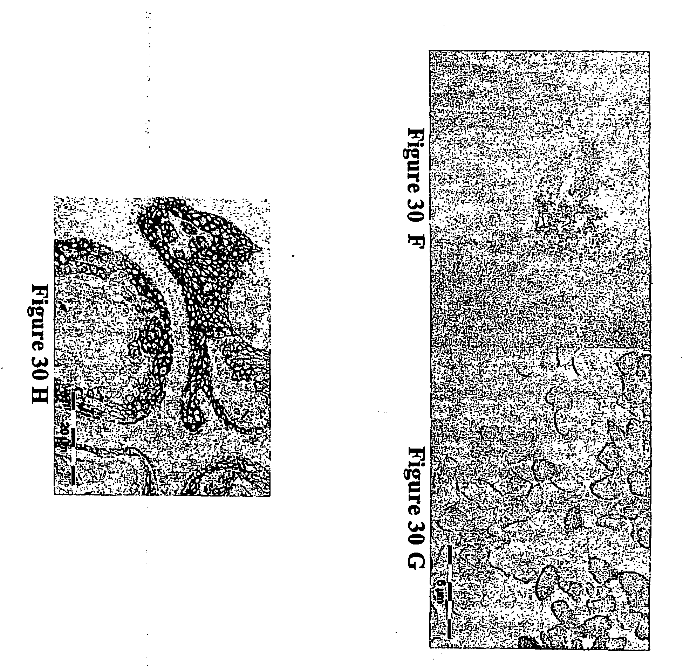

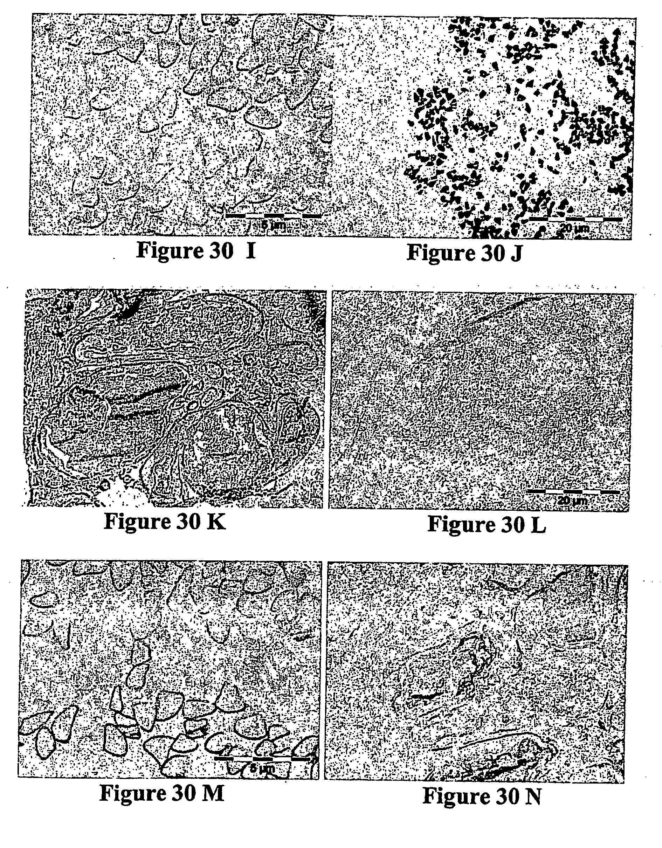

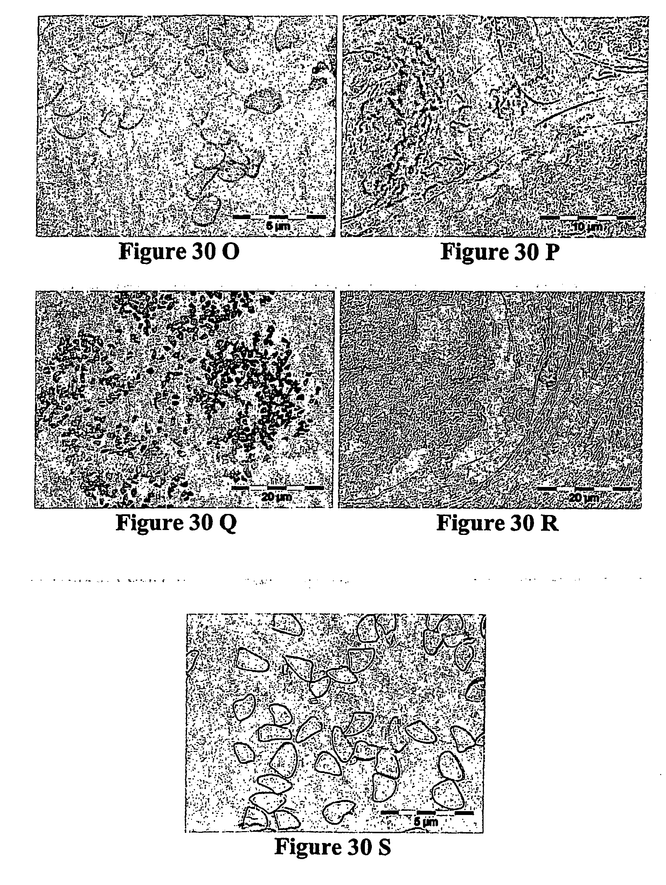

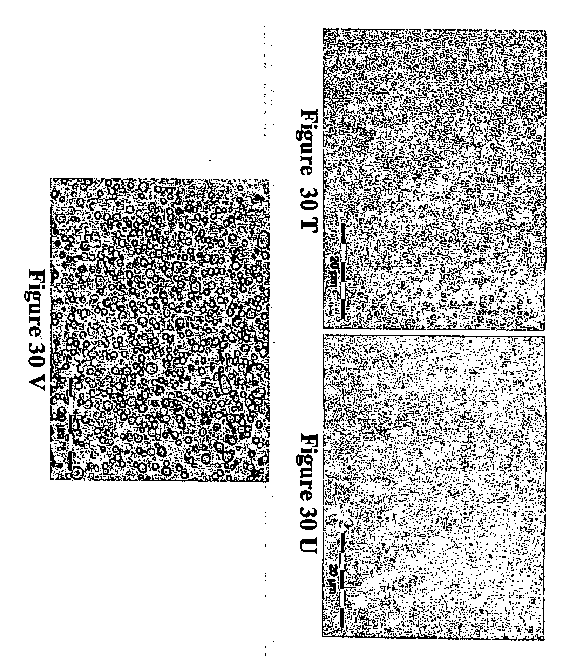

Figure 30 shows the HER2 staining results from Example 11.
Figure 30 A is a photomicrograph of stained slides with DAB stained breast tissue on the lower part of the slide. The fibre reference material is seen on the upper part of the slide. Figure 30 B to E is photomicrographs of the same stained slides using the anti DNP spiked primary reagent solution on the Herceptest ™ staining protocol. Figure 30 B and C is the DAB stained DNP fibres to the left/below and the red Lagertun fibres to the right/up, taken at 4 time and 40 time magnification, respectively. Figure 30 D and E is the DAB stained breast tissue, taken at 10 time and 4 time magnification, respectively.
Figure 30 F to I is photomicrographs of the same stained slides using the original Herceptest ™ staining protocol, without spiked primary reagent. Figure 30 F and G is the stained DNP fibres to the left/below and the red Lagertun fibres to the right/up, taken at 4 and 40-time magnification, respectively. Figure 30 H is the DAB stained breast tissue, taken at 10-time magnification.
Figure 30 I to L is photomicrographs of the same stained slides using the negative rabbit IgG antibody control reagent from the Herceptest ™ staining kit. Figure 30 I is the stained DNP fibres to the left/below and the red Lagertun fibres to the right/up, taken at 40-time magnification. Figure 30 J is the red Lagertun fibres taken at 10-time magnification. Figure 30 K and L is the DAB stained breast tissue, taken at 10 time and 4-time magnification, respectively.
Figure 30 M to N is photomicrographs of the same stained slides using rabbit anti DNP-HRP conjugate (DakoCytomation K5102) diluted 100 times instead of the antibody reagent from the Herceptest ™ staining protocol. Figure 30 M is the stained DNP fibres to the lefl/below and the red Lagertun fibres to the right/up, taken at 40-time magnification. Figure 30 N is the stained breast tissue, taken at 4-time magnification. Figure 30 O to R is photomicrographs of the same stained slides using mouse Envision HRP visualization conjugate instead of the anti rabbit EnVision visualization reagent in the Herceptest ^{™} staining protocol. Figure 30 O is the DAB stained DNP fibres to the left/below and the red Lagertun fibres to the right/up, taken at 40-time magnification. Figure 30 P is the DAB stained breast tissue, taken at 10-time magnification, respectively. Figure Q is the DAB stained red Portion fibres taken at 10-time magnification Figure 30 R is the stained breast tissue, taken at 4-time magnification. Figure 30 S is photomicrographs of Herceptest^{™} stained native Lagertun fibre.
Figure 30 T, U and V are photomicrographs of Herceptest^{™} stained o+, 1+ and 3+ reference cells, respectively.

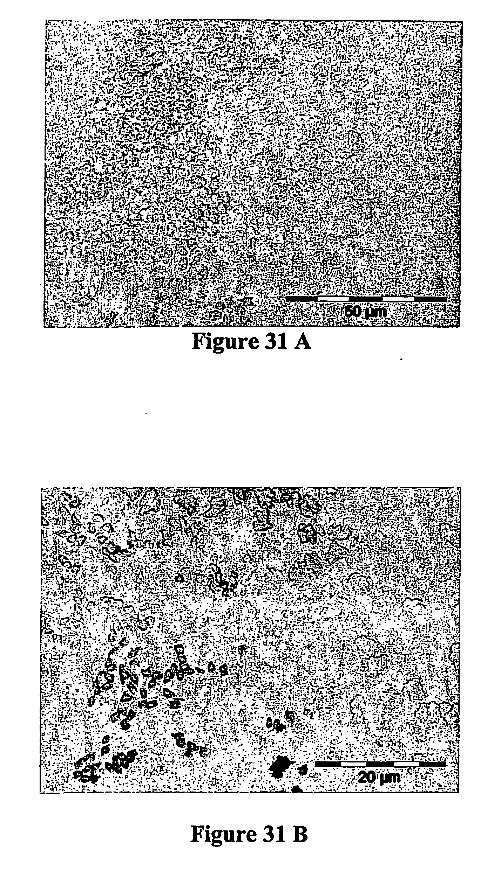

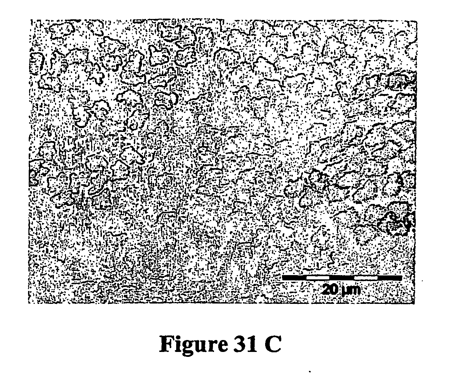

Figure 31 shows the results from Example 12: Photomicrographs of the red and blue collared Unifloss, Lagertun and LP floss fibers.
Figure 31A: 5 times magnification, Figure 31B: 10 time magnification, and Figure 31C: 40 time magnification.

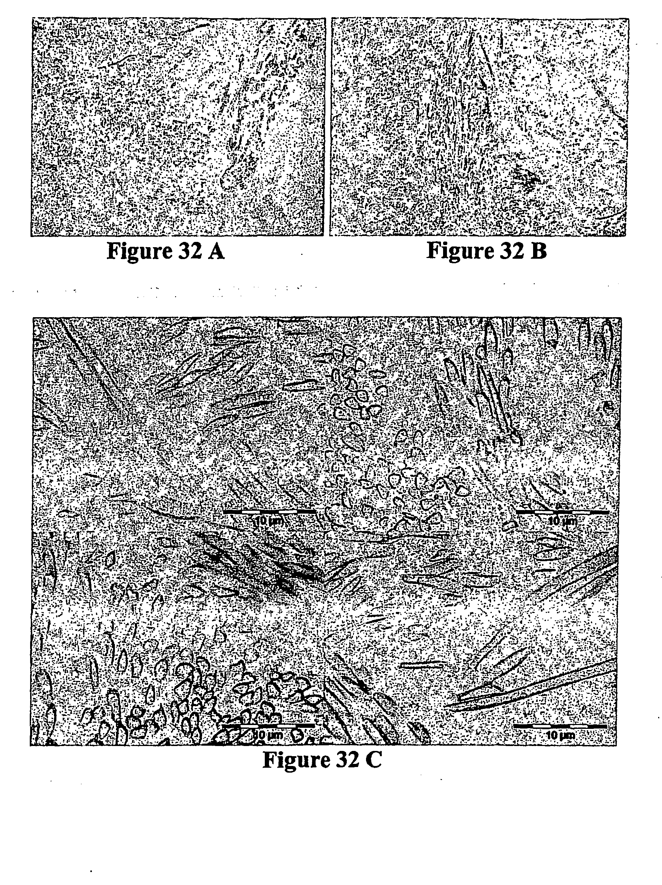

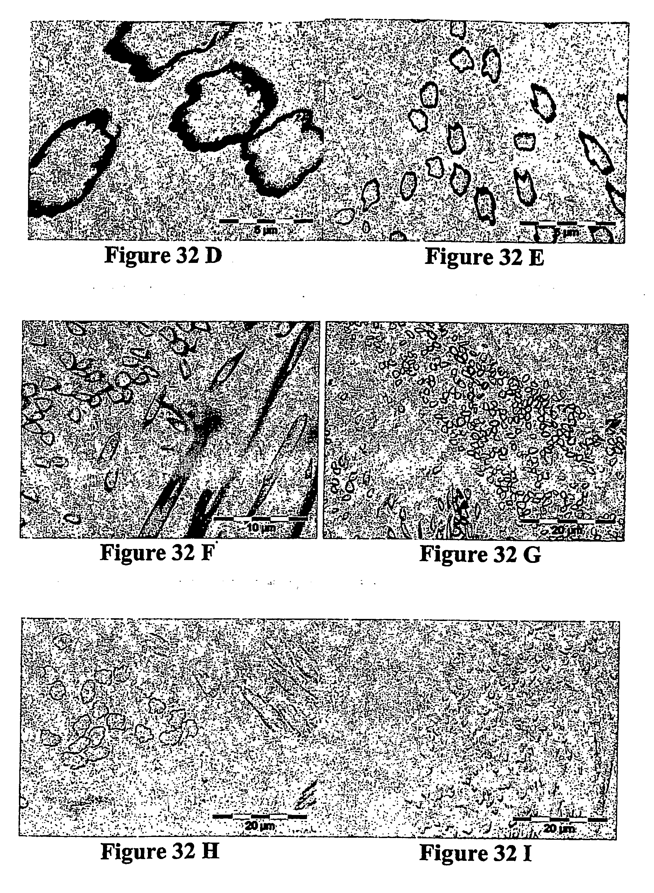

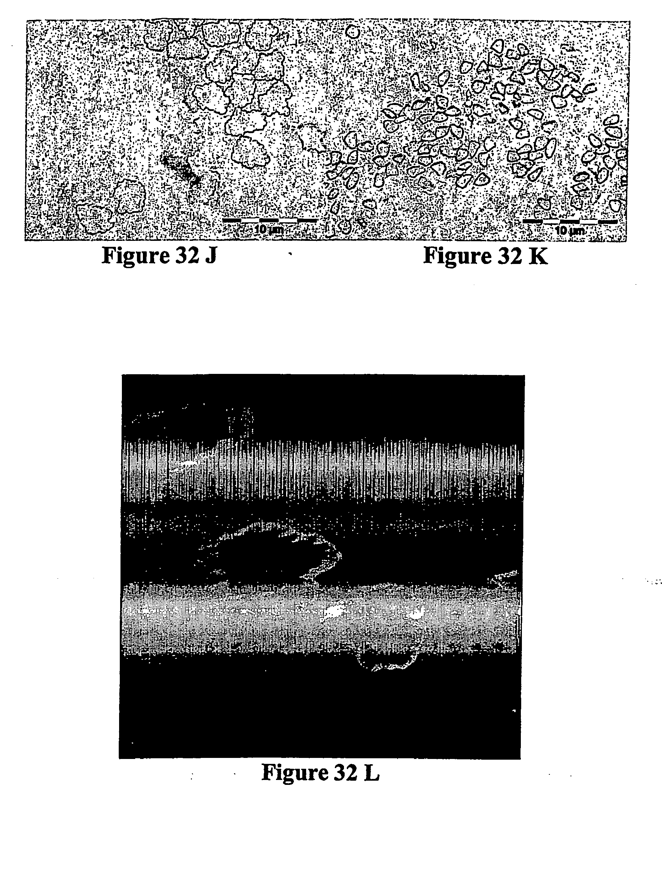

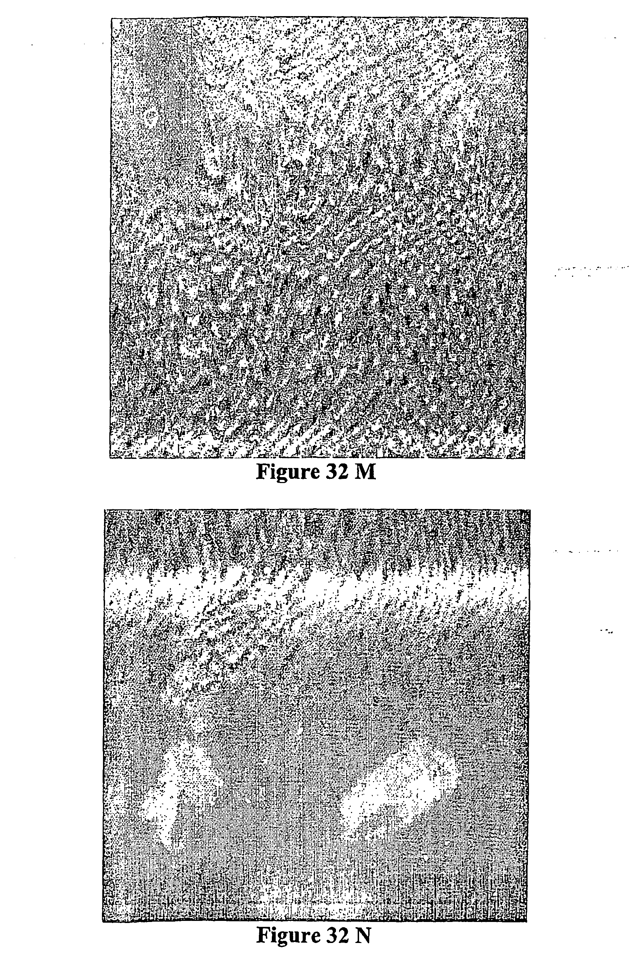

Figure 32 shows the results from Example 13: Figure 32 A and B is photomicrographs of immuno stained HER2 modified Lagertun fibres taken at 4 time magnification. Figure 32 C is 4 photomicrographs of the immuno stained HER2 modified Lagertun fibres taken at 20-time magnification. Figure 32 D and E is photomicrographs taken in the bright field microscope of fast red immuno stained randomly cut HER2 modified Uni floss fibres taken at 40 time (D) and 20 time (E) magnification, respectively. Figure 32 F and G is photomicrographs taken in the bright field microscope of fast red immuno stained randomly cut HER2 modified Lagertun fibres taken at 40 time (F) and 20 time (G) magnification. Figure 32 H and I is photomicrographs taken in the bright field microscope of negative control antibody fast red immuno stained randomly cut HER2 modified Unifloss (H) and Lagertun (I) fibres taken 20 time magnification. Figure 32 J and K is photomicrographs taken in the bright field microscope of fast red immuno stained randomly cut native Unifloss (J) and Lagertun (K) fibres taken 20 time (G) magnification.
Figure 32 L and M is photomicrographs taken in the fluorescence microscope of fast red immune stained randomly cut HER2 modified Unifloss (L) and Lagertun (M) fibres taken at 40 time magnification. Figure 32 N is photomicrographs taken in the fluorescence microscope of negative control antibody fast red immuno stained randomly cut HER2 modified Unifloss fibres taken 40-time magnification.

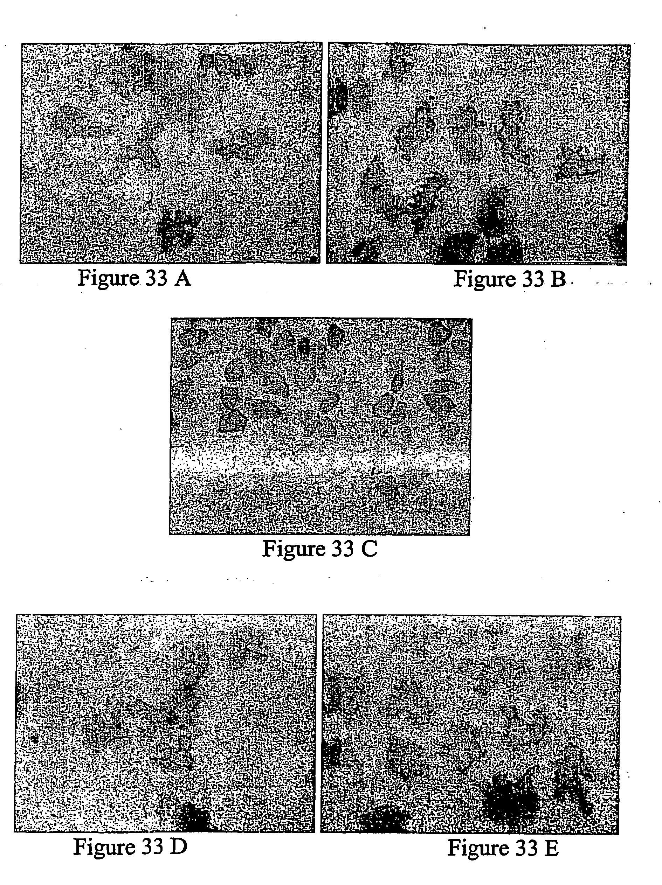

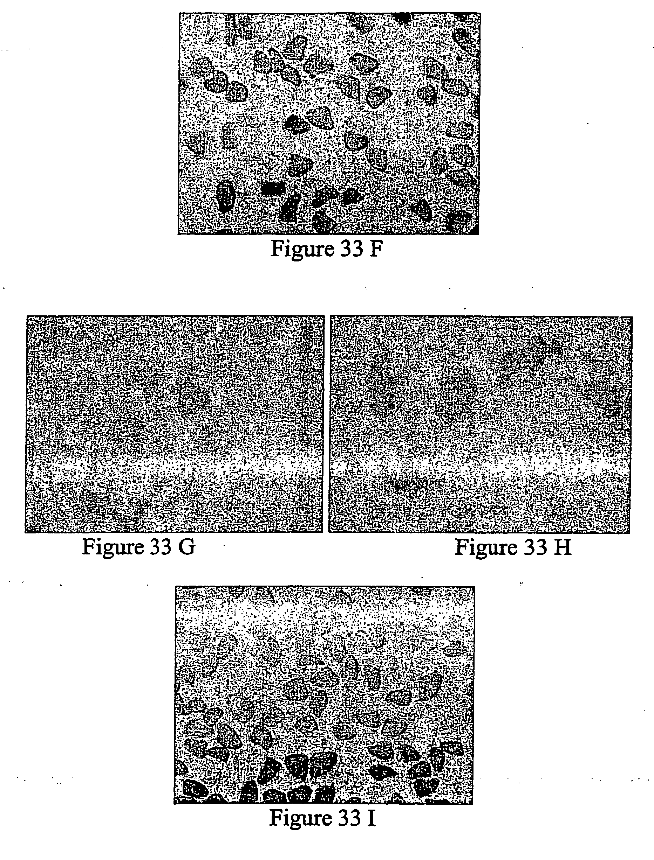

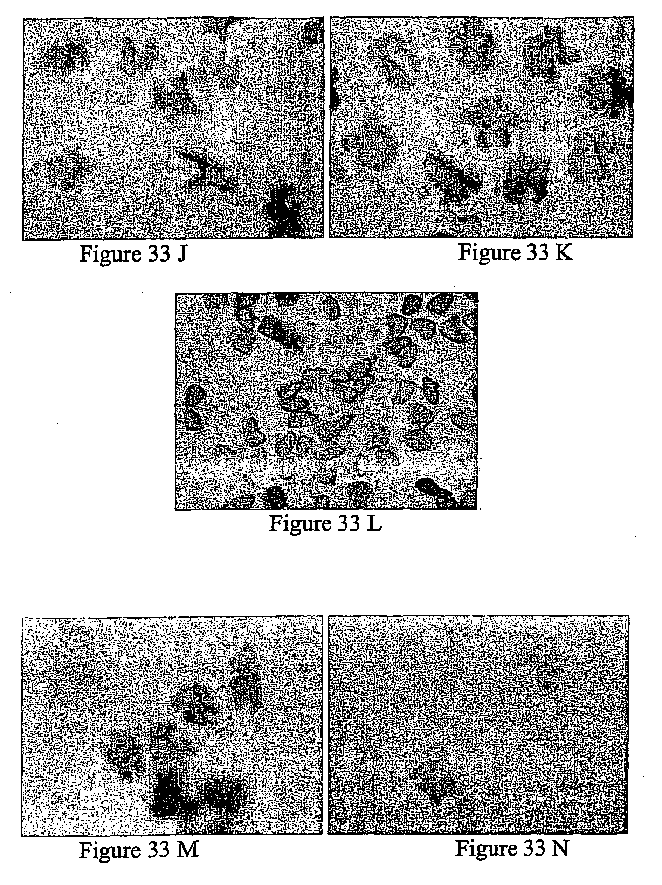

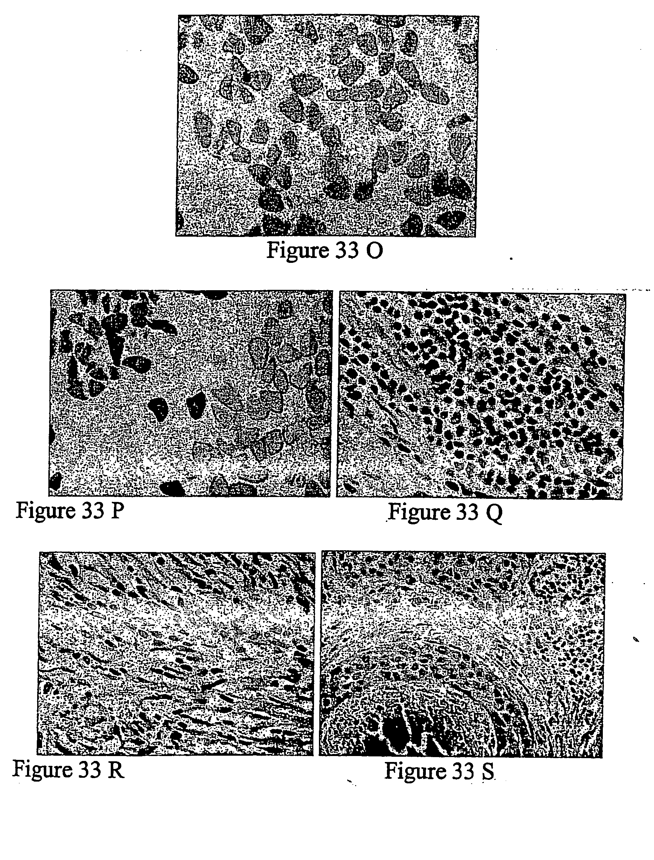

Figure 33 shows the results from Example 14: Photomicrographs of the Haematoxylin and Eosin stained slides taken at 40-time magnification, except for figure 33 S, which was taken at 20 times magnification.
Figure 33 A, B and C are Haematoxylin and Eosin stained native LP Floss, Unifloss and Lagertun fibres, respectively. Figure 33 D, E and F are Haematoxylin and Eosin stained hexan diisocyanate modified and hydrolysed LP Floss, Unifloss and Lagertun fibres, respectively. Figure 33 G, H and I are Haematoxylin and Eosin stained rabbit IgGmodified LP Floss, Unifloss and Lagertun fibres, respectively. Figure 33 J, K and L are Haematoxylin and Eosin stained fluorescein modified LP Floss, Unifloss and Lagertun fibres, respectively. Figure 33 M, N and O are Haematoxylin and Eosin stained DNP modified LP Floss, Unifloss and Lagertun fibres, respectively. Figure 33 P is Procion Red modified fibre bundles with DNP modified fibres (to the right). Figure 33 Q, R and S are HE stained FFPE breast tissue.

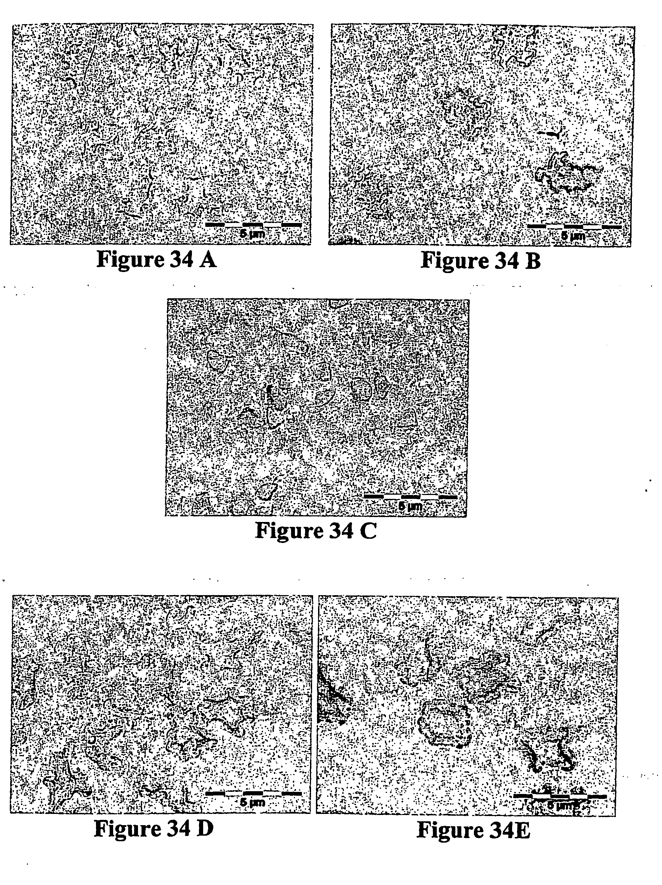

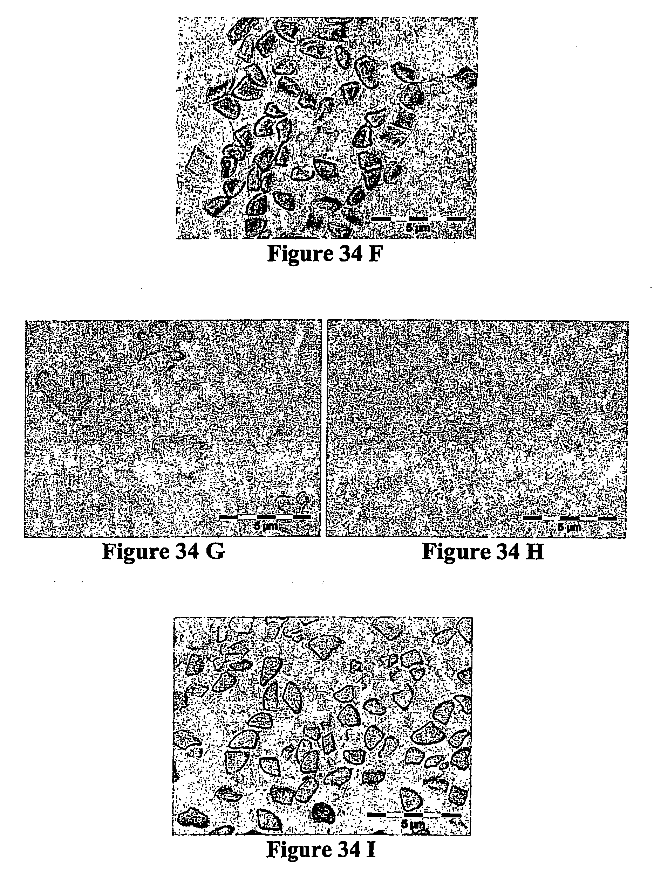

Figure 34 shows the Periodic Acid Schiff staining results from Example 15:
Figure 34 A, B and C is Periodic Acid Schiff stained native LP Floss, Unifloss and Lagertun fibres; respectively. Figure 34 D, E and F are Periodic Acid Schiff stained fluorescein modified LP Floss, Unifloss and Lagertun fibres, respectively. Figure 34 G, H and I are Periodic Acid Schiff stained DNP modified LP Floss, Unifloss and Lagertun fibres, respectively.

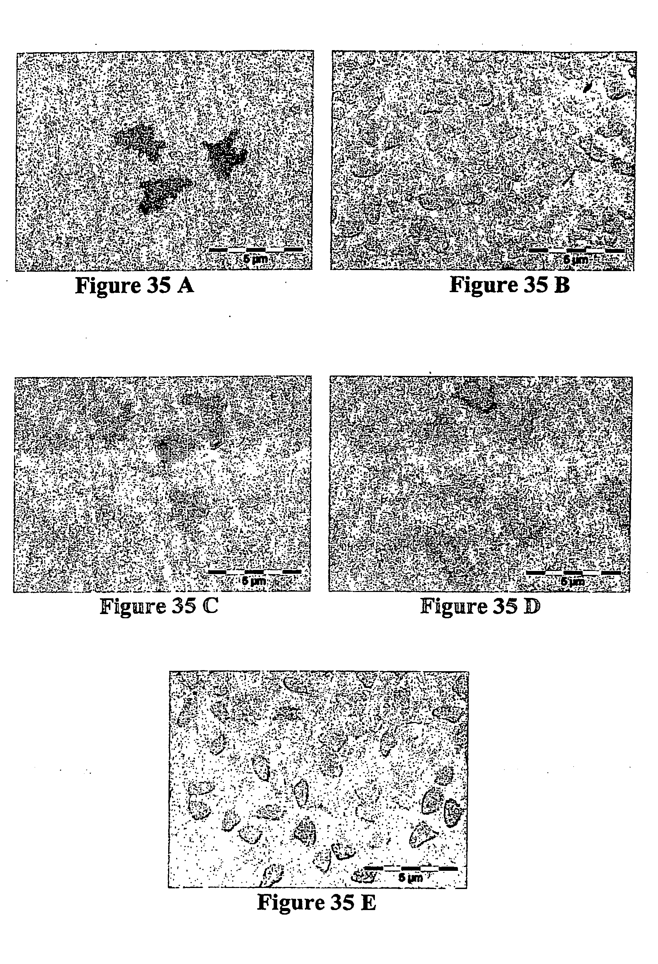

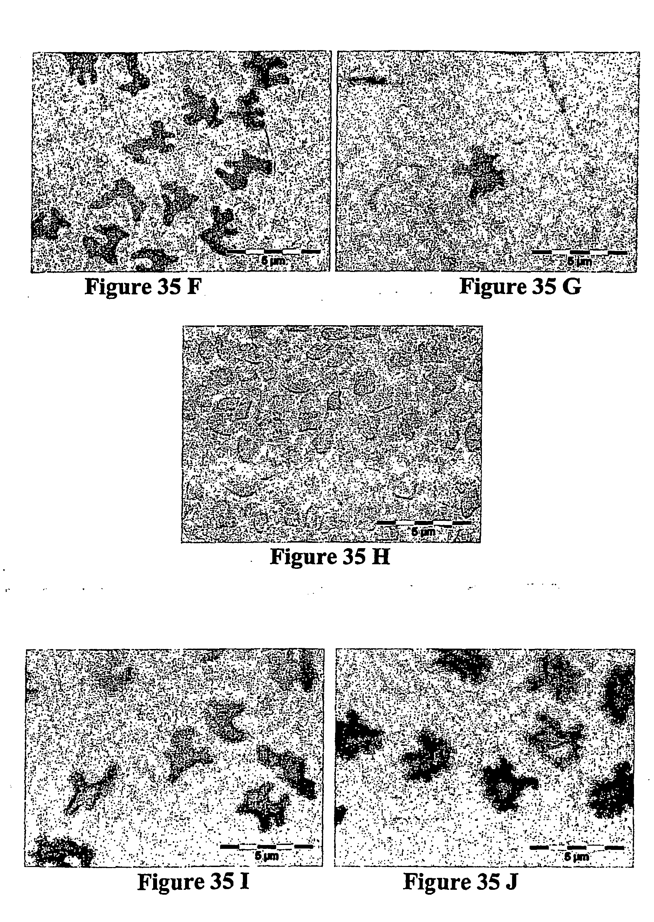

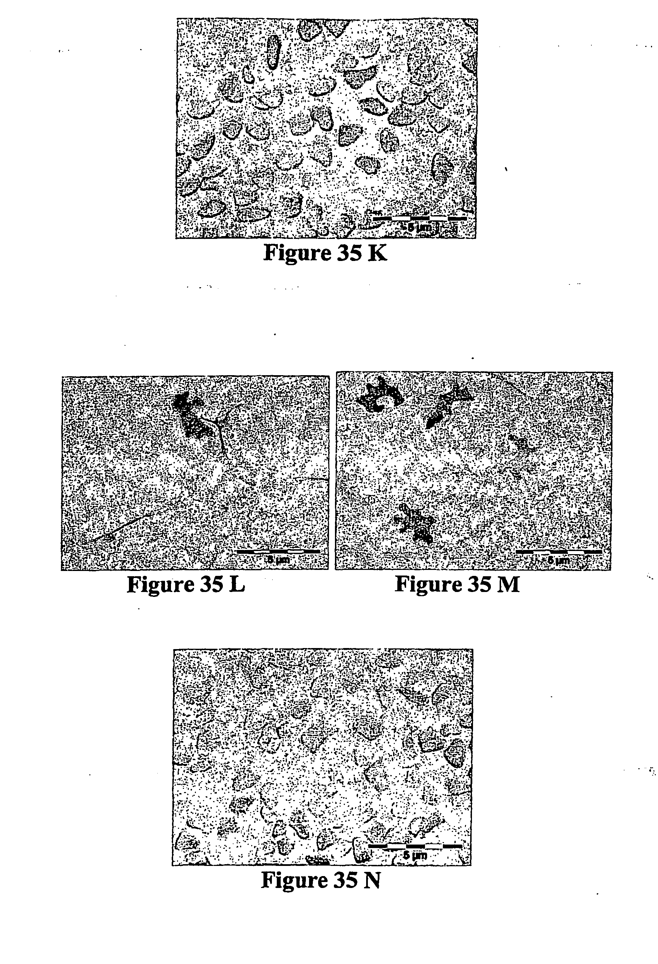

Figure 35 shows the Alcian Blue staining results from Example 15: Figure 35 A and B are Alcian Blue stained native Unifloss and Lagertun fibres, respectively. Figure 35 C, D and E are Alcian Blue stained hexan diisocyanate modified and hydrolysed LP Floss, Unifloss and Lagertun fibres, respectively. Figure 35 F, G and H are Alcian Blue stained rabbit IgG modified LP Floss, Unifloss and Lagertun fibres, respectively. Figure 35 I, J and K are Alcian Blue stained fluorescein modified LP Floss, Unifloss and Lagertun fibres, respectively. Figure 35 L, M and N are Alcian Blue stained DNP modified LP Floss, Unifloss and Lagertun fibres, respectively.

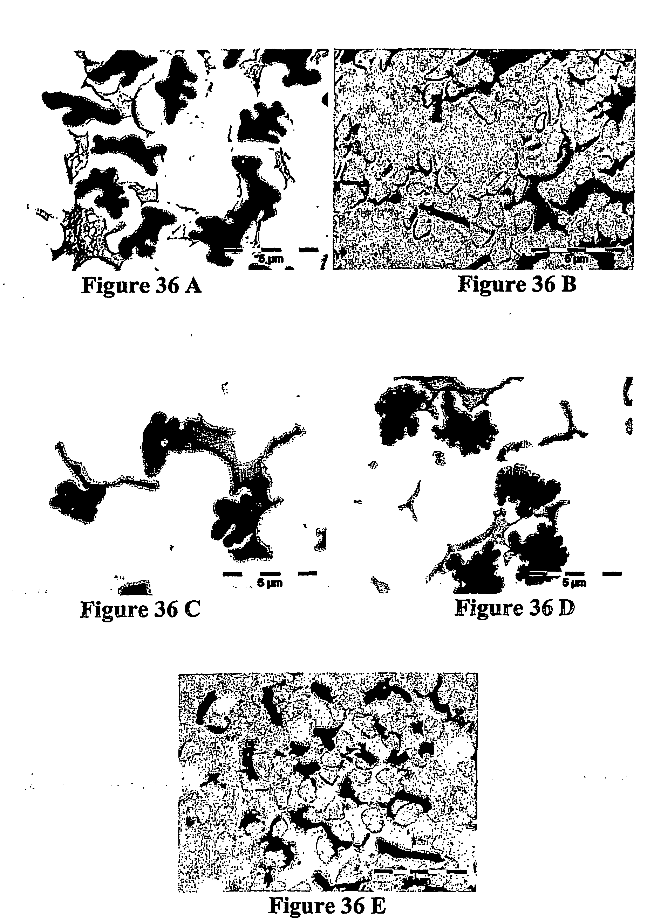

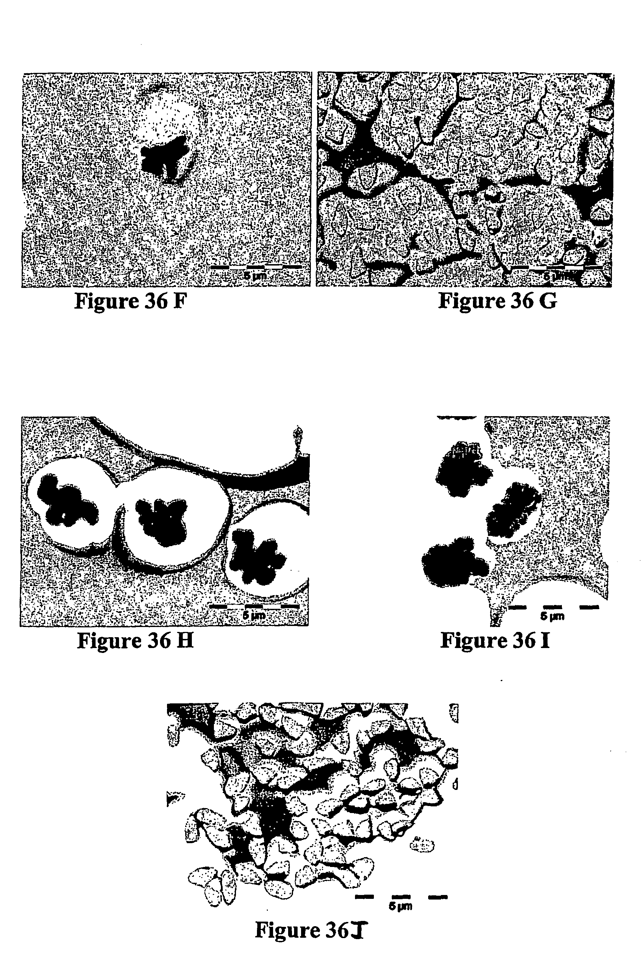

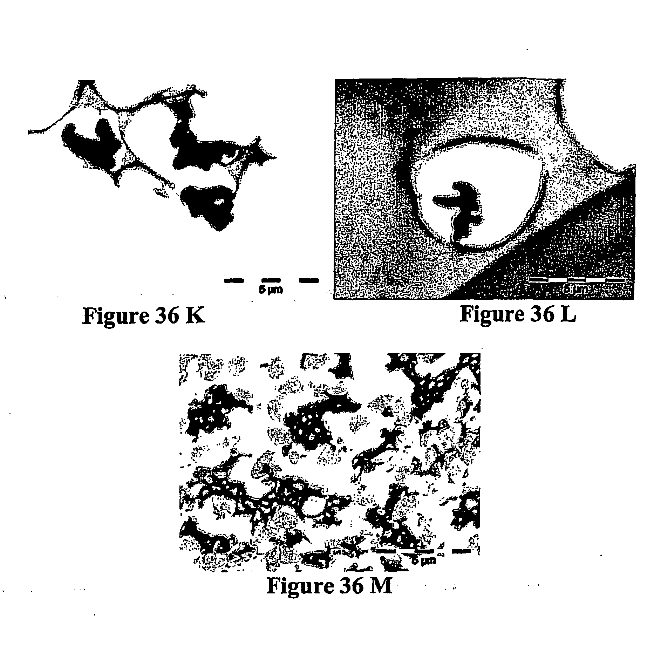

Figure 36 shows the Jones Basement membrane staining results from Example 15:
Figure 36 A and B are Jones Basement membrane stained native Unifloss and Lagertun fibres, respectively. Figure 36 C, D and E are Jones Basement membrane stained hexan diisocyanate modified and hydrolysed LP Floss, Unifloss and Lagertun fibres, respectively. Figure 36 F and G are Jones Basement membrane stained rabbit IgG modified LP Floss, Unifloss and Lagertun fibres, respectively. Figure 36 H, I and J are Jones Basement membrane stained fluorescein modified LP Floss, Unifloss and Lagertun fibres, respectively. Figure 36 K, L and M are Jones Basement membrane stained DNP modified LP Floss, Unifloss and Lagertun fibres, respectively.

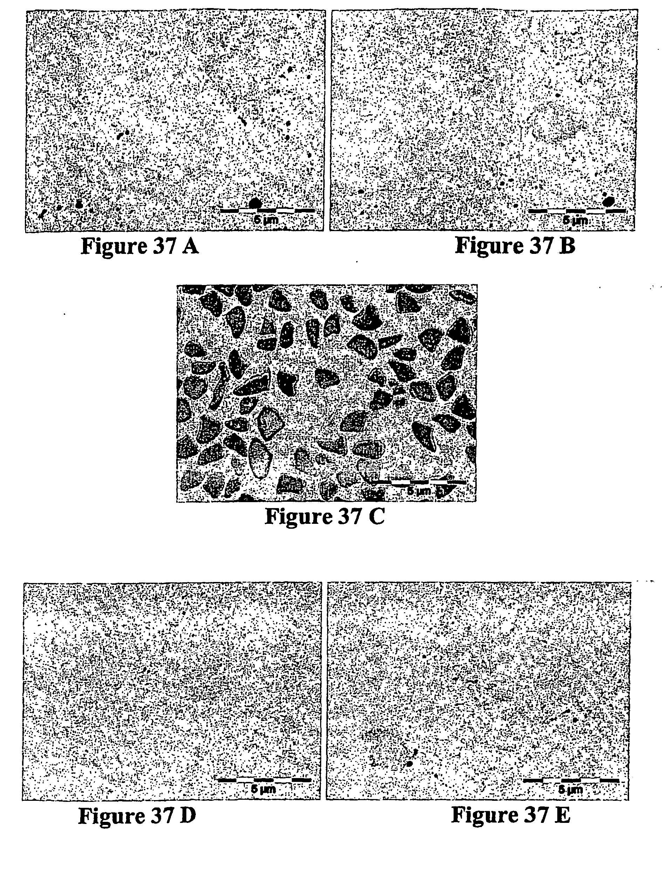

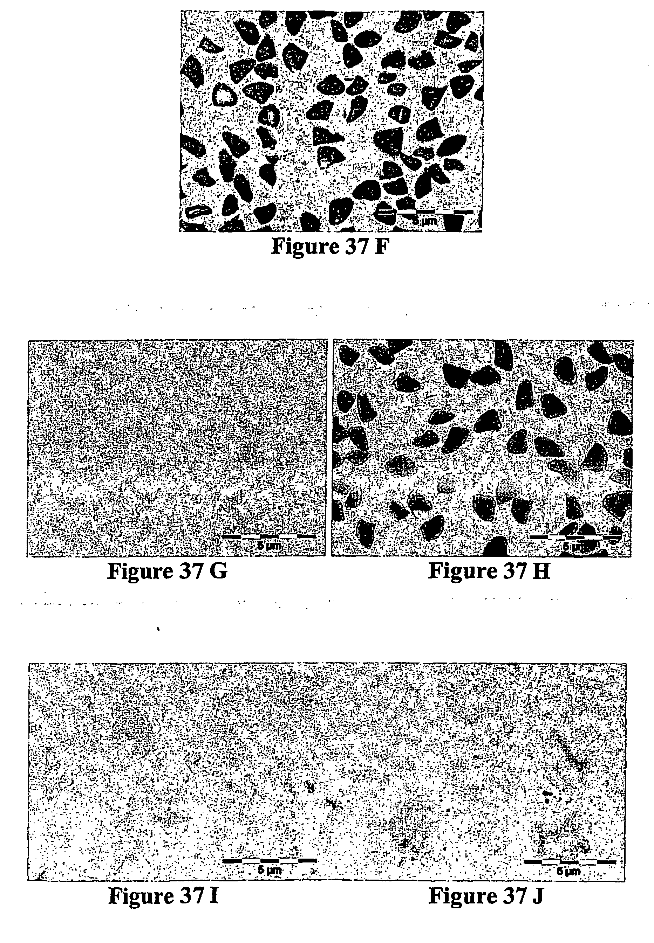

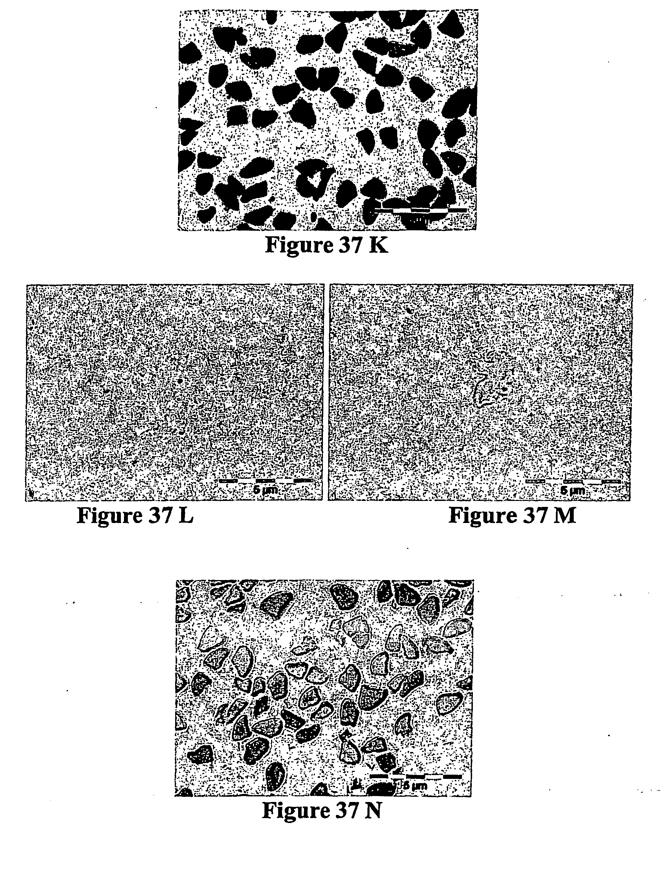

Figure 37 shows the Masson's Trichrome staining results from Example 15: Figure 37 A, B and C is Masson's Trichrome stained native LP floss, Unifloss and Lagertun fibres, respectively. Figure 37 D, E and F are Masson's Trichrome stained hexan diisocyanate modified and hydrolysed LP Floss, Unifloss and Lagertun fibres, respectively. Figure 37 G and H are Masson's Trichrome stained rabbit IgG modified LP Floss, Unifloss and Lagertun fibres, respectively. Figure 37 I, J and K are Masson's Trichrome stained fluorescein modified LP Floss, Unifloss and Lagertun fibres, respectively. Figure 37 L, M and N are Masson's Trichrome stained DNP modified LP Floss, Unifloss and Lagertun fibres, respectively.

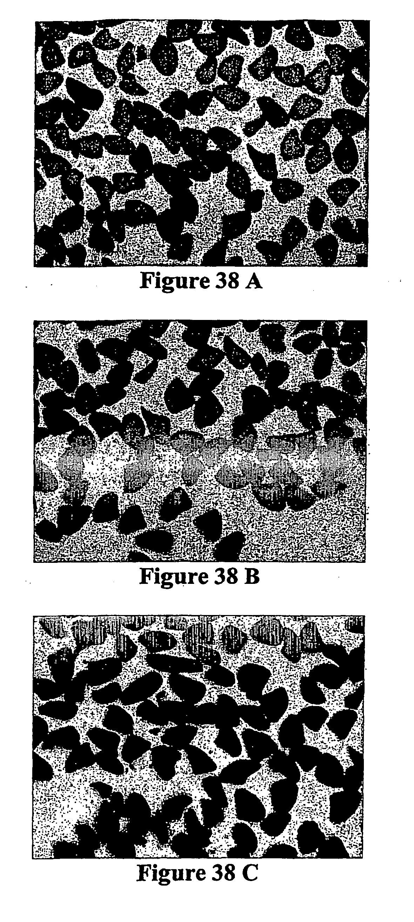

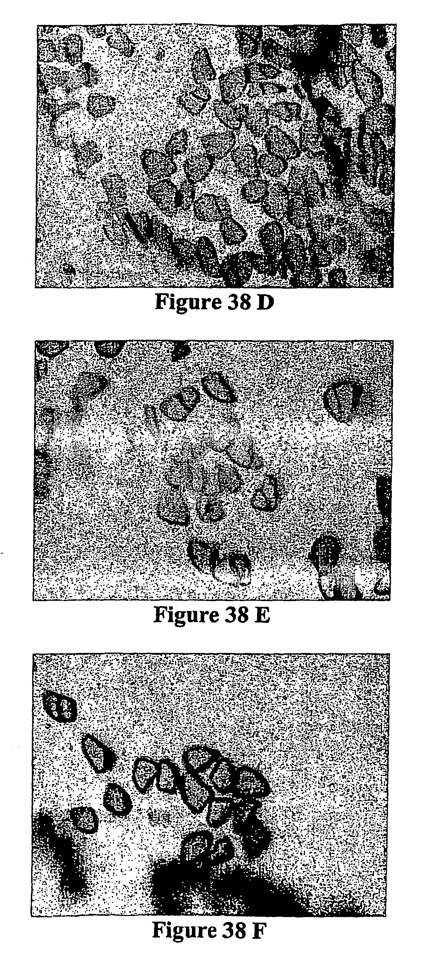

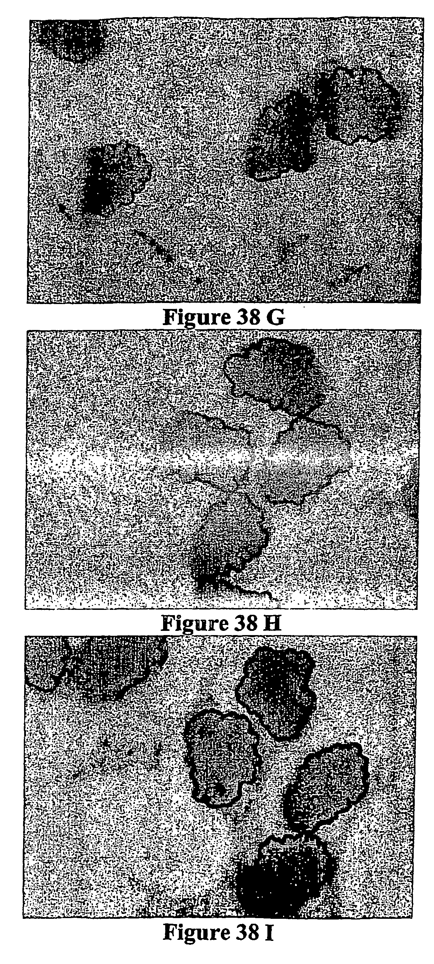

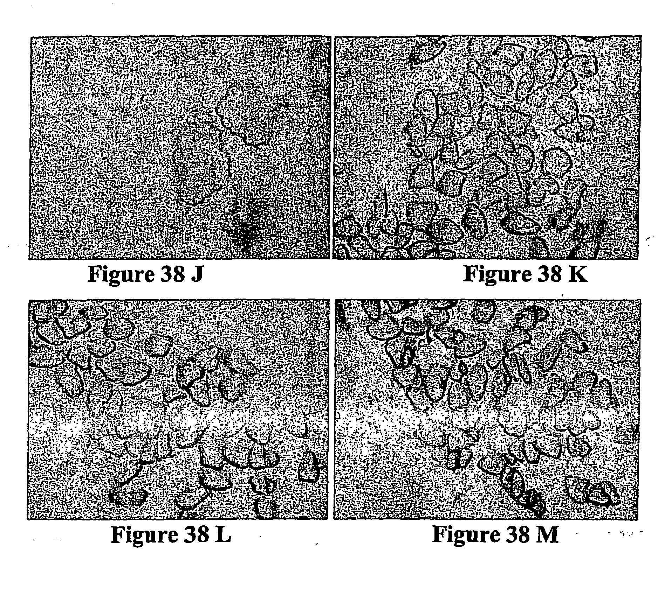

Figure 38 shows results from Example 16: Figure 38 are photomicrographs of Procion Red modified Lagertun fibres cut at 3 µm (A), 5 µm (B) and 7 µm (C). Figure 38 is photomicrographs of anti DNP Envision HRP/DAB stained DNP modified Lagertun fibres cut at 3 µm (D), 5 µm (E) and 7 µm (F). Figure 38 is photomicrographs of anti DNP Envision HRP/DAB stained DNP modified Unifloss fibres cut at 3 µm (G), 5 µm (H) and 7 µm (I). Figure 38 are photomicrographs of native uni floss fibre cut at 5 µm thickness (J), native Lagertun fibre cut at 5 µm thickness (K), negative primary antibody staining of DNP modified Lagertun fibre cut at 7 µm thickness (L) and negative Envision control staining of DNP modified Lagertun cut at 5 µm thickness (M).

### DETAILED DESCRIPTION

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, *Molecular Cloning: A Laboratory Manual,* Second Edition, Books 1-3, Cold Spring Harbor Laboratory Press; Ausubel, F. M. et al. (1995 and periodic supplements; *Current Protocols in Molecular Biology,* ch. 9, 13, and 16, John Wiley & Sons, New York, N.Y.); B. Roe, J. Crabtree, and A. Kahn, 1996, *DNA Isolation and Sequencing: Essential Techniques,* John Wiley & Sons; J. M. Polak and James O'D. McGee, 1990, *In situ Hybridization: Principles and Practice;* Oxford University Press; M. J. Gait (Editor), 1984, *Oligonucleotide Synthesis: A Practical Approach,* Irl Press; D. M. J. Lilley and J. E. Dahlberg, 1992, *Methods of Enzymology: DNA Structure Part A: Synthesis and Physical Analysis of DNA* Methods in Enzymology, Academic Press; Using Antibodies: A Laboratory Manual: Portable Protocol NO. I by Edward Harlow, David Lane, Ed Harlow (1999, Cold Spring Harbor Laboratory Press, ISBN 0-87969-544-7); Antibodies: A Laboratory Manual by Ed Harlow (Editor), David Lane (Editor) (1988, Cold Spring Harbor Laboratory Press, ISBN 0-87969-314-2), 1855. Handbook of Drug Screening, edited by Ramakrishna Seethala, Prabhavathi B. Fernandes (2001, New York, NY, Marcel Dekker, ISBN 0-8247-0562-9); and Lab Ref: A Handbook of Recipes, Reagents, and Other Reference Tools for Use at the Bench, Edited Jane Roskams and Linda Rodgers, 2002, Cold Spring Harbor Laboratory, ISBN 0-87969-630-3.

### REFERENCE STANDARD

We describe novel reference standards which may be used to standardise and grade samples in any staining procedure, including immunohistochemical (IHC) or in situ hybridisation (ISH) procedures, as well as methods of using and making the reference standards described here. Our reference systems are preferably essentially "cell-free", and may be manufactured using relatively simple procedures.

The reference standards as described here provide simple means to establish a "standard", in other words, an established value of a measurable property, by which a sample or test item may be judged. They may be used as colour standards, position standards, quantity standards, quality standards, or as diagnostic standards.

In particular, the reference standards described here allow the status or condition of a sample, or any components of the sample, to be determined. In some preferred embodiments, they allow the detection of whether a sample comprises diagnostically relevant levels, quantities or concentrations of a particular relevant antigen. Preferably, the reference standards as described here are used to gauge levels or amounts of detectable entity in tissues or preferably cells comprised in biological samples.

Thus, in preferred embodiments, the quantity of the detectable entity in the reference standard or planar section thereof is predetermined to correspond to a diagnostically relevant quantity or concentration.

By "diagnostically relevant" we mean a level or quantity of the detectable entity which when present in the biological sample, is an indicator of a condition or disease or symptom being possibly present in the sample or individual from which the sample was derived. Furthermore, it may indicate susceptibility to the disease or condition, etc. Detection of a "diagnostically relevant" level or quantity may be sufficient to indicate that the disease is likely to be present, though other assessments may be required to conclude medically that the person is afflicted with the disease. Thus, our methods are generally capable of providing an indication of the presence or quantity of a detectable entity in a sample, and may not be treated as methods of diagnosis *per se.*

It will be appreciated that the exact quantity or concentration of the detectable entity in the reference standard may vary depending on the condition or disease being considered. In general, the quantity or concentration of the detectable entity in the reference standard will correspond substantially to the level at which disease is expected to occur. Alternatively, or in addition, a smaller quantity or concentration of the detectable entity may be present the reference standard, and the user told that any quantity or concentration detected in the biological sample which is substantially (or quantitatively above this level) should be taken as an indicator of disease, etc.

The level, quantity etc of the detectable entity in the sample may in preferred embodiments be indicative of a "condition" or status of a cell or tissue comprised therein. Such level, quantity etc is therefore preferably indicative of the condition of the organism from which the cell or tissue is derived. The condition may be any state of the cell, tissue, organ or organism, whose detection may be desirable. Included are conditions which the cell, etc may adopt, as part of normal biological processes, such as part of a developmental or differentiation program. Although the term "condition" includes physiologically normal or undisturbed conditions, it preferably refers to non-physiologically standard conditions.

Preferred non-physiologically standard conditions include disease conditions, or any conditions which lead to disease. Preferably, the terms "diagnostically relevant level", "diagnostically relevant quantity" and "diagnostically relevant amount" should be taken to mean a level, quantity or amount of a detectable entity in a sample, which is indicative of a condition or disease in an individual from which the sample is taken.

The diagnostically relevant amount of a detectable entity will depend on the particular disease or condition in question, and may be established as standards. It will be understood that the skilled person will be aware of such standards, and will be able to determine the relevant diagnostically relevant amount depending on the disease or condition.

A reference standard as described here comprises at least two components: (i) a quantity of a relevant detectable entity and (ii) a support medium which supports the detectable entity. The detectable entity has an elongate path, and preferably adopts a generally elongate path in the embedding medium. The reference standard as described here is preferably in the form of a block.

In preferred embodiments, the reference standard is such that a detectable amount of the detectable entity is present in a defined region, preferably a reference area, in a cross section of the reference standard.

In highly preferred embodiments, the reference area present in the cross section of the reference standard has similar dimensions to a cell, for example, a prokaryotic cell or a eukaryotic cell, preferably an animal cell and most preferably a human cell. That is to say, the reference area in such embodiments has approximately the same size or shape of a cell, or both, such that the reference area has a shape which mimics a real cell in the sample. This enables a direct comparison to be easily made between a cell in a sample, and the reference area present in the slice or cross section. A user can then for example easily compare (by eye) the level of staining of a cell and the level of staining of the cross sectional profile, to make a judgement on whether that cell is "positive" or not.

Preferably, the reference area has a diameter of (or a greatest or maximum dimension of) between about 0.5 µm to 25 µm, more preferably between 1 µm and 20 µm, even more preferably between 1.5µm to 15 µm. The dimension can be up to, but preferably less than 30µm, more preferably 29µm or less, much more preferably 28µm or less, even more preferably 27µm or less, more preferably 26µm or less, or more preferably 25µm or less. Particularly preferred embodiments are those with diameters or greatest dimensions of, or substantially of, 0.5µm, 1µm, 2µm, 3µm, 4µm, 5µm, 6µm, 7µm, 8µm, 9µm, 10µm, 11µm, 12µm, 13µm, 14µm, 15µm, 16µm, 17µm, 18µm, 19µm, 20µm, 21µm, 22µm, 23µm, 24µm, 25µm.

In highly preferred embodiments, the detectable entity has an elongate path which is lengthwise in relation to the reference standard. The term "lengthwise" is understood to mean of, along, or in reference to the direction of the length. The elongate path therefore preferably runs or extends in the direction of the length of the reference standard.

A preferred configuration in which the detectable entity is disposed longitudinally in relation to the reference standard is shown in Figure 2A. Other configurations are possible, and are described in further detail below.

The detectable entity is one whose presence and preferably quantity is revealable, that is, its presence is demonstrable or its amount is measurable, either directly or indirectly. It may be visible to the naked eye, or visible with the aid of magnification such as the use of a microscope. The detectable entity may be visible only when stained with a dye. The detectable entity which is embedded or supported may take a variety of forms, as described in further detail below. The detectable entity is preferably one which is detectable by use of a binding agent, which is capable of binding to the detectable entity and revealing its presence. The detectable entity may in particular comprise protein, peptide or polypeptide, or nucleotide, nucleic acids, in particular, DNA and RNA.

Further details on detectable entities suitable for use in the reference standard described here are provided later in this document.

Preferably, the reference standard is such that a cross section of it includes a defined area comprising a known or pre-defined amount of detectable entity. Where the quantity of the detectable entity in the reference standard is known, it may be compared with that in the sample to establish the quantity or quality of the detectable entity in that sample, or preferably cells comprised in the sample.

In highly preferred embodiments, slices or sections of the reference standard are taken. These slices or sections should be treated as part of the invention described here.

Such slices or sections comprise defined areas comprising the detectable entity, as shown in Figure 2C. Multiple slices or sections may be made from a reference standard. The slices or sections may be treated like any section from FFPE material, to reveal the detectable entity. Treatment of the slices or sections of the reference standard together (preferably in parallel with) the sample ensures uniformity, and reduces or eliminates variations due to differences in protocols, materials, etc as discussed earlier in this document.

In preferred embodiments, the sections or slices are taken through one or more, preferably all, of these steps, preferably in parallel with the relevant FFPE section. This is illustrated in Figure 3.

The slices or sections do not need to be of uniform thickness, but may be. It will also be apparent that the staining intensity can be changed by the thickness of the slice. For example, various staining intensities could be obtained by using slices of different thickness. Thus, the staining level may be varied by varying the thickness of the section and therefore the amount of fibre material per area. As the fibres are homogeneous in length, this is a very simple method of controlling the staining intensity.

### REFERENCE STANDARD USES

As described above, the reference standards as described here provide simple means to establish a "standard", in other words, an established value of a measurable property of a detectable entity. The same or another property, of the same, similar, or different entity in a sample or test item may also be measured, and the values may be compared.

In the most general sense, the reference standard enables the presence of the detectable entity to be revealed. Thus, for some purposes, it is often enough to simply obtain information on the presence of the detectable entity in the reference standard. However, for other purposes, information on one or more characteristics of the detectable entity may be desired. Thus, for example, characteristics such as a dimension, quantity, quality, colour, orientation, position, reactivity (or any combination of any two or more these), etc may be determined. The referenc standards may also be used to validate one or more procedures in a method.

### Colour Standard

In one embodiment, the colour of the detectable entity is detected or determined. Thus, for example, it may be desired to have a colour standard in a series of staining experiments. In this case, the reference standard described here may be used to provide a "standard" colour, by inclusion of a detectable entity which has, or is stained to provide, a pre-determined colour.

Use of such a "colour standard" enables an operator to judge whether a sample, which may be stained, is similar to or different from the "standard" colour. For example, the sample when stained may be expected to produce a certain blue colour if positive, and the reference standard may therefore comprise a detectable entity which has or may be stained to produce such a blue colour. The colour in the sample may thus be compared to the blue colour provided by the reference standard to establish whether the sample should be regarded as positive or not.

Furthermore, the "colour standard" may also be used for example for calibration of optical machinery. Colour drifts or errors in colour detection which occur during use of optical machinery may therefore be prevented or adjusted for by comparing the response of the machinery to the standard colour and suitable adjustment if necessary. Two or more "standard" colours, for example of different wavelengths, may be included in the reference standard for more precise calibration.

### Position Standard

The position of the detectable entity within the reference standard may be detected or determined. Thus, the area comprising the expected colour may be detected by an operator or by machinery, to establish a reference point for a grid location in the sample, for example. The distance between such a reference point and a point in the sample may easily be measured, to provide information on distance, area or volume within the reference standard or sample. The reference standard or position standard may comprise two or more such position standards, preferably three or more position standards. Use of multiple colour locations, of the same or different colours, enables higher accuracy of dimensioning, positioning and navigation through triangulation.

### Quantity / Quality Standard

In other embodiments, the quantity of the detectable entity is detected or determined. This is most readily achieved by reaction with the binding agent, and the binding agent may be labelled for this purpose. Preferably, the binding agent binds to the detectable entity in a stoichiometric fashion. The intensity of the staining by the binding agent then gives information on the quantity or amount of detectable entity. However, it will be appreciated that other characteristics of the staining, and not just the intensity, may be just as important or useful.

### Validation Standard

In addition to the other uses described elsewhere in this document, the reference standard described here may be used for validating or verifying one or more procedural steps in a method. By validation and verification we specifically refer to a process by which the success, effectiveness or efficiency of a procedural step is measured.

In general, we disclose a method of validation of a procedure, the method comprising providing a reference standard for a detectable entity as disclosed in this document, applying the procedure to the reference standard or a portion thereof (in particular a slice or section of it), and detecting a change in a property of the detectable entity as a result of the procedure. The property of the detectable entity which is changed is preferably one which is indicative of the success or failure (or relative success or failure) of the procedure. In particular, the detectable entity may be modified in such a way that the procedure, where successful, removes the modification. Alternatively, or in addition, the detectable entity may be modified by the procedure. In each case, the modification is one which is easily detectable as a means to detect the success or failure of the procedure.

Therefore, we disclose a method of assessing the effectiveness or success of a procedure, the method comprising the steps of: (a) providing a reference standard as described in this document, in which a detectable property of the detectable entity is changed as a result of the procedure; (b) conducting the procedure on the reference standard; and (c) detecting a change in the detectable property of the detectable entity.

In one embodiment, a detectable property of the detectable entity is changed as a result of a successful procedure, which change in the detectable property of the detectable entity is detected to establish that the procedure is successful. Alternatively, or in addition, a detectable property of the detectable entity is changed as a result of an unsuccessful procedure, which change in the detectable property of the detectable entity is detected to establish that the procedure is not successful.

We also disclose the use of a reference standard as described in this document, as an antigen retrieval validation standard, a deparaffination standard, a blocking validation standard, a washing validation standard, a primary antibody validation standard, a secondary antibody validation standard, a calibration standard, or a diagnostic, standard. The detectable entity preferably comprises a property which is detectable, and which is changed as a result of the procedure, i.e., whether the procedure is successful or unsuccessful.

In particular, the reference standard described here may be used to validate any one or more of the steps employed in traditional IHC staining procedures. Such steps may include: Removal of paraffin, antigen retrieval (AR), blocking, endogenous biotin blocking (for example where a biotin based visualisation system is used), endogenous enzyme blocking (for example phosphatase or peroxidase activity), one or more washing steps, incubation with revealing agent such as a primary antibody, incubation with secondary visualisation components, chromogen staining (for example, enzyme catalysed), staining information acquisition and analysis.

In particular, the reference standard may be used to validate: i) Verifying the ability or functionality of the visualisation system to stain the cell population in the particular staining procedure, ii), verifying the ability or functionality of the primary antibody to stain the cell population in the particular staining procedure, iii) defining a staining threshold intensity for counting positively stained cells, iv) defining the diagnostic threshold intensity ratio between two or more stained populations, v) or verifying the function of individual reagents in the staining protocol, for example antigen retrieval, washing efficiency, blocking of peroxidase activity, and secondary visualisation reagents.

In highly preferred embodiments, the reference standard may be used as a validation standard for the steps of the addition of the primary antibody, the antigen retrieval step, the addition of the secondary visualization reagents, and staining information acquisition and analysis.

### General Procedural Validation Standard

In one particular embodiment, the detectable entity may comprise streptavidin or avidin. The streptavidin or biotin may be bound to the elongate path, such as the fibre. The resulting reference standard may then be used as a simple indicator for the correct addition of certain reagents, for example incubation with the correct primary antibody. By adding for example a small amount of biotinylated mouse antibody to the otherwise un-labelled primary antibody solution, the visualization system will stain the reference standard positive if the correct primary antibody is used on the particular slide.

In another particular embodiment, the detectable entity may comprise an immunoglobulin, for example a rabbit or mouse immunoglobulin or antibody. The immunoglobulin could for example be bound to the fibre in embodiments employing these. The ability of the secondary visualization systems to recognise and stain rabbit or mouse antibodies may thereby be validated.

In yet another particular embodiment, the detectable entity may comprise a hapten, for example DNP (2,4-dinitrophenyl (DNP) hapten). Other haptens which are suitable include: phosphorylcholine, dextran, NIP (4-Hydroxy-3-iodo-5-nitrophenylacetyl), NP (4-Hydroxy-3-nitrophenylacetyl), PC (Phosphoryl Choline) and TNP (2,4,6-Trinitrophenyl).

The hapten could for example be bound to the fibre in embodiments employing these. Such a procedural validation standard could be used to validate the addition of a primary antibody in a detection system. In this case, an amount of anti-hapten antibody would be used to "spike" the primary antibody composition. Addition of the primary antibody (containing the anti-hapten antibody) would allow allow the anti-hapten antibody to bind to and reveal the hapten conjugated to the fibre. If the hapten is revealed, it can be concluded that the primary antibody has been added properly to the test. Thus, the visualization system will stain the reference standard positive if the correct primary antibody is used on the particular slide.

The term "hapten" as used in this document should be understood to refer to a small molecule, not usually antigenic by itself, that can react with antibodies of appropriate specificity and elicit the formation of such antibodies when conjugated to a larger antigenic molecule, the carrier or schlepper. Where an anti-hapten antibody is used to spike the primary antibody, the anti-hapten antibody should preferably come from the same animal source as the primary antibody, e.g., a rabbit anti-hapten antibody should preferably be used to validate the addition of a rabbit anti-antigen antibody.

### Cutting Thickness Standard

The reference standard may be used as an standard to validate the correct thickness of a slice from the paraffin block. It is known for example, that it is important for a correct thickness of a paraffin slice to be cut by the microtome. Because of variations in temperature, different types or makes of microtome or blades used, different calibrations of such instruments across labs, however the thickness of a paraffin slice containing a sample can often be difficult or impossible to standardise.

A reference standard according to the description can be used to validate this procedure, by being sliced to the same thickness as the slide in question containing a sample. In other words, a paraffin block containing the sample is sliced to form a slice of a certain thickness, in parallel with a reference standard in the form of a block. Depending on the thickness of the slice of the reference standard, the detectable entity in the reference standard will have a different detectable property, such as higher optical density, darkness, etc. As the relationship between the thickness of the reference standard and the value of the detectable property is known, an indication of the expected value of the detectable property for the optimal slice depth can be provided to the user. Thus, the user establishes whether the correct thickness has been achieved by measuring the value of the detectable property in the sliced reference standard, and comparing it with the expected value.

### Antigen Retrieval Validation Standard

The reference standard may be used as an antigen retrieval validation standard. For this purpose, we disclose a method of assessing the effectiveness or success of an antigen retrieval procedure, the method comprising the steps of: (a) providing a reference standard as described in this document, in which a detectable property of the detectable entity is changed as a result of the antigen retrieval procedure, in which the detectable property of the detectable entity comprises the masking or unmasking of one or more epitopes; (b) conducting the antigen retrieval procedure on the reference standard; and (c) detecting a change in the detectable property of the detectable entity.

In highly preferred embodiments, the detectable entity in the reference standard is modified to mask one or more epitopes, some or all of which are unmasked in an antigen retrieval procedure which is successful.

Antigen retrieval ("AR") procedures can be standardized or monitored by employing reference standards comprising detectable entities which cannot normally be stained or in which the staining level depends strongly on the antigen retrieval process.

The detectable entity may be modified such that it completely or partially loses its antigenicity. In other words, one or more epitopes in the detectable entity may be masked artificially or naturally. Correct antigen retrieval unmasks the epitope(s) or antigens, and is revealed by correct staining of the detectable entity in subsequent procedures.

The masking or loss of antigenicity (which may affect one or more epitopes) may be effected chemically. For example, the same immunoglobulin modified fibre as described above could be fixed with for example formaldehyde. In particular, the detectable entity could be "masked" by over fixation with formaldehyde, resulting in the loss of most or all of the epitopes and low diffusion in the reference material.

Paraformaldehyde or any other fixatives as known in the art may also be used. Derivatives with acetylating, alkylating or otherwise masking reagents may also be employed for this purpose. Numerous methods are known from the organic chemistry literature, for example masking using Schiff Bases, esters, ethers or hemiacetal derivatives.

The masking may also be effected immunologically, by for example, the use of a suitable masking antibody or other binding agent. The reference material may therefore contain chemically and/or immunologically masked targets for either the primary antibody or the secondary visualization system.

Demasking or deprotection can be achieved by either chemoselective antigen retrieval or random antigen retrieval. Masked targets, which will only be stained when excessive antigen retrieval procedures are used, may also be employed

Such a reference standard can be used as an indicator of correct antigen retrieval. Correct antigen retrieval procedure will demask the immunoglobulin and stain this embodiment of the reference standard. Fibres with other proteins or peptides could be fixed with for example formaldehyde and thereby totally lose their antigenicity. Such a reference standard will be indicative of correct antigen retrieval. Correct antigen retrieval procedure will demask the immunoglobulin and stain this embodiment of the reference standard.

### Deparaffination Standard

The reference standard may be used as validation standard for deparaffination, i.e., for the step of removal of paraffin.

For this purpose, we disclose a method of assessing the effectiveness or success of a deparaffination procedure, the method comprising the steps of: (a) providing a reference standard as described in this document, in which a detectable property of the detectable entity is changed as a result of the deparaffination procedure, in which the detectable property of the detectable entity comprises the presence or quantity of detectable entity in the reference standard following the deparaffination procedure; (b) conducting the antigen retrieval procedure on the reference standard; and (c) detecting a change in the detectable property of the detectable entity.

In highly preferred embodiments, the detectable entity in the reference standard is soluble in the deparaffination medium, and in which at least a portion, preferably all, of the detectable entity is removed following a successful deparaffination procedure.

Thus, reference material with non-covalently attached and water insoluble targets detected by for example the secondary visualization systems can indicate insufficient deparaffination if they are stained.

For example, in traditional IHC procedures, deparaffination is done by washing with toluene or for example citrus or coconut oil followed by rehydration in alcohol/water solutions. Thus, a reference standard used for such a purpose may comprise detectable entities which are easily detached or removed or dislodged from their positions within the supporting medium. For example, they may be loosely attached, for example, by non-covalent means, to the fibres in certain embodiments. For such purposes, it is desirable that the detectable entity be water insoluble. If the deparaffination step is conducted properly, then the detectable entity should not be revealed by the reagents such as secondary visualisation systems.

Furthermore, a marker such as a dye may be added to the paraffin. Such a dye would preferably stain the detectable entity, or the fibre on which the detectable entity is attached. Where insufficient deparaffination has taken place, the presence of the dye will be easily visible to the human eye (or easily detected by machinery such as image analysis systems). This will indicate that insufficient deparaffination has taken place.

### Blocking Validation Standard

The reference standard described here may also be employed for validating any blocking steps, for example, blocking of endogenous activity, such as endogenous biotin activity or enzymatic activity.

Thus, for example, biotin or other haptens, which are naturally deposited in tissue, need to be blocked before one can use a visualization system using biotin or other haptens. It is because of the presence of endogenous biotin that secondary (for example goat anti mouse or goat anti rabbit) visualization systems are preferred, as they produce less "background" noise. For validating blocking, the reference material can contain biotin (or other haptens like Digoxigenin or DNP) used in the visualization system, which may be bound to the fibre where present. Positive staining of this reference material will indicate insufficient biotin blocking - due to for example non-reactive reagents, inefficient mixing/diffusion on the slide or too short incubation time.

The reference material may also contain covalently bound enzyme used in the enzyme catalysed staining system as the detectable entity. The typical enzymes used are peroxidase or phosphatase. For example, the detectable entity may comprise horseradish peroxidase. Alternatively, or in addition, where the reference standard comprises fibres, such fibres modified with for example horseradish peroxidase may be used for validating correct and efficient endogenous peroxide blocking. If the reference standard remains unstained after the last peroxide chromogen step, the blocking will be determined as being efficient.

Thus, positive staining of this reference material will indicate insufficient latent enzyme blocking - due to for example non-reactive reagents, reversible blocking, inefficient mixing/diffusion on the slide or too short incubation time. The material could be combined with the antigen retrieval reference material.

### Washing Validation Standard

The reference standard may also be used to validate the efficiency of any number of washing steps, for example, washing with buffer. The reference material could include a detectable entity which is insoluble in organic solvent (for example, toluene insoluble) and partly water insoluble for either the primary or secondary visualization system. The target should not be covalently bound in the reference material. It could be bound by ionic pairing or metal complex binding or by simply adsorption.

The target could have a large molecular weight in order to test the ability of washing buffers to diffuse into the material and remove the target. The molecular weight cut off ("MwCO") of the reference material (for example cellulose fibre) should be matched with the Mw of the targets by e.g selection of pore size or the degree of fixation.

Positive staining of this reference material will indicate insufficient washing - either due to for example number of washing steps, type of buffer used, inefficient mixing on the slide or too low temperature or diffusion times. For this purpose, the mounted sample tissue section and the reference material should preferably have the same thickness.

Alternatively, the target could be substituted with a detectable high molecular weight dye trapped in the reference material. The dye will be removed by efficient washing - and only be present if the washing is insufficient or ineffective. Alternatively, a reference standard comprising a fibre (as detectable entity) made of a partly water soluble material could be used. If the fibre disappears, the washing was efficient.

### Primary Antibody Validation Standard

The reference standard may be used as a standard for any incubation step, for example, the step of incubation with the correct primary antibody. One of the most critical human errors in the IHC staining is incubation with the wrong antibody.

The reference standard for use in validation of correct primary antibody addition or incubation could therefore comprise a detectable entity which is capable of binding (preferably specific binding) to a binding partner. The binding partner would be added to the primary antibody solution as a "marker", which would bind or specifically attach to the detectable entity and therefore indicate that the correct primary antibody was used. It will be appreciated that the primary antibody may be sold in a form which comprises the "marker".

As a particular example, the detectable entity may comprise or consist of streptavidin, and the "marker" comprise or consist of biotin. For example, the primary antibody may be supplemented with a biotinylated irrelevant mouse antibody, which would specifically attach to a detectable entity in the reference standard, for example, a detectable entity comprising or consisting of biotin. Where slices or sections of the reference standard are taken (as is preferred), the reference dot would be stained positive by the visualization system, if the correct antibody was used. It will be appreciated that the primary antibody could itself be modified with biotin, and function as the "marker", so that an additional "marker" is not strictly necessary.

### Secondary Antibody Validation Standard

For use in validating secondary antibody addition, the reference material could contain covalently bound mouse or rabbit antibodies or fractions hereof in various density. Positive and graded staining of this reference material will validate the functionality of the secondary visualization system. The same reference material could be combined with the reference material useful for validating the antigen retrieval step.

### Calibration Standard

Besides analysis of the various reference materials for graded primary or secondary staining, the reference system could consist of or contain permanent colours and physical shapes suited for calibrating cameras, optics and software algorithms.

Therefore, in yet another aspect, the reference standard can serve as a calibrator for any equipment, for example digital image processing equipment or any automatic image analysis system. This may be achieved for example by defining a particular colour, intensity or a particular number of events. This is particularly useful in automated scanners and microscopes. By combining a dyed material with an immunological or special staining, orientation and navigation on the slide microscope may be made easier.

Such reference material could help to define sizes, colours, colour spectra, boundaries between stained areas, counterstaining level and background.

It will be appreciated from the above that it is possible to construct a reference standard which is capable of being used to validate more than one of the procedural steps. For example, we disclose reference standards which are capable of validating 1, 2, 3, 4, 5, 6, 7, 8, 9, 10, 11, 12 or more procedural steps in any method. Such reference standards may suitable comprise a plurality (or more than one) detectable entity, which may be the same or different, as described above. The arrangement of the detectable entities in the reference standard is preferably such that the resulting slice or section taken of the reference standard comprises more than one "dot" or reference area, suitably arranged in an array.

Such reference standards may give rise to slices or sections which comprise for example a "spot array" of 9 different reference materials. For example, they may comprise spots with targets for the primary antibody in different density - giving graded staining levels; spots with lightly and graded fixed targets for the secondary visualization system - general validation of the antigen retrieval step; spots with lightly and graded fixed targets for the primary antibody - validation of the specific target antigen retrieval step or "threshold" antigen retrieval necessary for staining; spot with "over fixed" targets for the secondary visualization system - general validation of the antigen retrieval step; spots with targets for the secondary visualization system (for example mouse or rabbit Abs) in different density - general validation of the secondary visualization system; spots with peroxidase and/or phosphatase activity - validation of the endogen enzyme blocking step; spots with bound biotin - validation of the endogen biotin-blocking step (if for example LSAB type of visualization systems are used; spots with non-covalently bound and somewhat high molecular weight targets for the secondary visualization system - general validation of the washing steps; spots with distinct, homogeneous and permanent shape, size and colour - Calibration of the automatic image analysis system.

In highly preferred embodiments, the reference standard comprise an array of reference "spots" on the same slide as the patient sample tissue. In such an embodiment, it is possible for validation to be done automatically by the image analysis software.

### DIAGNOSTIC STANDARDS

In highly preferred embodiments, the reference standard is or may be used as a diagnostic standard. By this we mean that the detectable entity is detected for the purpose of revealing a condition of a cell, preferably a physiological or medical condition of the cell. It will however be appreciated that in some or more cases, the mere detection of a property of the detectable entity may be insufficient in itself to provide a medical diagnosis. Therefore, it will be appreciated that in some cases it may be desirable to conduct other tests in order to establish diagnosis.

In embodiments where the reference standard is employed as a diagnostic standard, the detectable entity suitably comprises an indicator of a state of a cell such as an indicator of the health or disease state of the cell, for example, a disease marker. Thus, a detectable entity may indicate, by its presence or quantity in a relevant sample, the state of the organism (such as its state of health or state of disease) from which the sample is taken. Disease markers, in particular, cancer markers, are discussed in more detail below.

Preferably, the reference standard is such that a cross section of it includes a defined area comprising an amount of detectable entity. This is shown in the illustration in Figure 2B. The amount of detectable entity in the reference standard or cross section may be compared with that in the sample to establish whether the latter is present in identical or similar amounts, or in lesser amounts, or in greater amounts, than that in the standard. However, it will be appreciated that while the reference standard described here is most useful to detect the presence and/or quantity of a detectable entity in a sample, it may be used more simply to indicate whether a detectable entity in a sample is the same as, or different from, the detectable entity of the reference standard.

The quantity or amount of detectable entity in the reference standard or cross section is preferably a known or pre-determined quantity. The quantity may be varied by controlling the quantity which is attached to or retained in the elongate path, as described in further detail below. Where slices or sections of the reference standard are taken, variation in quantity may also be achieved by changing the thickness (and hence the amount of detectable entity captured) of the section or slice.

In highly preferred embodiments, the quantity of a detectable entity in the reference standard comprises a diagnostically relevant quantity.

In contrast to the prior art, accurate and known amounts of detectable entity may be incorporated in the reference standard as described. Furthermore, sample-to-sample variation as present in the prior art may be overcome. The result is a more precise grading system.

In some embodiments, different quantities of the detectable entity are present in the reference standard. In a preferred embodiment, the reference standard comprises two or more quantities of the same detectable entity, in separate elongate paths. Preferably, a range of quantities of increasing amounts of the detectable entity is provided, preferably in an arithmetic or more preferably a logarithmic range. Preferably, the range encompasses a diagnostically or clinically relevant amount of detectable entity, i.e., an amount of detectable entity, which if present in the sample at about or above that amount, indicates that the sample contains, or is likely to contain, cells or tissues which are diseased or prone to disease.

Comparison of the amounts present in a cell or tissue or other biological sample with the reference standard according to this embodiment will provide an indication of whether the particular sample is "positive" or "negative" for the particular detectable entity. Where the detectable entity comprises a disease antigen, such as a cancer antigen (or expresses DNA/RNA, including messenger RNA, derived from a cancer gene) its presence or quantity may be used as a diagnostic for a relevant disease. We therefore provide a method of diagnosis of a disease in an individual, comprising comparing the presence or amount (or both) of a detectable entity in a biological sample from the individual with the amount in a reference standard as described here. Preferably, the comparison is done against a clinically relevant amount of detectable entity in the reference standard (i.e., an amount in a sample at or above which the presence of a disease is indicated, suspected or diagnosed).

In a particular embodiment, the detectable entity comprises HER2 antigen, the cell or tissue comprises breast tissue, and the disease which is diagnosed, or its presence indicated, comprises breast cancer. The detectable entity may also comprise other antigens, instead of or in addition to HER2, preferably selected from the group consisting of: ER, PR, p16, Ki-67 or EGFR (see also section "Detectable entity"). Mixtures of any two or more of these may be used. The detectable entity may comprise a nucleic acid encoding any of the above, or any detectable entity as specified in the section "Detectable Entity"; in particular, such reference standards comprising nucleic acids are useful for standardising *in situ* hybridisation. In particular, the reference standard may comprise a nucleic acid cancer marker, such as DNA/RNA cancer markers (for example an cancer mRNA marker).

Preferably, the reference standard comprises, in addition to a clinically or diagnostically relevant amount of detectable entity, a negative control, i.e. a defined area or volume or path comprising no detectable entity, or detectable entity at an undetectable quantity.

The diagnosed disease may be optionally treated, by administration of an appropriate therapeutic agent. Such an agent or drug may be one which is known to be effective for treating such a disease. In particular, the therapeutic agent may comprise an antibody, preferably an antibody against the detectable entity. Such an antibody may comprise the same antibody which was used for staining and detecting the detectable entity in the reference standard and/or the biological sample, or it may be a variant of it, such as a humanised antibody, or a single chain antibody such as an ScFv.

In particular, the detectable entity may comprise HER2, the binding agent may comprise any anti-HER2 antibody and the therapeutic agent may comprise a humanised anti-HER2 antibody such as Herceptin (Trastuzumab, Genentech). The detectable entity may comprise a HER2 nucleic acid, such as a HER2 RNA, HER2 mRNA or a HER2 DNA. The detectable entity may comprise any molecule such as a nucleic acid capable of binding to the HER2 nucleic acid, and may in particular comprise a sequence complementary to at least a portion of the HER2 nucleic acid.

HER2 and Herceptin are described in a number of publications, including Pegram M, Hsu S, Lewis G, et al. Inhibitory effects of combinations of HER-2/*neu* antibody and chemotherapeutic agents used for treatment of human breast cancers. *Oncogene.* 1999;18:2241-2251; Argiris A, DiGiovanna M. Synergistic interactions between tamoxifen and Herceptinú. *Proc Am Assoc Cancer Res*.2000;41:718. Abstract 4565; Pietras RJ, Fendly BM, Chazin VR, et al. Antibody to HER-2/*neu* receptor blocks DNA repair after cisplatin in human breast and ovarian cancer cells. *Oncogene.* 1994;9:1829-1838; Baselga J, Norton L, Albanell J, et al. Recombinant humanized anti-HER2 antibody (Herceptinú) enhances the antitumor activity of paclitaxel and doxorubicin against HER2/*neu* overexpressing human breast cancer xenografts. *Cancer Res.* 1998;58:2825-2831; Sliwkowski MX, Lofgren JA, Lewis GD, et al. Nonclinical studies addressing the mechanism of action of trastuzumab (Herceptin). *Semin Oncol.* 1999;26(suppl 12):60-70; Lewis GD, Figari I, Fendly B, et al. Differential responses of human tumor cell lines to anti-p185^{HER2} monoclonal antibodies. *Cancer Immunol Immunother.* 1993;37:255-263 and Pegram MD, Baly D, Wirth C, et al. Antibody dependent cell-mediated cytotoxicity in breast cancer patients in Phase III clinical trials of a humanized anti-HER2 antibody. *Proc Am Assoc Cancer Res.* 1997;38:602. Abstract 4044.

In other embodiments, more than one detectable entity is present in the reference standard. In particular, we envisage a plurality of different types of detectable entity in a single reference standard. Where more than one detectable entity is present in the reference standard, each of these may be in the same one elongate path, or more than one elongate path may be present. In the latter case, the or each elongate path may comprise one or more different detectable entities. The detectable entities may be present in the same or different quantities, in the or each elongate path.

Therefroe, two or more different detectable entities may be supported in the support medium, preferably embedded in the embedding medium, at least one of which is in a generally elongate path. Preferably, all the different detectable entities are in generally elongate paths. The reference standard may comprise a single or more than one quantity of a first detectable entity, and a single or more than one quantity of a second detectable entity. Multiple detectable entities, at the same or different quantities, may be present in the reference standard. Where more than one detectable entity is present, the reference medium preferably comprises at least one detectable entity at a quantity or amount which is diagnostically or clinically significant.

The reference standard may therefore comprise a quantity of first detectable entity therein in an elongate path. The reference standard may further comprise a quantity of a second detectable entity in the elongate path, or in a second elongate path. Furthermore, the reference standard may comprise a second different quantity of the or each detectable entity in the elongate path, or in a second or further elongate path. The multiple same or different detectable entities and / or quantities of such may be mixed or separated in space and / or time.

For example, the detectable entities may comprise HER2, together with another cancer antigen, such as *ras,* or in particular a breast cancer antigen such as a BRCA1 or BRCA2 protein. Alternatively or in addition, the reference standard comprises a quantity of tumour suppressor protein such as retinoblastoma (Rb) protein. The detectable entities may also comprise nucleic acids, such as HER2 nucleic acids, and other cancer antigen nucleic acids. The detectable entities may comprise one or more polypeptide detectable entities, together with one or more nucleic acid detectable entities.

Such embodiments of reference standards comprising a plurality of detectable entities are useful in applications where testing of a sample is conducted using more than one binding agent. Thus, we envisage the use of such reference standards in testing using "banks" or "panels" of antibodies/probes, for example. Such testing may be used to detect the presence or absence, or relative or absolute levels of different detectable entities in a sample, each of which may be diagnostically relevant. Such relative or comparative information is typically more useful than a single presence/absence test. Multiple testing in this way may be used to generate a "profile" of the patient or individual in question. Profiles generated from individuals suffering from (or suspected of suffering from), a disease or condition may be compared against matching profiles of "normal" or non-affected individuals. The profiles, or information generated by comparing profiles, may be used to diagnose (or aid in the diagnosis of) a disease or condition in that individual for the purpose of selecting the best possible treatment ("individualised therapy").

In embodiments where more than one elongate path is present, it may be advantageous to arrange the elongate paths in a bundle, or more than one bundle. The elongate paths may be arranged in such a way that it makes it easy to find the different reference dots and clusters. For example, an asymmetrical pattern of fibre bundles can help the user to orientate the slide correctly.

Embodiments in which multiple elongate paths comprising a single detectable entity at different amounts, or more than one different detectable entity, are shown in Figure2C.

Needless to say, where more than one detectable entity is present in the reference standard, the two or more detectable entities may be present in different elongate paths. Alternatively, more than one detectable entity may be present in a single path. For example, where the paths are defined by use of fibres, as described below, more than one detectable entity may be conjugated or attached to a fibre. Multiple fibres, each comprising a single, two or more detectable entities, may be used.

The or each detectable entity is present in an elongate path in the reference standard; this may be achieved by various means as described in detail further below. The or each elongate path may extend across at least a part of the reference standard, preferably from one end to another. For example, where the reference standard comprises an upper end and a lower end, the or each detectable entity may be disposed on an elongate path which extends from the upper end to the lower end (or vice versa).

### KITS AND DIAGNOSTIC KITS

The reference standard, or slices or sections of it may be packaged in a diagnostic kit. Thus, the reference standard when used as a diagnostic standard may conveniently be packaged as a kit, comprising a reference standard or a planar section thereof, together with one or more components, and optionally together with instructions for use. The instructions may in particular include a description of any of the methods for detecting the presence of, or establishing the quantity or concentration of, a detectable entity, as set out in this document.

The kit may comprise two or more slices or sections, comprising the same or different detectable entities, at the same or preferably different quantities, as described elsewhere in this document.

The kit may comprise a binding agent, such as an antibody, which is capable of specific binding to the detectable entity. The kit may comprise a microtome block with the reference standard, slices or sections mounted thereon. The kit may further comprise other reagents, such as detection, washing, or processing reagents, as well as instructions for use.

The kit may further comprise instructions for use, or other indicia, for example scoring aids such as charts or photographs of diseased and/or normal tissue. The indicia may in general indicate the level of signal which should be expected from the detectable entity in the biological sample, in order for it to be a relevant, e.g., diagnostically relevant level. This may comprise a picture, photomicrograph, or data relating to optical density, etc, depending on the method of detection. Indicia showing negative results may also be included. The indicia may also indicate the amount of variance between the reference signal and the detected signal from the biological sample which could be considered as acceptable for a positive or negative result. Where multiple reference standards or sections thereof are included in the kit, there may be more than one indicia included in the kit.

The kit may also comprise a therapeutic agent which is capable of treating or at least alleviating at least one of the symptoms of a disease. We also provide a combination of a reference standard as described here, or a section or slice thereof, together with a therapeutic agent.

In particular embodiments, the disease is one whose presence in an individual is indicated or suspected where a biological sample comprises a diagnostically relevant amount of detectable entity. In particular, the therapeutic agent may comprise an antibody against the detectable entity, or a nucleic acid capable of biding to the detectable entity, any other therapeutic agent which is known to be effective in treating or preventing the disease. For example, a kit or combination for detecting breast cancer may comprise a reference standard in which the detectable entity comprises HER2 antigen or HER2 nucleic acid. The kit may further comprise anti-HER2 antibody for detecting of that antigen, and may also further comprise any breast cancer drug, for example, Herceptin (a humanised monoclonal antibody which targets HER2 protein in metastatic breast cancer patients). Other embodiments of the kit include any antigen selected from the group consisting of: ER, PR, p16, Ki-67 or EGFR (se also section "Detectable entity").

In preferred embodiments, the detectable entity is molecular in nature (see description below), and the reference standard is therefore substantially free of cellular material. However, in some embodiments, cellular components, for example parts of a cell (for example, any subcellular compartment or organelle such as the nucleus, mitochondria, chloroplast, vacuole, etc) or whole cells themselves may be incorporated in the reference standard.

### DETECTABLE ENTITY

The detectable entity is one whose presence and preferably quantity is revealable, that is, its presence is demonstrable or its amount is measurable, either directly or indirectly. In general, the detectable entity can be anything which is capable of producing a detectable signal, or a revealable signal, whether by itself, naturally or when stimulated to do so.

The detectable entity may produce a signal alone or in conjunction with one or more other entities, for example, when contacted with revealing agents such as antibodies and/or secondary antibodies. The detectable entity may itself be labelled, as discussed elsewhere, using any of a variety of labels, for example, radioactive and non-radioactive labels, as known in the art. Further discussion of this aspect is contained in the section "Detection and Visualisation" below.

The reference standard preferably comprises the detectable entity in a relatively pure state, that is, substantially isolated from other molecules or compounds. The detectable entity is preferably free or substantially free from cellular components with which it may be normally associated. For example, the detectable entity may be in isolated form.

The detectable entity is preferably non-cellular in nature, but not necessarily non-cellular in origin. By this we mean that the detectable entity may originate from the cell, but is preferably one which has undergone some processing, for example purification or concentration, to isolate the detectable entity from at least one other cellular component. In particular, the detectable entity does not for example comprise raw cellular material or whole cells. Preferably, the detectable entity does not comprise substantial amounts of cellular structures, such as cell walls, cell membranes, organelles, cytoskeleton, etc. Preferably, in such embodiments, the detectable entity comprises "molecular" components in a relatively pure state, compared to their environment within the cell.

In other words, the detectable entity preferably comprises non-cellular material and/or non-cellular components. Ir preferably does not comprise substantial amounts of cellular material, for example, it is "cell-free". For this purpose, chemical synthesis or recombinant production of detectable entity is preferred.

The nature of the binding agent will depend on the detectable entity, but may comprise a non-specific or a preferably a specific binding agent. Thus, non-specific binding agents such as dyes, for example, dyes typically used to colour fabrics, may be employed as binding agents. However, specific binding agents are preferred.

The detectable entity may comprise a "small molecule", such as simple inorganic or organic compounds. The detectable entity may in particular comprise a hapten, such as di-nitrophenol (DNP). The detectable entity may include dyes, such as fluorescent dyes.

In highly preferred embodiments, the detectable entity comprises a nucleic acid, such as DNA, RNA, PNA or LNA, or a polypeptide, such as a protein or antigen, or other epitope-comprising polypeptide. Reference standards comprising protein, etc detectable entities are preferred for innumohistochemistry (IHC), while reference standards comprising nucleic acids, etc are preferred for *in situ* hybridisation (ISH).

Where the detectable entity comprises a nucleic acid, the binding agent may in particular comprise a nucleic acid probe. For this purpose, it may comprise a nucleic acid, such as DNA or RNA, or a derivative thereof, such as Peptide nucleic acid, PNA or Locked nucleic acid, LNA. The nucleic acid probe is preferably capable of specifically hybridising to a sequence in the detectable entity, preferably under stringent conditions. In particular, the nucleic acid probe may comprise at least a sequence complementary to a sequence in the detectable entity. Preferably, the nucleic acid binding agent or probe comprises a single stranded portion, or is denatured to expose binding sites.

Where the detectable entity comprises a protein such as an antigen, the binding agent preferably comprises any molecule capable of specifically binding to the protein. In particular, the binding agent may comprise an antibody (whether monoclonal or polyclonal) capable of specifically binding to the antigen.

In the above examples, nucleic acids are typically detected by binding agents comprising nucleic acids, while proteins are typically detected by antibodies. It will be appreciated, however, that detectable entity - binding agent pairs may be chosen based on protein nucleic acid interactions. Thus, it is known for example that nucleic acid binding proteins such as zinc finger proteins, HLH proteins, etc can bind to specific nucleic acids sequences. Thus, a nucleic acid binding protein may be used as a binding agent to detect a detectable entity comprising a cognate nucleic acid, and a nucleic acid may be used as a binding agent to detect a detectable entity comprising a cognate nucleic acid binding protein.

Preferably the detectable entity is selected from the group consisting of a protein, a polypeptide, a peptide, a phosphylated peptide, a phosphorylated peptide, a glucated peptide, a glycopeptide, a nucleic acid, a virus, a virus-like particle, a nucleotide, a ribonucleotide, a synthetic analogue of a nucleotide, a synthetic analogue of a ribonucleotide, a modified nucleotide, a modified ribonucleotide, an amino acid, an amino acid analogue, a modified amino acid, a modified amino acid analogue, asteroid, a proteoglycan, a lipid and a carbohydrate or a combination thereof (for example, chromosomal material comprising both protein and DNA components or a pair or set of effectors, wherein one or more convert another to active form, for example catalytically).

In preferred embodiments, the detectable entity comprises a polypeptide, or a nucleic acid. In highly preferred embodiments, the detectable entity suitably comprises an indicator of a state of a cell such as an indicator of the health or disease state of the cell.

For example, the presence or amount of the detectable entity may serve to indicate that the cell is in a healthy, normal or functional state. Preferably, however, the detectable entity is such that it is an indicator of an abnormal state of the cell, for example, a diseased state. In other words, if the detectable entity is present in a cell or tissue, it may be inferred that the cell or tissue or organ or individual comprising this is diseased. The detectable entity may be diagnostic of a single disease, or a number of diseases, or a syndrome such as AIDS, or a medical condition. Preferably, the disease, syndrome or condition is a treatable one.

Preferably, the presence, quantity or concentration of the detectable entity in a cell or tissue is detected to provide an indication of a condition of the cell or tissue, preferably a pathological condition of the cell or tissue. Therefore, in embodiments where the reference standard is employed as a diagnostic standard, the detectable entity may comprise any diagnostically relevant entity.

Thus, in this preferred embodiment, the detectable entity is essentially a marker of a pathological condition or a disease marker, the presence or quantity of which in a cell indicates that the cell is or is likely to be diseased. The presence of the detectable entity may be diagnostic, or it may serve as merely an indicator, which together with other indicators, for example a panel of indicators, points to the likelihood of disease. The quantity of the detectable entity in the cell or tissue may be significant, i.e., whether or not it is above a threshold level which is indicative of disease.

Preferably, the disease comprises cancer. The detectable entity is therefore preferably a cancer marker or cancer protein or cancer nucleic acid. A number of cancer and cancer related proteins are known in the art, for example, *ras,* BRCA1, HER2, *ATM, RhoC,* telomerase, etc. Carcinoembryonic antigen (CEA) is associated with digestive tract cancers (for example of the colon) as well as other malignant and non-malignant disorders. Examples of other cancer markers are set out below, and it will be appreciated that nucleic acids encoding these may also be used as detectable entities:
Prostate specific antigen (PSA) levels are elevated in prostate cancers and are sometimes enlarged prostate conditions (for example BPH) or prostatis.

CA 19.9 is mainly associated with gastrointestinal cancers. Sometimes increased values have also been observed in those patients with metastasis and in non-malignant conditions for example hepatitis, cirrhosis, pancreatitis.

HER2 is associated with breast cancers. Increased values of the HER2 protein, overexpression, are often associated with rapid growth of the tumour cells that may lead to metastatic conditions. Metastatic patents who overexpress HER2 protein may benefit from HERCEPTIN therapy (therapy with anti-HER2 antibodies).

Elevated CA 125 values are often associated with cancer of the ovaries. However non-malignant conditions such as endometriosis, first semester pregnancy, ovarian cysts or pelvic inflammatory disease can also cause elevated CA 125 levels. The link between family history and incidence of cancer has been well reported.

CA 15.3 values are often elevated in patients with breast cancers. When there is a history of cancer among family members, patients may be advised to also do a breast mammogram. Besides breast cancer, other non-malignant conditions (for example cirrhosis, benign diseases of ovaries & breast) have also been known to cause elevated CA 15.3 levels.

Alpha fetoprotein (AFP) levels are often elevated in liver cancers (hepatocellular) and testicular cancers (non-seminomatous). Raised levels are also present during pregnancy or some gastrointestinal cancers. AFP is also used in combination with other tests as a screening test for open neural tube defects.

Nasopharygeal carcinoma (NPC) is a non-lymphatous, non-glandular, squamous cell carcinoma arising from the epithelial cells of the nasopharynx..It is the most common form of nasophryngeal cancer, with a higher incidence in adults. Some clinical symptoms include nose or ear problems, blood in nasal mucous, neck lumps, enlarged lymph nodes (usually cervical) and sensation of nasal obstruction. Epstein Bar virus (EBV) has been shown to have a direct relationship with NPC where it can be detected in NPC tumours and patients with NPC tend to have higher titres of EBV specific antibodies than the general population. Early detection through screening usually results in favourable prognosis.

Tumour suppressor proteins, such as p53 and Rb may also be used as detectable entities.

The detectable entities may comprise immunoglobulin Kappa and Lambda light chains. The detectable entity or entities may comprise one or more prognostic markers like estrogen receptor (ER) alfa and beta and progesteron receptor (PR), p53 protein, Proliferation related proteins like Ki-67 and Proliferating cell nuclear antigen (PCNA). The detectable entity or entities may comprise one or more cell adhesion molecules like Cadherin E, and tumor suppressor proteins like p 16, p21, p27 and Rb. The detectable entity or entities may comprise one or more hematologic factors like CD3, CD15, CD20, CD30, CD34, CD45, CD45RO, CD99, Kappa and Lambda light chains and factor VIII. Nucleic acids encoding any of these may also be used as detectable entities.

The detectable entity or entities may comprise one or more epithelial differentiation markers like Prostate specific antigen (PSA), Prostate specific alkaline phosphatase (PSAP), cytokeratin, epithelial membrane antigen, carcinoembryonic antigen (CEA), polymorphic epithalial mucin, mesenchymal differentiation markers, Desmin, Actin, Vimentin, Collagen type IV. The detectable entity or entities may comprise one or more melanocytic markers like S-100, HMB45. The detectable entity or entities may comprise one or more of markers as CD117, CD133, CD45, CD4, CD8, CD19, CD20, CD56, CD13, CD33, CD235a, CD15, BerEP4, Neuron specific enolase, Glial fibrillary acidic protein, Chromogranin, Synaptophysin, c-Kit, Epidermal Growth Factor Receptors (EGFR), HER2/neu, HER3, and HER4 and their ligand like EGF and TGF-alfa and other receptor protein-tyrosine kinases like the Insulin Receptor (IR), the Platelet-derived growth factor receptor (PDGFR), and the Vascular endothelial growth factor receptors (VEGFR-1, VEGFR-2, and VEGFR-3), Apoptosis related proteins like M30, Bcl-2, p53, caspases and Fas.

It will be appreciated that it is not strictly necessary to use the proteins of the above detectable entities in the reference standards as described here, and that it is possible to detect the antigens by use of nucleic acids encoding the proteins. Therefore, it should be appreciated that the reference standard may comprise a detectable entity which is a nucleic acid capable of encoding any of the polypeptide detectable entities as described above. Needless to say, the binding agent or revealing agent in this case would preferably comprise a nucleic acid, preferably a nucleic acid capable of specific binding to at least a portion of the nucleic acid detectable entity, preferably a complementary nucleic acid.

Where the detectable entity comprises a polypeptide, it may be unmodified, or comprise one or more post-translational modifications, preferably phosphorylation. For example, the detectable entity may comprise phosphorylated HER2 or phosphorylated EGFR. The detectable entity may also suitably comprise an antigen, preferably a diagnostically relevant antigen, which is detectable by binding to a relevant antibody. Any suitable antigen may be employed, and a skilled person will know which antigens are suitable for which diagnostic purposes.

As used herein, the term "detectable entity" includes but is not limited to an atom or molecule, wherein a molecule may be inorganic or organic, a hapten, a biological effector molecule and/or a nucleic acid encoding an agent such as a biological effector molecule, a protein, a polypeptide, a peptide, a phosphylated peptide, a phosphorylated peptide, a glucated peptide, a glycopeptide, a nucleic acid, a virus, a virus-like particle, a nucleotide, a ribonucleotide, a nucleic acid, a DNA, an RNA, a peptide nucleic acid (PNA), locked nucleic acid (LNA), a synthetic analogue of a nucleotide, a synthetic analogue of a ribonucleotide, a modified nucleotide, a modified ribonucleotide, an amino acid, an amino acid analogue, a modified amino acid, a modified amino acid analogue, a steroid, a proteoglycan, a lipid and a carbohydrate. The detectable entity may be in solution or in suspension (for example, in crystalline, colloidal or other particulate form). The detectable entity may be in the form of a monomer, dimer, oligomer, etc, or otherwise in a complex.

It will be appreciated that it is not necessary for a single detectable entity to be used, and that it is possible to use two or more detectable entities in the reference standard. Accordingly, the term "detectable entity" also includes mixtures, fusions, combinations and conjugates, of atoms, molecules etc as disclosed herein. For example, a detectable entity may include but is not limited to: a nucleic acid combined with a polypeptide; two or more polypeptides conjugated to each other; a protein conjugated to a biologically active molecule (which may be a small molecule such as a prodrug); or a combination of any of these with a biologically active molecule.

In preferred embodiments, the detectable entity comprises a "biological effector molecule" or "biologically active molecule". These terms refer to an entity that has activity in a biological system, including, but not limited to, a protein, polypeptide or peptide including, but not limited to, a structural protein, an enzyme, a cytokine (such as an interferon and/or an interleukin) an antibiotic, a polyclonal or monoclonal antibody, or an effective part thereof, such as a F(ab)2, F(ab') or Fv fragment, which antibody or part thereof may be natural, synthetic or humanised, a peptide hormone, a receptor, a signalling molecule or other protein; a nucleic acid, as defined below, including, but not limited to, an oligonucleotide or modified oligonucleotide, an antisense oligonucleotide or modified antisense oligonucleotide, cDNA, genomic DNA, an artificial or natural chromosome (for example a yeast artificial chromosome) or a part thereof, RNA, including mRNA, tRNA, rRNA or a ribozyme, or a peptide nucleic acid (PNA), locked nucleic acid (LNA), a virus or virus-like particles; a nucleotide or ribonucleotide or synthetic analogue thereof, which may be modified or unmodified; an amino acid or analogue thereof, which may be modified or unmodified; a non-peptide (for example, steroid) hormone; a proteoglycan; a lipid; or a carbohydrate. Small molecules, including inorganic and organic chemicals, are also of use as detectable entities. In a particularly preferred embodiment, the biologically active molecule is a pharmaceutically active detectable entity, for example, an isotope.

The detectable entity may emit a detectable signal, such as light or other electromagnetic radiation. The detectable entity may be a radio-isotope as known in the art, for example ³²P or ³⁵S or ⁹⁹Tc, or a molecule such as a nucleic acid, polypeptide, or other molecule as explained below conjugated with such a radio-isotope. The detectable entity may be opaque to radiation, such as X-ray radiation. The detectable entity may also comprise a targeting means by which it is directed to a particular cell, tissue, organ or other compartment within the body of an animal. For example, the detectable entity may comprise a radiolabelled antibody specific for defined molecules, tissues or cells in an organism.

The detectable entity may comprise a dye, including azo dyes, organic pigments, cibracron blue, etc.

It will be appreciated that the detectable entity may comprise one or more entities as set out above, and in particular may comprise two or more or a plurality of any of the above entities, or combinations of one or more of the above entities.

### ELONGATE PATH

The reference standard described here comprises the detectable entity in a generally elongate path in the support medium. The detectable entity therefore has a generally elongate shape in the reference standard as described here.

It will be appreciated from the above that the "elongate path" or the "elongate shape" does not refer to the specific shape of the molecules or atoms making up the detectable entity, as these can be of any shape. Rather, the term should be taken to refer to the general disposition or localisation of the detectable entity within the support medium. For example, the mass or bulk of the detectable entity, for example, in a contiguous state, preferably has an "elongate shape" as it is disposed in the support medium.

By "elongate", we mean a shape which is generally longer than wide; therefore, such elongate shapes have at least two linear dimensions that differ by a significant amount. Preferably, the longest dimension of the detectable entity is significantly greater than the shortest dimension.

Preferably, the ratio of the longest dimension to the shortest dimension is 5:1 or more, more preferably at least 10:1, most preferably at least 20:1, 50:1, or 100: 1 or more. The ratio may in particular be 5:1 or more, 6:1 or more, 7:1 or more, 8:1 or more, 9:1 or more, 10:1 or more, 15:1 or more, 20:1 or more, 25:1 or more, 30:1 or more, 40:1 or more, 45:1 or more, 50:1 or more, 55:1 or more, 60:1 or more, 65:1 or more, 70:1 or more, 75:1 or more, 80:1 or more, 85:1 or more, 90:1 or more, 95:1 or more, or 100:1 or more.

Therefore, where applicable, preferably the length of the path is at least 10x its width or diameter, preferably more than 10x its width or diameter, preferably more than 20x, most preferably, more than 100x or more.

However, embodiments in which the path is more compact or uniform or regular in shape, while still being elongate, for example, a balloon shape, a cigar shape, a sausage shape, a disc shape, a teardrop shape, a ball shape or an elliptical shape, are also envisaged. However, regular shapes in which the linear dimensions are approximately the same, or are comparable, or in which the ratio of the longest dimension to the shortest dimension is less than 5:1 are not included in the reference standards described here.

The path may be non-linear, and may have a curved orientation comprising one or more curved portions. It may be curly or wavy in shape, for example as illustrated in Figure 4A. However, in preferred embodiments, the elongate path is straight or substantially straight. The detectable entity in such preferred embodiments therefore adopts a linear configuration.

In particular, the path may have a rod-like shape or orientation. For example, the path may comprise a linear rod. Where more than one elongate path is comprised in the detectable entity, the or each detectable entity adopts a rod-like path in the support or embedding medium. The rods are preferably parallel in orientation, so that the parallel rods form a bundle in the support or embedding medium. The or each detectable entity is preferably substantially longitudinal in orientation. However, the rods may be parallel along a short edge of the the support or embedding medium.

In some embodiments, the path adopted by the detectable entity is non-uniform in cross section. The path may therefore have a lens shape or a sausage shape. However, in preferred embodiments, the path has a uniform cross section across at least a substantial portion of the path, preferably substantially the length of the path. The cross sectional area of the path is therefore substantially the same across all portions of the detectable entity in such embodiments. However, embodiments where the detectable entity is not uniformly distributed along the path may be envisaged. For example, the detectable entity may for example adopt a "beads on a string" configuration, as shown in Figure 4A.

The cross sectional profile of the path, i.e., the defined area comprising the detectable entity in cross section, may have a variety of configurations. Preferably, the defined area is circular or elliptical or ellipsoid in shape. However, the defined area may have a pill shape, a regular shape or an irregular shape. Different cross sectional profiles may be established by use of appropriately profiled paths.

In highly preferred embodiments, the cross sectional profile is similar to the size or shape of a cell, or both, for example, a prokaryotic cell or a eukaryotic cell, preferably an animal cell and most preferably a human cell. Thus, a cross sectional profile in a slice of the reference standard will present a shape which mimics a real cell in the sample. This enables a direct comparison to be easily made between a cell in a sample, and the cross sectional profile present in the slice. A user can for example easily compare (by eye) the level of staining of a cell and the level of staining of the cross sectional profile, to make a judgement on whether that cell is "positive" or not.

Preferably, the cross sectional profile of the path, or the shortest dimension of the path, has a diameter of (or a greatest dimension of) between about 0.5 µm to 100 µm; more preferably between 1 µm and 100 µm, even more preferably between 10 µm to 20 µm and most preferably between 2 µm to 20 µm. Particularly preferred embodiments are those with diameters or greatest dimensions of, or substantially of, 1µm, 2µm, 3µm, 4µm, 5µm, 6µm, 7µm, 8µm, 9µm, 10µm, 11µm, 12µm, 13µm, 14µm, 15µm, 16µm, 17µm, 18µm, 19µm or 20µm.

It will be appreciated that these sizes are not limiting, and the user will know to vary the size depending on the application. Furthermore, where the reference standard comprises more than one detectable entity, or more than one path, or both, it will be appreciated that each of these may independently have the features and properties set out above. Furthermore, where the reference standard comprises two or more paths, these may be stained at the same density, or at different densities.

Where the reference standard comprises two or more paths, these may be stained at the same density, or at different densities.

In preferred embodiments, the paths comprising the detectable entity are established in the support medium by means of a fibre comprising the detectable entity, or by establishing a channel in the embedding medium comprising the detectable entity. Furthermore, the use of "hollow fibres, "block copolymers" and macrostructures (in particular self-assemblying macrostructures) is envisaged. Each of these methods is described in further detail in the sections below.

### FIBRES

In a preferred embodiment, the detectable entity is attached to an elongate fibre, and the fibre comprising the entity is supported in the support medium. Where an embedding medium is employed in preferred embodiments, the fibre comprising the detectable entity is embedded in the embedding medium.

The term "fibre" as used in this document, should be taken to include any natural or man-made fibre, or strand or string. Fibres are typically thin and have an elongate shape. Fibres include natural fibres such as animal hair, keratin, etc. The term furthermore includes any polymer of for example actin or tubulin, which is assembled to form a long thin strand. Fibres made from extrusion or pulling are included, as are elongate micellar structures. Indeed, any macrostructure formed for example by self assembly of components is included. However, synthethic and natural fibres used in fabrics, or in the clothing industry, are particularly preferred.

The detectable entity may be attached, coupled, fused, mixed, combined, or otherwise joined to a fibre. The attachment, etc between the detectable entity and the fibre may be permanent or transient, and may involve covalent or non-covalent interactions (including hydrogen bonding, ionic interactions, hydrophobic forces, Van der Waals interactions, etc).

While the term "attached" implies a permanent or semi-permanent association between the fibre and the detectable entity, it should not be taken to be limited to these possibilities. Instead, attachment should be taken to refer to any association between the detectable entity and the fibre, however, transient or loose, so long as that association is sufficient for the detectable entity to adopt a path which is substantially defined by the positioning of fibre during and subsequent to supporting or embedding. All that is important is that the fibre holds the detectable entity in place during the step or steps of supporting or preferably embedding in the medium.

The fibre may merely retain the detectable entity, or otherwise prevent it from diffusing or being washed away. The retention may be transient, or it may persist through a substantial portion of the supporting or embedding step, preferably throughout the course of that step. The use of a mordant, as known in the art for coupling dyes and other molecules to fibres, may be advantageous.

In preferred embodiments, the detectable entity is covalently attached to the fibre. In such preferred embodiments, the detectable entity is chemically coupled or cross-linked to one or more molecules making up the fibre. Preferred methods of chemical coupling are described in further detail below, in the section headed "Coupling". Needless to say, the amount of detectable entity coupled to the fibre in this embodiment will control how much detectable entity is presented in cross-section, and hence the staining level achieved.

Furthermore, in certain embodiments, it may be desirable to include spacing means between the detectable entity and the fibre, or components of the fibre. Such spacing means may suitably comprise linkers or spacers as known in the art. The purpose of the spacing means is to space the detectable entity from the fibre (or other component of the path), to avoid for example steric hindrance and to promote detection of the detectable entity. Accordingly, depending on the application, the use of shorter or longer spacers may be preferred.

The spacing means may comprise linkers or spacers which are polymers of differing lengths (the length of which may be controlled by controlling the degree of polymerisation). Numerous spacers and linkers are known in the art, and the skilled person will know how to choose and use these, depending on the application. The skilled person will also know what spacer length to use.

The spacers may be made for example ofpolyethylenglycol, PEG derivatives or polyalkanes or homo poly amino acids. Dextrans and dendrimers, as known in the art, may also be used. In particular, the linkers or spacers may comprise nucleotide polymers (nucleic acids, polynucleotides, etc) or amino acid polymers (proteins, peptides, polypeptides, etc).

For example, where the detectable entity comprises a peptide or polypeptide, this may be synthesised or expressed (e.g., as a fusion protein) together with additional amino acid residues (these making up the spacer or linker). The linker or spacer need not be contigous with the detectable entity, however, but may itself be coupled to it, whether covalently or non-covalently (as described above) using any suitable means, for example by use of the cross-linkers described below.

It will be appreciated that where peptides are used as spacers, the configuration and length of the peptide spacers is limited only by the peptide synthesis methods themselves. Thus, for example, the flexibility of peptide synthesis methods allows the synthesis of long, short and branched peptides, including peptides with natural and un-natural occurring amino acids. In particular, the use of branched spacers, for example, branched peptide structures, is desirable, as it enables more than one molecule of a detectable entity to be attached to a spacer or linker. Furthermore, spacers with branched or tree-structures enable the coupling or attachment of more than one type of detectable entity. For example, a first detectable entity may be coupled to one arm of the spacer, a second detectable entity coupled to a second arm, etc. Furthermore, different quantities of the same or different detectable entity may be coupled to each arm.

The fibre may comprise any suitable material. Preferably, the fibre is a spun fibre. The fibre may be natural in origin, or synthetic. Natural and synthetic fibres and sources for obtaining them are well known in the art. Examples of natural fibres suitable for use include cotton, linen, silk, cellulose, keratin, etc. Examples of synthetic fibres suitable for use include Rayon, Nylon, polyester, polycarbamate, etc. Nylon comprises a polyamide fibre, and any other suitable polyamide fibre may also be used. Rayon is comprises mainly of cellulose, and other cellulose fibres as known in the art may also be suitably employed for the purposes described here.

Blends comprising amounts of two or more of these fibres may also be employed, and blended fibres and blending techniques will be known in the art. For example, textile fibres. Suitable blends of polyamide and cellulose, polyamide/polyesters or polyester/silk may be employed. Preferably, the fibre will have at least some mechanical resistance, at least some resistance to chemical attack, or to heat treatment, or any combination of these.

Synthetic fibres which may be used may include (but are not limited to) polyester (for example Dacron and Terylene), polyacrylonitrile (for example Orlon), acrylic, polyamid (for example Nylon); Polyolefin, polypropylene, poly(ethylene terephthalate), poly(1,4-dimethylenecyclohexane terephthalate, poly(tetramethylene terephthalate), polyurethane, poly(vinyl chloride), poly(vinylidene chloride), poly(vinyl alcohol), postchlorinated Poly(vinyl chloride) (CPVC) and polytetrafluoroethylene.

Copolymers of these fibres, including for example copolymers consisting of polyester and polyethylene (for example Karat), may also be used. Of especial interest are the thinnest of industrial textile fibres - so called micro fibres of for example polyester, nylon or acrylic polymers. Other fibres which may be used include those of semi natural origin like rayon, acetate rayon, cellulose, milk protein (for example Aralac) or wood pulp (for example Lyocell or Tencel). The synthetic fibres may be manufactured by wet, dry or melt spinning.

Also of use are natural fibres including and not restricted to the group of wool fibres from animal coats of sheep, goats, rabbits, alpacas, lama etc., cotton, silk, for example from the cocoon of the silkworm, linen from flax, hemp, ramie, sisal and jute.

In preferred embodiments, a fibre is embedded in a paraffin-embedding medium. A flowchart showing how a reference standard according to this embodiment may be made is shown in Figures 7 and 8. The agarose-embedding step, described in further detail below, is optional.

More than one fibre may be present in the reference standard. For example, two or more fibres, each comprising a different detectable entity may be employed. Furthermore, different quantities of the same detectable entity may be provided on different fibres. Finally, more than one detectable entity may be conjugated or attached to a fibre. Multiple fibres, each comprising a single, two or more detectable entities, at identical or different quantities, may be used.

The or each fibre comprising the or each detectable entity may extend through substantially the reference standard, or at least through a portion of it. For example, where the reference standard is in the form of a rectangular box, the or each fibre may extend from one end to another (i.e., across substantially the entire length of the rectangular box).

Preferably, the channel has a diameter of between about 0.5 µm to 100 µm, more preferably between 1 µm and 100 µm, even more preferably between 10 µm to 20 µm and most preferably between 2 µm to 20 µm.

The detectable entity or each fibre may be dyed using any one of various dyes known in the art, for easier identification and/or location. Where two or more fibres are comprised in the reference standard, each of the fibres if dyed is preferably dyed using a different colour, to allow easier distinction between the fibres.

As described above, the or each fibre may be embedded in the same embedding medium as the sample (for example, a cell or tissue to be assayed) itself, at the same time, before, or after, the sample is embedded in that medium. In preferred embodiments, the or each fibre is co-embedded in an embedding medium comprising paraffin. Preferably, such fibre embedding takes place as part of the paraffin embedding process of the sample in FFPE.

### SWELLING

In some embodiments, the fibres may be at least partially swollen before or during association with the detectable entity. In preferred embodiments, the fibre may be allowed to partially or completely swell before or during chemical coupling to the detectable entity.

Although the term "swelling" may in some contexts be taken as reference to the uptake of solvents in a insoluble polymer gel, our use of the term is meant to be more general, and specifically including mechanical, physical or chemical treatment to increase the volume or surface area or accessibility to sites, preferably internal sites, of the elongate path such as a fibre.

Swelling of the fibre enables the detectable entity to access interior of the fibre as well as surface portions. The degree of penetration of the detectable entity into core portions of the fibre, and hence coupling thereto, may then be modulated or controlled. The resulting different modulated distribution patterns of the detectable entity in the fibre may be used for different detectable entities, depending on their distribution in the cell.

The fibre may be swollen by various means. For example, the fibre may treated mechanically, by being unrolled for example. The fibre may be opened up mechanically, such as by teasing apart. The degree of swelling or shrinking may be controlled by controlling the amount of unrolling or teasing apart, as the case may be.

Preferably, however, the fibre is allowed to swell by being exposed to a swelling agent, the absorption or adsorption of which enables the volume of the fibre to be changed; preferably increased. A preferred example of a swelling agent is water. Where a swelling agent is used, the degree of swelling may be modulated by various methods. For example, the amount of swelling agent, such as water, which is exposed to a fixed amount of fibre may be varied. Furthermore, the time or temperature, or both, of exposure of the swelling agent or water to the fibre may also be varied, in conjunction with, or instead of controlling the amount exposed.

In preferred embodiments, the coupling between the detectable entity and the fibre is such that it optimally takes place in a non-aqueous medium such as an organic medium. A preferred organic medium is toluene, xylene, dichloromethan, acetone or dimthylformamide, as these are compatible with preferred coupling and activation reagents, for example vinylsulfone, azlactones, cyanuric chloride, dichlorotriazine, chlorotriazine, isocyanates, N-hydrozyl succinimide esters, aldehydes, epoxides, carbonyl diimadazole, cyanogenbromide, tresylchloride, bromoacetyl and alkyl bromide.

In such a case, the amount or degree of swelling may conveniently be controlled by adding amounts of different solvents into the non-aqueous medium. In other words, the extent of swelling may be controlled by allowing coupling in a mixture of non-aqeuous medium and water in varying proportions or by mixing different non aquous solvents like toluene and alcohols.

Mixtures of solvents, whether organic, inorganic, water miscible, water immiscible, etc, may also be used. The solvent mixtures could be any mixable solvents - for example preferable alcohols and water, acetone and water, alcohols and toluene, toluene and dimethylformamide - or any mixture thereof, which are compatible with the fibres and the coupling chemistry used.

In such a case, the amount or degree of swelling may conveniently be controlled by adding amounts of water into the non-aqueous medium. In other words, the extend of swelling may be controlled by allowing coupling in a mixture of non-aqeuous medium and water in varying proportions. The more water present, the higher the amount of swelling.

Figure 6A shows an embodiment in which the fibre has been allowed to swell substantially completely during or before coupling. The detectable entity then has access to the interior or core of the fibre, and can couple thereto, resulting in at least some staining in such core portions. In extreme cases, the detectable entity is coupled to substantially all portions in a cross section of the fibre. Exposure to a relevant antibody results in homogenous staining (i.e., staining at both core and peripheral regions of the fibre). The cross section profile of the fibre and reference standard therefore has a uniform distribution of detectable entity. Such an embodiment is preferred where the detectable entity is known to have a distribution in both the cell membrane as well as the cytoplasm, or even across substantially all portions of the cell.

Where the detectable entity is known or suspected to have some distribution in the cytoplasm, but perhaps the majority being present in the cell membrane, such a distribution or pattern of staining may be mimicked by enabling or allowing the fibre to swell partially during or after coupling. Partial swelling produces fibres in which cross sections have substantially more detectable entity at surface portions compared to core portions, as shown in Figure 6B. Antibody staining is non-homogenous, and is concentrated on the peripheral or surface regions of the fibre, with limited internal staining.

Where swelling is not allowed to take place, the molecules of the detectable entity will only react with and couple to surface portions of the fibre. The detectable entity is only able to access and couple to the peripheral or surface portions of the fibre. No coupling takes place in the internal or core portion of the fibre, resulting in staining which is substantially restricted to the surface (dense surface staining, no internal staining).

This results in a fibre with a cross sectional distribution of detectable entity which is substantially restricted to outer portions of the fibre, with substantially no staining in interior or core portions of the fibre. The resulting profile will therefore have a "ring" shape. Such a distribution is shown in Figure 6C. Such a ring shape may be useful where the detectable entity is known in a cell to be restricted to the cell surface, as the staining pattern will in both cases be similar. Reference standards as described in which no swelling is allowed to take place may therefore be usefully employed as standards to gauge the presence, quantity and/or distribution of a detectable entity which is a membrane protein or a cell-surface receptor, for example.

Furthermore, it will be appreciated that the different distributions achieved as described above by modulating swelling may be employed for the purposes of monitoring cell entry of an agent. Thus, for example, they can be used to track whether a particular agent, such as a drug, is capable of passing through the cell membrane and penetration into the cell. The efficiency of a membrane translocation sequence (MTS) such as *Drosophila* Antennapaedia protein or HIV TAT (or their fragments) may be monitored this way.

The elongate path where defined by means of a fibre is established in the medium in a number of ways. Most simply, the medium is formed around the fibre comprising the detectable entity attached thereto. For example, an embedding medium such as paraffin may be treated with heat and melted, and the molten embedding medium poured around the fibre; once solidified, the embedding medium will encase the fibre within it. In such situations, it may be desirable during the process for the fibre to be held in place with a scaffold or other support for example. Such a scaffold may hold the fibre taut (under tension), and may have the facility to carry more than one fibre, preferably multiple fibres in substantially parallel orientation. Once the embedding medium has solidified, the scaffold may be released from the fibres.

Furthermore, the fibres may be embedded or supported in another medium prior to being embedded in the embedding medium. Such an embodiment is illustrated in the flowchart shown in Figures 7 and 8, which include an agarose embedding step. In this embodiment, the fibre is held in place in the context of an agarose gel, which may be melted and poured around the fibre to encase it. The fibre may be held in place during this procedure with a scaffold, as described above. A strip of the agarose gel may then be cut which includes the encased fibre. The agarose strip is then itself embedded in the embedding medium, for example, by melting, pouring and solidifying the embedding medium as described above. The advantage of such an embodiment is that the agarose gel is handled more easily, and retains its elongate form, more readily than the bare fibre. Needless to say, the agarose gel should have adequate stiffness, and concentrations of agarose of between 0.5 % to 1 %, 2 %, 3 %, 4 %, 5 % or more may be required.

The use of agarose strips is illustrated in the photographs making up Figure 9. Fibres are first strung on a frame and tensioned. The frame is then placed in a container, and molten agarose poured over the frame. The agarose is allowed to set, and the set agarose removed. Strips of agarose containing individual fibres are cut from the agarose block, and the agarose blocks containing individual fibres are embedded in paraffin. It will however be appreciated that the embedding of the fibres in agarose is done for the purpose of retaining the fibres in the desired conformation (here, an elongate conformation) while the paraffin is setting. Therefore, the agarose embedding steps are optional, so long as the fibres can be retained in desired conformations for and during the paraffin-embedding step.

It will also be apparent that fibres embedded in agarose (without paraffin embedding) may be themselves used as the reference standard. In such an embodiment, the embedding medium is agarose itself. The agarose block containing embedded fibres may be cut into sections as described elsewhere in this document, to produce slices or sections for subsequent fixing and staining procedures. For this purpose, the agarose blocks may be frozen, so that slicing is made easier.

### CHANNELS

In an alternative embodiment, the elongate path of the detectable entity is defined by a channel in the support medium. In such an embodiment, an elongate channel is opened in the support medium in which the detectable entity is placed. Multiple channels may be formed, for supporting different amounts of a detectable entity, or different detectable entities, or both.

We further provide an embodiment, in which channels are provided in the form of hollow fibres (see below); in such an embodiment, the channels may be provided in both the hollow fibres themselves, or in the support medium (for example, a paraffin block), or both, optionally in combination with fibres as described above.

The channels may be formed in the support medium by any means, such as carving or drilling, for example. The channels may be formed by puncturing the support medium using a thin needle. Ordinary metal needles may be used, or needles made of polymer. Drinking straws of suitable stiffness may also be used to punch holes in support medium if it is soft enough, for example, paraffin.

Alternatively, or in addition, the channels may be formed by positioning placeholders, for example, rods (made of metal or other material) in a liquefied embedding medium. The embedding medium is allowed to solidify, and the placeholders are removed. Suitable moulds comprising single or multiple placeholders may be employed for this purpose. Such a technique is useful for making channels in paraffin, for example. Use of laser radiation to burn or carve out the channels is also possible.

The quantity of detectable entity may then be located in the channels by any suitable means. For example, where the detectable entity is in a solid state, it may merely be placed in the channel and packed or compressed if necessary. Where the detectable entity is in the form of a powder or grains, use of glue or other medium to bind these together may be envisaged. Similarly, the detectable entity may be dissolved in a liquid medium, and the liquid medium injected into the channels and allowed to solidify. For example, a molten solution of agarose may be made and the detectable entity dissolved or suspended in it. The agarose solution may be injected or otherwise placed in the channel, and allowed to solidify. The detectable entity may be dissolved or suspended in a monomer solution, the monomer solution injected or placed into the channel, and allowed to polymerise.

Preferably, the channel has a diameter of between about 0.5 µm to 100 µm, more preferably between 1 µm and 100 µm, even more preferably between 10 µm to 20 µm and most preferably between 2 µm to 20 µm.

The reference standard as described here may be produced either singly, or batch-wise during manufacturing. Batch-wise production is preferred.

In some embodiments, the channel is formed in the same embedding medium as the sample. Thus, any of the steps descried above for forming the channel may be conducted on the embedding medium which is to hold the sample. Standard procedures for FFPE embedding may be modified accordingly to effect this. For example, channels may be formed in the sample containing embedding medium by positioning placeholders as described above in a liquefied embedding medium such as paraffin. The paraffin is allowed to solidify and envelope both the sample and the placeholder, and the placeholders are removed. Alternatively, a paraffin block comprising the sample may be made beforehand, and a channel formed in the block by drilling. The detectable entity is then placed in the channel as described above.

### HOLLOW FIBRES

It will be appreciated that the reference standard may make use of hollow fibres.

Hollow fibres as the term is used in this document, are fibres, which have one or more holes running through their entire length. They are made by e.g. a dry/wet phase separation process by extruding a polymer solution through a spinneret having an annulus or annular orifice together with a bore-forming fluid or coagulant.

The polymer used is typically silicone, polypropylene, cellulose, cellulose derivatives, polysulfone or polyesters. Hollow fibres are widely used for e.g. membrane filtering devices, textiles or upholstering.

As hollow fibres are often made by simple extrusion, the inner holes can be empty or filled with another material during the manufacturing process. This makes hollow fibres attractive for use in the reference material of the invention. After sectioning, the fibres will exhibit a predictable morphology, which can mimic cells or cell structures.

Therefore, the detectable entity may comprise or be bound to a hollow fibre in the reference standard (the hollow fibre being a support for the detectable entity). Furthermore, where the hollow fibre comprises holes or channels, these holes or channels may form paths for the detectable entity. In other words, the elongate path of the detectable entity is defined by a channel in the hollow fibre. The detectable entity may then be located in the hollow fibre channel or channels as described above in the "Channels" section.

In such embodiments, therefore, it will be appreciated that the hollow fibre itself may be regarded as the reference standard.

Figure 10 illustrates various hollow fibres suitable for use in the reference standard described here. Figures 10A and 10B show profiles or cross sections of hollow fibres. The hollows or channels are clearly visible. Figure 10C shows an embodiment in which a number of hollow fibres, which may comprise one or more same or different detectable entities coupled onto each or all of the hollow fibres. Alternatively, or in addition, the or each hollow or channel of the or each hollow fibre may comprise the same or different detectable entity.

Core-shell fibre poses some of the same properties as hollow fibres, and may be used in the reference standard described here. Core-shell fibres consist of a core material surrounded by a shell made of a different material. The two materials can be combinations of e.g. PVC, Teflon, polyamides, cellulose, cellulose derivatives, polysulfone, polyesters or polycarbonates.

### BLOCK CO-POLYMERS

Self-assembling macromolecules can provide materials with predictable structure and morphology. One important class of macromolecules are block copolymers. Accordingly, the use of macromolecules, for example self-assemblying molecules, as well as block copolymers is envisaged in the reference standard described here.

Block co-polymers are produced by joining two or more chemically distinct homopolymer blocks, each a linear series of identical monomers. Using combinations of multiple homopolymer blocks, one can produce materials with tailored physical properties.

Some block copolymers spontaneously self-assemble into micro domain structures due to micro phase separation. The repeating structures can be e.g. spheres, cylinders, double gyroid, double diamond or lamellar structures.

As the different polymer blocks have different chemical properties, one can e.g. further selectively modify one of the blocks and leave the other unmodified.

Fibres of self-assembling block copolymers are attractive for use in the reference material of the invention. After sectioning, the highly ordered structures in the fibres will give a predictable staining pattern and morphology, which can mimic cells or cell structures.

### REFERENCE STANDARD SHAPE

The reference standard described here may take any suitable shape.

The reference standard may have an amorphous shape, for example, an elongate amorphous shape, but preferably it has a defined shape. It may take the form generally of a sphere, but preferably, the reference standard is of polyhedral shape.

More preferably, the reference standard has substantially a shape selected from the group consisting of: a regular polyhedron, a prism, a right prism, a regular right prism, a cuboid, a rectangular box and a cube.

The reference standard preferably has an elongate shape, such that it possesses a long axis. In preferred embodiments, the (or each where more than one is present) elongate path defined by the detectable entity is located along a long axis, or substantially parallel to it. Such a longitudinal orientation is preferred.

In highly preferred embodiments, the reference standard has a cuboid shape. A cuboid as the term is used here refers to a closed box composed of three pairs of rectangular faces placed opposite each other and joined at right angles to each other, also known as a rectangular parallelepiped. The cuboid is also a right prism, a special case of the parallelepiped, and corresponds to what in everyday parlance is known as a (rectangular) "box". In such preferred embodiments, the or each elongate path defining the detectable entity is in a substantially parallel orientation to a long edge of the cuboid.

Where slices or sections are taken of the reference standard, these are preferably taken at planes which are substantially at right angles to the elongate path adopted by the detectable entity. Alternatively, or in combination, the sections or slices may be taken transversely with respect to the orientation of the reference standard.

### SUPPORT MEDIUM

Any material or medium which is capable of supporting the detectable entity may be used as a "support medium".

Preferably, such a support medium is capable of supporting the detectable entity so that it retains its shape or configuration substantially. In preferred embodiments, the support medium supports the detectable entity in an elongate path. The support medium may comprise a solid, semi-solid, or a gel. The support medium may be comprised of a matrix or web, or network or grid, on which the detectable entity is supported, for example. The matrix, web etc may be comprised of any suitable fibrous material, for example, carbon fibre or fibreglass. A loose matrix, web, etc may be employed, providing a substantially open structure. Alternatively, a more dense structure may be preferred in certain embodiments.

In highly preferred embodiments, the support medium comprises an embedding medium. The embedding medium preferably surrounds or envelopes at least a portion of the detectable entity, preferably its entirety. Any embedding medium as known in the art, such as an immunohistochemical (IHC) or an *in situ* hybridisation (ISH) embedding medium, may be employed for this purpose. Polymers, preferably made by polymerisation of monomers, may be used, as described below. Preferred examples of such embedding media comprise paraffin and agarose.

The support or embedding medium may be homogenous, or it may be heterogeneous and comprise other material. This "other material" may comprise cells, parts of cells, tissue fragments, dyes, granular materials, etc.

The purpose of including this other material is to produce a "ground" or "background" in any slice or section taken of the reference standard; the presence of "ground" enables the defined region comprising the detectable entity to be more easily detected or located, i.e., it increases the contrast. Furthermore, the presence of the "ground" enables the viewer to make a more accurate determination of the presence or quantity of the detectable entity within the support medium. This is because of the well known "optical effect", in which the eye perceives two signals of identical intensities as being different intensities, depending on the background. Thus, for example, in a clinical sample, the signal to be detected by the eye (e.g., a cell which is stained) may have a background comprising other cells, other cells of different types, blood vessels; bone tissue, etc. Accordingly, the use of the other material within the support medium ensures that the eye will perceive signals from the reference standard of a similar intensity to those from the sample in question as being similar or identical, rather than of different intensity.

The support or embedding medium may further comprise orientation means. The orientation means may comprise any visible indication within the medium which aids or enables the user to determine its orientation or direction or position. The orientation means may in particular comprise reticulations, or a network of lines, within the medium. The orientation means may comprise one or more generally parallel lines in one or more planes. Preferably, a three-dimensional network of such planes each comprising parallel lines is included. The matrix may therefore include visible structures that look like for example chicken wire net to help the user to navigate over the slide, as pathologists are trained to look for "chicken wire" structures.

The support or embedding medium may comprise biological material, such as cells, tissues, organs, etc, or any part of these, such as organelles, cellular structures, etc. The biological material may completely surround the detectable entity, or be positionally spaced from it. In either configuration, a planar section or slice will comprise a defined region comprising the detectable entity, together with a section of the tissue, etc. In the former configuration, the defined region is located within the section of the tissue, and may allow easier comparisons to be made between the two.

### EMBEDDING MEDIUM

In highly preferred embodiments, the support medium embeds the detectable entity, and may therefore be regarded as an "embedding medium". The embedding medium may comprise any suitable material, for example, a material which is used to embed tissues or samples in immunohistochemistry, such as paraffin.

Preferably, the embedding medium is inert. More preferably, the embedding medium is transparent to radiation, preferably transparent to visible light.

While paraffin is the most commonly used embedding medium, any other type of suitable embedding medium may be used. Included are materials such as Araldite M, Dammar Resin, Divinylbenzene" Durcupan (Fluka), Epoxy Embedding Medium, Ethylene glycol dimethacrylate, Glycol methacrylate, Histocryl embedding resin, Lowicryl HM20, Butyl methacrylate, Hydroxypropyl methacrylate, Methyl methacrylate, Paraffin wax, Paraplast. Embedding media may be formed from polymerisation of monomers such as methacrylic acid monomer and styrene monomer. Further details of forming embedding media through polymerisation are provided in the section headed "Polymers" below.

The embedding medium may comprise ice, i.e., a frozen section comprising frozen water in which the tissue, etc is embedded. Therefore, in certain embodiments, the elongate path is supported in the same embedding medium as the sample tissue, cell, organ itself. Such embodiments are advantageous because only a single cut section needs to be handled, instead of one for the reference standard and one for the sample.

Such embodiments of the reference standards may be made achieved by embedding a fibre as described below in the same embedding medium as the sample. Alternatively, and as described in further detail below, the elongate path of the detectable entity is defined by a channel in that same support medium. It will be appreciated that the elongate path may be defined in the support medium at the same time, before, or after, the sample is embedded in that medium. In other words, the fibre may be embedded (and/or the channel cut) in the support medium at any time relative to the embedding of the sample in the support medium. Preferably, where the elongate path comprises a fibre, this is embedded as part of the paraffin embedding process in FFPE.

### SECTIONS AND MOUNTING

The reference standard may be provided in a single piece, and used without further processing. Preferably, however, the reference standard may be cut into slices or sections, and these sections or slices being used as standards themselves. As illustrated in Figure 2C, the slices or sections comprise defined areas comprising the detectable entity, and multiple slices or sections may be made from a reference standard.

It will be appreciated that the defined areas in the slices or sections which comprise the detectable entity may take the form of elongate shapes, and as these are supported by the supporting medium, they may themselves preferably be treated as "reference standards" as described in this document.

The slices or sections may be taken using any suitable means (for example, a microtome such as a bench microtome or a rocking microtome). The thickness of the slices or sections are typically those of standard microtome slices. Preferably, the slices or sections are between about 0.5 to 300 µm, preferably between 5 to 200 µm, preferably between about 10 to 100 µm, preferably between about 1 to 100 µm, preferably between about 20 to 30 µm, most preferably between about 2 to 10 µm thick. In a highly preferred embodiment, the slices or sections are 5 µm thick or thereabouts.

Multiple sections or slices of the reference standard may be taken in a similar manner as for any FFPE sample. The resulting section or slice of the reference standard may be treated in the same way as any other fixed and embedded tissue or cell sample.

The slice or section of the reference standard may be mounted on a suitable support to aid handling. Such a support may suitably comprise a slide, such as a microscope slide, made of glass or other material. The section or slice of the reference section may be mounted in a similar manner to a section of a FFPE embedded material. Such mounting techniques are known in the art. The section or slice may be mounted in a temporary, permanent or semi-permanent manner, and may in particular be fixed to the support, by adhesive or surface tension for example.

The slice or section may also be placed on a liner, which is typically a thin planar piece of material able to support the slice or section. The slice or section may be shipped or sold with the liner, either one slice or section on a single piece of liner, or a plurality of slices or sections. For example, such a liner may be made of paper, cardboard, plastic, cellulose acetate, etc. The surface of the liner may be treated to prevent adhesion of the slice or section, for example, by silicone. A preferred embodiment of such a liner therefore comprises siliconized paper. Use of such a liner enables the slice or section to be easily mounted on a microscope slide, preferably next to the sample from the patient.

The methods for attaching or mounting sections to slides include using clean slides and relying on the capillary attraction and no adhesives. Other techniques include glues like egg-white glycerine, glycerine-gelatine mixtures, polyvinyl acetate glue, chrome-alum gelatine and poly lysine coating. Heating or "burning" of the section as a means of facilitating mounting of the section should be used with caution, as the tissue can be destroyed.

The support may comprise indicia to aid the quantitation of the detectable entity. Such indicia may preferably be located in the vicinity of the area comprising the detectable entity. The indicia may relate to quantity of detectable entity, or the grade assigned to that quantity or the nature of detectable entity

The amount of staining in the slice or section may then be compared to that in the sample to provide an assessment of the significance of the level of staining of the latter. It will be appreciated that although it is preferred that the slice or section be taken of the reference standard be subject to the processing procedures, it may be desirable in some embodiments for the reference standard itself to be taken through processing.

### DETECTION AND VISUALISATION

The reference standard may comprise the detectable entity in an already visualisable state; in other words a detectable entity in a form which does not need to be processed further to identify its presence or quantity. The detectable label may therefore comprise a label such as a fluorescent or radioactive label, or otherwise tagged with an agent which is capable of emitting radiation. The label preferably emits light; most preferably, the light is emitted as a result of fluorescence. The detectable entity may be detected by detecting signal emission (or a change in signal emission) by the detectable label, and the detection may further comprise exciting the detectable label and monitoring fluorescence emission. The amount of the signal emitted may be measured to indicate the amount of detectable entity present.

In preferred embodiments, however, the reference standard comprises a detectable entity which is not substantially modified from its native form, for example, unlabelled. In such embodiments, the presence and/or quantity of the detectable entity is only revealed on further processing of the reference standard, for example, by application of the steps conventionally taken to reveal a detectable entity in a biological sample.

Thus, application of a primary revealing means such as a binding agent which binds to the detectable entity, preferably specifically, may be used. The detectable entity, the binding agent, or the combination of the two may be further detected by the use of secondary revealing means. Where the binding agent comprises an antibody and the detectable entity comprises an antigen, the antibody, antigen, or the antigen-antibody complex, may be revealed by a secondary antibody. The secondary antibody may be conjugated to an enzyme, which is capable of producing a signal when reacted to a chromogenic substrate. It will be appreciated that any step or series of steps which may be used to reveal, detect or quantify a detectable entity in a biological sample may be applied to the reference standard or its sections. Preferably, such step(s) are those which are conventionally employed in immunohistochemistry, for example, detection steps to detect the entity in FFPE sections.

The section or slice of the reference standard may in particular be subjected to any one or more of the procedures used to stain a FFPE embedded sample or section thereof. Thus, the slice or section of the reference standard is intended to be, and may be subjected to any one or more of the typical post embedding procedures used to stain tissue samples, in order to reveal the antigen. In preferred embodiments, the section or slice is subjected to all or substantially all of such procedures. Preferably, therefore, the section or slice is subjected to any one or more, preferably all, of the following: mounting onto a slide, baking, deparaffination, rehydration, antigen retrieval, blocking, exposure to antibody, exposure to primary antibody, washing, exposure to secondary antibody-enzyme conjugate, exposure to enzyme substrate, exposure to chromogen substrate, and counter staining.

Baking refers to gently heating the slide with the paraffin slice on it. The paraffin partly melts away and the tissue sticks to the glass surface.

In preferred embodiments, the detectable entity is exposed to an antibody, and binding of antibody visualised in any of several ways. For a general introduction to different immunocytochemistry visualization techniques, see for example Lars-Inge Larsson "*Immunocytochemistry: Theory and Practice",* CRC Press inc., Baca Raton, Florida, 1988, ISBN 0-8493-6078-1, and John D. Pound (ed); "*Immunochemical Protocols, vol 80*", in the series: "Methods in Molecular Biology", Humana Press, Totowa, New Jersey, 1998, ISBN 0-89603-493-3.

The most commonly used detection methods in immunohistochemistry are direct visualisation of fluorescence or gold particles and enzyme mediated colorimetric detection.

For direct fluorescent studies, the labels can for example be 5-(and 6)-carboxyfluorescein, 5- or 6-carboxyfluorescein, 6-(fluorescein)-5-(and 6)-carboxamido hexanoic acid, fluorescein isothiocyanate (FITC), rhodamine, tetramethylrhodamine, and dyes such as Cy2, Cy3, and Cy5, optionally substituted coumarin including AMCA, PerCP, phycobiliproteins including R-phycoerythrin (RPE) and allophycoerythrin (APC), Texas Red, Princeston Red, Green fluorescent protein (GFP) and analogues thereof, and conjugates of R-phycoerythrin or allophycoerythrin and for example Cy5 or Texas Red, and and inorganic fluorescent labels based on semiconductor nanocrystals (like quantum dot and Qdot^{™} nanocrystals), and time-resolved fluorescent labels based on lanthanides like Eu3+ and Sm3+.

Colloidal gold or silver can be used as direct labels for immunocytochemical studies for electron microscopy and light microscopy. Amplification of the signal can be obtained by further silver enhancement of the colloidal gold particles.

The general enzymatic methods use labelled avidin or streptavidin-biotin (LAB or LSAB), avidin or streptavidin-biotin complex (ABC), enzyme anti-enzyme complex (PAP and APAAP), direct dextran polymer based antibody-enzyme complex (EPOS, DakoCytomation); indirect dextran polymer based antibody-enzyme complex (EnVision, DakoCytomation) or double bridge enzyme anti-enzyme complex.

The enzymatic staining uses enzymatic labels such as horse radish peroxidase (HRP), alkaline phosphatase (AP), beta-galactosidase (GAL), glucose-6-phosphate dehydrogenase, beta-N-acetylglucosaminidase, invertase, Xanthine Oxidase, firefly luciferase and glucose oxidase (GO).

Examples of commonly used substrates for horse radish peroxidase include 3,3'-diaminobenzidine (DAB), diaminobenzidine with nickel enhancement, 3-amino-9-ethylcarbazole (AEC), Benzidine dihydrochloride (BDHC), Hanker-Yates reagent (HYR), Indophane blue (IB), tetramethylbenzidine (TMB), 4-chloro-1-naphtol (CN), □-naphtol pyronin(□-NP), o-dianisidine (OD), 5-bmmo-4-chloro-3-indolylphosphate (BCIP), Nitro blue tetrazolium (NBT), 2-(p-iodophenyl)-3-p-nitrophenyl-5-phenyl tetrazolium chloride (INT), tetranitro blue tetrazolium (TNBT), 5-bromo-4-chloro-3-indoxyl-beta-D-galactoside/ferro-ferricyanide (BCIG/FF).

Examples of commonly used substrates for Alkaline Phosphatase include Naphthol-AS-B1-phosphate/fast red TR (NABP/FR), Naphthol-AS-MX-phosphate/fast red TR (NAMP/FR), Naphthol-AS-B1-phosphate/fast red TR (NABP/FR), Naphthol-AS-MX-phosphate/fast red TR (NAMP/FR), Naphthol-AS-B1-phosphate/new fuschin (NABP/NF), bromochloroindolyl phosphate/nitroblue tetrazolium (BCIP/NBT), 5-Bromo-4-chloro-3-indolyl-b-d-galactopyranoside (BCIG).

One of the most potent detection systems is the catalysed reporter deposition (CARD); this amplification method is based on the deposition of labelled tyramide on tissue through the enzymatic action ofHRP. After HRP-immunostaining, labelled tyramide is applied and bound near the site of HRP-activity. The bound and labelled tyramide is then visualised by traditional fluorescence or colorimetric enzyme mediated detection.

The labelling compounds mentioned above can in general be applied to both probes and antibodies (or any other substance used to detect a desired target).

The method of viewing the stained specimens includes bright field microscopes or scanners, fluorescent microscopes or scanners, transmission electron microscope (TEM) or scanning electron microscope (SEM).

Automated staining systems have been introduced to reduce cost, increase uniformity of slide preparation, reduce laborious routine work and most significantly reduce procedural human errors.

The current automated systems can handle any immunochemical assay including assays relying on immunofluorescence, indirect immunoassay procedures, enzyme or gold staining methods. They perform all steps of the immunohistochemical assay irrespective of complexity or their order, at the prescribed time and temperature.

Immunocytochemistry techniques have traditionally used specific antibodies for identification and visualisation of specific antigens. The technique is complex, many steps and molecules with high affinities for specific staining are needed.

"Special stains", described in a separate section below may also be used, either alone or in combination with immunohistochemical visualisation.

### STAINING AND IMMUNOSTAINING

In the following, some of the individual steps in a staining procedure are described. Each of, some of, or all of these steps may be applied to the reference standard, or a slice or section thereof.

Fixatives are needed to preserve cells and tissues in a reproducible and life-like manner. To achieve this, tissue blocks, sections, or smears are immersed in a fixative fluid, or in the case of smears, are dried. Fixatives stabilise cells and tissues thereby protecting them from the rigors of processing and staining techniques.

Any suitable fixing agent may be used, for example, ethanol, acetic acid, picric acid, 2-Propanol, 3,3'-Diaminobenzidine tetrahydrochloride Dihydrate, Acetoin (mixture of monomer) and dimer, Acrolein, Crotonaldehyde( (cis+trans), Formaldehyde, Glutaraldehyde, Glyoxal, Potassium dichromate, Potassium permanganate, Osmium tetroxide, Paraformaldehyde, Mercuric chloride, Tolylene-2,4-diisocyanate, Trichloroacetic acid, Tungstic acid. Preferred types of fixative include formalin (aqueous formaldehyde) and neutral buffered formalin (NBF) is among the most commonly used. Other preferred fixatives include glutaraldehyde, acrolein, carbodiimide, imidates, benzoequinone, osmic acid and osmium tetraoxide.

Fresh biopsy specimens, cytological preparations (including touch preparations and blood smears), frozen sections and tissues for immunohistochemical analysis are commonly fixed in organic solvents, including ethanol, acetic acid, methanol and/or acetone.

### ANTIBODIES

In preferred embodiments, an antibody capable of binding to the detectable entity is employed to reveal its presence.

Antibodies comprise immunoglobulin molecules. Immunoglobulin molecules are in the broadest sense members of the immunoglobulin superfamily, a family of polypeptides comprising the immunoglobulin fold characteristic of antibody molecules, which contains two β sheets and, usually, a conserved disulphide bond. Members of the immunoglobulin superfamily are involved in many aspects of cellular and non-cellular interactions *in vivo,* including widespread roles in the immune system (for example, antibodies, T-cell receptor molecules and the like), involvement in cell adhesion (for example the ICAM molecules) and intracellular signalling (for example, receptor molecules, such as the PDGF receptor). The methods described here of detecting detectable entities and of using the reference standard may therefore make use of any immunoglobulin superfamily molecule which is capable of binding to a target. Peptides or fragments derived from immunoglobulins may also be used.

Antibodies, as used herein, refers to complete antibodies or antibody fragments capable of binding to a selected target, and including Fv, ScFv, F(ab') and F(ab')₂, monoclonal and polyclonal antibodies, engineered antibodies including chimeric, CDR-grafted and humanised antibodies, and artificially selected antibodies produced using phage display or alternative techniques. Small fragments, such as Fv and ScFv, possess advantageous properties for diagnostic and therapeutic applications on account of their small size and consequent superior tissue distribution. Preferably, the antibody is a single chain antibody or ScFv.

The antibodies may be altered antibodies comprising an effector protein such as a toxin or a label. Use of labelled antibodies allows the imaging of the distribution of the antibody *in vivo.* Such labels may be radioactive labels or radioopaque labels, such as metal particles, which are readily visualisable within the body of a patient. Moreover, they may be fluorescent labels (such as the ones described here) or other labels which are visualisable on tissue samples removed from patients. Antibodies with effector groups may be linked to any association means as described above.

Antibodies may be obtained from animal serum, or, in the case of monoclonal antibodies or fragments thereof, produced in cell culture. Recombinant DNA technology may be used to produce the antibodies according to established procedure, in bacterial, yeast, insect or preferably mammalian cell culture. The selected cell culture system preferably secretes the antibody product.

Growing of hybridoma cells or mammalian host cells in vitro is carried out in suitable culture media, which are the customary standard culture media, for example Dulbecco's Modified Eagle Medium (DMEM) or RPMI 1640 medium, optionally replenished by a mammalian serum, for example foetal calf serum, or trace elements and growth sustaining supplements, for example feeder cells such as normal mouse peritoneal exudate cells, spleen cells, bone marrow macrophages, 2-aminoethanol, insulin, transferrin, low density lipoprotein, oleic acid, or the like. Multiplication of host cells which are bacterial cells or yeast cells is likewise carried out in suitable culture media known in the art, for example for bacteria in medium LB, NZCYM, NZYM, NZM, Terrific Broth; SOB, SOC, 2 x YT, or M9 Minimal Medium, and for yeast in medium YPD, YEPD, Minimal Medium, or Complete Minimal Dropout Medium.

Use of insect cells as hosts for the expression of proteins has advantages in that the cloning and expression process is relatively easy and quick. In addition, there is a high probability of obtaining a correctly folded and biologically active protein when compared to bacterial or yeast expression. Insect cells may be cultured in serum free medium, which is cheaper and safer compared to serum containing medium. Recombinant baculovirus may be used as an expression vector, and the construct used to transfect a host cell line, which may be any of a number of lepidopteran cell lines, in particular *Spodoptera frugiperda Sf9,* as known in the art. Reviews of expression of recombinant proteins in insect host cells are provided by Altmann et al. (1999), *Glycoconj J* 1999, 16, 109-23 and Kost and Condreay (1999), *Curr Opin Biotechnol,* 10, 428-33.

*In vitro* production provides relatively pure antibody preparations and allows scale-up to give large amounts of the desired antibodies. Techniques for bacterial cell, yeast, insect and mammalian cell cultivation are known in the art and include homogeneous suspension culture, for example in an airlift reactor or in a continuous stirrer reactor, or immobilised or entrapped cell culture, for example in hollow fibres, microcapsules, on agarose microbeads or ceramic cartridges.

Large quantities of the desired antibodies can also be obtained by multiplying mammalian cells *in vivo.* For this purpose, hybridoma cells producing the desired antibodies are injected into histocompatible mammals to cause growth of antibody-producing tumours. Optionally, the animals are primed with a hydrocarbon, especially mineral oils such as pristane (tetramethyl-pentadecane), prior to the injection. After one to three weeks, the antibodies are isolated from the body fluids of those mammals. For example, hybridoma cells obtained by fusion of suitable myeloma cells with antibody-producing spleen cells from Balb/c mice, or transfected cells derived from hybridoma cell line Sp2/0 that produce the desired antibodies are injected intraperitoneally into Balb/c mice optionally pre-treated with pristane, and, after one to two weeks, ascitic fluid is taken from the animals.

The foregoing, and other, techniques are discussed in, for example, Kohler and Milstein, (1975) Nature 256:495-497; US 4,376,110; Harlow and Lane, Antibodies: a Laboratory Manual, (1988) Cold Spring Harbor. Techniques for the preparation of recombinant antibody molecules is described in the above references and also in, for example, EP 0623679; EP 0368684 and EP 0436597.

The cell culture supernatants are screened for the desired antibodies, preferentially by immunofluorescent staining of cells expressing the desired target by immunoblotting, by an enzyme immunoassay, for example a sandwich assay or a dot-assay, or a radioimmunoassay.

For isolation of the antibodies, the immunoglobulins in the culture supernatants or in the ascitic fluid may be concentrated, for example by precipitation with ammonium sulphate, dialysis against hygroscopic material such as polyethylene glycol, filtration through selective membranes, or the like. If necessary and/or desired, the antibodies are purified by the customary chromatography methods, for example gel filtration, ionexchange chromatography, chromatography over DEAE-cellulose and/or immunoaffinity chromatography, for example affinity chromatography with the a protein containing a target or with Protein-A.

Antibodies generated according to the foregoing procedures may be cloned by isolation of nucleic acid from cells, according to standard procedures. Usefully, nucleic acids variable domains of the antibodies may be isolated and used to construct antibody fragments, such as scFv.

The methods described here preferably employ recombinant nucleic acids comprising an insert coding for a heavy chain variable domain and/or for a light chain variable domain of antibodies. By definition such nucleic acids comprise coding single stranded nucleic acids, double stranded nucleic acids consisting of the coding nucleic acids and of complementary nucleic acids thereto, or these complementary (single stranded) nucleic acids themselves.

Furthermore, nucleic acids encoding a heavy chain variable domain and/or for a light chain variable domain of antibodies can be enzymatically or chemically synthesised nucleic acids having the authentic sequence coding for a naturally-occurring heavy chain variable domain and/or for the light chain variable domain, or a mutant thereof. A mutant of the authentic sequence is a nucleic acid encoding a heavy chain variable domain and/or a light chain variable domain of the above-mentioned antibodies in which one or more amino acids are deleted or exchanged with one or more other amino acids. Preferably the modification(s) are outside the complementary determining regions (CDRs) of the heavy chain variable domain and/or of the light chain variable domain of the antibody. Such a mutant nucleic acid is also intended to be a silent mutant wherein one or more nucleotides are replaced by other nucleotides with the new codons coding for the same amino acid(s). Such a mutant sequence is also a degenerated sequence. Degenerated sequences are degenerated within the meaning of the genetic code in that an unlimited number of nucleotides are replaced by other nucleotides without resulting in a change of the amino acid sequence originally encoded. Such degenerated sequences may be useful due to their different restriction sites and/or frequency of particular codons which are preferred by the specific host, particularly yeast, bacterial or mammalian cells, to obtain an optimal expression of the heavy chain variable domain and/or a light chain variable domain.

The term mutant is intended to include a DNA mutant obtained by *in vitro* or *in vivo* mutagenesis of DNA according to methods known in the art:
Recombinant DNA technology may be used to improve antibodies. Thus, chimeric antibodies may be constructed in order to decrease the immunogenicity thereof in diagnostic or therapeutic applications. Moreover, immunogenicity may be minimised by humanising the antibodies by CDR grafting [European Patent 0 239 400 (Winter)] and, optionally, framework modification [European Patent 0239400; Riechmann *et al.,* (1988) Nature 322:323-327; and as reviewed in international patent application WO 90/07861 (Protein Design Labs)].
Recombinant nucleic acids may be employed comprising an insert coding for a heavy chain variable domain of an antibody fused to a human constant domain γ, for example γ1, y2, γ3 or y4, preferably γ1 or γ4. Likewise recombinant DNAs comprising an insert coding for a light chain variable domain of an antibody fused to a human constant domain κ or λ, preferably κ may also be used.

More preferably, CDR-grafted antibodies, which are preferably CDR-grafted light chain and heavy chain variable domains only, may be used. Advantageously, the heavy chain variable domain and the light chain variable domain are linked by way of a spacer group, optionally comprising a signal sequence facilitating the processing of the antibody in the host cell and/or a DNA coding for a peptide facilitating the purification of the antibody and/or a cleavage site and/or a peptide spacer and/or an effector molecule. Such antibodies are known as ScFvs.

Antibodies may moreover be generated by mutagenesis of antibody genes to produce artificial repertoires of antibodies. This technique allows the preparation of antibody libraries, as discussed further below; antibody libraries are also available commercially. Hence, artificial repertoires of immunoglobulins, preferably artificial ScFv repertoires, are used as an immunoglobulin source.

Isolated or cloned antibodies may be linked to other molecules, for example nucleic acid or protein association means by chemical coupling, using protocols known in the art (for example, Harlow and Lane, Antibodies: a Laboratory Manual, (1988) Cold Spring Harbor, and Maniatis, T., Fritsch, E. F. and Sambrook, J. (1991), Molecular Cloning: A Laboratory Manual. Cold Spring Harbor, New York, Cold Spring Harbor Laboratory Press).

### NUCLEIC ACID PROBES

As noted above, the detectable entity may comprise a nucleic acid, and such reference standard embodiments comprising nucleic acid detectable entities are suitably used as standards in *in situ* hybridisation. In such embodiments, a nucleic acid probe capable of binding to the nucleic acid detectable entity is employed to reveal its presence.

The nucleic acid probe in particular preferably comprises at least a sequence that is capable of hybridising to a sequence in the detectable entity. In particular, it may comprise at least a sequence that is complementary to such a sequence. The nucleic acid probe is preferably single stranded, and may comprise in particular single stranded DNA or single stranded RNA.

The term "hybridisation" as used in this document refers to "the process by which a strand of nucleic acid joins with a complementary strand through base pairing". Preferably, the nucleic acid probe includes sequences that are capable of hybridising under stringent conditions (for example 50°C and 0.2x SSC {1x SSC = 0.15 M NaCl, 0.015 M Na₃citrate pH 7.0}) to at least a nucleotide sequence in the detectable entity. More preferably, the nucleic acid probe includes sequences that are capable of hybridising under high stringent conditions (for example 65°C and 0.1xSSC {1xSSC = 0.15 M NaCl, 0.015 M Na₃citrate pH 7.0}).

Nucleic acid probes capable of selectively hybridising to a nucleotide sequence in the detectable entity, or to their complement, will be generally at least 75 %, preferably at least 85 or 90 % and more preferably at least 95 % or 98 % homologous to the corresponding complementary nucleotide sequence in the detectable entity over a region of at least 20, preferably at least 25 or 30, for instance at least 40, 60 or 100 or more contiguous nucleotides. Preferred probes comprise at least one region preferably at least 80 or 90 % and more preferably at least 95 % homologous to the nucleotide sequence in the detectable entity

The term "selectively hybridizable" means that the nucleic acid probe is capable of hybridising to the target nucleic acid sequence at a level significantly above background. The background hybridization may occur because of other nucleotide sequences present, for example, in the sample being processed for ISH. In this event, background implies a level of signal generated by interaction between the probe and a non-specific DNA member of the library which is less than 10 fold, preferably less than 100 fold as intense as the specific interaction observed with the target DNA. The intensity of interaction may be measured, for example, by radiolabelling the probe, for example with ³²P.

Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridization can be used to identify probes comprising identical nucleotide sequences while an intermediate (or low) stringency hybridization can be used to identify probes comprising similar or related polynucleotide sequences.

Nucleotide sequences which are not 100 % homologous to a sequence in the detectable entity but which may be used as probes can be obtained in a number of ways. Conserved sequences can be predicted, for example, by aligning the amino acid sequences from several variants/homologues. Sequence alignments can be performed using computer software known in the art. For example the GCG Wisconsin PileUp program is widely used.

The probes may be produced recombinantly, synthetically, or by any means available to those of skill in the art. In general, probes will be produced by synthetic means, involving a step-wise manufacture of the desired nucleic acid sequence one nucleotide at a time. Techniques for accomplishing this using automated techniques are readily available in the art.

Longer nucleotide sequences for use as nucleic acid probes will generally be produced using recombinant means, for example using a PCR (polymerase chain reaction) cloning techniques. This will involve making a pair of primers (for example of about 15 to 30 nucleotides) flanking a region of the targeting sequence which it is desired to clone, bringing the primers into contact with mRNA or cDNA obtained from an animal or human cell, performing a polymerase chain reaction (PCR) under conditions which bring about amplification of the desired region, isolating the amplified fragment (for example by purifying the reaction mixture on an agarose gel) and recovering the amplified DNA. The primers may be designed to contain suitable restriction enzyme recognition sites so that the amplified DNA can be cloned into a suitable cloning vector.

The nucleic acid probes may be labelled by various means, as known in the art. For example, the probe may be labelled using radioactive labels such as ³¹P, ³³P or ³²S, or non-radioactively, using labels such as digoxigenin, or fluorescent labels, a great many of which are known in the art.

### STAINS AND DYES

Although antibodies are preferred as binding agents for use in revealing the detectable entity, other suitable stains or dyes may be used to reveal the target. For example, dyes typically used to colour fabrics may be used to stain the target or detectable entity, or the fibre itself. Such dyes will typically bind in a stoichiometric fashion, such that the more detectable entity or fibre present, the more dye is bound. However, simple staining to reveal presence and/or location may often provide enough information.

### "Special " Stains and Dyes

It should be understood, therefore, that the reference standard described here may also be stained not only using immunological recognition using for example antibodies, but also by means of chemical, which we refer to as "special stains".

The most common used are the general Haematoxylin-Eosin (H & E) staining, the Gomori methenamine silver stain (GMS) useful for identifying for example carbohydrates from fungi and the Periodic Acid-Schiff (PAS) stain useful for identifying for example glycogen, acid and neutral mucosubstances, fungal cell wall, basement membranes, collagen fibres and reticular fibres,

There are numerous special stains formulations, variations and combinations, including the Trichrome Blue, Masson's Trichrome, Prussian Blue, Giemsa, Diff-Quik, Reticulum, Congo Red, Alcian Blue, Steiner, AFB, PAP, Gram, Mucicarmine, Verhoeff-van Gieson, Elastic, Carbol Fuchsin and Golgi's stains.

It should also be understood, that some of the above-mentioned special stains will specifically stain target probes like for example some carbohydrates or hydrophobic moities, which are easily introduced into the reference standard described here. Furthermore, any combination of one or more immunological stains and one or more special staining may also be carried out.

For example, suitable stains and dyes may include any of the following: 1,4-Phenylenediamine, 2,3,5-Triphenyltetrazolium chloride, 2,4-Dinitro-5-fluoroaniline, 2-Naphthol (beta), 3,3'-Diaminobenzidine, 4-Chloro-1-naphthol, 4-Chloro-1-naphthol, Acridine Orange, Acridine Orange hydrochloride Hydrate, Silver proteinate, Alcian Blue 8GX, Alizarin, Alizarin Red S, Alkali Blue 4 B, Ammonium molybdate Tetrahydrate, Aniline hydrochloride, Auramine O, Azocarmine B, Azocarmine G, Azophloxine, Azure A, Azure B, Azure II, Azure II-Eosin, Azure Mixture sicc. Giemsa Stain, Bengal Rose B, Benzopurpurine 4B, Prussian Blue soluble, Prussian Blue insoluble, Bismarck Brown Y (G), Bismarck Brown R, Bismuth(III) nitrate basic, Lead(II) acetate Trihydrate, Lead(II) citrate TrihydrateLead(II) nitrate, Lead(II) nitrate, Lead(II) tartrate, Lead tetraacetate, Borax Carmin Solution ((acc. to Grenacher), Brilliant Green, Brilliant Cresyl Blue, 'Brilliant Cresyl Blue', Brilliant Cresyl Blue Solution, Bromocresol Green andcomplexometry, Bromocresol Green Sodium salt, Bromocresol Purple, Bromophenol Blue, Bromosulfalein, Bromothymol Blue Sodium salt, Carbol-Fuchsin Dye Powder, Carbol-Fuchsin Solution (according to Kinyoun), Carbol-Fuchsin Solution (according toZiehl-Neelsen), Carbol-Gentianaviolet Solution, Carbol-Methylene Blue Solution, Carmine, Carminic acid, Celestine Blue, Quinacrine Mustard dihydrochloride, Quinoline Yellow, Chlorazol Black, Chromium(VI) oxide, Chromium(VI) oxide, Chromotrop 2 R andcomplexometry, Chrysoidine G, Cobaltous chloride anhydrous, Cobalt naphthenate, ~10% Co, Cyanosine, Cytochrome c from horse heart lyophil.salt-free powder, Cytochrome c from horse heart, Cytochrome c from horse heart, Cytochrome c from bovine heart, Cytochrome c from pigeon breast muscle, Differential Stain Solution, Direct Red, Direct Red 80, Fast Blue B Salt, Fast Blue BB Salt, Fast Blue RR Salt, Fast Green FCF, Fast Red 3 GL Salt, Fast Red RC Salt, Fast Violet B Salt, Eosin alcohol soluble, Eosin Yellowish, Eosin Yellowish Solution, Eosin-Hematoxylin Solution ((acc. to Ehrlich), Eosin Methylene Blue (acc. to Leishman), Eosin Methylene Blue (acc. to Wright), Eosin Methylene Blue, Eosin Methylene Blue Solution (acc. ToWright), Eosin Methylene Blue Solution (acc. to Wright-Lillie), Eosin Scarlet, Eriochrome Red B, Erythrosin extra Bluish, Acetic acid Solution, Ethyl Violet, Evans Blue Fluka, Dye Sol. (acc. to Boroviczeny A: Toluidine Blue-Safranine fix), Dye Sol. (acc. to Boroviczeny B: EosineSolution), Ferritin from horse spleen, Fat Red Bluish, Fat Black, Fluorescein isothiocyanate, Fuchsin, Fuchsin Solution, Gallocyanine, Gentian Violet, Giemsa-Solution, Gold chloride Hydrate (~52% Au), Gold chloride Hydrate (ACS, >49% Au), Gold Solution, colloidal, Hemalaun, Hematein, Hematoxylin, Hematoxylin Solution A (acc. to Weigert), Hematoxylin Solution B (acc. to Weigert, Hematoxylin Solution (acc. to Boehmer), Hematoxylin Solution, (acc. to Delafield), Hematoxylin Solution (acc. to Mayer), Hanker-Yates Reagent, Hayem's Solution, Hesperidin, Indigocarmine, Indium(III) chloride Hydrate, Indium(in) chloride anhydrous, Iodonitrotetrazolium chloride, iso-Chloridazon solution, Janus GreenB, Potassium dichromate, Potassium hexahydroxoantimonate (V), Potassium permanganate, Potassium permanganate (Hg <0.000005%, ACS), Carmine Solution ammoniacal (acc. to Best), Carmine Solution acidic (acc. to Mayer), Nuclear Fast Red, Congo Red,indicator, Cresol Red, Cresyl Violet acetate, Crystal Violet indicator, Crystal Violet Solution, Lactophenol Blue Solution, Lanthanum nitrate Hexahydrate, Light Green SF Yellowish, Lipid Crimson, Lithium carbonate, Lugol Solution, Malachite green oxalate, May-Grunwald Solution, Metanil Yellow, Methylene Blue Zinc chloride Double salt, Methylene Blue, Methylene Blue Concentrate (acc. to Ehr-lich), Methylene Blue Solution alkaline (acc. to Löffler), Methylene green zinc double salt, Methyl Green, Methyl Orange, Methyl Violet, Morin, Mucicarmine, N-(4-Amino-2,5-diethoxyphenyl)benzamide, N,N-Dimethylaniline, N,N-Dimethyl-p-toluidine, Naphthol AS-acetate, Naphthol AS-BI-beta-D-glucuronide, (the following Naphthol derivatives are enzyme (AP) substrates), Naphthol AS-BI-phosphate Disodium saltHeptahydrate, Naphthol AS-BI-phosphate, Naphthol AS-BI-phosphate, Naphthol AS-BS-phosphate, Naphthol AS-chloroacetate, Naphthol AS-D-acetate, Naphthol AS-D-chloroacetate, Naphthol AS-E-acetate, Naphthol AS-E-phosphate, Naphthol AS-MX-acetate, Naphthol AS-MX-phosphate Disodium salt Nonahydrate, Naphthol AS-MX-phosphate, forhistology, Naphthol AS-phosphate, forhistology, Naphthol AS-TR-phosphate, Naphthol AS-TR-phosphate, Naphthol Blue Black, Naphthol Yellow S, Naphthol Green Bandcomplexometry, Sodium tungstate Dihydrate, Oil of cloves, Neotetrazolium chloride, New Coccine, New Fuchsine, New Methylene Blue N, Neutral Red, Nigrosin B alcohol soluble, Nigrosin watersoluble, Nile Blue A, Nile Blue chloride, Ninhydrin, Ninhydrin, Nitrazine Yellow, Nitrotetrazolium Blue chloride, Nitrotetrazolium Blue chloride, Nitrotetrazolium Blue chlor, Orange G, Orange G Solution (alcoholic), Orcein, Orcein, Palladium(II) chloride anhydrous, Palladium(II) oxide Hydrate, Parafuchsin, Parafuchsin hydrochloride, Peroxidase from horse radish (lyoph.powd.salt-free ~100 U/mg), Phenosafranine, Phosphomolybdic acid Ammonium salt Hydrate, Phosphomolybdicacid Hydrate, Phosphomolybdic acid Sodium salt Hydrate, Phosphorus pentoxide, Phosphotungstic acid Hydrate, Phthalocyanine, Picric acid moistened withwater (H2O ~40%), Pinacyanol iodide, Platinum(IV) oxide Hydrate, Ponceau BS, Ponceau S, Pyridine, Pyronine Y (G), Resorufin, Rhodamine B, Ruthenium(III) chloride anhydrous, Ruthenium Red, Safranin T, Safranin Solution (acc. to Olt), Acid Fuchsin Calcium salt, Acid Fuchsin indicator, Scarlet R, Schiff's Reagent for Aldehydes, Silver, Codex France, colloidal, Silver nitrate, Sirius Rose BB, 'Stains-all', Sudan Blue II, Sudan Orange G, Sudan Red B, Sudan Black B, Sulforhodamine B acid chloride, Tartrazine, Tetranitroblue tetrazolium chloride, Tetrazolium Blue chloride, Tetrazolium Violet, ThaHium(I) nitrate, Thiazole Yellow G, adsorption indicator, Thiazolyl Blue Tetrazolium bromide, Thiocarbohydrazide, Thioflavine T, Thionine acetate, Toluidine Blue, Tropaeolin 000 No. 1, Tropaeolin 000 No. 2, Trypan Blue, Trypan BlueSolution, 0.4%, Tuerk Solution, Uranyl acetate Dihydrate, Uranyl nitrate Hexahydrate, Variamine Blue B salt, Vesuvine Solution (acc. to Neisser), Victoria Blue B, Water Blue, Weigert's Solution, Tungstosilicic acid Hydrate, Wright Stain, Xylenecyanol FF (redox indicator).

### ANTIGEN RETRIEVAL

To facilitate the specific recognition in fixed tissue, it is often necessary to retrieve or unmask the targets through pre-treatment of the specimens to increase reactivity of the majority of targets. This procedure is referred to as "antigen retrieval", "target retrieval" or "epitope retrieval", "target unmasking" or "antigen unmasking". An extensive review of antigen retrieval (antigen unmasking) may be found in Shi S-R, Cote RJ, Taylor CR. Antigen retrieval immunocytochemistry: past, present, future. J Histochem Cytochem 1997: 45(3);327-343.

Antigen retrieval or target retrieval includes a variety of methods by which the availability of the target for interaction with a specific detection reagent is maximised. The most common techniques are enzymatic digestion with a proteolytic enzyme (for example Proteinase, pronase, pepsin, papain, trypsin or neuraminidase) in an appropriate buffer or heat induced epitope retrieval (HIER) using microwave irradiation, heating in a regular oven, autoclaving or pressure cooking in an appropriately pH stabilised buffer, usually containing EDTA, EGTA, Tris-HCl, citrate, urea, glycin-HCl or boric acid.

Detergents may be added to the HIER buffer to increase the epitope retrieval or added to the dilution media and/or rinsing buffers to lower unspecific binding.

Additionally, the signal-to-noise ratio may be increased by different physical methods, including application of vacuum and ultrasound, or freezing and thawing of the sections before or during incubation of the reagents.

Endogenous biotin binding sites or endogenous enzyme activity (for example phosphatase, catalase or peroxidase) can be removed as a step in the staining procedure.

Similarly, blocking of unspecific binding sites with inert proteins like, HSA, BSA, ovalbumine, fetal calf serum or other sera; or detergents like Tween20, Triton X-100, Saponin, Brij or Pluronics is widely used. Blocking unspecific binding sites in the tissue or cells with unlabelled and target non-specific versions of the specific reagents.

### OTHER TECHNIQUES

In staining procedures using the so-called "free floating techniques", a tissue section is brought into contact with different reagents and wash buffers in suspension or freely floating in appropriate containers, for example micro centrifuge tubes.

The tissue sections can be transferred from tube to tube with different reagents and buffers during the staining procedure using for example a "fishing hook like" device, a spatula or a glass ring.

The different reagents and buffer can also be changed by gentle decantation or vacuum suction. Alternatively, containers with the tissue sections can be emptied into a special staining net, like the Coming "Netwells" and the tissue section washed before being transferred back into the tube for the next staining step.

All the individual staining procedure steps, including for example fixation, antigen retrieval, washing, incubation with blocking reagents, immuno-specific reagents and for example the enzymatic catalysed development of the coloured stains, are done while the tissue section is floating freely or withheld on nets. After development of the stain, the tissue section is mounted on slides, dried, before being counterstained and cover slipped before being analysed in for example a microscope.

Occasionally, the tissue section is mounted on slides following the critical incubation with the immuno-specific reagents. The rest of the staining process is then conducted on the slide mounted tissue sections.

The free-floating method has been used mainly on thick tissue sections. It is important that sections never dry out during the staining process.

Advantages of the free-floating method include even and good penetration of the immunohistochemical staining reagents. The free-floating method allows for high concentrations of reagents and good mixing.

### HISTOLOGICAL MATERIALS, TECHNIQUES AND ANALYSIS

There are in general two categories of histological materials. The reference standard described here may be suitably employed for analysis of each type.

The first category includes preparations, which are fresh tissues and/or cells, which generally are not fixed with aldehyde-based fixatives. These specimens commonly include biopsy materials, which may be analysed while the surgical procedure is in progress (frozen sections), cytological preparations (including e. g. touch preparations and blood smears), and tissues, which are to be histochemically analysed. Such specimens are either placed directly on a slide or cover slip, or frozen and sectioned onto slides. Such specimens are then fixed, usually with an alcohol- or acetone-based fixative, and stained.

The more common second category includes a fixed, paraffin-embedded tissue specimen, often archive material. These are often referred to as "formalin-fixed and paraffin-embedded" (FFPE) specimens. These specimens are fixed, usually using a formalin-based fixative, dehydrated by using for example xylene, embedded in a suitable embedding medium such as paraffin or plastic (for example Epon, Araldite, Lowicryl, LR White or polyacrylamide), sectioned onto a slide, deparaffinised or otherwise treated, rehydrated, and stained.

Biological samples may be treated using the cytological and histological techniques described here, prior to being stained and compared to the reference standard.

The sample may be purified or concentrated, or cells may be isolated prior to analysis. The sample may also be embedded into paraffin and sectioned prior to analysis. Such procedures are readily known to the person skilled in the art.

The sample may be mounted on a support. By the term "mounted" is meant placed on or attached to a substantially planar support. Any suitable support may be employed. Included is placing the tissue or cell sample on a support, for example for viewing on a microscope slide. The sample can be attached to further prevent it from falling or sliding off during handling of the support. The method of attachment to the support includes relying on the physical, capillary attraction, adhesives and chemically binding. The sample may be fixed or not fixed.

In particular, the support may be a glass slide, a membrane, a filter, a polymer slide, a chamber slide, a dish, or a petridish.

The sample or parts thereof may suitably be grown or cultured directly on the support prior to analysis. Examples of suitable culture media includes culture media of biological origin such as blood serum or tissue extract; chemically defined synthetic media; or mixtures thereof. Cell cultures are usually grown either as single layers of cells on for example a glass or plastic surface, in flasks or on chamber slides, or as a suspension in a liquid or semisolid medium. The cells can be transferred to and mounted onto a more suitable support, for example a glass slide. If grown on a chamber slide, which is suitable for for example viewing in a microscope, the cells can potentially remain on the support.

However, the cells need not be grown or cultured prior to analysis. Often the sample will be analysed directly without culturing. It is to be understood that samples for direct analysis may undergo the processing procedures described above.

The sample may, either directly or after having undergone one or more processing steps, be analysed in primarily two major types of methods, *in situ* methods (*in situ* analyses) and *in vitro* methods (*in vitro* analyses).

In this context, *in situ* methods are to be understood as assays, in which the morphology of the sample cells is essentially preserved. By "essentially preserved" is meant that the overall morphology is preserved, making it possible to identify some or all of the structural compositions of the tissue or cells. Examples are analysis of smears, biopsies, touch preparations and spreading of the sample onto the support. Samples may be subjected to i.a. fixation, permeabilisation, or other processing steps prior to analysis.

*In vitro* methods are to be understood as methods, in which the overall morphology is not preserved. In the case of *in vitro* methods, the sample is subjected to a treatment, which disrupts the morphology of the cell structure. Such treatments are known to the person skilled in the art and include treatment with organic solvents, treatment with strong chaotropic reagents such as high concentrations of guanidine thiocyanate, enzyme treatment, detergent treatment, bead beating, heat treatment, sonication and/or application of a French press.

### DETAILED PROCEDURES

The following section provides a detailed description of procedures for making FFPE samples, as well as antibody staining and detection. It is taken from the reference textbook Antibodies: A Laboratory Manual by Ed Harlow (Editor), David Lane (Editor) (1988, Cold Spring Harbor Laboratory Press, ISBN 0-87969-314-2), 1855.

### Preparing Paraformaldehyde

Standard commercial 37 % solutions of formaldehyde are stabilised with 10-15 % methanol to inhibit polymerisation of the formaldehyde to paraformaldehyde. For most fixatives, paraformaldehyde should be used in place of the commercial formaldehyde. Paraformaldehyde dissolves in water, releasing formaldehyde in the process. To prepare a 4 % solution of paraformaldehyde, add 8 g to 100 mL of water. Heat to 60°C in a fume hood. Add a few drops of 1 N NaOH to help dissolve. When the solid has completely dissolved, let the solution cool to room temperature, add 100 mL of 2x PBS. This stock solution should be prepared fresh daily.

### Preparing Paraffin Embedded Tissue Sections

1. Cut small blocks approximately 1 cm² x 0.4 cm. 2. Place in either freshly prepared 4 % paraformaldehyde or Bouin's fixative (To prepare 1 L, dissolve 2 g of picric acid in 500 mL of deionized water. Filter through Whatman No. 1, or equivalent. Add 20 g of paraformaldehyde, and heat to 60°C in a fume hood. Add a few drops of 1 N NaOH to dissolve. Cool and add 500 mL of 2x PBS). 3. Incubate for 2 hr. to overnight. 4. Follow standard paraffin embedding procedures. 5. Collect 4 µm sections onto clean glass slides. 6. Place in 60°C oven for 30 min. 7. Dewax in xylene. Change two times, 3 min. each. 8. Rehydrate by passing through graded alcohols (two changes, absolute ethanol, 3 min. each, followed by two changes, 95 % ethanol, 3 min. each). 9. Rinse in water.

Antibodies can now be applied to the specimen, as described below.

Where peroxidase labelled detection methods are to be used, it may be necessary to block or inhibit endogenous enzyme activity within the specimen before the application of antibody. To block endogenous peroxidase activity, the specimen is incubated with a solution of 4 parts methanol to 1 part of 3 % hydrogen peroxide for 20 min. Hydrogen peroxide is generally supplied as a 30 % solution and should be stored at 4°C, at which it will last about 1 month. For specimens containing high peroxidase activities, such as spleen or bone marrow, better results may be obtained by using a solution of 0.1 % phenylhydrazine hydrochloride in Phosphate Buffered Saline (PBS).

### Fixation

All fixation protocols must (1) prevent antigen leakage, (2) permeabilize the cell to allow access of the antibody, (3) keep the antigen in such a form that it can be recognised efficiently by the antibody, and (4) maintain the cell structure.

A wide range of fixatives are in common use, and the correct choice of method will depend on the nature of the antigen being examined and on the properties of the antibody preparation. Fixation methods fall generally into two classes, organic solvents and cross-linking reagents. Organic solvents such as alcohols and acetone remove lipids and dehydrate the cells, precipitating the proteins on the cellular architecture. Cross-linking reagents form intermolecular bridges, normally through free amino groups, thus creating a network of linked antigens. Both methods may denature protein antigens, and for this reason, antibodies prepared against denatured proteins may be more useful for cell staining. In some instances, anti-denatured protein antibodies are the only ones that can work.

### Fixing Attached Cells in Paraformaldehyde or Glutaraldehyde

Fixation in protein cross-linking reagents such as paraformaldehyde or glutaraldehyde preserves cell structure better than organic solvents but may reduce the antigenicity of some cell components. Simple fixation with paraformaldehyde or glutaraldehyde does not allow access of the antibody to the specimen and therefore is followed by a permeabilization step using an organic solvent or non-ionic detergent. Using the organic solvent is easy, but it can destroy certain elements of the cell architecture, although the prior fixation with paraformaldehyde does help to preserve the cellular structure. If preservation of cell structure is important, the best first choice would be to use a non-ionic detergent.
1. Prior to the staining, prepare either the paraformaldehyde or glutaraldehyde solution. For paraformaldehyde, prepare a 4 % solution. For glutaraldehyde, prepare a 1 % solution (electron microscopic grade) in PBS. Caution Glutaraldehyde is toxic. Work in a fume hood. 2. Wash the coverslip, slide, or plate gently in PBS. 3. For paraformaldehyde, incubate in a 4 % solution for 10 min. at room temperature. For glutaraldehyde, incubate in a 1 % solution for 1 hr. at room temperature in a fume hood. 4. Wash the cells twice with PBS. At this stage, cells fixed with glutaraldehyde can be removed from the fume hood. 5. Permeabilize the fixed cells by incubating in any of the following: (i) 0.2 % Triton X-100 in PBS for 2 min. at room temperature. Some antigens may need as long as 15 min. Check this for each antigen; (ii) Methanol for 2 min. at room temperature; or (iii) Acetone for 30 sec. at room temperature (for cells grown on tissue culture plates, use 50 % acetone/50 % methanol). (Optional) For glutaraldehyde, block free reactive aldehyde groups by incubating with 0.2 M ethanolamine pH 7.5 for 2 hr. at room temperature or by incubating with three changes of 5 min. each with 0.5 mg/mL sodium borohydride in PBS. In some cases this may also help paraformaldehyde-fixed cells, but in general is not necessary. 6. Rinse gently in PBS with four changes over 5 min.

The sample is now ready for the application of antibodies, as described below.

### Unmasking Hidden Epitopes with Proteases

Fixation in formaldehyde or glutaraldehyde may mask or change some epitopes. These epitopes can often be re-exposed by a gentle incubation of the sample in proteases. Trypsin works well. Incubate the specimen in a 0.1% trypsin, 0.1 % CaCl₂, 20 mM Tris pH 7.8 solution for 2-20 min. at room temperature. Stop the digestion by rinsing the specimen under the cold tap for 5 min.

### Binding Antibodies to Attached Cells

Antibodies generally are applied directly to the area of the cells of tissues that is being studied.
1. Cells are fixed and washed. Place coverslips, slides or plates in a humidified chamber. Slides or coverslips can be placed in a petri dish containing a water-saturated filter. Coverslips are best placed on a layer of parafilm; this helps to stop the antibody solution from rolling off the edge of the coverslip and makes it easy to pick up the coverslips with fine forceps, as the parafilm is compressible. 2. Add the first antibody solution. All dilutions must be carried out in protein-containing solutions. For example, use PBS containing 3 % BSA. For unlabelled primary antibodies: Monoclonal antibodies are best applied as tissue culture supernatants (specific antibody concentration of 20-50 mg/L, use neat). Ascites fluids, purified monoclonal and polyclonal antibodies, and crude polyclonal sera should be tested at a range of dilutions aimed at producing specific antibody concentrations between 0.1-10 mg/L. If the specific antibody concentration of the antibody sample is unknown, prepare and test 1/10, 1/100, 1/1000, and 1/10,000 dilutions of the starting material. For labelled primary antibodies: Primary antibodies can be labelled with enzymes, fluorochromes, or iodine. They should be assayed at several dilutions in preliminary tests to determine the correct working range. Too-high concentrations will yield high backgrounds; too-low concentration will depend on both the abundance of the antigen under study and the specificity of the antibody. 3. Incubate the coverslips, slides, or plates for a minimum of 30 min. at room temperature in the humidified chamber. For some reactions, prolonged incubations of up to 24 hr. can increase sensitivity. 4. Wash in three changes of PBS over 5 min. This buffer may be supplemented with 1 % Triton X-100 or NP-40 to help with any background problems.

If the first antibody is labelled, the specimen is now ready for the detection step. Otherwise:
5. Apply the labelled secondary reagent. It is essential to carry out all dilutions in a protein-containing solution such as 3 % BSA in PBS or 1% immunoglobulin in PBS (prepared from the same species as the detection reagent). Useful secondary reagents include anti-immunoglobulin antibodies, protein A, or protein G. They can be labelled with enzymes, fluorochromes, gold, or iodine. Labelled secondary reagents can be purchased from several suppliers or can be prepared. For enzyme-labelled reagents: If using a commercial preparation, test dilutions of the secondary antibodies 1/50 to 1/1000. Alkaline phosphatase-labelled reagents should be handled using Tris-buffered saline, not PBS. **For fluorochrome-labelled reagents:** If using commercial preparations, test dilutions between 1/10 to 1/300. **For gold-labelled reagents**: Wash the gold particles once in PBS. Dilute in PBS containing 1 % gelatin and add to the specimen. For iodine-labelled reagents: Add the iodinated antibody at approximately 0.1 mg/L. Usually, specific activities between 10 and 100 Ci/g are used.
6. Incubate with the labelled secondary reagent for a minimum of 20 min. at room temperature in the humidified chamber. For gold-labelled reagents, observe periodically under the microscope until a satisfactory signal is obtained. 7. Wash in three changes of PBS (or Tris saline) over 5 min.

The specimen is now ready for the detection step.

### Detection Using Enzyme-Labelled Reagents

Enzyme-labelled reagents are detected using soluble chromogenic substrates that precipitate following enzyme action, yielding an insoluble coloured product at the site of enzyme-localisation (Avrameas and Uriel 1966; Nakane and Pierce 1967a,b; Avrameas 1972). A range of substrates is available for each enzyme, and the following protocols represent some of the most useful alternatives. A wide range of conjugated reagents are available commercially or can be prepared as described. Enzymes can be coupled to anti-immunoglobulin antibodies, protein A, protein G, avidin, or streptavidin.

### Horseradish Peroxidase-Labelled Reagents

A range of substrates are useful, including diaminobenzidine, chloronaphthol, and aminoethylcarbazole. In preferred embodiments, the use of diaminobenzidine is indicated.

### Diaminobenzidine

Diaminobenzidine (DAB) is the most commonly used substrate and one of the most sensitive for horseradish peroxidase. It yields an intense brown product that is insoluble in both water and alcohol. DAB staining is compatible with a wide range of common histological stains.
1. Dissolve 6 mg of DAB (use DAB tetrahydrochloride) in 10 mL of 0.05 M Tris buffer pH 7.6. 2. Add 0.1 mL of 3 % solution of H₂O₂ in H₂O. H₂O₂ generally is supplied as a 30 % solution and should be stored at 4°C, at which it will last about 1 month. 3. If a precipitate appears, filter through Whatman No. 1 filter paper (or equivalent). 4. Apply to specimen, incubate for 1-20 min. Stop the reaction by washing in water. (**Optional**) Counterstain if necessary. 5. Mount in DPX.

### Diaminobenzidine/Metal

The diaminobenzidine substrate for horseradish peroxidase can be made more sensitive by adding metal salts such as cobalt or nickel to the substrate solution. The reaction product is slate gray to black, and the products are stable in both water and alcohol. DAB/metal staining is compatible with a wide range of common histological stains.
1. Dissolve 6 mg of DAB (use DAB tetrahydrochloride) in 9 mL of 0.05 M Tris buffer pH 7.6. 2. Add 1 mL of a 0.3 % W/V stock solution of nickel chloride in H₂O (the same amount of cobalt chloride can be used as an alternative). 3. Add 0.1 mL of a 3 % solution of H₂O₂ in H₂O. H₂O generally is supplied as a 30 % solution and should be stored at 4°C, at which it will last about 1 month. 4. If a precipitate appears, filter through Whatman No. 1 filter paper (or equivalent). 5. Apply to specimen, incubate for 1-20 min. Stop the reaction by washing in H₂O. (**Optional**) Counterstain if necessary. 6. Mount in DPX.

### COUPLING

The detectable entity may be coupled to the fibre by a number of methods. For example, the detectable entity may be coupled to the fibre by the use of cyanogen bromide.

Chemical crosslinkers are used to covalently modify proteins for studying ligand-receptor interactions, conformational changes in tertiary structure, or for protein labeling. Crosslinkers are divided into homobifunctional crosslinkers, containing two identical reactive groups, or heterobifunctional crosslinkers, with two different reactive groups. Heterobifunctional crosslinkers allow sequential conjugations, minimizing polymerization.

| *Homobifunctional* | | | | | |
|---|---|---|---|---|---|
| **Reagent** | **code No.** | **Modifi ed Group** | **Solubility** | **Comments** | **Refs** |
| BMME | 442635-Y | -SH | DMF, Acetone | Homobifunctional crosslinker useful for formation of conjugates via thiol groups. | Weston, P.D., et al. 1980. Biochem. Biophys Acta. **612**, 40. |
| BSOCOE S | 203851-Y | -NH2 | Water | Base cleavable crosslinker useful for studying receptors and mapping surface polypeptide antigens on lymphocytes. | Howard, A.D., et al. 1985. J. BioL Chem.**260,** 10833. |
| DSP | 322133-Y | -NH2 | Water | Thiol cleavable crosslinker used to immobilize proteins on supports containing amino groups. | Lee, W.T., and Conrad, D.H. 1985. J. Immunol.**134,** 518. |
| DSS | 322131-Y | -NH2 | Water | Non-cleavable, membrane impermeable crosslinker widely used for conjugating radiolabeled ligands to cell surface receptors and for detecting conformational changes in membrane proteins. | D'Souza, S.E., et al. 1988. J. Biol. Chem.**263,** 3943. |
| EGS | 324550-Y | -NH2 | DMSO | Hydroxylamine cleavable reagent for crosslinking and reversible immobilization of proteins through their primary amine groups. Useful for studying structure-function relationships. | Geisler, N., et al. 1992. Eur. J. Biochem.**206**, 841. 14. Moenner, M., et al. 1986. Proc. Natl. Acad. Sci. USA83, 5024. |
| EGS, Water Soluble | 324551-Y | -NH2 | Water | Water soluble version of EGS that reacts rapidly with dilute proteins at neutral pH. Crosslinked proteins are readily cleaved with hydroxylamine at pH 8.5 for 3-6 hours, 37°C. | Yanagi, T., et al. 1989. Agric. Biol. Chem.**53**, 525. |
| Glutarald ehyde | 354400-Y | -OH | Water | Used for crosslinking proteins and polyhydroxy materials. Conjugates haptens to carrier proteins; also used as a tissue fixative. | Harlow, E., and Lane, D. 1988. Antibodies: A Laboratory Manual, Cold Spring Harbor Publications, N.Y., p. 349. |
| SATA | 573100-Y | -NH2 | DMSO | Introduces protected thiols via primary amines. When treated with hydroxylamine, yields a free sulhydryl group that can be conjugated to maleimide-modified proteins. | Duncan, R.J.S., et al. 1983. Anal. Biochem.**132,** 68. |

| *Heterobifunctional* | | | | | |
|---|---|---|---|---|---|
| **Reagent** | **code No.** | **Modifi ed Group** | **Solubility** | **Comments** | **Refs** |
| GMBS | 442630-Y | -NH2, -SH | DMSO | Heterobifunctional crosslinker useful for preparing enzyme-antibody conjugates (for example □-gal-IgG) and for immobilizing enzymes on solid supports. | Kitagwa, T., et al. 1983. J. Biochem94, 1160.19. Rusin, K.M., et al. 1992. Biosens. Bioelectron.**7,** 367. |
| MBS | 442625-Y 442626-Y | -NH2, -SH -NH2, -SH | DMSO, Water | Thiol cleavable, heterobifunctional reagent especially useful for preparing peptide-carrier conjugates and conjugating toxins to antibodies. | Green, N., et al. 1982. Cell **28**, 477. |
| PMPI | 528250-Y | -SH2, -OH | DMSO, DMF | Used in the preparation of alkaline phosphatase conjugates of estradiol, progesterone, serine-enriched peptides, and vitamin B12. | Aithal, H.N., et al. 1988. J. Immunol. Methods**112,** 63. |
| SMCC | 573114-Y 573115-Y | -NH2, -SH -NH2, -SH | DMF, AN Acetonitrile Water | Heterobifunctional reagent for enzyme labeling of antibodies and antibody fragments. The cyclohexane bridge provides extra stability to the maleimide group. Ideal reagent for preserving enzyme activity and antibody specificity after coupling. | Annunziato, M.E., et al. 1993. Bioconjugate Chem.**4,** 212. |
| SPDP | 573112-Y | -NH2, -SH | DMF, AN Acetonitrile | Introduces protected thiol groups to amine groups. Thiolated proteins can be coupled to a second molecule via an iodoacetamide or maleimide group, or to a second pyridyldisulfide containing molecule. | Caruelle, D., et al. 1988. Anal. Biochem.**173,** 328. |

Each of these reagents may be obtained from a number of manufacturers, for example, from Calbiochem (code No. in column 2), or Piece Chemical Company.

The fibre may be activated prior to coupling, to increase its reactivity. In preferred embodiments, the fibre is activated using chloroacetic acid followed by coupling using EDAC/NHS-OH. Fibres may also be activated using hexane di isocyanate to give primary amino group. Such activated fibres may be used in combination with any hetero bifunctional cross linker. The fibre in certain embodiments is activated using divinyl sulfon. Such activated fibres comprise moieties which can react with amino or thiol groups, on a peptide, for example.

The fibre may also be activated using tresyl chloride, giving moieties which are capable of reacting with amino or thiol groups. The fibre may also be activated using cyanogen chlorid, giving moieties which can react with amino or thiol groups

### PEPTIDE COUPLING

In preferred embodiments, the detectable entity comprises a peptide. Coupling of peptides to fibres is described in detail in this section.

Peptides can be obtained by solid phase synthesis methods. The first stage of the technique, first introduced by Merrifield (R.B. Merrifield, Solid Phase Peptide Synthesis. The synthesis of a Tetrapeptide., J. Am. Chem. Soc. 85, page 2149-2154, (1963) and R.B. Merrifield, Solid Phase Synthesis, Science 232, page 341-347, (1986)) consists of peptide chain assembly with protected amino acid derivatives on a polymeric support. The second stage of the technique is the cleavage of the peptide from the support with the concurrent cleavage of all side chain protecting groups to give the crude free peptide. To achieve larger peptides, these processes can be repeated sequentially.

The flexibility of the method allows the synthesis of long, short and branched peptides, including peptides with natural and un-natural occurring amino acids, different linkers and so-called spacers. The spacers typically being ofpolyethylenglycol, PEG derivatives or polyalkanes or homo poly amino acids. The solid phase synthesis method allows for the preparation of peptides terminated with reactive functionalities, for example free thiols, for chemo selective coupling schemes to the fibre material.

A sequence of amino acids can be repeated in the final peptide sequence to enhance the immunoreactivity with a specific antibody. The repetitive and reactive sequence can be spaced with irrelevant amino acid sequences in a linear peptide. Also, by synthesizing branched or dendritic peptide constructs, like the multiple antigen peptides (MAP), the immuno reactivity can be enhanced.

For a review of the general methodology, including the different chemical protection schemes and solid and soluble supports, see for example G. Barany, N. Kneib-Cordonier, D. G. Mullen, Solid-phase peptide synthesis: A silver anniversary report, Int. J. Peptide Protein Res. 30, page 705-739, (1987), and G.B. Fields, R. L. Noble, Solid phase peptide synthesis utilizing 9-fluorenylmethoxycarbonyl amino acids, Int. J. Peptide Protein Res. 35, page 161-214 (1990)

Other methods for obtaining peptides include enzymatic fragment ligation, genetic engineering techniques as for example site-directed mutagenesis. Genetic engineering of oligonucleotides, PCR-products, or cloned fragments of DNA material encoding relevant amino acid sequence using standard DNA cloning techniques has been a well established methods of obtaining polypeptides. Alternatively, the peptides can be obtained after isolation from natural sources, such as by protein purification and digestion.

Conjugation of the target molecule (for example, peptide) may be achieved by forming covalent bonds or using strong binding pairs, for example ion binding, biotin-avidin. Examples of other binding entities than streptavidin, avidin and derivatives and biotin and biotin analogues, are the leucine zipper domain of AP-1 (Jun and fos), hexa-his (metal chelate moiety), hexa-hat GST (glutathione S-Transferase) glutathione affinity, trivalent vancomycin, D-Ala-D-Ala, lectines that binds to a diversity of compounds, including carbohydrates, lipids and proteins, for example Con A (Canavalia ensiformis) and WGA (Whet germ agglutinin) and tetranectin or Protein A or G. These and other methods are well known to any skilled in the art of conjugation.

Covalent conjugation confers several advantages, including increased resistance to degradation.

The coupling method useful for conjugation is dependent on the chemical structure of the target and the fibre involved. Typical chemical reagents used are so-called zero length cross linkers, homobifunctional, heterobifunctional or polymeric cross linkers.

Zero length cross linkers like 1-Ethyl-3-(3-dimethylaminopropyl) carbodiimide (EDAC) or 1-cyclohexyl-3-(2-morpholinoethyl)carbodiimide metho-p-toluenesulfonate (CHMC) and other carbodiimides can facilitate direct coupling between for example Glu or Asp to Lysine residues and for example the N terminus of a peptide.

Homobifunctional cross linkers like glutar(di)aldehydes, imidates, bis-diazotized benzidines, bis(imido esters), bis(succinimidyl esters), diisocyanates, diacid chlorides, divinylsulfone or similar, allows amino or hydroxyl groups to be bound covalent together through a short linker molecule. Formaldehyde or glutar(di)aldehyde can also facilitate cross-linking between fibre and peptide.

The use of heterobifunctional cross linkers is described in more detail for cross linking peptides to a fibre by a methology known to any skilled in the art of conjugation.

Heterobifunctional cross linkers have the advantage of providing greater control over the cross-linking than methods which rely on for example homobifunctional cross linkers.

The most common schemes for forming a heteroconjugate involve the indirect coupling of an amine group on one bio molecule to a thiol group on a second bio molecule, usually by a two- or three-step reaction sequence. The high reactivity of thiols and their relative rarity in many biomolecules make thiol groups ideal targets for controlled chemical cross-linking.

If a thiol is not present, thiolgoups can be introduced by several methods. One common method including the use of succinimidyl 3-(2-pyridyldithio)propionate (SPDP) followed by reduction of the 3-(2-pyridyldithio)propionyl conjugate with DTT or TCEP. Reduction releases the chromophore 2-pyridinethione, which can be used to determine the degree of thiolation.

Alternatively, the degree of thiolation can be measured using 5,5'-dithiobis-(2-nitrobenzoic acid) (DTNB, Ellman's reagent) which stoichiometrically yields the chromophore 5-mercapto-2-nitrobenzoic acid upon reaction with a thiol group.

Heterobifunctional cross linkers typically contain an activated carboxyl group at one end which can react with amino groups and a maleimido or Iodoacetamides group at the opposite end which reacts readily with the sulfhydryl group of cysteine residues.

Two frequently used heterobifunctional crosslinkers are N-gamma-Maleimidobutyryloxysuccinimide ester (GMBS) and Succinimidyl 4-(N-maleimidomethyl)cyclohexane-1-carbonate (SMCC).

It should be understood, that the cross linker may contain a photoactivated reactive moiety. The photo reactive moiety acts as a masked reactive group. By using a photoreaktive coupling method, it is possible to bring the target molecule into specific part of for example the fibre before using the photo reactive group for the covalent coupling.

Typically, the peptide is synthesized with a single cysteine residue at either the Nor C-termini. Alternatively, the internal Cys residues or Cys residues on a linker can be used. If the peptide contains no thiol group, then one or more can be introduced using one of several thiolation methods, typically by modifying one of the amino groups.

It should be understood that coupling of target probes, like for example peptides, are not limited by the use of thiol selective coupling schemes.

Other useful chemical moieties for both chemo selective or random conjugation schemes include carboxyl, hydroxyl, aromatic, phenolic or amino groups. Especially amino groups are useful, as they are very reactive at relevant pH, can form strong chemical bonds and are widely distributed in biological material, including silk fibres, proteins, and peptides.

The possibility to employ conjugation schemes using the amino group in the N-termini of peptides, including the amino group in the side chain of lysine or polylysine is of special relevance to the compositions and methods described here.

The cross linker is first reacted with the amino groups on the fibre, followed by removal of the unreacted cross linker using for example a decanting or centrifugation. The activated carrier is then reacted with the Cys-containing peptide. Excess peptide is removed using for example a desalting column, dialysis or centrifugation. The amount of peptide or cross linker attached can be assessed by various direct or indirect analytical methods.

The conjugation sequence can be reversed by first attaching the heterobifunctional cross linker to the peptide, before attaching to thiols on the fibre.

During conjugation reaction, the free thiols are often protected against spontaneous oxidation by the addition of EDTA, EGTA or tributylphosphine or similar or by using protective atmosphere.

Other methods of covalent cross-linking include the use of homo or heterofunctionel polymeric cross linkers. Examples of reagents include tresyl or vinylsulfone activated dextrans or activated polyacrylic acid polymers or derivatives. Especially divinyl is preferred for activation of for example hydroxyl groups on fibres, as the resulting second vinylsulfone is highly reactive towards thiols.

The amount of coupled peptide can be determined by several methods, including incorporating one beta-alanine residue immediately adjacent to the cysteine residue on the peptide. Amino acid analysis may then be used to determine the amount of beta-alanine present after purification of the resulting conjugate.

The cross linkers may offer the possibility to include a tracer or detectable moiety. This moiety can be used to measure the amount of cross linker bound to the bio molecule. The tracer can be fluorescent, radioactive, a hapten or any other detectable molecule.

### POLYMERS

In certain embodiments, the supporting medium may be in the form of an embedding medium. Such an embedding medium may be produced by polymerisation of monomers, as described in detail in this section.

Polymers made from polymerisable monomers have wide spread applications. For example, polymers are used as additives for coating applications, such as paints and adhesives.

Polymers are prepared by polymerising one or more types of polymerisable monomers, such as by emulsion polymerisation, solution polymerisation, suspension polymerisation or bulk polymerisation. The monomer(s) may be polymerised in the presence of optional ingredients such as any one of emulsifiers, stabilisers, surface active agents, initiators (such as photoinitiators), inhibitors, dispersants, oxidising agents, reducing agents, viscosity modifiers, catalysts, binders, activators, accelerators, tackifiers, plasticizers, saponification agents, chain transfer agents, surfactants, fillers, dyes, metal salts, and solvents.

There are numerous references on polymerisation of polymerisable monomers. For example, some teachings may be found in "Emulsion Polymerization: Theory and Practice" by D. C. Blackley (published by Wiley in 1975) and "Emulsion Polymerization" by F. A. Bovey *et al.* (published by Interscience Publishers in 1965). For example, a polymer can be prepared from monomers such as methyl acrylate, ethyl acrylate, butyl acrylate, 2-ethylhexyl acrylate, decyl acrylate, methyl methacrylate, ethyl methacrylate, butyl methacrylate, styrene, butadiene, ethylene, vinyl acetate, vinyl esters, C₉, C₁₀ and C₁₁ tertiary monocarboxylic acids, vinyl chloride, vinyl pyridine, vinyl pyrrolidine, vinylidene chloride, acrylonitrile, chloroprene, acrylic acid, methacrylic acid, itaconic acid, maleic acid and fumaric acid.

Examples of further teachings on polymerisation of polymerisable monomers may be found in "Vinyl and Related Polymers" by C.E. Schildknecht (New York: John Wiley & Sons 1952) and "Monomeric Acrylic Esters" by E.H. Riddle (New York: Reinhold Publishing Corp. 1954), and by A.G. Alexander (J Oil Colour Chemists' Association [1962] 45 12) and G.G. Greth and J.E. Wilson (J Appl Polymer Sci [1961] **5** 135).

More recent teachings regarding polymerisation methods may be found in EP-A-0622378, EP-A-0634428, EP-A-0623632, EP-A-0635522, EP-A-0633273, EP-A-0632157, EP-A-0630908, EP-A-0630641, EP-A-0628614, EP-A-0628610, EP-A-0622449, EP-A-0626430 and EP-A-0625529.

By the term "cross-linker" we mean a compound which increases the degree of cross-linking of a monomer during polymerisation when compared to the polymerisation of the monomer in the absence of the cross-linker.

The monomers may be provided in the form of a polymerisable composition. The polymerisable composition may also comprise conventional additional components such as any one or more of emulsifiers, stabilisers, surface active agents, initiators (such as photoinitiators), inhibitors, dispersants, oxidising agents, reducing agents, viscosity modifiers, catalysts, binders, activators, accelerators, tackifiers, plasticizers, saponification agents, chain transfer agents, cross-linking agents, surfactants, fillers, dyes, metal salts, and solvents.

By way of example, the surfactants and dispersants can be salts of fatty rosin and naphthenic acids, condensation products of naphthalene sulphonic acid and formaldehyde of low molecular weight, carboxylic polymers and copolymers of the appropriate hydrophile-lipophile balance, higher alkyl sulfates, such as sodium lauryl sulfate, alkyl aryl sulfonates, such as dodecylbenzene sulfonate, sodium or potassium isopropylbenzene sulfonates or isopropylnaphthalene sulfonates; sulfosuccinates, such as sodium dioctylsulfosuccinate alkali metal higher alkyl sulfosuccinates, *for example* sodium octyl sulfosuccinate, sodium N-methyl-N-palmitoyl-taurate, sodium oleyl isethionate, alkali metal salts of alkylarylpolyethoxyethanol sulfates or sulfonates, *for example* sodium t-octylphenoxy-polyethoxyethyl sulfate having 1 to 5 oxyethylene units. Typical polymerisation inhibitors that can be used include hydroquinone, monomethyl ether, benzoquinone, phenothiazine and methylene blue.

In a preferred embodiment the dye is selected from the group consisting of 2-hydroxybenzophenone, oxidiazoles, salicylic acid, resorcinol monobenzoate, benzotriazole, preferably 2H-benzotriazole, benzothiazoloazine, preferably 2N-benzothiazoloazine, α-cyano-β-phenylcinnamic acid, polyalkypiperidine and derivatives thereof.

Preferably, the dye is selected from benzotriazole, in particular 2H-benzotriazole and derivatives thereof.

The composition may comprise one or more additional comonomers. Examples of the one or more additional comonomers that can be used include one of: (alkyl and cycloalkyl) acrylates; (alkyl and cycloalkyl) methacrylates; free-radical polymerisable olefinic acids, including alkoxy-, alkylphenpxy-, alkylphenoxy-(polyethyleneoxide)-, vinyl ester-, amine substituted (including quaternary ammonium salts thereof), nitrile-, halo-, hydroxy-, and acid substituted (for example phospho- or sulpho-) derivatives thereof; and other suitable ethylenically unsaturated polymerisable moieties; including combinations thereof. Preferably the alkyl and cycloalkyl groups contain up to 20 carbon atoms, for example (C₁-C₂₀ alkyl and C₁-C₂₀ cycloalkyl) acrylates, and (C₁-C₂₀ alkyl and C₁-C₂₀ cycloalkyl) methacrylates. In more detail, typical comonomers include any one of methyl acrylate, ethyl acrylate, n-propyl acrylate, isopropyl acrylate, n-butyl acrylate, isobutyl acrylate, t-butyl acrylate, isobomyl acrylate, pentyl acrylate, hexyl acrylate, octyl acrylate, iso-octyl acrylate, nonyl acrylate, lauryl acrylate, stearyl acrylate, eicosyl acrylate, 2-ethylhexyl acrylate, cyclohexyl acrylate, cycloheptyl acrylate, methyl methacrylate, ethyl methacrylate, hydroxymethylacrylate, hydroxymethylmethacrylate, propyl methacrylate, n-butyl methacrylate, t-butyl methacrylate, isobutyl methacrylate, pentyl methacrylate, hexyl methacrylate, cyclohexyl methacrylate, 2-ethylhexyl methacrylate, isobornyl methacrylate, heptyl methacrylate, cycloheptyl methacrylate, octyl methacrylate, iso-octyl methacrylate, nonyl methacrylate, decyl methacrylate, lauryl methacrylate, eicosyl methacrylate, dodecyl acrylate, pentadecyl acrylate, cetyl acrylate, stearyl acrylate, eicosyl acrylate, isodecyl acrylate, vinyl stearate, nonylphenoxy-(ethyleneoxide)₁₋₂₀ acrylate, octadecene, hexadecene, tetradecene, dodecene, dodecyl methacrylate, pentadecyl methacrylate, cetyl methacrylate, stearyl methacrylate, eicosyl methacrylate, isodecyl methacrylate, nonylphenoxy-(ethyloneoxide)₁₋₂₀ methacrylate, acrylic acid, methacrylic acid, fumaric acid, crotonic acid, itaconic acid, fumaric anhydride, crotonic anhydride, itaconic anhydride, maleic acid, maleic anhydride, styrene, alpha-methyl styrene, vinyl toluene, acrylonitrile, methacrylonitrile, ethylene, vinyl acetate, vinyl chloride, vinylidene chloride, acrylamide, methacrylamide, methacrylamide 2-cyanoethyl acrylate, 2-cyanoethyl methacrylate, dimethylaminoethyl methacrylate, dimethylaminopropyl methacrylate t-butylaminoethyl methacrylate, glycidyl acrylate, glycidyl methacrylate, glyceryl acrylate, glyceryl methacrylate, benzyl acrylate, benzyl methacrylate, phenyl acrylate, phenyl methacrylate, vinyl pyridine, vinyl pyrrolidine, siloxanes, silanes and mixtures thereof. Other polymerisable monomers are disclosed in US-A-2879178, US-A-3037006, US-A-3502627, US-A-3037969 and US-A-3497485.

Preferred comonomers include any one of glyceryl methacrylate (GMA), (2,2 dimethyl-1,3-dioxolan-4-yl) methyl methacrylate (GMAK), hydroxy ethyl methacrylate (HEMA), methacrylic acid, acrylic acid, GYMA, N-vinyl pyrrolidone, alkyl methacrylates (such as C₁₋₂₀ alkyl methacrylates, more preferably C₁₋₁₅ alkyl methacrylates, more preferably C₁₋₁₀ alkyl methacrylates, more preferably C₁₋₅ alkyl methacrylates, such as methyl methacrylate), alkyl acrylates (such as C₁₋₂₀ alkyl acrylates, more preferably C₁₋₁₅ alkyl acrylates, more preferably C₁₋₁₀ alkyl acrylates, more preferably C₁₋₅ alkyl acrylates, such as methyl acrylate), aryl methacrylates, aryl acrylates, diacetone acrylamide, acrylamide, methacrylamide, N-alkyl acrylamides (such as C₁₋₂₀ N-alkyl acrylamides, more preferably C₁₋₁₅ N-alkyl acrylamides, more preferably C₁₋₁₀ N-alkyl acrylamides, more preferably C₁₋₅ N-alkyl acrylamides, such as methyl acrylamide), N-alkyl methacrylamides (such as C₁₋₂₀ N-alkyl methacrylamides, more preferably C₁₋₁₅ N-alkyl methacrylamides, more preferably C₁₋₁₀ N-alkyl methacrylamides, more preferably C₁₋₅ N-alkyl methacrylamides, such as methyl methacrylamide), vinyl acetate, vinyl esters, styrene, other substituted olefins, N-dialkyl acrylamides (such as C₁₋₂₀ N-dialkyl acrylamides, more preferably C₁₋₁₅ N-dialkyl acrylamides, more preferably C₁₋₁₀ N-dialkyl acrylamides, more preferably C₁₋₅ N-dialkyl acrylamides, such as N N dimethyl acrylamide), N-dialkyl methacrylamides (such as C₁₋₂₀ N-dialkyl methacrylamides, more preferably C₁₋₁₅ N-dialkyl methacrylamides, more preferably C₁₋₁₀ N-dialkyl methacrylamides, more preferably C₁₋₅ N-dialkyl methacrylamides, such as N N dimethyl methacrylamide), 3-methacryloxypropyl tris (trimethysilyl siloxy) silane (TRIS monomer), fluoro substituted alkyl and aryl acrylates and methacrylates (preferably wherein the alkyl is C₁₋₂₀ alkyl, more preferably C₁₋₁₅ alkyl, more preferably C₁₋₁₀ alkyl, more preferably C₁₋₅ alkyl), and combinations thereof.

More preferred comonomers include any one of glyceryl methacrylate (GMA), (2,2 dimethyl-1,3-dioxolan-4-yl) methyl methacrylate (GMAK), 2-hydroxy ethyl methacrylate (2-HEMA), methacrylic acid, acrylic acid and glycidyl methacrylate, or combinations thereof.

The lists of comonomers also include substituted derivatives of those monomers, such as halogenated monomers, especially fluorinated monomer derivatives, and acetal and ketal derivatives.

The polymerisable monomer of the composition and the one or more additional comonomers may be selected so that the composition consists essentially of GMA and HEMA.

Any typical, suitable polymerisation method may be used. The preferred method is free radical polymerisation, thermal or UV initiated.

### FURTHER ASPECTS

Further aspects and embodiments of the invention are now set out in the following numbered Paragraphs; it is to be understood that the invention encompasses these aspects:
Paragraph 1. A reference standard for a detectable entity, the reference standard comprising: (a) a support medium; and (b) a quantity of a detectable entity supported by the support medium; in which the detectable entity has an elongate path;in which a detectable amount of the detectable entity is present in a defined region in a cross section of the reference standard.
Paragraph 2. A reference standard according to Paragraph 1, in which the defined region is present in at least one other cross section of the reference standard, preferably comprising a similar amount of detectable entity.
Paragraph 3. A reference standard according to Paragraph 1 or 2, in which the detectable entity has a substantially uniform distribution along the elongate path.
Paragraph 4. A reference standard according to Paragraph 1, 2 or 3, in which the support medium comprises an embedding medium, in which the detectable entity is embedded.
Paragraph 5. A reference standard according to any preceding Paragraph, in which the detectable entity is substantially free of cellular material.
Paragraph 6. A reference standard according to any preceding Paragraph, in which the detectable entity comprises a diagnostically relevant target.
Paragraph 7. A reference standard according to any preceding Paragraph, in which the detectable entity comprises an antigen, an epitope, a peptide, a polypeptide, a protein, a nucleic acid, or two or more or a plurality of any of the above, or combinations of one or more of the above.
Paragraph 8. A reference standard according to any preceding Paragraph, in which the presence and/or quantity of the detectable entity is revealable by a binding agent, preferably a labelled binding agent.
Paragraph 9. A reference standard according to Paragraph 8, in which the binding agent is selected from the group consisting of: an antibody, preferably an antibody capable of specific binding to the detectable entity, a nucleic acid such as a DNA or an RNA, preferably a nucleic acid capable of specific binding to the detectable entity, a protein nucleic acid (PNA), a dye, a special stain, Haematoxylin-Eosin (H & E), Gomori methenamine silver stain (GMS), Periodic Acid-Schiff (PAS) stain, Trichrome Blue, Masson's Trichrome, Prussian Blue, Giemsa, Diff-Quik, Reticulum, Congo Red, Alcian Blue, Steiner, AFB, PAP, Gram, Mucicarmine, Verhoeff-van Gieson, Elastic, Carbol Fuchsin and Golgi's stains.
Paragraph 10. A reference standard according to any preceding Paragraph, in which the presence of the detectable entity in a cell, tissue, organ or organism is indicative of a disease or a condition.
Paragraph 11. A reference standard according to any preceding Paragraph, in which the detectable entity is present at substantially the same area, quantity or concentration in all transverse planar sections of the reference standard.
Paragraph 12. A reference standard according to any preceding Paragraph, in which the defined region includes a reference area, the reference area comprising the detectable entity at a pre-defined amount.
Paragraph 13. A reference standard according to Paragraph 11, in which the amount of the detectable entity in the reference area is compared to the amount of the detectable entity in a sample to determine the presence, quantity or concentration of the detectable entity in the sample.
Paragraph 14. A reference standard according to any preceding Paragraph, in which the reference standard is in the shape of a rectangular box, and the detectable entity is in the form of a substantially linear rod disposed along or substantially parallel to a long axis of the reference standard.
Paragraph 15. A reference standard according to any preceding Paragraph, in which the detectable entity is attached, preferably chemically coupled, to an elongate fibre.
Paragraph 16. A reference standard for a detectable entity, comprising: (a) an embedding medium in a preferably substantially rectangular box shape; and (b) elongate fibre with a quantity of detectable entity coupled thereto; in which the elongate fibre is embedded lengthwise in the embedding medium.
Paragraph 17. A reference standard according to Paragraph 14 or 15, in which the fibre is selected from the group consisting of: a polyamide fibre, a cellulose fibre, a polycarbamate fibre, a silk fibre, a polyester fibre, a Nylon fibre, a Rayon fibre, and blends thereof.
Paragraph 18. A reference standard according to Paragraph 14, 15 or 16, in which the detectable entity is substantially uniform at both core and surface portions of the fibre.
Paragraph 19. A reference standard according to Paragraph 14, 15 or 16, in which the detectable entity at surface portions of the fibre is present in greater amount than at core portions.
Paragraph 20. A reference standard according to any of Paragraphs 14, 15 or 16, in which core portions of the fibre comprise substantially no detectable entity.
Paragraph 21. A reference standard according to any of Paragraphs 1 to 13, in which the detectable entity is formed in an elongate channel in the embedding medium.
Paragraph 22. A reference standard according to any of Paragraphs 14 to 21, in which the fibre or channel has a uniform cross sectional area across substantially its entire length.
Paragraph 23. A reference standard according to any of Paragraphs 14 to 22, in which the fibre or channel has a diameter of between about 0.5µm to 100µm, preferably between 2µm to 20µm.
Paragraph 24. A reference standard according to any preceding Paragraph, which comprises two or more linear fibres or channels in substantially parallel orientation, preferably in a bundle.
Paragraph 25. A reference standard according to any preceding Paragraph, which comprises two or more different detectable entities, each of which is disposed in or on an individual fibre or channel.
Paragraph 26. A reference standard according to any preceding Paragraph, comprising two or more fibres or channels each comprising the same detectable entity.
Paragraph 27. A reference standard according to any preceding Paragraph, comprising two or more fibres or channels comprising different amounts of detectable entity on each.
Paragraph 28. A reference standard according to any preceding Paragraph, in which a planar section of the reference standard comprises a plurality of areas on which are presented the detectable entity at different density.
Paragraph 29. A reference standard according to any preceding Paragraph, in which a planar section of the reference standard comprises a first area comprising the detectable entity substantially at a diagnostically significant density.
Paragraph 30. A reference standard according to any preceding Paragraph, further comprising a control comprising a fibre or channel which comprises substantially no detectable entity.
Paragraph 31. A reference standard according to any preceding Paragraph, in which the detectable entity is selected from the group consisting of: a hapten, a biologically active molecule, an antigen, an epitope, a protein, a polypeptide, a peptide, an antibody, a nucleic acid, a virus, a virus-like particle, a nucleotide, a ribonucleotide, a deoxyribonucleotide, a modified deoxyribonucleotide, a heteroduplex, a nanoparticle, a synthetic analogue of a nucleotide, a synthetic analogue of a ribonucleotide, a modified nucleotide, a modified ribonucleotide, an amino acid, an amino acid analogue, a modified amino acid, a modified amino acid analogue, a steroid, a proteoglycan, a lipid, a carbohydrate, a dye, and mixtures, fusions, combinations or conjugates of the above.
Paragraph 32. A reference standard according to any preceding Paragraph, in which the detectable entity comprises any one or more of HER2, ER, PR, p16, Ki-67 and EGFR protein, and nucleic acids encoding such.
Paragraph 33. A reference standard according to any preceding Paragraph, in which the embedding medium is selected from the group consisting of: ice, wax, paraffin, acrylic resin, methacrylate resin, epoxy, Epon, Araldite, Lowicryl, K4M and LR White and Durcupan.
Paragraph 34. A reference standard for a detectable entity comprising an surrounding medium together with a quantity of detectable entity located in the surrounding medium in a defined amount, in which the detectable entity is disposed in a generally lengthwise configuration in the surrounding medium.
Paragraph 35. An amount of detectable agent arranged in a defined volume in a matrix, in which the agent is substantially free from cellular materials, such that a planar section of the matrix presents the detectable agent in a pre-determined quantity.
Paragraph 36. A kit comprising a reference standard according to any preceding Paragraph, together with a binding agent capable of specific binding to the detectable entity, optionally together with instructions for use.
Paragraph 37. A planar section, preferably a transverse planar section, preferably of substantially uniform thickness, of a reference standard according to any preceding Paragraph.
Paragraph 38. A support, preferably a slide such as a microscope slide, comprising a planar section according to Paragraph 37 mounted thereon.
Paragraph 39. A reference standard, kit or a planar section according to any preceding Paragraph, in which the reference standard has been stained, preferably with an antibody or a nucleic acid probe.
Paragraph 40. A diagnostic kit for detecting the presence or amount of a detectable entity in a biological sample, comprising: (a) a reference standard, planar section or slide according to any preceding Paragraph; (b) a binding agent capable of specific binding to the detectable entity; and optionally (c) instructions for use.
Paragraph 41. A combination of a reference standard, planar section, slide or diagnostic kit according to any of Paragraphs 1 to 40 together with a therapeutic agent capable of treating or alleviating at least one of the symptoms of a disease or condition in an individual.
Paragraph 42. A combination according to Paragraph 41, in which the individual is diagnosed as suffering from or susceptible to the disease or condition, if the amount of detectable entity in the biological sample or component is similar to or greater than that in the reference standard.
Paragraph 43. A diagnostic kit according to Paragraph 40 or a combination according to Paragraph 41 or 42, in which the binding agent or therapeutic agent comprises an antibody against the detectable entity.
Paragraph 44. Use of a reference standard, a planar section or a kit according to any preceding Paragraph, for determining the presence or amount of a detectable entity in a biological sample.
Paragraph 45. A method of comparing the amount of a detectable entity in a biological sample with a reference standard, the method comprising the steps of: (a) providing a biological sample and obtaining a first signal indicative of the amount of detectable entity in the biological sample, or a component thereof; (b) providing a reference standard, a planar section or a kit according to any of Paragraphs 1 to 39; (c) obtaining a second reference signal indicative of the amount of detectable entity in the reference standard or planar section thereof; and (d) comparing the first signal obtained in (a) against the reference signal.
Paragraph 46. A method according to Paragraph 45, in which the detectable signal is selected from the group consisting of: radiation, optical density, reflectance, radioactivity, fluorescence, enzymatic activity.
Paragraph 47. A method according to Paragraph 45 or 46, in which the reference standard or planar section thereof is subjected to the same one or more steps or conditions, preferably substantially all, as the biological sample.
Paragraph 48. A method according to Paragraph 45, 46 or 47, in which the reference standard or planar section thereof is processed through one or more, preferably all, of the following steps: mounting onto a slide, baking, deparaffination, rehydration, antigen retrieval, blocking, exposure to antibody, exposure to primary antibody, exposure to nucleic acid probe, washing, exposure to secondary antibody-enzyme conjugate, exposure to enzyme substrate, exposure to chromogen substrate, and counter staining.
Paragraph 49. A method or use according to Paragraphs 45 to 48, in which the biological sample comprises a cell, tissue or organ, preferably a cell, tissue or organ of an organism suspected of suffering a disease or condition.
Paragraph 50. A method of diagnosis of a disease or a condition in an individual, the method comprising the steps of: (a) obtaining a biological sample from the individual; and (b) comparing the amount of a detectable entity in a biological sample or component thereof with a reference standard, in a method according to Paragraph 43; in which the individual is diagnosed as suffering from or susceptible to the disease or condition, if the amount of detectable entity in the biological sample or component is similar to or greater than that in the reference standard.
Paragraph 51. A method of treatment of a disease or a condition in an individual, the method comprising the steps of diagnosing the disease or condition in an individual in a method according to Paragraph 50, and administering a therapeutic agent to the individual.
Paragraph 52. A method of treatment according to Paragraph 51, in which the therapeutic agent comprises an antibody capable of binding to the detectable entity.
Paragraph 53. A method of producing a reference standard for a detectable entity, the method comprising the steps of: (a) providing a support medium; and (b) supporting a quantity of detectable entity therein in an elongate path.
Paragraph 54. A method according to Paragraph 53, which further comprises supporting a quantity of a second detectable entity in the elongate path, or in a second elongate path.
Paragraph 55. A method according to Paragraph 53 or 54, which further comprises supporting a second different quantity of the or each detectable entity in the elongate path, or in a second or further elongate path.
Paragraph 56. A method according to Paragraph 53, 54 or 55, in which the support medium comprises an embedding medium, and the or each detectable entity is supported by embedding in the embedding medium.
Paragraph 57. A method according to any of Paragraphs 53 to 56, which further comprises one or more steps selected from the following: (a) forming a quantity of a second detectable entity in a generally elongate path, and embedding the second detectable entity in the embedding medium; and (b) forming a second different quantity of the detectable entity in a generally elongate path, and embedding the second quantity of detectable entity in the embedding medium.
Paragraph 58. A method according to any of Paragraphs 53 to 57, in which the elongate path of the or each detectable entity is defined by attaching, preferably covalently coupling, the or each detectable entity to an elongate fibre.
Paragraph 59. A method according to Paragraph 58, in which the or each detectable entity is allowed to attach to surface portions, but not substantially to core portions, of the fibre.
Paragraph 60. A method according to Paragraph 58 or 59, in which the fibre is allowed to at least partially swell prior to or during attachment of detectable entity, such that the or each detectable entity is capable of accessing and covalently coupling to core portions of the fibre.
Paragraph 61. A method according to Paragraph 58, 59 or 60, in which the or each detectable entity is attached to the fibre by being covalently coupled to polymer chains attached to the surface of the fibre.
Paragraph 62. A method of producing a reference standard for a detectable entity, the method comprising the steps of providing an elongate fibre, attaching or covalently coupling a quantity of detectable entity to the fibre, and embedding the fibre in a lengthwise orientation in an embedding medium.
Paragraph 63. A method according to any of Paragraphs 58 to 62, in which the fibre is selected from the group consisting of: a polyamide fibre, a cellulose fibre, a polycarbamate fibre, a silk fibre, a polyester fibre, a Nylon fibre, a Rayon fibre, and blends thereof.
Paragraph 64. A method according to any of Paragraphs 53 to 57, in which the elongate path of the or each detectable entity in step (b) is defined by forming a channel in the embedding medium, and filling the channel with detectable entity.
Paragraph 65. A method according to Paragraph 64, in which detectable entity in liquid form, preferably in a solution of agarose or agar, is injected into the channel and allowed to solidify.
Paragraph 66. A method according to any of Paragraphs 53 to 55, in which the embedding medium is selected from the group consisting of: ice, wax, paraffin, acrylic resin, methacrylate resin, epoxy, Epon, Araldite, Lowicryl, K4M and LR White and Durcupan.
Paragraph 67. A reference standard for a detectable entity, the reference standard comprising: (a) a support medium; and (b) a quantity of a detectable entity supported by the support medium; in which the detectable entity has an elongate path.
Paragraph 68. A reference standard according to Paragraph 67, in which a detectable amount of the detectable entity is present in a defined region in a cross section of the reference standard.
Paragraph 69. A reference standard according to any of Paragraphs 2 to 33, 67 or 68, in which the detectable amount of the detectable entity in at least two of the cross sections is similar.
Paragraph 70. A reference standard according to any of Paragraphs 2 to 33, 67, 68 or 69, in which the detectable amount of the detectable entity in each cross section is similar.
Paragraph 71. A reference standard for a detectable entity, comprising: (a) an embedding medium; (b) a quantity of a detectable entity disposed therein in a generally elongate path.
Paragraph 72. A reference standard according to Paragraph 71, in which the detectable entity is present in a defined area, quantity or concentration in a cross section.
Paragraph 73. A method of producing a reference standard for a detectable entity, the method comprising the steps of: (a) providing an embedding medium; (b) forming a quantity of detectable entity in a generally elongate path; and (c) embedding the detectable entity in the embedding medium.
Paragraph 74. A reference standard for a detectable entity, the reference standard comprising: (a) a support medium; and (b) a quantity of a detectable entity supported by the support medium; in which the detectable entity comprises non-cellular components and / or non-cellular material.
Paragraph 75. A reference standard according to Paragraph 74, in which a detectable amount of the detectable entity is present in a defined region in a cross section of the reference standard.
Paragraph 76. A reference standard as Paragraphed in Paragraph 74 or 75, further comprising any of the features of Paragraphs 2 to 34, 69 or 70.
Paragraph 77. A method of assessing the effectiveness or success of a procedure, the method comprising the steps of: (a) providing a reference standard according to any preceding Paragraph, in which a detectable property of the detectable entity is changed as a result of the procedure; (b) conducting the procedure on the reference standard; and (c) detecting a change in the detectable property of the detectable entity.
Paragraph 78. A method according to Paragraph 77, in which a detectable property of the detectable entity is changed as a result of a successful procedure, which change in the detectable property of the detectable entity is detected to establish that the procedure is successful.
Paragraph 79. A method according to Paragraph 77, in which a detectable property of the detectable entity is changed as a result of an unsuccessful procedure, which change in the detectable property of the detectable entity is detected to establish that the procedure is not successful.
Paragraph 80. A method of validating a procedure according to Paragraph 77, 78 or 79, in which the procedure is selected from the group consisting of: an *in situ* hybridisation procedure, an immunohistochemical procedure, deparaffination, antigen retrieval, blocking, endogenous biotin blocking, endogenous enzyme blocking, a washing step, incubation with revealing agent such as a primary antibody, incubation with secondary visualisation components, chromogen staining, staining information acquisition and analysis.
Paragraph 81. A method according to Paragraph 77, 78, 79 or 80, in which the procedure is an antigen retrieval procedure, and in which the detectable property of the detectable entity comprises the masking or unmasking of one or more epitopes.
Paragraph 82. A method according to Paragraph 77, 78, 79, 80 or 81, in which the detectable entity in the reference standard is modified to mask one or more epitopes, some or all of which are unmasked in an antigen retrieval procedure which is successful.
Paragraph 83. A method according to Paragraph 77, 78, 79 or 80, in which the procedure is an deparaffination procedure, and in which the detectable property of the detectable entity comprises the presence or quantity of detectable entity in the reference standard following the deparaffination procedure.
Paragraph 84. A method according to Paragraph 77, 78, 79, 80 or 83, in which the detectable entity in the reference standard is soluble in the deparaffination medium, and in which at least a portion, preferably all, of the detectable entity is removed following a successful deparaffination procedure.
Paragraph 85. Use of a reference standard as Paragraphed in any preceding Paragraph as an antigen retrieval validation standard, a deparaffination standard, a blocking validation standard, a washing validation standard, a primary antibody validation standard, a secondary antibody validation standard, a calibration standard, or a diagnostic standard.
Paragraph 86. A reference standard comprising a detectable entity localised in an elongate path in a support medium.
Paragraph 87. A reference standard according to Paragraph 86, further comprising any one or more of the features set out above in Paragraphs 1 to 85.
Paragraph 88. A reference standard, or a method of producing such, or a method using a reference standard according to any preceding Paragraph, in which the detectable entity spaced from an elongate fibre to which it is attached by spacer means, such as a spacer or coupler.
Paragraph 89. A reference standard substantially as hereinbefore described with reference to and as shown in Figures 2 to 38 of the accompanying drawings.
Paragraph 90. A planar section preferably of substantially uniform thickness of a reference standard substantially as hereinbefore described with reference to and as shown in Figures 2 to 38 of the accompanying drawings.
Paragraph 91. Use of a reference standard or a planar section for determining the presence or amount of a detectable entity in a biological sample, such use substantially as hereinbefore described with reference to and as shown in Figures 2 to 38 of the accompanying drawings.
Paragraph 92. A method of determining the amount of a detectable entity in a biological sample substantially as hereinbefore described with reference to and as shown in Figures 2 to 38 of the accompanying drawings.
Paragraph 93. A method of diagnosis of a disease or a condition in an individual substantially as hereinbefore described with reference to and as shown in Figures 2 to 38 of the accompanying drawings.

### EXAMPLES

To illustrate the general utility and some of the general aspects described here, several reference materials have been prepared and evaluated, including the use of various fibre materials, attachment of haptens, dyes, and methods for embedding the material to allow good attachment to slides.

The reference material has been made as paraffin embedded (FFPE) preparations and evaluated in a bright field microscope.

### Example 1. Preparation of Fluorescein and DNP Reference Material

The following Example describes the preparation of fluorescein and dinitrophenyl (DNP) containing model reference material: using three different fibres, and coupled in aqueous or organic solvents at different concentrations to illustrate i) variation in staining intensity and ii) localization of the stain by swelling of the fibres.

Both the fluorescein and the Dinitrophenyl moiety are well known general haptens for diagnostic assays. The haptens are both recognizable using specific antibodies.

The model haptens are selected to illustrate the use of two different coupling chemistries: the activated aryl halogen and the iso thio cyanate reactive groups.

### The three different fibres used are:

Unifloss fibres (50 % nylon, 50 % rayon, 15 Yds, 600 1X, single strand floss, blue, UNI Products J.G. Côté inc., QC, Canada, obtained from LP Fly Shop, DK-8900 Randers, Denmark),

LP floss fibres (Rayon silk, cellulose, white, LP Fly Shop, DK-8900 Randers, Denmark), and

Lagertun fibres (natural silk, white, obtained from LP Fly Shop, Made in France, spooled in USA, Multi strand silk 8 Yds)

All the fibres are cut to 40 cm in length and knots tied at the ends to avoid the fibre bundles to roll up and to identify them from each others.

The three different fibres are put in the same reaction vessels (5 mL dark glass bottles with screw caps) in each of the following four coupling experiments:

### 1A. Coupling of DNP in Aqueous Solution

A 1.0 M stock solution of Sanger's reagent, (2,4 dinitro fluoro benzene, F-DNP, Sigma code No. D 1529; 36.4 mg in 200 µL dry N-Methyl-Pyrrilidone, NMP, Lab-Scan, code No C 2538) is prepared.

Water, NMP and carbonate buffer, pH 9.0 are added to the three reaction vessels with fibres and allowed to swell for 20 min. before Sanger's reagent is added from the stock solution.

The total reaction solutions (3.0 mL) comprise 50 mM carbonate, pH 9.0, 20 % NMP in water and 5, 10 or 25 mM F-DNP, respectively.

After 18 hr. in a 30°C water bath, the nine fibres are washed twice with HEPES buffer (10 mM HEPES, Sigma code No. H-3375, 100 mM NaCl, pH 7.0) for 2 min., followed by washing twice in deionized water and four times with absolute ethanol.

The Unifloss and LP floss fibres are unchanged from the unmodified fibres, whereas the Lagertun fibres for all 3 preparations are more yellow than the unmodified fibre.

### 1B. Coupling of DNP in Organic Solution

Toluene (Lab-Scan, code No. C 2522), NMP and triethylamine (Aldrich code No. 13,206-0) is added to two reaction vessels with fibres and allowed to swell for 20 min. before Sanger's reagent is added from the stock solution.

The total reaction solutions (3.0 mL) comprise 10 mM triethylamine, 20 % NMP in toluene and 5 or 10 mM F-DNP, respectively.

After 18 hr. in a 30°C water bath, the six fibres are washed once with NMP for 2 min., followed by washing four times with toluene and four times with absolute ethanol.

All six fibres are visually unchanged, as compared to the unmodified fibres.

### 1C. Coupling of FITC in Aqueous Solution

A 1.0 M stock solution of FITC, (Fluorescein isothiocyanate isomer 1, Molecular Probes, Eugene, USA code. No. F-1906) 65.1 mg in 170 µL NMP is prepared.

Water, NMP and carbonate buffer are added to three reaction vessels with fibres and allowed to swell for 20 min. before FITC is added from the stock solution.

The total reaction solutions (3.0 mL) is 50 mM carbonate, pH 9.0, 20 % NMP in water and 5, 10 or 25 mM FITC, respectively. Reaction time, conditions and washing are as for Example 1A.

The resulting Unifloss fibres appeared olive green, and the LP floss fibres and Lagertun fibres light yellow for all 3 preparations, as compared to the unmodified fibres.

### 1D. Coupling of FITC in Organic Solution

Toluene, NMP and triethylamine are added to two reaction vessels with fibres and allowed to swell for 20 min. before FITC is added from the stock solution.

The total reaction solutions (3.0 mL) comprises 10 mM triethylamine, 20 % NMP in toluene and 5 or 10 mM FITC, respectively. Reaction time, conditions and washing are as for Example 1B.

All six fibres are visually unchanged, as compared to the unmodified fibres.

All the 30 fibre preparations are stored in absolute ethanol (2-8°C) before being embedded, cut, stained and examined.

Unmodified Unifloss, LP floss and Lagertun fibres are stored in a similar fashion as negative controls.

### Example 2. Embedding of Reference Material

The following Example describes the general method of preparing paraffin blocks using different fibres, including the fibres prepared in Example 1.

The modified or unmodified fibres are mounted on a 9 x 9 cm steel frame (Figure 11A) and placed in a 10 x 10 x 2 cm metal container with a steel basket to help remove the gel with later (Figure 11B).

An agarose solution (80 mL, 2 % by weight in deionized water, HSA 1000 protein grade, FMC BioPolymer/Litex, obtained from Medinova Scientific A/S, Hellerup, Denmark) is heated to 60°C in a water bath before being poured over the fibres (Figure 11C).

The gel solution is allowed to cool to room temperature and solidify.

The resulting solidified gel is taken out of the container (Figure 11D) and cut from the steel frame using a surgical knife. The gel slab is further cut into approximately 5 x 5 mm thick rectangular pieces in approximately 1.5-2.0 cm in length (Figure 11E). The fibres are embedded in the centre of the agarose gel pieces.

Each gel piece is transferred to a container (30 mL Polystyrene Nunc/Nalgene test tube) with Neutral buffered formalin, NBF (20 mL, 50 mM Tris-HCl, 0,15 M NaCl, pH 7,6, adjusted to 4 % formaldehyde from a 37 % Formaldehyde (Merck code No. 4003), 10 % Methanol stock solution) and left overnight in a ventilated laboratory hood (18 hr., at room temperature)

The formalin fixed agarose gel embedded fibres are dehydrated by placing each in a marked histocapsule (Sekura ProHosp: Mega-cassette code No. 59040, approximately 32x26x10 mm), and successively placing the capsules in 70 % ethanol for 15 min., twice in 96 % ethanol for 15 min., twice in 99 % ethanol for 15 min. and twice in xylene for 15 min.

All the shrunk and slightly yellowish gels pieces are transferred to melted paraffin (Merck code No. 7337.9020, melting point 56-58°C) and left over night at 60°C.)

The gel pieces are transferred to fresh warm paraffin and left there for additional two hours before being embedded with paraffin in a cast (Sekura, ProHosp 4166 mega, approximately 31x23x13, 55 mm) and cooled to form the final paraffin blocks.

The marked paraffin blocks containing the embedded fibres are stored at room temperature before being cut, mounted and stained.

### Example 3. Immuno Staining of Fluorescein and DNP Reference Material

The following Example describes the cutting and the staining of fluorescein and DNP modified model reference material with graded staining intensity and controlled localization of the stain.

Paraffin blocks from Example 2 are mounted in a microtome (Leica 0355 model RM2065, Feather S35 knives, set at 5 micrometer) with the lengths of the fibres perpendicular to the cutting direction. The first few mm are fast cut away before the paraffin sections are cut in 5 µm thickness at room temperature and collected. The paraffin sections are gently stretched on a 60°C hot water bath before being mounted onto marked microscope glass slides (Superfrost plus, Menzel-gläser code No. 041300).

The slides are dried, baked in an oven at 60°C, excess and melted paraffin is wiped away with a tissue.

The slides are deparaffinated by successive incubating twice in xylene for 2-5 min., twice in 96 % ethanol for 2-5 min., twice in 70 % ethanol for 2-5 min. and once in TBS (50 mM Tris-HCl (Tris(hydroxymethyl)aminomethan p.a., Crystal Chem inc., Il, USA), 150 mM NaCl, pH 7.6) for 5 min.

To ensure good coverage of reagent on the material, the area on the slide with reference material is encircled with a silicone barrier ("DakoPen", DakoCytomation code No. S 2002).

The slides are transferred to a rack in a small chamber to avoid drying out during the following procedural steps.

All the slides are incubated with a blocking buffer (Casein based blocking buffer concentrate, Sigma-Genosys Ltd, UK, code No. SU-07-250, 15 min.), washed three times with TBS for 1 min. followed by blocking any peroxidase activity by incubating with ChemMate™ Peroxidase-Blocking Solution (DakoCytomation code No. S 2023), before being washed once with TBS for 2 min.

The immunovisualisation of the DNP modified fibres is done using the Horseradish labelled F(ab') rabbit antibody against DNP (DakoCytomation code No. P 5102, dilution 1:100, 100 µL per slide, incubation for 60 min.).

The slides are washed gently three times in the TBS buffer for 2 min., followed by incubation with a diaminobenzidine chromogenic substrate system (DAB+, DakoCytomation code No. 3468) for 10 min.

The immunovisualisation of fluorescein modified and unmodified fibres is done exactly as for the DNP modified material, except using the Horse radish labelled F(ab') rabbit antibody against FITC (DakoCytomation code No. P 5100 dilution 1:100, 100 µL per slide, incubation for 60 min.).

All the slides are washed twice with the TBS buffer, followed by washing once with distilled water. The slides are cover slipped using an aqueous mounted media, Faramount (DakoCytomation code No. S 3025), and examined in a bright field microscope (Leica DM LB) at 10x or 40x magnification, using light strength setting 8, digitally photographed (Olympus DP50-CU) and the pictures white background corrected.

Figure 12 shows an overall comparison of human breast tissue stained with the state of the art Herceptest ™ (Figure 12A), Lagertun silk fibres modified with DNP and stained using anti DNP-HRP/DAB (Figure 12B) and stained HER2 +3 reference cells from the Herceptest ™ (Figure 12C). The overall morphology and appearance of the reference standard is similar to that of the conventional tissue staining using reference cells, in terms of size, shape and distribution. The silk fibres are more homogeneous in size compared to the Herceptest reference cells. The human tissue has a more complicated morphology compared to both of the reference systems.

### Results

Figure 13 shows the resulting staining at 10x magnification. Figure 13A: Unifloss, Figure 13B: LP floss and Figure 13C: Lagertun fibres coupled with 25 mM F-DNP in aqueous solution, and in comparison Figure 13D: Unmodified Unifloss fibres as negative control.

The Unifloss and LP floss fibres are only very weakly stained on the outer surfaces - or edges of the fibres. The lagertun fibres are positively stained (approx. 2+) both on the surface and in the interior. The staining is homogeneous for all the fibres.

In conclusion, the Unifloss and LP floss fibres modified with Sanger's reagent in aqueous solution are stained to a degree which is not significant different from the background, whereas the lagertun silk fibres is stained homogeneously and to a high degree.

Figure 14 shows the resulting staining viewed at 10x magnification. Figure 14A: Unifloss, Figure 14B: LP floss and Figure 14C: Lagertun fibres coupled with 10 mM FITC in toluene, and in comparison, Figure I4D:Unmodified Unifloss fibres as negative control.

The Unifloss and LP floss fibres are only very weakly stained on the surfaces - almost to a degree, which is not significantly different from the background. The lagertun fibres are weakly stained predominately on the surface (approx. 1+) and appear unstained in the interior. The staining is homogeneous for all the fibres.

In conclusion, the Unifloss and LP floss fibres modified with FITC in the toluene solution are stained to a degree, which is not significant, different from the background. The lagertun silk fibres are stained homogeneously and almost only on the outer surface of the fibre. This is different from the staining pattern for the aqueous/F-DNP modified Lagertun fibres pictured in Figure 13C indicating a change in localization due to the different swelling properties of silk in water and toluene. Silk do not swell much in toluene.

As a consequence, during coupling in toluene, the reactive groups could not reach the inner part of the unswelled silk fibre. This is illustrated in Figure 14C.

### Example 4. Immuno Staining of Fluorescein and DNP Reference Material Using a Secondary Visualization System

The following Example describes the staining of fluorescein and DNP modified model reference material with graded staining intensity and controlled localization of the stain, using a secondary visualization system, which is more sensitive than the primary or direct staining system used in Example 3 above.

The reference material is cut, mounted on slides, and deparaffinated as described in Example 3 above.

Similarly, the blocking with protein and blocking for peroxidase activity is as for Example 3.

The overall procedure being first incubating with a conjugates containing a primary antibody against DNP or Fluorescein. Followed by washing, incubation with a polymeric dextran conjugate mixture containing horseradish peroxidase and both goat anti rabbit and goat anti mouse and staining with a HRP chromogen.

In more detail, the DNP modified fibres are incubated for 60 min. with HRP labelled F(ab') rabbit antibody against DNP (DakoCytomation code No. P 5102, dilution 1:100, 100 µL per slide).

The FITC modified fibres are incubated for 60 min. with HRP labelled F(ab')rabbit antibody against FITC (DakoCytomation code No. P 5100, dilution 1:100, 100 µL per slide).

All slides are washed gently three times in the TBS buffer for 2 min., followed by incubation with Envision+/HRP conjugate (DakoCytomation code No. K 5007,100 µL per slide) for 30 min.

The slides are washed gently three times in the TBS buffer for 2 min., followed by incubation with a diaminobenzidine chromogenic substrate system (DAB+, DakoCytomation code No. K 3468) for 10 min.

All the slides are washed, cover slipped, examined in the microscope and photographed as in Example 3.

Figure 15 shows the staining of (a) Unifloss, (b) LP floss and (c) Lagertun fibres coupled with 10 mM FITC in toluene and (d) Unmodified Unifloss at 10x magnification. All treated as FFPE samples and stained with anti FITC-HRP/EnVision+/DAB+ (DakoCytomation code Nos. P 5100 and K 4010/4011).

The Unifloss and LP floss fibres are stained exclusively on the surfaces (approx. approx. 1+ - 1 ½ +). The lagertun fibres are stained with higher intensity (approx. 2+) on the outer surface. The staining is homogeneous for all the fibres.

Figure 16 shows the staining of Unifloss fibres coupled with Sanger's reagent (F-DNP) at different conditions: a) Coupled with 5 mM F-DNP in aqueous solvent, b) Coupled with 25 mM F-DNP in aqueous solvent, c) Coupled with 5 mM F-DNP in toluene, d) Coupled with 10 mM F-DNP in toluene and e) unmodified fibres.

The Unifloss fibres modified in aqueous solution are stained both on the surface and in the interior of the fibre. The intensity increases from approximately 1/2+ using 5 mM F-DNP to 1+ using 25 mM F-DNP.

The Unifloss fibres modified in toluene are stained almost exclusively on the surfaces. The fibres modified with 5 mM F-DNP have a staining intensity just slightly more than the unmodified fibres. Whereas the fibres modified with 10 mM F-DNP are more stained (approx. ½-1+).

The staining is homogeneous for all the fibres. The unmodified fibres are not stained. Only a shadow from the fibre can be seen near the edges.

Figure 17 shows the staining of LP Floss fibres coupled with Sanger's reagent (F-DNP) at different conditions: a) Coupled with 5 mM F-DNP in aqueous solvent, b) Coupled with 25 mM F-DNP in aqueous solvent, c) Coupled with 5 mM F-DNP in toluene, and d) Coupled with 10 mM F-DNP in toluene.

The LP fibres modified in aqueous solution are stained homogeneous though the fibre. The intensity increases from approximately 1+ using 5 mM F-DNP to 2+ using 25 mM F-DNP.

The LP fibres modified in toluene are stained at low intensity on the surfaces. The fibres modified with 10 mM F-DNP are staining slightly more than the fibres modified with 5 mM F-DNP. The staining is homogeneous for all the fibres.

Figure 18 shows the staining of Lagertun silk fibres coupled with Sanger's reagent (F-DNP) at different conditions: a) Coupled with 5 mM F-DNP in aqueous solvent, b) Coupled with 25 mM F-DNP in aqueous solvent, c) Coupled with 5 mM F-DNP in toluene, and d) Coupled with 10 mM F-DNP in toluene.

The Lagertun silk fibres modified in aqueous solution are stained with high intensity both on the surface and in the interior. The fibres modified with low concentration of F-DNP stained at approx 2.0 - 2.5 + on the surface and approx. 1+ in the interior. The fibres modified with 25 mM F-DNP are stained with even higher intensity: approx. 3+ on the surface and 2+ in the interior.

The Lagertun fibres modified in toluene are only weakly stained mainly on the surfaces. The fibres modified with 25 mM F-DNP are staining almost to the same degree as the fibres modified with 5 mM F-DNP. The staining is homogeneous for all the fibres.

In conclusion, the various stainings described above shows that it is possible to stain with different intensities depending on the modification conditions, here concentration of the two reactive haptens, FITC and Sanger's reagent.

Also, it is possible to stain the target specifically with low intensity, but clearly above the background.

This is often very desired, as the diagnostic interesting threshold staining for many IHC stainings are approximately 1+, and should be clearly distinguishable from unspecific staining. This is can be very difficult to obtain, as weak specific staining often drowns in the unspecific background staining.

The staining intensity also depends on the hapten used and the solvents used during the modification. Apparently, the FITC modified fibres are stained weaker than the F-DNP modified fibres. In addition, better swelling during modification results in a higher staining intensity.

The localization of the stain is dependable on the swelling properties of the solvent during modification. In general, none of the fibres swelled in toluene, and are consequently predominantly stained on the surfaces.

The Lagertun silk fibres swelled in aqueous solvents, and are therefore also stained in the interior of the fibre.

LP floss and Uni floss are stained with different intensities on the surface and in the middle of the fibre when using aqueous conditions during the modification.

### Example 5. Comparison of Size and Shape

The following Example compares the size and shape of the reference material with the reference systems included in the HercepTest™.

Stained Unifloss, LP floss and Lagertun fibres slides pictured on Figure 16B, Figure 17B and Figure 28B (all coupled with 25 mM F-DNP at aqueous conditions, from Example 4) are size measured using the software "Olympus DP-soft, version 3.1".

The average of 10 measurements at 40x magnification in the bright field microscope gave an average diameter of 3.3; 3.5 and 1.6 µm for the DNP stained Unifloss, LP floss and Lagertun, respectively.

Figure 19 illustrates the size and shape of all three fibres, with a scale bar being inserted using the above-mentioned software.

Figure 20A is a picture taken at 10x magnification of the 3+ level reference cells stained for HER2 using HercepTest™ (DakoCytomation code No. K 5204).

Figure 20B is a picture taken of the stained negative control reference cells used in the same kit at the same magnification.

The 3+ cell line (Figure 20A) gives strong (3+) membrane staining and no cytoplasmic staining. The staining is homogenous and it can be seen that the preparation contains few dead cells and some cell debris.

Figure 21 is a picture taken at 10x magnification of the DAB+ stained and haematoxylin counterstained EGFR reference cells from the DakoCytomation EGFR PharmDx kit (code No. K 1492).

The picture shows strong membrane staining (3+) and some weak cytoplasmic staining (1+). The staining is homogenous and the preparation contains some dead cells and some cell debris.

The size, distribution and shape of the stained fibres in Figure 14 to 19 can be compared with the reference cells used in the HercepTest™ and the EGFR PharmDx kits (Figure 20 and 21).

The size measurements of the fibres indicate that the individual dots are of roughly the same size as the cells used in the for example HercepTest™. Especially the Unifloss and LP floss fibres are of the same size are as the Lagertun fibres are significantly smaller.

Also, the size distribution is more homogenous and narrow as compared to the HercepTest™ cells (Figure 20), where the cells are cut randomly, resulting in various sizes of cell fragments and cell.

In contrast to the reference cell lines, none of the fibres have visible debris or debris like fragments.

The Unifloss fibres appear as spherical dots with an uneven and flossy edge. The LP floss is much more even at the edges, but not as spherical.

The Lagertun fibres are even at the edges and slightly oval, resembling many crystals.

The shape of the fibres do not perfectly resembles natural cells, but the size and overall contours are cell like.

### Example 6. Colouring Fibres Using Reactive Dyes

The Example illustrates the preparation of permanent collared reference material and their ability to withstand deparaffination and epitope retrieval.

2.0 m of Unifloss, Uni Floss and Lagertum fibres are placed separately in test tubes with a reactive red dye solution (33 mg Procion Red MX-5B, Aldrich, product No. 40,436-5, 615.34 Da, in 662.5 µL deionized water) or reactive blue dye solution (Cibacron Blue 3 GA, Sigma-Aldrich co., product No. C-9534, 774.2 DA in 662.5 µL deionized water). All closed test tubes are heated on a60°C water bath for 30 min. and shaken every 10 min.

To each test tube is added sodium cloride (25 µL, 4M) and sodium carbonate (312.5 µL, 0.8 M, pH 9.0) and incubated for 2 hr. at 80°C while being shaken every 10 min.

The fibres are washed with tap water until the no dye leaked from the fibres. The fibres are stored in deionized water containing sodium azide (15 mM, 2-8°C) before being embedded in agarose gel, cut in to gel blocks, dehydrated and paraffin embedded as in Example 2 above.

The paraffin blocks containing the dyed fibres are stored at room temperature before being cut and mounted onto slides as in Example 3.

The slides are divided into three groups: The first slides are cover slipped just before the deparafination step, the second group is deparafinated before being cover slipped, and the third group is deparafinated and subjected to a epitope retrieval step (DakoCytomation, code No. S 1700) in a water bath at just below boiling point (95-99°C) for 40 min., allowed to cool to room temperature for 20 min., washed once with TBS for 5 min. before being cover slipped.

The deparafination and cover slipping is done as in Example 3.

All the cover slipped slides are examined in the bright field microscope at 10x magnification and at light setting 8 in the microscope.

In general, all the fibres are highly collared and the dye contents the same after deparaffination and epitope retrieval. Strongly indicating the dyes are stable towards the standard IHC procedural steps.

Also, the lagertun silk fibres are much stronger dyed red or blue than the two other fibres.

In more detail, for the Procion Red modified LP floss and Unifloss fibres:
Figure 22 are digital pictures of Procion Red modified LP floss before deparaffination, after deparaffinaition and after the epitope retrieval step, respectively.
Figure 23 are digital pictures ofProcion Red modified Uni floss before deparaffination, after deparaffinaition and after the epitope retrieval step, respectively.

Surprisingly, the red colour is clearly visible and apparently of the same strength or level after the different steps. The dying of the fibres is therefore permanent and stable during the standard IHC steps.

The Unifloss appears in general to be less coloured than the LP floss.

The LP floss fibres seem to be dyed slightly more homogeneously than Unifloss fibres.

In Figure 23B and to some degree in Figure 23C, the LP floss fibres seems to have been cut not perfectly perpendicular to fibres lengths, resulting in oval and not spherical dots.

### Example 7. The Effect of Various Embedding Media and Glass Slides

The following Example illustrates how the reference material remains intact on the slides after chemically harsh antigen retrieval procedures.

Various additives in the embedding media are combined with various glass slides and using various routine epitope retrieval methods.

In this Example unmodified fibres (unifloss, LP floss and Lagertun) are used.

The three different fibres are mounted on the steel frame and embedded in different agarose gel formulations: (i) 2 % agarose, (ii) 4 % agarose, (iii) 2 % agarose, 0.25 % PVA (Fluka code No. 81386), (iv) 2 % agarose, 0.25 % PEI (Fluka code No. 03880).

The agarose is the same as in Example 3.

The gel embedded fibres are fixed, dehydrated, paraffin embedded, cut, and mounted on slides as described in Example 3.

The slides used are: (1) Super Frost (Menzel-gläser code No. 041300), (2) ChemMate™ Capillary Gap Microscope Slides (DakoCytomation code No. S 2024), (3) DakoCytomation Silanized Slides (code No. S 3003), (4) Poly-L-Lysine slides (Electron Microscpy Sciences, code No. 63410-01)

The sections mounted on the different slides are deparaffinated, examined in the microscope, photographed and treated with one of the following epitope retrieval procedures.
(a) High pH epitope retrieval buffer pH 9.9 (DakoCytomation code No. S 3308), 40 min. in a 95-99°C water bath, allowed to cool to room temperature for 20 min., washed once with TBS for 5 min. before being cover slipped.
(b) A epitope retrieval buffer, citrate, pH 6.1 (DakoCytomation code No. S 1700), same procedure as (a), expect for heating for 20 min. in the water bath and wash in deionized water.
(c) Citrate pH 6,2 (10 mM), same procedure as (a)
(d) ChemMate™ Buffer for Antigen Retrieval, citrate, pH 6,0 (DakoCytomation code No. S 2031), same procedure as (a)

After the antigen retrieval procedure, all the labelled slides are cover slipped and examined in a broad field microscope and again photographed.

All the combinations of fibre, gel formulation, slide and antigen retrieval is done in duplicates.

The number of dots from each fibre bundle on each slide is estimated by comparing photographs before and after the antigen retrieval.

In the Tables 1-4, the results are summarized for each of the four epitope retrieval procedures.

The number of remaining dots after the deparaffination and epitope retrieval are given as approximate values. <10 %: Less than 10 % of dots remained. <20 %: Less than 20 % of dots remained. >20 %: More than 20 % of dots remained. >90 %: More than 20 % of dots remained.

Figure 24 and 25 are representative of the photographs taken before and after deparaffination and antigen retrieval.

Figure 24 is pictures of Unifloss fibres embedded in 2 % agarose with 0.25 % PEI mounted on ChemMate™ Slides before and after deparaffination and antigen retrieval with DakoCytomation citrate antigen retrieval buffer, pH 6.1, taken at 10x magnification (Experiment 8 - ChemMate™ Capillary Gap Microscope Slide in the Table 2).

The Unifloss fibres can be seen as small spheres in between the agaraose and paraffin matrix on the slide (Figure 24A). After deparaffination and antigen retrieval, the fibres can be seen more clearly as individual dots (Figure 24B).

Approximately 50-60 % of the individual dots remained attached to the slide after deparaffination and antigen retrieval.

Figure 25 are pictures ofLP fibres embedded in 2% agarose with 0.25% PEI, mounted on Poly-L-Lysine Slides before and after deparaffination and antigen retrieval using high pH buffer (DakoCytomation code No. S 3308), taken at 10x magnification. (Experiment 20 - poly-L-Lysine slides in Table 1).

More than 90 % of the individual dots remained attached to the slide after deparaffination and antigen retrieval.

The shadow to the left on Figure 25A is the edge of the paraffin slap, and does not interfere with the estimation of the number of fibres in the bundle.

It can be seen from the Tables, that high pH epitope retrieval (DakoCytomation code No. S 3308 pH 9,9) in general is the harshest to the reference material, almost irrespectively of the fibre type.

The influence of the agarose gel formulation strongly indicates, that 2 % agarose with 0.25 % PEI is far superior to the three other formulations. The 4 % agarose is better than 2 % agarose and 2 % agarose with 0.25 % PVA.

In general, the best performing slides in these experiments are poly-L-lysine slides, which are better than ChemMate™ and Superfrost slides, which are both better than the silan coated slides.

It is somewhat surprising that the individual dots in general stay attached to the slides after the antigen retrieval procedural step.

It is furthermore surprising that the combination of for example 2 % agarose with PEI, lysine coated slides and the most harsh antigen retrieval condition allow the majority of the individual dots to remain attached to the slide.

In comparison, it is not uncommon that 50% of natural cells can fall of slides during antigen retrieval.

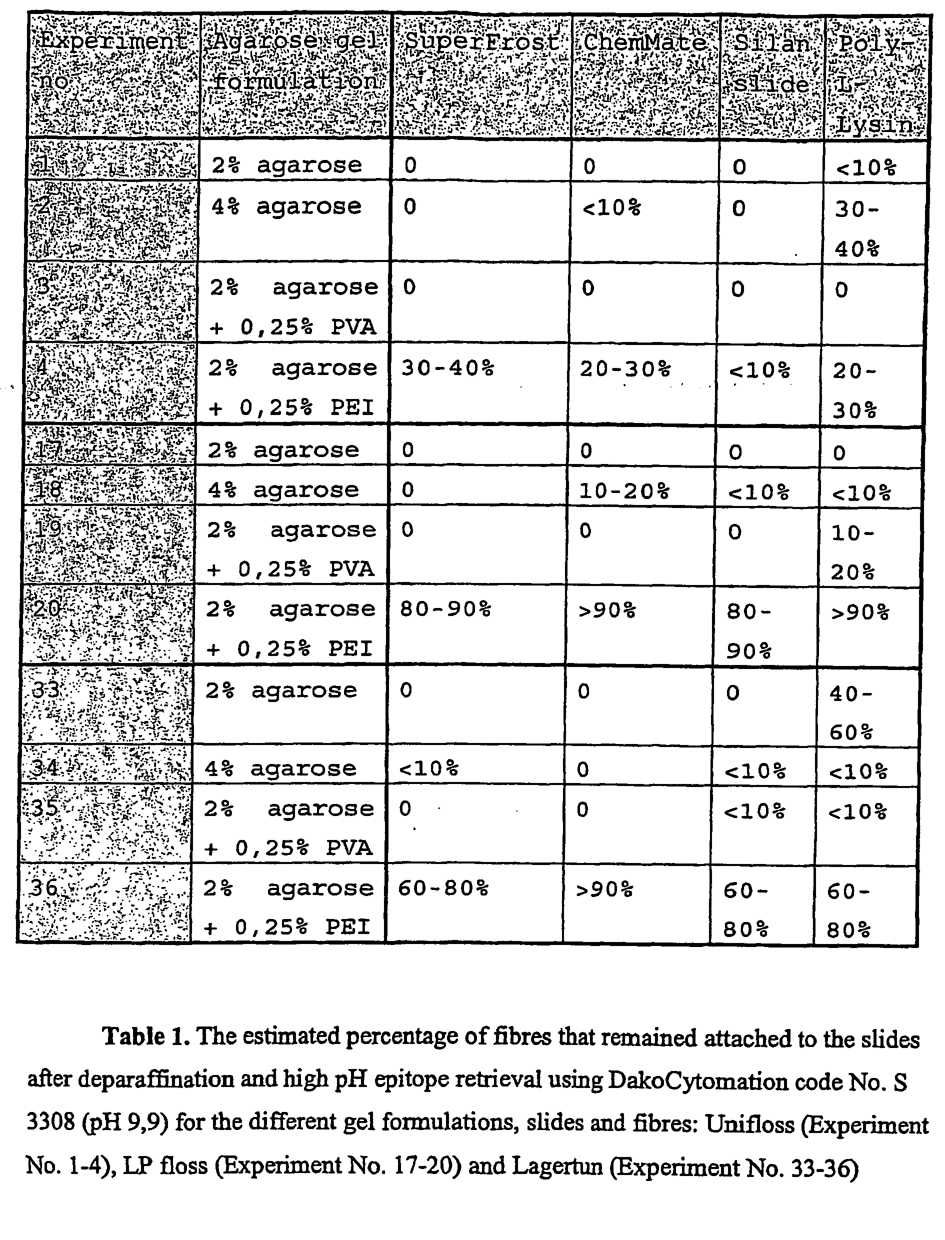

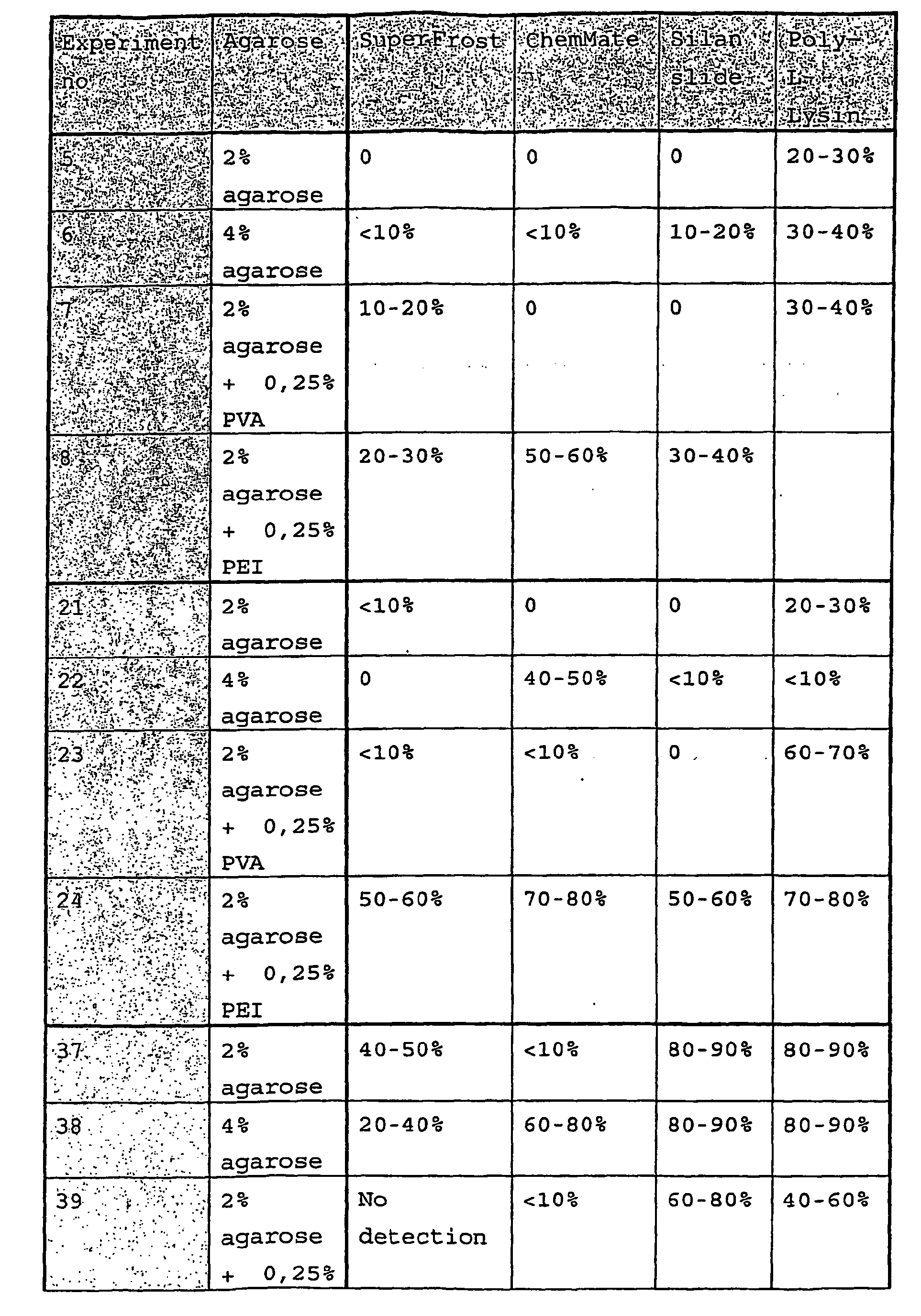

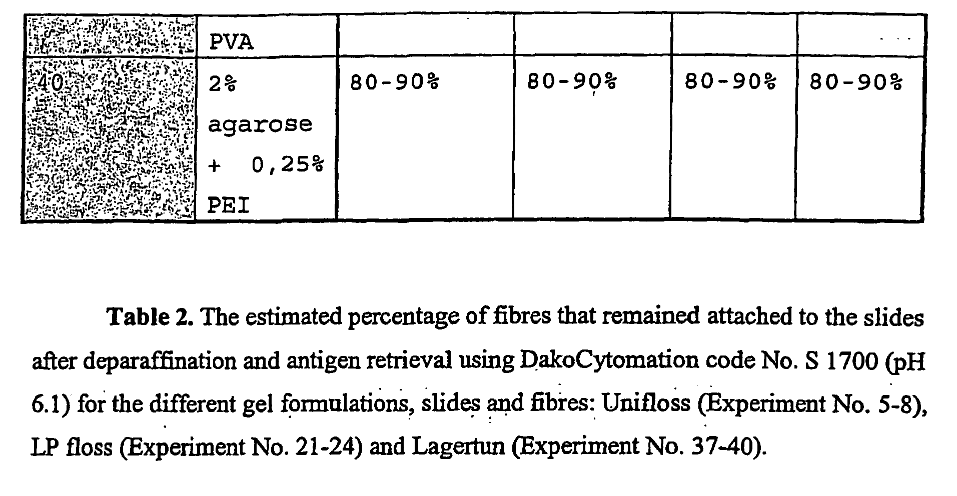

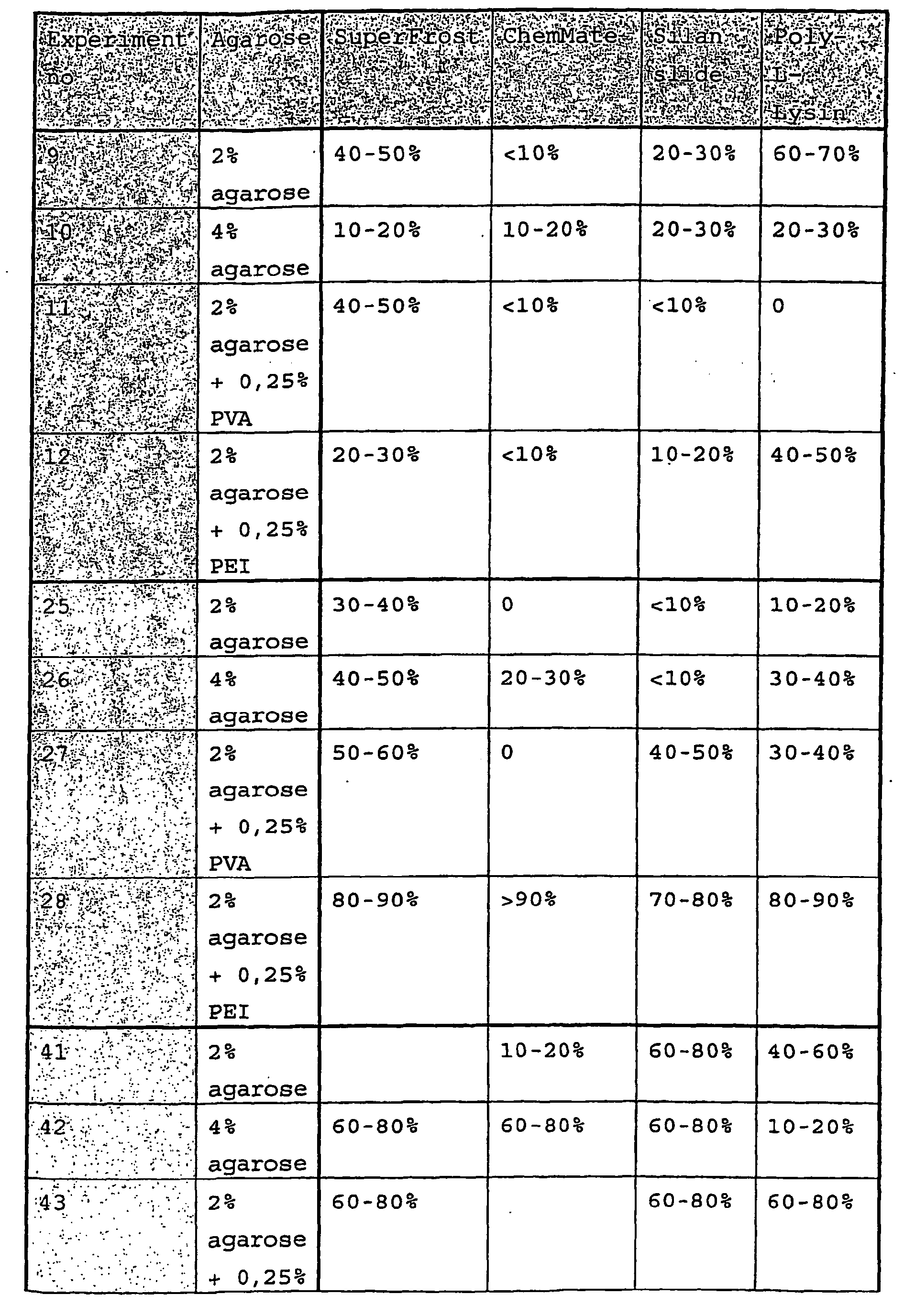

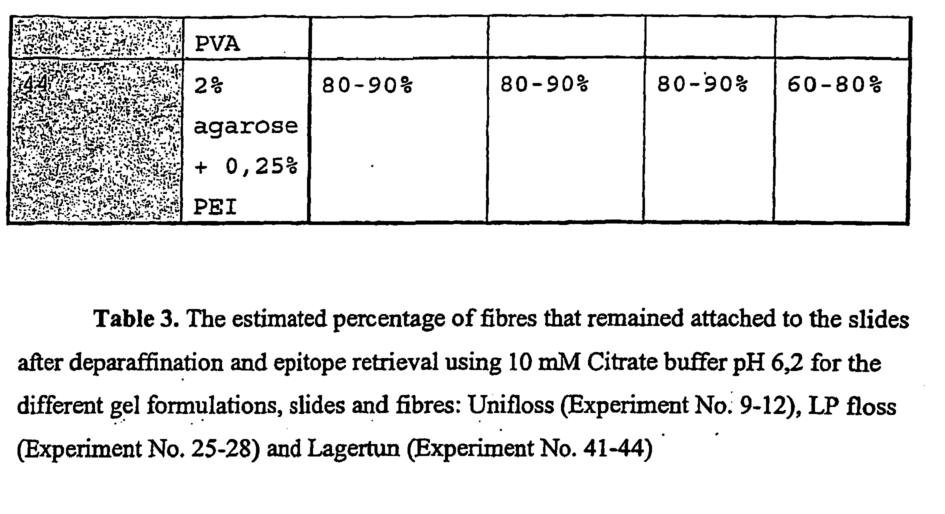

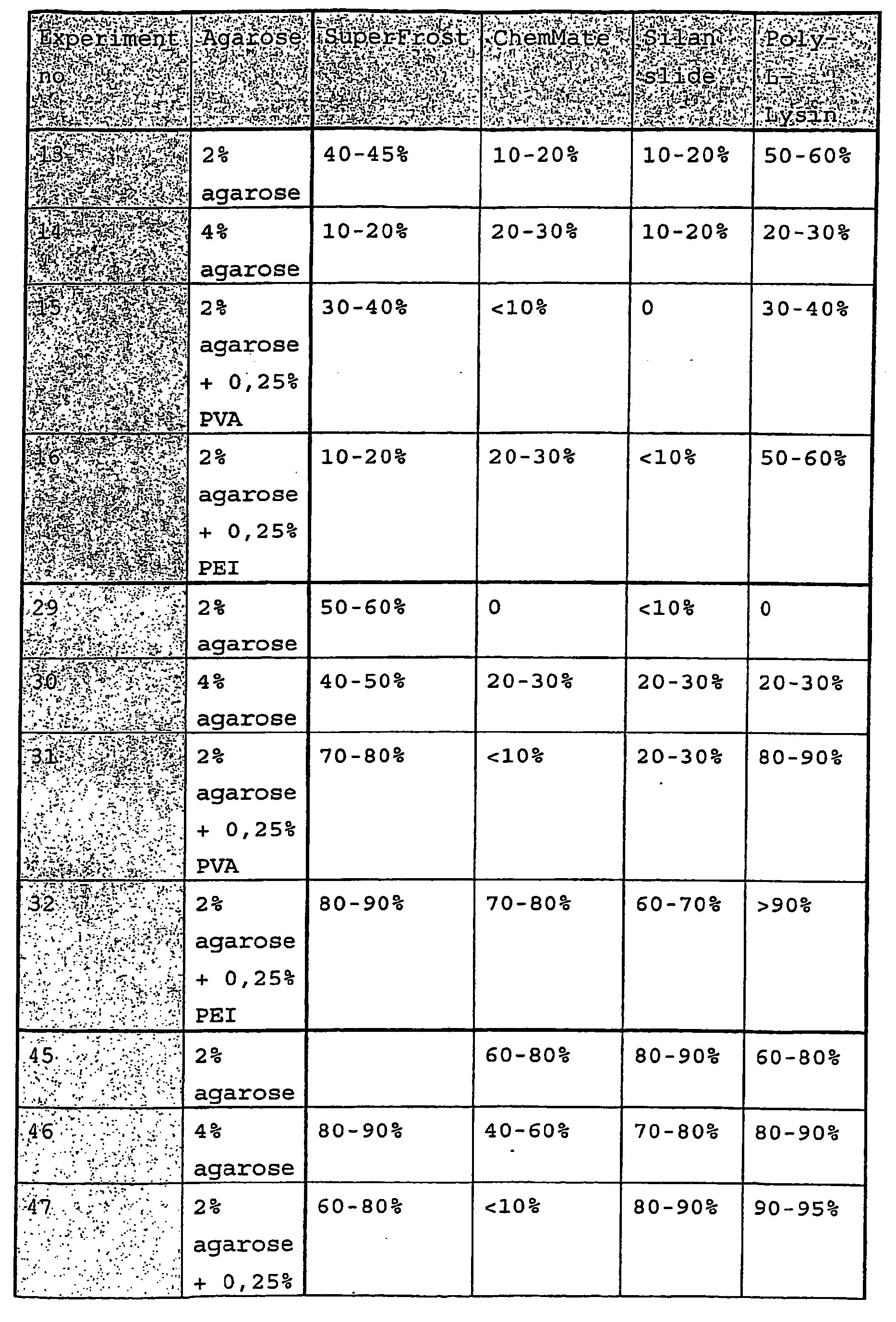

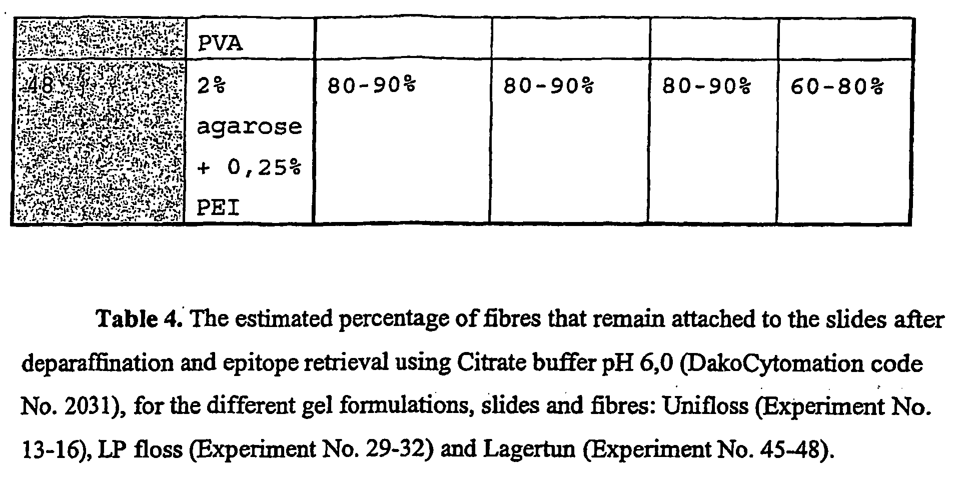

### Example 8. Preparation and Immunostaining of Igg Reference Material

The example illustrates the use of CDI activated and IgG or biotinylated IgG modified fibres as control for the function of the visualization system.

The fibres are functionalised with carbonyl diimidazol. The resulting reactive imidazole carbamate introduced onto the fibres is used to directly couple rabbit IgG or biotinylated rabbit IgG. The fibres are immunovisualized with EnVision/DAB plus. In more detail:
20 cm long pieces of Lagertun fibre are washed with dry DMF (Aldrich Chemical co, dried over 4A mol. sieves), followed by washing in DMF:acetone (1:1, Aldrich Chemical co, acetone dried over solid potasiumcarbonate) for 5 minutes.

Each fibre is gently shaken in a closed container for 60 minutes at room temperature with 1.0 ml. of a freshly prepared carbonyl diimidazol solution (CDI, Aldrich Chemical co., cat. No. 11, 553-3, 10 % w/v, 1:1 DMF:acetone).

IgG or biotinylated IgG (rabbit IgG, DakoCytomation x0936, Biotinylated rabbit anti mouse IgG, E0354 is dialyzed against 0.10 M NaCl with (10 kDa MwCO, 3 ml: 1000 ml, 4 changes) before being concentrated to 12,5 and 8.0 g/l, respectively.

The fibres are taken from the CDI reaction container, excess liquid removed on a paper filter and directly transferred to solutions of IgG or biotinylated IgG, before being adding carbonate buffer (in total, 5 g/l IgG, 0.10 M NaCl, 50 mM carbonate, pH 9.0) and gently shaking overnight (17 hours) at room temperature.

Any remaining active groups are quenched by washing with ethanol amine (10 mM ethanol amine, 50 mM carbonate, pH 9.0) for 6 hours.

In parallel, fibres are treated without the addition of CDI.

The modified fibres are removed from the containers, excess liquid removed on a paper filter and the fibres washed once in water and stored at 2-8 °C before being formaldehyde fixed, embedded in agarose, dehydrated, paraffin embedded, cut and mounted onto poly lysine coated slides and deparaffinated according to the general procedure.

Some of the slides are antigen retrieved at 95 °C in 40 minutes using Dakocytomation S 1700AR buffer.

The slides with unmodified fibres or fibres modified with IgG are stained by first blocking with Genosys buffer, TBS buffer washing three times, blocking with Chemmate peroxidase blocking buffer (DakoCytomation S2023), TBS buffer washing three times and incubation with EnVision dual link (DakoCytomation K5007, against mouse and rabbit IgG) or EnVision anti mouse (DakoCytomation K4001, against mouse IgG), three times TBS washing, DAB incubation for 10 minutes followed by TBS washing three times and once with tap water. The slides are mounted with cover slides and inspected in the microscope. All the above were done according to the general procedure.

The slides with fibres modified with biotinylated IgG are stained by incubation with Streptavidine-horse radish peroxidase (DakoCytomation PO397, diluted 1000 times in TBS) instead of the EnVision conjugates.

Figure 26 are photomicrographs, taken at 40-time magnification, showing the DAB staining of the Lagertun fibres:
The CDI activated fibres coupled with rabbit IgG and stained with rabbit Envision HRP/DAB (Figure 26A).
The GDI activated fibres coupled with rabbit IgG and antigen retrieved and stained with rabbit Envision HRP/DAB (Figure 26B).
The CDI activated fibres coupled with biotinylated rabbit IgG and stained with Streptavidine HRP/DAB (Figure 26C).
Native fibres, treated without CDI, with rabbit IgG and stained with Envision HRPIDAB (Figure 26D).
Native fibres stained with Envision HRP/DAB (Figure 26E).
The CDI activated fibres coupled with rabbit IgG, AR treated and stained with anti mouse Envision HRP/DAB (Figure 26F).

The Lagertun silk fibres coupled with rabbit IgG and stained with rabbit Envision HRP/DAB give clear staining located exclusively on the edge of the fibres (Figure 26 A). The staining intensity is approximately 0,5 +.

Antigen retrieval of the same fibres (Figure 26B) give the same staining localization and only higher staining intensity.

The Lagertun silk fibres coupled with biotinylated rabbit IgG and stained with Streptavidine HRP/DAB give clear staining located exclusively on the edge of the fibres (Figure 26 C). The staining intensity is approximately 0,25 +.

The Lagertun fibres treated without the CDI activation (Figure 26 D), native fibre (Figure 26E) and fibre coupled with rabbit IgG, AR treated and stained with anti mouse Envision HRP/DAB all give no visible DAB staining. In conclusion, the unspecific background is insignificant.

The carbonyl diimidazole activation method allows for easy preparation of the reference material. The modification is only on the outer surface of the fibre.

The two different visualization systems (EnVision and SA-HRP) both give specific staining. The intensity is not the same, which is due to the different targets and different nature of the visualization systems.

The example illustrates the preparation and staining of reference material useful for the verification of the functionality of the secondary or indirect immunovisualization system.

### Example 9. Preparation and Immunostaining of Her2 Reference Material

The example illustrates the preparation and immunostaining of HER2 peptide modified fibres as control for the function of the primary antibody and visualization system. The target peptide being coupled to an amino linker though a chemo selective heterobifunctional cross linker.

In more detail:
The fibres are modified with primary amino groups attached to a linker by first reaction with hexan diisocyanate followed by hydrolysis.

The amino group is functionalised with GMBS, followed by coupling the thiol containing peptide. The fibres are immunovisualized with HercepTest™ visualization system.

In even more detail,
6 pieces of 20 cm long pieces of each fibre, Lagertun, LP floss and Unifloss, are washed with dry DMSO (Aldrich Chemical co, dried over 4A mol. Sieves,), and left for 3 hours to swell in DMSO.

The fibres are treated with a 1,6-hexan diisocyanate solution (HDI, Aldrich Chemical Co., D12, 470-2, 10 % W/V in DMSO) overnight at room temperature while being gently shaken.

The fibres are washed twice in DMSO, before the isocyanate groups are hydrolysed by treating the fibres overnight with a DMSO/water solution (1:1) at room temperature. The fibres are washed in DMSO and stored in water at 2-8 °C before being coupling to peptide.

The amino group modified fibres are partly dried on filter paper before being treated with a N-succinimidyl-4-maleimidobutyrate solution (10 mm GMBS, Pierce cat. No. 22309, 10 mM dipropyl ethylamine, DIPEA, Aldrich, DMSO) for 120 minutes at room temperature while being gently shaken.

The fibres are removed from the reaction vessel and excess solvent removed with filter paper. The modified fibres are washed twice with DMSO before the peptide is coupled to the thiol active malemido group.

As a negative coupling control, fibres are treated in parallel with no addition of GMBS.

A freshly prepared Her2 peptide solution is added to each fibre (in total 0.10 g peptide/ml, 5 mM EDTA, 500 mM HEPES, 30% DMSO, pH 8.0) and gently shaken for 60 minutes at room temperature.

The Her2 peptide is obtained from Neosystems, (Strasbourg, T-16-C-SP991729E) is a 16 amino acid peptide (1678 Da) with cysteine at the C terminal and contains a HER2 peptide motif recognized by DakoCytomation HercepTest™ kit.

Excess reactive groups are quenched by shaking with ethanol amine (100 mm, 100 mM HEPES, pH 8.0) for 10 minutes.

The fibres are washed twice with a HEPES buffer (100 mM, pH 8.0) and stored at 2-8 °C before being embedded in agarose with 0.05% PEI, fixed, dehydrated, paraffin embedded, cut and mounted on poly lysine slides as described in the previous examples.

Half of the slides are antigen retrieved using S 1700 for 40 minutes (DakoCytomation)

All the slides are stained according to the instructions in the Herceptest^{™} kit (DakoCytomation K5205)

In short, the slides are incubated with rabbit anti HER2 followed by incubation with an EnVision HRP anti rabbit visualization system using DAB as chromogen.

In parallel, native fibres and fibres not treated with GMBS are stained.
Figure 27 show photomicrographs of the stained slides taken at 40-time magnification.
Figure 27A to Figure27E show HER2 modified LP floss (A), Uni floss (B), and Lagertun fibres (C), HER2 modified Uni floss with Antigen Retrieval (D) and HER2 modified Lagertun including Antigen Retrieval (E)
Figure 27F is a negative coupling control using non-activated Lagertun fibres treated in parallel.
Figure 27G to Figure 27I show negative primary antibody control of HER2 modified LP floss (G), Uni floss (H), and Lagertun fibres (I)

The native and unmodified fibres did not show any staining.

Figure 27J show the stained 0, 1+ and 3+ control cell lines included in the HercepTest™ kit, taken at 10 time magnification

All three fibres types give distinct DAB staining located on the edge of the fibres.

(Figures 27A - C). The staining intensity is approximately 1.0 +.

Antigen retrieval of the same HER2 modified Unifloss and Lagertun fibres (Figure 27D and Figure27E) give the same staining localization and higher staining intensity. Some of the individual dots are fallen off the slides after AR treatment.

The negative coupling control, negative primary antibody and native fibres (Figure 27F - Figure 27I) did not give any staining, indicating very low general background.

The obtained staining intensity is of practical utility, as threshold staining values between 0.5-1.5 + is typically of diagnostically relevance.

By comparison with the reference cells, it can be seen that the reference system of the invention appears more uniform with respect to both staining localization and intensity.

In conclusion, the HDI activation and chemo selective coupling method allowed for preparation of the HER2 peptide containing reference material. The staining is mainly located on the outer surface of the fibre.

The example illustrates the preparation and staining of reference material useful for verification of the functionality of the immunovisualization system.

### Example 10. Preparation and Immunostaining of Graded Her2 and P16 Reference Material

The example illustrates the preparation and graded immunostaining of HER2 peptide and p16 peptide modified fibres as control for e.g. the function of the primary antibody and visualization system.

Mixtures of the two peptides are coupled to an amino linker though a chemo selective heterobifunctional cross linker.

20 cm long pieces of Unifloss malemido group modified fibre are prepared as in example 9. In short, the fibres are treated with HDI, hydrolysed and reacted with GMBS.

As a negative coupling control, fibres are treated in parallel with no addition of GMBS.

A number of freshly prepared HER2-p16 peptide mixtures prepared (in total 0.20 g peptide/ml, 5 mM EDTA, 100 mM HEPES, 30% DMSO, pH 8.0, 1000 micro litre), according to the scheme:
a) 0,20 g/l Her2 peptide + 0,00 g/l p 16 peptide
b) 0,19 g/l Her2 peptide + 0,01 g/l p 16 peptide
c) 0,15 g/l Her2 peptide + 0,05 g/l p 16 peptide
d) 0,10 g/l Her2 peptide + 0,10 g/l p 16 peptide
e) 0,05 g/l Her2 peptide + 0,15 g/l p16 peptide
f) 0,01 g/l Her2 peptide + 0,19 g/l p16 peptide
g) 0,00 g/l Her2 peptide + 0,20 g/l p16 peptide

The freshly prepared HER2-p 16 peptide mixtures is added to each fibre and gently shaken for 60 minutes at room temperature.

The Her2 peptide is obtained from Neosystems, (Strasbourg, T-16-C-SP991729E) is a 16 amino acid peptide (1678 Da) with cystein in the C terminal and contained a HER2 peptide motif recognized by DakoCytomation HercepTest™ kit.

The p16 peptide is synthesized by solid phase peptide synthesis using a Boc/TFA strategy, purified by HPLC and identified by MALDI-TOF analysis. The 17 amino acid peptide (2114 Da) contains cystein amide in the C terminal, a fluorescein in the amino terminal and a peptide motif recognized by the DakoCytomation CINtec ™ p16-INK4 Histology Kit.

Excess reactive groups are quenched by shaking with ethanol amine (100 mM, 100 mM HEPES, pH 8.0) for 10 minutes.

The fibres are washed twice with a HEPES buffer (100 mM, pH 8.0) and stored at 2-8 °C before being embedded in agarose with 0.05% PEI, fixed, dehydrated, paraffin embedded, cut and mounted on poly lysine slides as described in the previous examples.

All the slides are stained according to the instructions in the Herceptest ^{™}kit (DakoCytomation K5205) and CINtec ™ p16-INK4 Histology Kit (DakoCytomation K5334), except for the antigen retrieval step.

In short, the slides are incubated with rabbit anti HER2 or mouse anti p16 antibody followed by incubation with an EnVision HRP anti rabbit/mouse visualization system using DAB as chromogen.

In parallel, native fibres and fibres not treated with GMBS are stained and negative control antibody and visualization staining is used as controls.

Also, the reference cell lines included in the Herceptest ™ are stained after antigen retrieval according to the procedure.
Figure 28 show photomicrographs of the HER2 stained slides taken at 40-time magnification.
Figure 28 A, B, C, D, E and F are photomicrographs of the HER2 stained slides prepared using 0,20; 0,19; 0,15; 0,10; 0,05 and 0,01 g/l HER2 peptide, respectively.
Figure 28G is the coupling control that is, the slide with no HER2 peptide and only 0,20 g/l p16
Figure 28H is the coupling control prepared without addition of GMBS or peptides.
Figure 28I is the primary antibody negative control using nonsense rabbit antibody (DakoCytomation K5205)
Figure 28J is the native unmodified fibre control.
Figure 28K is the secondary visualization negative control using anti mouse IgG Envision HRP (DakoCytomation K4006)
Figure 28L, M and N are the stained reference cells included in the Herceptest ™ with 0+, 1+ and 3+ cells, respectively.

The slide with only p16 peptide and no Her2 peptide (Figure 28G) give no or insignificant staining.

The slides with graded levels of Her2 target (Figure 28A - F) give distinct DAB staining located mainly on the edge of the fibres.

The staining intensity is approximately 1,5+; 1,5+; 1,5+; 1,0-1,5+; 1,0 + and 0,5+, respectively.

The negative control slides with no GMBS activation, nonsense primary antibody, native fibre and nonsense secondary visualization system, Figure 28H, I, J and K show no visible DAB staining, indicating very low general background.
Figure 29 show photomicrographs of the p 16 stained slides taken at 40-time magnification.
Figure 29A is the coupling control, that is, the slide with no p16 peptide and only 0,20 g/l HER2
Figure 29B, C, D, E, F and G show photomicrographs of the p16 stained slides prepared using 0,01; 0,05; 0,10; 0,15; 0,19; and 0,20 g/l p16 peptide, respectively.
Figure 29H is a coupling control prepared without addition of GMBS.
Figure 29I is the primary antibody negative control using nonsense rabbit antibody (part of the CINtec ™ p16-INK4 Histology Kit)
Figure 29J is the native unmodified fibre control.
Figure 29K is the secondary visualization negative control using anti rabbit IgG Envision HRP (DakoCytomation 4003)

The slide with only HER2 peptide and no p16 peptide (Figure 29A) give no or insignificant staining.

The slides with graded levels of p16 target (Figure 29B - G) give distinct DAB staining located mainly on the edge of the fibres

The staining intensity is approximately 0-0,5 +; 1.0 +, 1.0-1,5 +, 1,5-2,0 +, 1,5 + and 1,5 +, respectively.

For some on the p 16 slides, some unspecific staining outside the fibres could be observed. This could be due to conjugate or antibody adhesion to dried out agarose residues. The staining on the slides can easily be identified from the DAB staining in the agarose matrix.

The negative control slides with no GMBS activation, nonsense primary antibody, native fibre and nonsense secondary visualization system, Figure 29H, I, J and K give no visible staining, indicating very low general background.

The example illustrates the preparation and staining of reference material useful for verification of the functionality of the immunovisualization system.

The obtained staining intensity is of practical utility, as threshold staining values between 0.5 and 1.5 + is typically of diagnostically relevance and often technically difficult to obtain.

It could be noted that the dynamic range for the staining appears to be at approximately 0.01 to 0.15 g/l peptide range. That is, the staining is approximately the same for the fibres conjugated with more than 0.15 g/l peptide. It could be coincidental this is the same for both peptides.

The two peptides did no seem to influence each other's. Thereby, e.g. the HER2 peptide could be coupled to the fibre and act as a nonsense peptide for the p16 staining. This is of importance for the production of the material, as one often prefers to have a constant peptide concentration during conjugation in a production standard operation procedure. The subsequent wanted staining level by adjusted by adding nonsense peptide.

By comparison with the Herceptest ™ reference cells, it can be seen that the reference system of the invention appears more uniform with respect to both staining localization and intensity.

In conclusion, it is possible to prepare a reference material with graded levels of HER2 and p16 with very low unspecific background, using a chemo selective coupling scheme and mixtures of cystein functionalised peptides.

### Example 11. Preparation and Immunostaining of Control Material for Correct Addition of Primary Reagent During the Staining Procedure

The example illustrates the use of coloured and hapten modified fibres for use as a control system for correct addition of primary antibody solution to the slide.

Procion red modified Lagertun fibres and DNP modified (aqueous reaction conditions, 25 mM F-DNP) Lagertun fibre are prepared as in example 6 and 1.

The Procion red modified Lagertun fibres and DNP modified Lagertun and Unifloss fibre are bundled and embedded together as described previously. The resulting paraffin block contained red fibres and colourless DNP modified fibre close together.

The primary antibody solution containing rabbit anti HER2 IgG is spiked with rabbit anti DNP IgG HRP conjugate (2772 ml Ra-a-Her2 IgG from the Herceptest ™ kit, DakoCytomation K5205, and 0,028 ml Ra-a-DNP-HRP, DakoCytomation 5102.

FFPE Mammae (breast) tissue sections and sections from the block containing Procion red and DNP modified fibre is mounted on microscope slides. The fibres are mounted next to the tissue sections.

The slides are baked, deparaffinated and treated as previously described.

Slides are immuno stained according to the instructions in the Herceptest kit (DakoCytomation K5205), except for use of the spiked primary antibody solution instead of the original HER2 antibody solution.

Also, slides are stained exactly according to the instructions in the Herceptest kit (DakoCytomation K5205).

In short, the slides are incubated with primary antibody solution followed by incubation with an EnVision HRP anti rabbit visualization system using DAB as chromogen.

In parallel, slides with native fibres are stained.

Also, slides are stained with a negative antibody control antibody, and a negative control for the visualization system.
Figure 30A is a photomicrograph of stained slides. The DAB stained breast tissue is seen on the lower part of the slide. The fibre reference material is seen on the upper part of the slide.
Figure 30B to E show photomicrographs of the same stained slides using the anti DNP spiked primary reagent solution on the Herceptest ™ staining protocol.
Figure 30B and C is the DAB stained DNP fibres to the left/below and the red Lagertun fibres to the right/up, taken at 4 time and 40 time magnification, respectively.
Figure 30D and E is the DAB stained breast tissue, taken at 10 time and 4 time magnification, respectively.
Figure 30F to I show photomicrographs of the same stained slides using the original Herceptest ™ staining protocol, without spiked primary reagent.
Figure 30F and G is the stained DNP fibres to the left/below and the red Lagertun fibres to the right/up, taken at 4 and 40-time magnification, respectively.
Figure 30H is the DAB stained breast tissue, taken at 10-time magnification.
Figure 30I to L show photomicrographs of the same stained slides using the negative rabbit IgG antibody control reagent from the Herceptest ™ staining kit.
Figure 30I is the stained DNP fibres to the left/below and the red Lagertun fibres to the right/up, taken at 40-time magnification.
Figure 30J is the red Lagertun fibres taken at 10-time magnification.
Figure 30K and L is the DAB stained breast tissue, taken at 10 time and 4-time magnification, respectively.
Figure 30M to N show photomicrographs of the same stained slides using rabbit anti DNP-HRP conjugate (DakoCytomation K5102) diluted 100 times instead of the antibody reagent from the Herceptest ™ staining protocol.
Figure 30M is the stained DNP fibres to the left/below and the red Lagertun fibres to the right/up, taken at 40-time magnification.
Figure 30N is the stained breast tissue, taken at 4-time magnification.
Figure 300 to R show photomicrographs of the same stained slides using mouse Envision HRP visualization conjugate instead of the anti rabbit EnVision visualization reagent in the Herceptest ™ staining protocol.
Figure 300 is the DAB stained DNP fibres to the lefl/below and the red Lagertun fibres to the right/up, taken at 40-time magnification.
Figure 30P is the DAB stained breast tissue, taken at 10-time magnification, respectively.
Figure Q is the DAB stained red Portion fibres taken at 10-time magnification
Figure 30R is the stained breast tissue, taken at 4-time magnification.
Figure 30S show photomicrographs of Herceptest ™ stained native Lagertun fibre.
Figure 30T, U and V are photomicrographs ofHerceptest ™ stained o+, 1+ and 3+ reference cells, respectively.

### By studying the stained slides, it can be summarized that

The anti-DNP IgG spiked primary reagent in the Herceptest ™ stains the DNP fibre to approximately 1+ in intensity and at the same time the breast tissue.

The original Herceptest staining kit does not stain the DNP fibre, but stains the tissue.

Nonsense antibody does not stain the breast tissue, nor the DNP modified fibre.

Anti-DNP HRP IgG stained the DNP modified fibre and not the tissue. It also stained using the nonsense secondary visualization system. This is expected, as the anti-DNP conjugate contained HRP, which is a substrate for DAB.

By spiking with a HRP containing anti-DNP conjugate, the effect of the correct secondary antibody visualization system could be partly cancelled.

The herceptest^{™} kit worked satisfactory, according to the reference cells from the kit.

The red coloured fibre is not affected by the different staining procedures.

In conclusion, the example illustrates the possibility to make a simple control material for the correct application of correct primary reagent to the slide.

The red fibre easily identified the control material in this example. The positive DAB staining verified the correct addition of primary antibody reagent.

The coloured fibres make it possible to fast find the reference fibre on the slide. One can envision, that automated image processing systems can find the reference dots and check if the staining is above threshold intensity and thereby confirm the correct addition of the primary reagent.

### Example 12. Preparation of Bundles of Reference Material Containing Various Colours and Shapes

The example illustrates combinations of multiple collared fibres as indicators for position on the slide and shape.

Unifloss, LP floss and Lagertun fibres modified as in example 6 with red dye (Procion Red MX-5B) or blue dye (Cibacron Blue 3 GA) are bundled with fibres modified with HER2 modified fibres from example 9 and embedded in agarose with 0.05% PEI, fixed, dehydrated, paraffin embedded, cut and mounted on poly lysine slides as described in the previous examples.

Figure 31A, B and C show photomicrographs of the red and blue collared Unifloss, Lagertun and LP floss fibers taken at 5 (A), 10 (B) and 40 (C) time magnification.

The various shapes and colours can clearly be identified and separated from each other's.

The example illustrates the possibility to use the coloured fibres to help orient the slide and to calibrate an automated image analysis system.

### Example 13. Preparation and Immunostaining of randomly oriented reference material containing HER2 target.

The example illustrates the preparation of staining reference material using short fibres randomly cut, and staining controls using a fluorescent chromogen.

HER2 modified fibres from example 9 are cut in 2-5 mm pieces and embedded in agarose with 0.05% PEI in a small tube, before being fixed, dehydrated, paraffin embedded, cut and mounted on poly lysine slides as described in the previous examples.

Half of the slides with HER2 modified fibres are DAB stained using the Herceptest ™ according to the instructions.
Figure 32A and B show photomicrographs of immuno stained HER2 modified Lagertun fibres taken at 4 time magnification.
Figure 32C is 4 photomicrographs of the immuno stained HER2 modified Lagertun fibres taken at 20-time magnification.

The negative antibody control and negative Envision control did not show any staining (Not shown).

The other half of the slides are stained using the Herceptest according to the instructions except for the use of Alkaline phosphatase (AP) Envision dual link (DakoCytomation K4017) and Fast red chromogen (DakoCytomation K0597). The stained slides are mounted with Faramount aqueous mounting media and examined in both bright field and fluorescence microscope.
Figure 32D and E show photomicrographs taken in the bright field microscope of fast red immuno stained randomly cut HER2 modified Uni floss fibres taken at 40 time (D) and 20 time (E) magnification, respectively.
Figure 32F and G show photomicrographs taken in the bright field microscope of fast red immuno stained randomly cut HER2 modified Lagertun fibres taken at 40 time (F) and 20 time (G) magnification.
Figure 32H and I show photomicrographs taken in the bright field microscope of negative control antibody fast red immuno stained randomly cut HER2 modified Unifloss (H) and Lagertun (I) fibres taken 20 time magnification.
Figure 32J and K show photomicrographs taken in the bright field microscope of fast red immuno stained randomly cut native Unifloss (J) and Lagertun (K) fibres taken 20 time (G) magnification.
Figure 32L and M show photomicrographs taken in the fluorescence microscope of fast red immuno stained randomly cut HER2 modified Unifloss (L) and Lagertun (M) fibres taken at 40 time magnification.
Figure 32N show photomicrographs taken in the fluorescence microscope of negative control antibody fast red immuno stained randomly cut HER2 modified Unifloss fibres taken 40-time magnification.

In summary, the random oriented fibres make it more difficult to determine the staining intensity.

The Fast red chromogen appears strong and very distinct localized on the edge of the fibre.

The fluorescent staining pattern is similar to the DAB and Fast red staining seen in the bright field microscope. The fibres have some auto fluorescence, which is seen as a blue haze in the middle of the fibre.

The negative control staining give no visible staining on the fibre. Some weak unspecific staining can be detected outside the Uni floss fibre.

### Example 14. Preparation and Haematoxylin and Eosin Staining of Various Modified Reference Material

The example illustrates the staining of various native and modified fibres using non-immunological stains, Haematoxylin and Eosin ("HE") often used as a general counter stain.

Different FFPE blocks containing fibres are cut, mounted and stained:

The native fibres in FFPE blocks, HDI treated and hydrolysed fibre blocks from example 9.

CDI activated and rabbit IgG modified LP Floss, Unifloss and Lagertun fibres from example 8.

DNP coupled LP Floss, Unifloss and Lagertun fibres from example 1 (Aqueous reaction conditions, 25 mM F-DNP)

Fluorescein modified HDI fibre. The amino groups are introduced as described above in example 9 by treatment with HDI followed by hydrolysis. The three different amino functionalised fibres are added a fluorescein N-hydroxy succinimide ester solution (Molecular Probes, Eugene, Oregon, USA, cat no. C-1311, in total 10 mM FLU-NHS, 20% DMSO, 50 mm HEPES, pH 8.0) and shaken gently overnight at room temperature. Any remaining active groups are quenched by shaking with ethanol amine for 30 minutes (10 mM ethanol amine, 50 mM carbonate, pH 9.0). The fluorescein-modified fibres are washed three times with DMSO and water. The fibres are stored in water at 2-8 °C being embedded in agarose with 0.05% PEI, fixed, dehydrated, paraffin embedded, cut and mounted on poly lysine slides as described in the previous examples.

The blocks containing the native and modified fibre are cut, mounted on slides and deparaffinated as described previously.

The slides are placed in a vertical slide holder and immersed into a bath of filtered Mayer's Haematoxylin (Bie&Bertsen, Lab00254, Roedovre, Denmark) for 5 minutes at room temperature. The slides are washed under running tap water for 5 minutes before being immersed in a freshly prepared Eosin solution (10 ml 1% Eosin in 70% ethanol (Bie&Bertsen, Roedovre, Denmark) mixed with 190 ml 70% ethanol and 3 ml 0,20 M HCl).

The slide holder is removed from the solution, and excess reagent removed with filter paper. The slides are dehydrated by treatment in a series of ethanol baths and xylene (twice in 96% ethanol, twice in 99,9% ethanol and twice in xylene).

The slides are air dried and mounted with a permanent mounting media (DakoCytomation S3026)

For comparison, FFPE breast tissue is Haematoxylin and Eosin stained in parallel.
Figure 33 show photomicrographs of the Haematoxylin and Eosin stained slides taken at 40-time magnification, except for Figure 33S, which is taken at 20 times magnification.
Figure 33A, B and C are Haematoxylin and Eosin stained native LP Floss, Unifloss and Lagertun fibres, respectively.
Figure 33D, E and F are Haematoxylin and Eosin stained hexan diisocyanate modified and hydrolysed LP Floss, Unifloss and Lagertun fibres, respectively.
Figure 33G, H and I are Haematoxylin and Eosin stained rabbit IgG modified LP Floss, Unifloss and Lagertun fibres, respectively.
Figure 33J, K and L are Haematoxylin and Eosin stained fluorescein modified LP Floss, Unifloss and Lagertun fibres, respectively.
Figure 33M, N and O are Haematoxylin and Eosin stained DNP modified LP Floss, Unifloss and Lagertun fibres, respectively.
Figure 33P is Procion Red modified fibre bundles with DNP modified fibres (to the right)
Figure 33Q, R and S are HE stained FFPE breast tissue for comparison.

I general, the staining pattern is homogeneously distributed in the fibre materials.

The Lagertun fibres are all slightly more blue/red coloured, compared to the other native and modified fibre.

The fluorescein-modified fibres appeared to be taken up more colour than the native of otherwise modified fibres. This could be due to the both highly hydrophobic and charged fluorescein molecule.

In general, the Haematoxylin and Eosin staining did not give strong and localized staining patterns for the different fibre materials. This is highly advantageous, as Haematoxylin and Eosin staining is used as a general counter staining to help identify the general morphology.

### Example 15. Examples of Special Staining of Various Modified Reference Material

The following example illustrates the staining of various native and modified fibre using examples of non-immunological stains so-called special stains.

The FFPE blocks from the previous example, 14, containing the native and modified fibre, are cut and mounted on poly lysine slides as previously described.

The slides are mounted on the Artisan (Cytologix, Cambridge, MA and DakoCytomation) automated stainer capable of performing multiple special stains. The slides are stained on the Artisan instrument according to the kit instructions, using:
Periodic Acid Schiff stain (DakoCytomation cat. No AR165),
Alcian Blue pH 2,5 stain (DakoCytomation cat. No AR160),
Jones Basement membrane stain (DakoCytomation cat. No AR180),or
Masson's Trichrome stain (DakoCytomation cat. No AR173)

After staining, the slides are washed for 5 minutes in 99,9% ethanol and air dried for two hours before being mounted with a permanent mounting media (DakoCytomation cat. S3026) and stored overnight before being inspected and digitally photographed.
Figure 34, 35, 36 and 37 are photomicrographs of the special stained slides taken at 40-time magnification.
Figure 34A, B and C is Periodic Acid Schiff stained native LP Floss, Unifloss and Lagertun fibres, respectively.
Figure 34D, E and F are Periodic Acid Schiff stained fluorescein modified LP Floss, Unifloss and Lagertun fibres, respectively.
Figure 34G, H and I are Periodic Acid Schiff stained DNP modified LP Floss, Unifloss and Lagertun fibres, respectively.
Figure 35A and B are Alcian Blue stained native Unifloss and Lagertun fibres, respectively.
Figure 35C, D and E are Alcian Blue stained hexan diisocyanate modified and hydrolysed LP Floss, Unifloss and Lagertun fibres, respectively.
Figure 35F, G and H are Alcian Blue stained rabbit IgG modified LP Floss, Unifloss and Lagertun fibres, respectively.
Figure 35I, J and K are Alcian Blue stained fluorescein modified LP Floss, Unifloss and Lagertun fibres, respectively.
Figure 35L, M and N are Alcian Blue stained DNP modified LP Floss, Unifloss and Lagertun fibres, respectively.
Figure 36A and B are Jones Basement membrane stained native Unifloss and Lagertun fibres, respectively.
Figure 36C, D and E are Jones Basement membrane hexan diisocyanate modified and hydrolysed LP Floss, Unifloss and Lagertun fibres, respectively.
Figure 36F and G are Jones Basement membrane stained rabbit IgG modified LP Floss, Unifloss and Lagertun fibres, respectively.
Figure 36H, I and J are Jones Basement membrane stained fluorescein modified LP Floss, Unifloss and Lagertun fibres, respectively.
Figure 36K, L and M are Jones Basement membrane stained DNP modified LP Floss, Unifloss and Lagertun fibres, respectively.
Figure 37A, B and C is Masson's Trichrome stained native LP floss, Unifloss and Lagertun fibres, respectively.
Figure 37D, E and F are Masson's Trichrome stained hexan diisocyanate modified and hydrolysed LP Floss, Unifloss and Lagertun fibres, respectively.
Figure 37G and H are Masson's Trichrome stained rabbit IgG modified LP Floss, Unifloss and Lagertun fibres, respectively.
Figure 37I, J and K are Masson's Trichrome stained fluorescein modified LP Floss, Unifloss and Lagertun fibres, respectively.
Figure 37L, M and N are Masson's Trichrome stained DNP modified LP Floss, Unifloss and Lagertun fibres, respectively.

In general, there are significant differences in the staining intensities and distributed for the different fibres.

The Periodic Acid Schiff stain did not stain the native fibres much. The fluorescein modified LP floss and Uni Floss fibre have distinct reddish stain near the edges, whereas the Lagertun fibre is more homogeneously and stronger stained.

The DNP modified LP floss and Uni Floss fibre have little or no stain due to the Periodic Acid Schiff stain, whereas the DNP modified Lagertun have a homogeneous reddish stain.

The Alcian blue stain in general coloured all the Uni floss fibre blue in the interior and reddish on the edge, whereas the Lagertun n fibre only appeared weak homogeneously reddish.

The LP floss appeared blue, with some differences for the various modifications. The HDI and hydrolysed fibre appear reddish, whereas the IgG modified LP floss is blue.

The fluorescein modified LP floss has a reddish stain on the edge and a blue interior. The same can be seen even more strongly on the fluorescein modified Uni floss fibre. Some blue background staining can be observed between the fibres for all the Alcian blue stains.

All the Uni floss and LP floss fibre are strongly stained red by the Jones Basement membrane stain. The different Lagertun fibre only appears weakly reddish.

The blue background is very intense for all the Jones Basement membrane stainings, though all the fibres can be easily identified.

The Masson's Trichrome stain give strong bright red stains on all the Lagertun fibre, whereas the Unifloss and LP floss did not stain at all. The DNP modified fibres are stained somewhat weaker compared with the other Lagertun fibres.

In conclusion, the example illustrates the possibility to use the reference material
for e.g. the verification of the functionality of special stains. The nature of the original fibre seems to be more important for the staining intensity, colour and overall appearance, than the various chemical modifications.

### Example 16. Preparation, Immunostaining and Use of Reference Material Cut At Different Thickness

This example illustrates the use of the reference material for monitoring and verifying the thickness of the cut sections.

FFPE blocks containing permanently dyed fibres and DNP modified fibres are cut on a microtome in different thickness, mounted on slides, immuno stained and evaluated in a microscope.

In more detail:
Unifloss and Lagertun fibres modified as in example 6 with red dye (Procion Red MX-5B) are bundled with fibres modified with DNP modified Lagertun and Uniflos fibres from example 6 and 1 using 25 mM DNP during the aqueous coupling and embedded in agarose with 0.05% PEI, fixed, dehydrated and paraffin embedded, as described in the previous examples.
the three types of fibres are embedded together, resulting in paraffin blocks with permanently dyed fibres and uncoloured fibres with DNP hapten covalent attached.
in parallel, FPPE blocks containing native Unifloss and Lagertun fibre are cut and mounted on slides.
the blocks are cut in 3, 5 or 7 µm thickness on a standard microtome before being mounted on poly lysine slides as described in the previous examples.
the slides are immunostained as in example 4 using rabbit anti DNP-HRP and anti rabbit Envision HRP/ DAB plus.
in parallel, slides are stained using a negative primary antibody control (DakoCytomation rabbit negative control, N1699) or negative secondary visualization system control (anti mouse EnVision HRP/DAB plus) as described in the previous examples.
the resulting stained slides are inspected at 40-time magnification.

Figure 38 are photomicrographs of Procion Red modified Lagertun fibres cut at 3 µm (a), 5 µm (B) and 7 µm (C).
Figure 38 show photomicrographs of anti DNP Envision HRP/DAB stained DNP modified Lagertun fibres cut at 3 µm (D), 5 µm (E) and 7 µm (F).
Figure 38 show photomicrographs of anti DNP Envision HRP/DAB stained DNP modified Unifloss fibres cut at 3 µm (G), 5 µm (H) and 7 µm (I).
Figure 38 are photomicrographs of native uni floss fibre cut at 5 µm thickness (J), native Lagertun fibre cut at 5 µm thickness (K), negative primary antibody staining of DNP modified Lagertun fibre cut at 7 µm thickness (L) and negative Envision control staining of DNP modified Lagertun cut at 5 µm thickness (M).

The staining intensity is scored to approximately 0,5-1,0+; 0,5-1,0+ and 1,0-1,5+ for the DNP modified Lagertun fibres (Figure 38 D, E and F) cut at 3 µm (D), 5 µm (E) and 7 µm (F), respectively. The DNP modified Unifloss fibres (Figure 38 G, H and I) is scored to approximately 0,5+; 1,0+ and 1,5+ for the fibres cut at 3 µm (G), 5 µm (H) and 7 µm (I), respectively. The DNP modified Unifloss fibre appeared slightly easier to grade than the lagertun fibre.

the Figure 38D shows both the DAB stained fibres and the permanently dyed fibre.
the Procion red modified fibres are homogeneous coloured throughout the fibre, and do not to be affected by the immunostaining.
the DAB staining is located mainly in the edge with some diffuse staining in the interior.

Some unspecific staining is seen between the fibres. The background appears to be located in the agarose matrix. Some of the photomicrographs show fibres slightly out of optical focus.
it should be understood that the fibres are uniform in lengths and therefore will be mounted on the slides as columns with different heights.
a faint shadow can be seen as a dark rim on the fibres. The thicker the cut section, the more visible does the shadow seems. The precipitated DAB stain is though easily visible located and quantified.
the negative antibody control and negative Envision control did not show any staining
the various shapes and colours can clearly be identified and separated from each other's.
in conclusion, it is possible to cut the reference material of the invention in different thickness and obtain different intensities, both for directly dyed material and for the immuno stained material.
the example illustrates the possibility to use immunostained fibres to help to verify the microtome cutting thickness.
the example further illustrates the possibility to use the coloured fibres to help orient the slide and to calibrate an automated image analysis system with respect to shape, size and colour of the reference material.

## Claims

1. A method comprising:
(a) providing a reference standard or a planar section thereof, the reference standard comprising:
(i) a support medium; and
(ii) a quantity of a detectable entity supported by the support medium;
in which the detectable entity has an elongate path the cross-sectional profile of which has a size of between 0.5 µm to 100 µm; in which a predetermined amount of the detectable entity is present in a defined region in a cross section of the reference standard;
(b) obtaining a first reference signal indicative of the presence or quantity of detectable entity in the reference standard, planar section thereof, or the defined region;
(c) providing a biological sample and obtaining a second signal indicative of the presence or quantity of detectable entity in the biological sample, or a component thereof; and
(d) comparing the reference signal obtained in (b) against the second signal obtained in (c).

2. A method of obtaining an indication of the presence, quantity or concentration of a detectable entity in a biological sample according to Claim 1, in which the reference signal is compared to the second signal to determine the presence, quantity or concentration of the detectable entity in the sample.

3. A method according to Claim 1 or 2, in which the reference standard or planar section thereof is subjected to the same one or more steps or conditions, preferably substantially all, as the biological sample.

4. A method according to Claim 1, 2 or 3, in which the reference standard or planar section thereof is processed through one or more, preferably all, of the following steps: mounting onto a slide, baking, deparaffination, rehydration, antigen retrieval, blocking, exposure to antibody, exposure to primary antibody, exposure to nucleic acid probe, washing, exposure to secondary antibody-enzyme conjugate, exposure to enzyme substrate, exposure to chromogen substrate and counter staining.

5. A method according to any preceding claim, in which the detectable entity is selected from the group consisting of: a hapten, a biologically active molecule, an antigen, an epitope, a protein, a polypeptide, a peptide, an antibody, a nucleic acid, a virus, a virus-like particle, a nucleotide, a ribonucleotide, a deoxyribonucleotide, a modified deoxyribonucleotide, a heteroduplex, a nanoparticle, a synthetic analogue of a nucleotide, a synthetic analogue of a ribonucleotide, a modified nucleotide, a modified ribonucleotide, an amino acid, an amino acid analogue, a modified amino acid, a modified amino acid analogue, a steroid, a proteoglycan, a lipid, a carbohydrate, a dye, a diagnostically relevant target, preferably selected from the group consisting of: an antigen, an epitope, a peptide, a polypeptide, a protein, a nucleic acid, or two or more or a plurality of any of the above, or mixtures, fusions, combinations or conjugates of one or more of the above.

6. A method according to any preceding claim, in which the detectable entity comprises any one or more of HER2, ER, PR, p16, Ki-67 and EGFR protein, and nucleic acids encoding such.

7. A method according to any preceding claim, further comprising providing a second reference standard or a planar section thereof, obtaining a second reference signal indicative of the quantity of detectable entity in the second reference standard, planar section thereof or the defined region; and comparing the second signal obtained in (c) against the first reference signal and the second reference signal.

8. A method according to any preceding claim, in which the presence and/or quantity of the detectable entity is revealable by a binding agent, preferably a labelled binding agent, preferably selected from the group consisting of: an antibody, preferably an antibody capable of specific binding to the detectable entity, a nucleic acid such as a DNA or an RNA, preferably a nucleic acid capable of specific binding to the detectable entity, a protein nucleic acid (PNA), a dye, a special stain, Haematoxylin-Eosin (H & E), Gomori methenamine silver stain (GMS), Periodic Acid-Schiff (PAS) stain, Trichrome Blue, Masson's Trichrome, Prussian Blue, Giemsa, Diff-Quik, Reticulum, Congo Red, Alcian Blue, Steiner, AFB, PAP, Gram, Mucicarmine, Verhoeff-van Gieson, Elastic, Carbol Fuchsin and Golgi's stains.

9. A method according to any preceding claim, in which the detectable signal is selected from the group consisting of: radiation, optical density, reflectance, radioactivity, fluorescence, enzymatic activity.

10. A method according to any preceding claim, in which:
(a) the reference standard is in the shape of a rectangular box, and the detectable entity is in the form of a substantially linear rod disposed along or substantially parallel to a long axis of the reference standard; or
(b) the defined region is present in at least one other cross section of the reference standard, preferably comprising a similar amount of detectable entity; or
(c) the detectable entity has a substantially uniform distribution along the elongate path; or
(d) the detectable entity is present at substantially the same area, quantity or concentration in all transverse planar sections of the reference standard;
or any combination of the above.

11. A method according to any preceding claim, in which the support medium comprises an embedding medium, in which the detectable entity is embedded, the embedding medium preferably being selected from the group consisting of: ice, wax, paraffin, acrylic resin, methacrylate resin, epoxy, Epon, Araldite, Lowicryl, K4M and LR White and Durcupan.

12. A method according to any preceding claim, in which the detectable entity is attached, preferably chemically coupled, to an elongate fibre, preferably selected from the group consisting of: a polyamide fibre, a cellulose fibre, a polycarbamate fibre, a silk fibre, a polyester fibre, a Nylon fibre, a Rayon fibre and blends thereof.

13. A method according to Claim 12, in which the detectable entity is substantially uniform at both core and surface portions of the fibre.

14. A method according to Claim 12, in which the detectable entity at surface portions of the fibre is present in greater amount than at core portions, preferably in which core portions of the fibre comprise substantially no detectable entity.

15. A method according to any of Claims 1 to 11, in which the detectable entity is formed in an elongate channel in the embedding medium.

16. A method according to any of Claims 12 to 15, in which the fibre or channel has a uniform cross sectional area across substantially its entire length, preferably in which the fibre or channel has a diameter of between about 0.5µm to 25µm, preferably between 1.5µm to 15µm.

17. A method according to any preceding claim, in which the reference standard comprises:
(a) two or more linear fibres or channels in substantially parallel orientation, preferably in a bundle; or
(b) two or more different detectable entities, each of which is disposed in or on an individual fibre or channel; or
(c) two or more fibres or channels each comprising the same detectable entity, preferably comprising different amounts of detectable entity on each;
or any combination of the above.

18. A method according to any preceding claim, in which a planar section of the reference standard comprises a plurality of areas on which are presented the detectable entity at different density.

19. A method according to any preceding claim, in which a planar section of the reference standard comprises a first area comprising the detectable entity substantially at a diagnostically significant density, preferably further comprising a control comprising a fibre or channel which comprises substantially no detectable entity.

20. A method according to any preceding claim for obtaining an indication useful,in the assessment of the likelihood of a disease or a condition in an individual, in which the presence of the detectable entity, or the presence of a predetermined quantity of the detectable entity, in the biological sample is indicative of the likelihood of a disease or a condition in the biological sample or an individual from which the sample is taken.

21. A method according to any preceding claim, in which the biological sample comprises a cell, tissue or organ, preferably a cell, tissue or organ of an organism suspected of suffering a disease or condition.

22. A method of providing an indication useful in the diagnosis of a disease or a condition in an individual, the method comprising comparing the amount of a detectable entity in a biological sample from the individual or component thereof with a reference standard in a method according to any preceding claim, in which the individual is indicated as likely to be suffering from or susceptible to the disease or condition if the amount of detectable entity in the biological sample or component is similar to or greater than that in the reference standard.

23. A method of assessing the effectiveness or success of a procedure, the method comprising the steps of:
(a) providing a reference standard or a planar section thereof, the reference standard comprising:
(i) a support medium; and
(ii) a quantity of a detectable entity supported by the support medium;
in which the detectable entity has an elongate path the cross-sectional profile of which has a size of between 0.5 µm to 100 µm; in which a predetermined amount of the detectable entity is present in a defined region in a cross section of the reference standard; and
in which a detectable property of the detectable entity is changed as a result of the procedure;
(b) conducting the procedure on the reference standard; and
(c) detecting a change in the detectable property of the detectable entity.

24. A method according to Claim 23 for assessing the effectiveness or success of a procedure carried out on a sample, in which the procedure is conducted on the reference standard or planar section substantially in parallel with the sample.

25. A method according to Claim 23 or 24, in which a detectable property of the detectable entity is changed as a result of a successful procedure, which change in the detectable property of the detectable entity is detected to establish that the procedure is successful.

26. A method according to Claim 23 or 24, in which a detectable property of the detectable entity is changed as a result of an unsuccessful procedure, which change in the detectable property of the detectable entity is detected to establish that the procedure is not successful.

27. A method according to any of Claims 23 to 26, in which the procedure is selected from the group consisting of: an *in situ* hybridisation procedure, an immunohistochemical procedure, deparaffination, antigen retrieval, blocking, endogenous biotin blocking, endogenous enzyme blocking, a washing step, incubation with revealing agent such as a primary antibody, incubation with secondary visualisation components, chromogen staining, staining information acquisition and analysis.

28. A method according to any of Claims 23 to 27, in which the procedure is an antigen retrieval procedure, and in which the detectable property of the detectable entity comprises the masking or unmasking of one or more epitopes.

29. A method according to any of Claims 23 to 27, in which the detectable entity in the reference standard is modified to mask one or more epitopes, some or all of which are unmasked in an antigen retrieval procedure which is successful.

30. A method according to any of Claims 23 to 27, in which the procedure is an deparaffination procedure, and in which the detectable property of the detectable entity comprises the presence or quantity of detectable entity in the reference standard following the deparaffination procedure.

31. A method according to any of Claims 23 to 27, in which the detectable entity in the reference standard is soluble in the deparaffination medium, and in which at least a portion, preferably all, of the detectable entity is removed following a successful deparaffination procedure.

32. A method according to any of Claims 23 to 27, in which the effectiveness or success of a two or more procedures are assessed preferably in parallel.

33. Use of a reference standard for validating a procedure, in which the reference standard comprises:
(i) a support medium; and
(ii) a quantity of a detectable entity supported by the support medium;
in which the detectable entity has an elongate path the cross-sectional profile of which has a size of between 0.5 µm to 100 µm; in which a predetermined amount of the detectable entity is present in a defined region in a cross section of the reference standard, and in which a detectable property of the detectable entity is changed as a result of the procedure.

34. Use according to Claim 33, in which the reference standard is used as an antigen retrieval validation standard, a deparaffination standard, a blocking validation standard, a washing validation standard, a primary antibody validation standard, a secondary antibody validation standard, a calibration standard, or a diagnostic standard.

35. A kit comprising a reference standard or a planar section thereof together with instructions for use in a method according to any preceding claim, in which the reference standard comprises:
(i) a support medium; and
(ii) a quantity of a detectable entity supported by the support medium;
in which the detectable entity has an elongate path the cross-sectional profile of which has a size of between 0.5 µm to 100 µm; in which a predetermined amount of the detectable entity is present in a defined region in a cross section of the reference standard.

36. A kit according to Claim 35, further comprising a binding agent capable of specific binding to the detectable entity, which binding agent generates a signal indicative of the presence or quantity of detectable entity in the reference standard, planar section thereof, or the defined region.

37. A diagnostic kit for detecting a diagnostically relevant presence or amount of a detectable entity in a biological sample, the diagnostic kit comprising a kit according to Claim 35 or 36.

38. A kit or diagnostic kit according to any of Claims 35 to 37 together with a therapeutic agent capable of treating or alleviating at least one of the symptoms of a disease or condition in an individual, preferably comprising an antibody against the detectable entity.

39. A set of two or more planar sections each derived from a reference standard, which reference standard comprises:
(i) a support medium; and
(ii) a quantity of a detectable entity supported by the support medium;
in which the detectable entity has an elongate path the cross-sectional profile of which has a size of between 0.5 µm to 100 µm; in which a predetermined amount of the detectable entity is present in a defined region in a cross section of the reference standard, in which at least two planar sections in the set comprise a different amount of detectable entity.

40. A set of two or more planar sections each comprising a defined region comprising detectable entity, in which the quantity of the detectable entity in the defined region is different between at least two of the planar sections in the set, each planar section being derived from a reference standard comprising:
(i) a support medium; and
(ii) a quantity of a detectable entity supported by the support medium;
in which the detectable entity has an elongate path the cross-sectional profile of which has a size of between 0.5 µm to 100 µm.

## Patentansprüche

1. Verfahren, welches folgendes umfaßt:
(a) Bereitstellen eines Referenzstandards oder eines ebenen Schnitts davon, wobei der Referenzstandard folgendes umfaßt:
(i) ein Trägermedium und
(ii) eine Menge einer detektierbaren Einheit, die von dem Trägermedium getragen wird,
wobei die detektierbare Einheit einen länglichen Pfad beschreibt, dessen Querschnittsprofil eine Größe von zwischen 0,5 µm und 100 µm hat, wobei eine vorbestimmte Menge der detektierbaren Einheit in einem definierten Bereich in einem Querschnitt des Referenzstandards vorliegt,
(b) Erhalten eines ersten Referenzsignals, welches das Vorliegen oder die Menge einer detektierbaren Einheit in dem Referenzstandard, dem ebenen Schnitt davon oder dem definierten Bereich anzeigt,
(c) Bereitstellen einer biologischen Probe und Erhalten eines zweiten Signals, welches das Vorliegen oder die Menge der detektierbaren Einheit in der biologischen Probe oder einer Komponente davon anzeigt, und
(d) Vergleichen des in (b) erhaltenen Referenzsignals mit dem in (c) erhaltenen zweiten Signal.

2. Verfahren zum Erhalten einer Anzeige bezüglich des Vorliegens, der Menge oder der Konzentration einer detektierbaren Einheit in einer biologischen Probe nach Anspruch 1, wobei das Referenzsignal mit dem zweiten Signal verglichen wird, um das Vorliegen, die Menge oder die Konzentration der detektierbaren Einheit in der Probe zu bestimmen.

3. Verfahren nach Anspruch 1 oder 2, wobei der Referenzstandard oder der ebene Schnitt davon der oder den gleichen und vorzugsweise im wesentlichen allen Stufe(n) oder Bedingung(en) unterworfen wird wie die biologische Probe.

4. Verfahren nach einem der Ansprüche 1, 2 oder 3, wobei der Referenzstandard oder der ebene Schnitt davon durch eine oder mehrere und vorzugsweise alle der folgenden Stufen verarbeitet wird: Auflegen auf einen Objektträger, Backen, Entparaffinieren, Rehydratisieren, Antigengewinnung, Blockieren, Aussetzen an Antikörper, Aussetzen an primären Antikörper, Aussetzen an Nukleinsäuresonde, Waschen, Aussetzen an sekundäres Antikörper-Enzym-Konjugat, Aussetzen an Enzymsubstrat, Aussetzen an Chromogensubstrat und Kontrastfärbung.

5. Verfahren nach einem der vorangegangenen Ansprüche, wobei die detektierbare Einheit aus der Gruppe ausgewählt ist, bestehend aus einem Hapten, einem biologisch aktiven Molekül, einem Antigen, einem Epitop, einem Protein, einem Polypeptid, einem Peptid, einem Antikörper, einer Nukleinsäure, einem Virus, einem virusartigen Partikel, einem Nukleotid, einem Ribonukleotid, einem Desoxyribonukleotid, einem modifizierten Desoxyribonukleotid, einem Heteroduplex, einem Nanopartikel, einem synthetischen Analogen eines Nukleotids, einem synthetischen Analogen eines Ribonukleotids, einem modifizierten Nukleotid, einem modifizierten Ribonukleotid, einer Aminosäure, einem Aminosäureanalogen, einer modifizierten Aminosäure, einem modifizierten Aminosäureanalogen, einem Steroid, einem Proteoglycan, einem Lipid, einem Kohlenhydrat, einem Farbstoff, einem diagnostisch relevanten Ziel, vorzugsweise ausgewählt aus der Gruppe, bestehend aus einem Antigen, einem Epitop, einem Peptid, einem Polypeptid, einem Protein, einer Nukleinsäure oder zwei oder mehreren aus einer Mehrzahl von irgendwelchen der oben genannten oder Gemischen, Fusionen, Kombinationen oder Konjugaten von einem oder mehreren der oben genannten.

6. Verfahren nach einem der vorangegangenen Ansprüche, wobei die detektierbare Einheit irgendeines oder mehrere von HER2-, ER-, PR-, p16-, Ki-67- und EGFR-Protein und diese codierende Nukleinsäuren umfaßt.

7. Verfahren nach einem der vorangegangenen Ansprüche, welches weiterhin die Bereitstellung eines zweiten Referenzstandards oder eines ebenen Schnitts davon, das Erhalten eines zweiten Referenzsignals, welches die Menge der detektierbaren Einheit in dem zweiten Referenzstandard, dem ebenen Schnitt davon oder dem definierten Bereich anzeigt, und das Vergleichen des in (c) erhaltenen zweiten Signals mit dem ersten Referenzsignal und dem zweiten Referenzsignal umfaßt.

8. Verfahren nach einem der vorangegangenen Ansprüche, wobei das Vorliegen und/oder die Menge der detektierbaren Einheit durch ein Bindemittel, vorzugsweise ein markiertes Bindemittel erfaßt werden kann, welches vorzugsweise aus der Gruppe ausgewählt ist, bestehend aus einem Antikörper, vorzugsweise einem Antikörper, der spezifisch an die detektierbare Einheit binden kann, einer Nukleinsäure, wie einer DNA oder einer RNA, vorzugsweise einer Nukleinsäure, die spezifisch an die detektierbare Einheit binden kann, einer Proteinnukleinsäure (PNA), einem Farbstoff, einem speziellen Färbemittel, Hämatoxylin-Eosin (H & E), Gomori-Methenaminsilber-Färbemittel (GMS), Periodic-Acid-Schiff- (PAS-) Färbemittel, Trichromblau, Massons Trichrome, Preußischblau, Giemsa, Diff-Quik, Reticulum, Kongo-Rot, Alcian-Blau, Steiner, AFB, PAP, Gram, Mucikarmin, Verhoeff-van Gieson, Elastic, Carbol-Fuchsin und Golgi-Färbemittel.

9. Verfahren nach einem der vorangegangenen Ansprüche, wobei das detektierbare Signal aus der Gruppe ausgewählt ist, bestehend aus Strahlung, optischer Dichte, Reflexion, Radioaktivität, Fluoreszenz und enzymatischer Aktivität.

10. Verfahren nach einem der vorangegangenen Ansprüche, wobei
(a) der Referenzstandard die Form eines rechteckigen Kastens hat und die detektierbare Einheit die Form einer im wesentlichen linearen Stange hat, die entlang oder im wesentlichen parallel zu einer Längsachse des Referenzstandards angeordnet ist,
(b) der definierte Bereich in wenigstens einem anderen Querschnitt des Referenzstandards vorliegt, welcher vorzugsweise eine ähnliche Menge der detektierbaren Einheit umfaßt, und
(c) die detektierbare Einheit entlang des länglichen Pfades im wesentlichen einheitlich verteilt ist, oder
(d) die detektierbare Einheit in allen quer geführten ebenen Schnitten des Referenzstandards in im wesentlichen demselben Bereich, derselben Menge oder Konzentration vorliegt, oder irgendeine Kombination der obigen.

11. Verfahren nach einem der vorangegangenen Ansprüche, wobei das Trägermedium ein Einbettungsmedium umfaßt, in welches die detektierbare Einheit eingebettet ist, wobei das Einbettungsmedium vorzugsweise aus der Gruppe ausgewählt ist, bestehend aus Eis, Wachs, Paraffin, Acrylharz, Methacrylatharz, Epoxid, Epon, Araldit, Lowicryl, K4M und LR-Weiß und Durcupan.

12. Verfahren nach einem der vorangegangenen Ansprüche, wobei die detektierbare Einheit an eine längliche Faser, vorzugsweise ausgewählt aus der Gruppe, bestehend aus einer Polyamidfaser, einer Zellulosefaser, einer Polycarbamatfaser, einer Seidenfaser, einer Polyesterfaser, einer Nylonfaser, einer Rayonfaser und Gemischen davon, angefügt, vorzugsweise chemisch gekoppelt ist.

13. Verfahren nach Anspruch 12, wobei die detektierbare Einheit sowohl an den Kern- als auch in den Oberflächenabschnitten der Faser im wesentlichen gleichmäßig verteilt ist.

14. Verfahren nach Anspruch 12, wobei die detektierbare Einheit an den Oberflächenabschnitten der Faser in größeren Mengen vorliegt als in den Kernabschnitten, wobei vorzugsweise die Kernabschnitte der Faser im wesentlichen keine detektierbare Einheit umfassen.

15. Verfahren nach einem der Ansprüche 1 bis 11, wobei die detektierbare Einheit in einem länglichen Kanal in dem Einbettungsmedium gebildet ist.

16. Verfahren nach einem der Ansprüche 12 bis 15, wobei die Faser oder der Kanal im wesentlichen über ihre bzw. seine gesamte Länge hinweg eine gleichmäßige Querschnittsfläche hat, wobei die Faser oder der Kanal vorzugsweise einen Durchmesser von zwischen etwa 0,5 µm bis 25 µm, bevorzugt zwischen 1,5 µm und 15 µm hat.

17. Verfahren nach einem der vorangegangenen Ansprüche, wobei der Referenzstandard folgendes umfaßt:
(a) zwei oder mehr lineare Fasern oder Kanäle in im wesentlichen paralleler Ausrichtung, vorzugsweise in einem Bündel, oder
(b) zwei oder mehr verschiedene detektierbare Einheiten, von denen jede in oder auf einer einzelnen Faser oder einem einzelnen Kanal angeordnet ist, oder
(c) zwei oder mehr Fasern oder Kanäle, die jeweils dieselbe detektierbare Einheit umfassen, die vorzugsweise jeweils verschiedene Mengen der detektierbaren Einheit aufweisen, oder irgendeine Kombination der obigen.

18. Verfahren nach einem der vorangegangenen Ansprüche, wobei ein ebener Schnitt des Referenzstandards eine Mehrzahl von Bereichen umfaßt, in welchen die detektierbare Einheit in unterschiedlichen Dichten präsentiert wird.

19. Verfahren nach einem der vorangegangenen Ansprüche, wobei ein ebener Schnitt des Referenzstandards einen ersten Bereich umfaßt, welcher die detektierbare Einheit im wesentlichen in einer diagnostisch signifikanten Dichte umfaßt, und vorzugsweise weiterhin eine Kontrolle umfaßt, welche eine Faser oder einen Kanal umfaßt, die bzw. der im wesentlichen keine detektierbare Einheit umfaßt.

20. Verfahren nach einem der vorangegangenen Ansprüche zum Erhalten einer Anzeige, die für die Bestimmung der Wahrscheinlichkeit einer Erkrankung oder eines Zustands in einem Individuum geeignet ist, wobei das Vorliegen der detektierbaren Einheit oder das Vorliegen einer vorbestimmten Menge der detektierbaren Einheit in der biologischen Probe die Wahrscheinlichkeit einer Erkrankung oder eines Zustands in der biologischen Probe oder in einem Individuum, von dem die Probe genommen wurde, anzeigt.

21. Verfahren nach einem der vorangegangenen Ansprüche, wobei die biologische Probe eine Zelle, ein Gewebe oder ein Organ, vorzugsweise eine Zelle, ein Gewebe oder ein Organ eines Organismus, von vermutet wird, daß er an einer Erkrankung oder einem Zustand leidet, umfaßt.

22. Verfahren zum Bereitstellen einer Anzeige, die bei der Diagnose einer Erkrankung oder eines Zustands in einem Individuum geeignet ist, wobei das Verfahren umfaßt, daß man die Menge einer detektierbaren Einheit in einer biologischen Probe von dem Individuum oder einer Komponente davon mit einem Referenzstandard in einem Verfahren gemäß einem der vorangegangenen Ansprüche vergleicht, wobei angezeigt wird, daß das Individuum wahrscheinlich an der Erkrankung oder dem Zustand leidet oder dafür empfänglich ist, wenn die Menge der detektierbaren Einheit in der biologischen Probe oder der Komponente ähnlich derjenigen oder größer als diejenige in dem Referenzstandard ist.

23. Verfahren zum Bestimmen der Wirksamkeit oder des Erfolgs einer Maßnahme, wobei das Verfahren die folgenden Stufen umfaßt:
(a) Bereitstellen eines Referenzstandards oder eines ebenen Schnitts davon, wobei der Referenzstandard folgendes umfaßt:
(i) ein Trägermedium und
(ii) eine Menge einer detektierbaren Einheit, die von dem Trägermedium getragen wird,
wobei die detektierbare Einheit einen länglichen Pfad beschreibt, dessen Querschnittsprofil eine Größe von zwischen 0,5 µm und 100 µm hat, wobei eine vorbestimmte Menge der detektierbaren Einheit in einem definierten Bereich in einem Querschnitt des Referenzstandards vorliegt, und
wobei eine detektierbare Eigenschaft der detektierbaren Einheit als Ergebnis der Maßnahme verändert wird,
(b) Durchführen der Maßnahme an dem Referenzstandard und
(c) Erfassen einer Veränderung in der detektierbaren Eigenschaft der detektierbaren Einheit.

24. Verfahren nach Anspruch 23 zum Feststellen der Wirksamkeit oder des Erfolgs einer an einer Probe durchgeführten Maßnahme, wobei die Maßnahme an dem Referenzstandard oder einem ebenen Schnitt, der im wesentlichen parallel zu der Probe ist, durchgeführt wird.

25. Verfahren nach Anspruch 23 oder 24, wobei eine detektierbare Eigenschaft der detektierbaren Einheit als Ergebnis einer erfolgreichen Maßnahme verändert wird, wobei die Veränderung in der detektierbaren Eigenschaft der detektierbaren Einheit erfaßt wird, um festzustellen, daß die Maßnahme erfolgreich war.

26. Verfahren nach Anspruch 23 oder 24, wobei eine detektierbare Eigenschaft der detektierbaren Einheit als Ergebnis einer nicht erfolgreichen Maßnahme verändert wird, wobei die Veränderung in der detektierbaren Eigenschaft der detektierbaren Einheit erfaßt wird, um festzustellen, daß die Maßnahme nicht erfolgreich war.

27. Verfahren nach einem der Ansprüche 23 bis 26, wobei die Maßnahme aus der Gruppe ausgewählt ist, bestehend aus einer *in situ*-Hybridisierungsmaßnahme, einer immunohistochemischen Maßnahme, Entparaffinierung, Antigengewinnung, Blockieren, endogener Biotin-Blockierung, endogener Enzymblockierung, einer Waschstufe, Inkubation mit einem Aufdeckungsmittel, wie einem primären Antikörper, Inkubation mit sekundären Visualisierungskomponenten, Chromogenfärbung, Gewinnung von Färbungsinformationen und Analyse.

28. Verfahren nach einem der Ansprüche 23 bis 27, wobei die Maßnahme eine Antigengewinnungsmaßnahme ist und wobei die detektierbare Eigenschaft der detektierbaren Einheit das Maskieren oder Demaskieren eines oder mehrerer Epitope umfaßt.

29. Verfahren nach einem der Ansprüche 23 bis 27, wobei die detektierbare Einheit in dem Referenzstandard modifiziert wird, um ein oder mehrere Epitope zu maskieren, von denen einige oder alle in einer erfolgreichen Antigengewinnungsmaßnahme demaskiert werden.

30. Verfahren nach einem der Ansprüche 23 bis 27, wobei die Maßnahme eine Entparaffinierungsmaßnahme ist und wobei die detektierbare Eigenschaft der detektierbaren Einheit das Vorliegen oder die Menge der detektierbaren Einheit in dem Referenzstandard nach der Entparaffinierungsmaßnahme umfaßt.

31. Verfahren nach einem der Ansprüche 23 bis 27, wobei die detektierbare Einheit in dem Referenzstandard in dem Entparaffinierungsmedium löslich ist und wobei wenigstens ein Teil der und vorzugsweise die gesamte detektierbare Einheit im Anschluß an eine erfolgreiche Entparaffinierungsmaßnahme entfernt wird.

32. Verfahren nach einem der Ansprüche 23 bis 27, wobei die Wirksamkeit oder der Erfolg zweier oder mehrerer Maßnahmen vorzugsweise parallel bestimmt wird.

33. Verwendung eines Referenzstandards zur Validierung einer Maßnahme, wobei der Referenzstandard folgendes umfaßt:
(i) ein Trägermedium und
(ii) eine Menge einer detektierbaren Einheit, die von dem Trägermedium getragen wird,
wobei die detektierbare Einheit einen länglichen Pfad beschreibt, dessen Querschnittsprofil eine Größe von zwischen 0,5 µm und 100 µm hat, wobei eine vorbestimmte Menge der detektierbaren Einheit in einem definierten Bereich in einem Querschnitt des Referenzstandards vorliegt und wobei eine detektierbare Eigenschaft der detektierbaren Einheit als Ergebnis der Maßnahme verändert wird.

34. Verwendung nach Anspruch 33, wobei der Referenzstandard als ein Validierungsstandard für die Antigengewinnung, ein Entparaffinierungsstandard, ein Validierungsstandard für die Blockierung, ein Validierungsstandard für das Waschen, ein Validierungsstandard für primäre Antikörper, ein Validierungsstandard für sekundäre Antikörper, ein Kalibrierungsstandard oder ein diagnostischer Standard verwendet wird.

35. Kit, welcher einen Referenzstandard oder einen ebenen Schnitt davon zusammen mit Anweisungen für die Verwendung in einem Verfahren gemäß einem der vorangegangenen Ansprüche umfaßt, wobei der Referenzstandard folgendes umfaßt:
(i) ein Trägermedium und
(ii) eine Menge einer detektierbaren Einheit, die von dem Trägermedium getragen wird,
wobei die detektierbare Einheit einen länglichen Pfad beschreibt, dessen Querschnittsprofil eine Größe von zwischen 0,5 µm und 100 µm hat, wobei eine vorbestimmte Menge der detektierbaren Einheit in einem definierten Bereich in einem Querschnitt des Referenzstandards vorliegt.

36. Kit nach Anspruch 35, welcher weiterhin ein Bindemittel umfaßt, welches spezifisch an die detektierbare Einheit binden kann, wobei das Bindemittel ein Signal erzeugt, welches das Vorliegen oder die Menge der detektierbaren Einheit in dem Referenzstandard, dem ebenen Schnitt davon oder dem definierten Bereich anzeigt.

37. Diagnostischer Kit zum Detektieren eines diagnostisch relevanten Vorliegens oder der Menge einer detektierbaren Einheit in einer biologischen Probe, wobei der diagnostische Kit einen Kit nach Anspruch 35 oder 36 umfaßt.

38. Kit oder diagnostischer Kit nach einem der Ansprüche 35 bis 37 zusammen mit einem therapeutischen Mittel, welches wenigstens eines der Symptome einer Erkrankung oder eines Zustands in einem Individuum behandeln oder lindern kann, und der vorzugsweise einen Antikörper gegen die detektierbare Einheit umfaßt.

39. Satz von zwei oder mehreren ebenen Schnitten, die jeweils von einem Referenzstandard erhalten wurden, wobei der Referenzstandard folgendes umfaßt:
(i) ein Trägermedium und
(ii) eine Menge einer detektierbaren Einheit, die von dem Trägermedium getragen wird,
wobei die detektierbare Einheit einen länglichen Pfad beschreibt, dessen Querschnittsprofil eine Größe von zwischen 0,5 µm und 100 µm hat, wobei eine vorbestimmte Menge der detektierbaren Einheit in einem definierten Bereich in einem Querschnitt des Referenzstandards vorliegt, wobei wenigstens zwei ebene Schnitte in dem Satz eine unterschiedliche Menge der detektierbaren Einheit umfassen.

40. Satz von zwei oder mehreren ebenen Schnitten, welche einen definierten Bereich umfassen, der eine detektierbare Einheit aufweist, wobei die Menge der detektierbaren Einheit in dem definierten Bereich zwischen wenigstens zweien der ebenen Schnitt in dem Satz unterscheidet, wobei jeder ebene Schnitt von einem Referenzstandard erhalten wurde, welcher folgendes umfaßt:
(i) ein Trägermedium und
(ii) eine Menge einer detektierbaren Einheit, die von dem Trägermedium getragen wird,
wobei die detektierbare Einheit einen länglichen Pfad beschreibt, dessen Querschnittsprofil eine Größe von zwischen 0,5 µm und 100 µm hat.

## Revendications

1. Processus comprenant :
(a) la fourniture d'un étalon de référence ou de la coupe plane de celui-ci, l'étalon de référence comprenant :
(i) un support ; et
(ii) une quantité d'une entité détectable portée par le support ;
dans lequel l'entité détectable a la forme d'une tige allongée dont la coupe transversale a une dimension comprise entre 0,5 µm et 100 µm ; dans lequel une quantité prédéterminée de l'entité détectable est présente dans une zone définie de la coupe transversale de l'étalon de référence ;
(b) l'obtention d'un premier signal de référence indiquant la présence ou la quantité de l'entité détectable dans l'étalon de référence, dans la coupe plane de celui-ci, ou dans la zone désignée ;
(c) la fourniture d'un échantillon biologique et l'obtention d'un second signal indiquant la présence ou la quantité de l'entité détectable dans l'échantillon biologique, ou dans un de ses composants ; et
(d) la comparaison du signal de référence obtenu en (b) avec le second signal obtenu en (c).

2. Processus d'obtention de l'indication de la présence, de la quantité ou de la concentration d'une entité détectable dans un échantillon biologique selon la revendication 1, dans lequel le signal de référence est comparé au second signal pour déterminer la présence, la quantité ou la concentration de l'entité détectable dans l'échantillon.

3. Processus selon la revendication 1 ou 2, dans lequel l'étalon de référence ou la coupe plane de celui-ci est soumis à la même ou aux mêmes étapes ou conditions, de préférence sensiblement toutes, que l'échantillon biologique.

4. Processus selon la revendication 1, 2 ou 3, dans lequel l'étalon de référence ou la coupe plane de celui-ci est traité en passant par une ou plusieurs des étapes suivantes, de préférence toutes : montage sur une plaque, cuisson, déparaffinage, réhydratation, démasquage des antigènes, blocage, exposition à l'anticorps, exposition à l'anticorps primaire, exposition à la sonde d'acide nucléique, lavage, exposition au couplage anticorps secondaire - enzyme, exposition au substrat de l'enzyme, exposition au substrat chromogène et coloration de contraste.

5. Processus selon l'une quelconque des revendications précédentes, dans lequel l'entité détectable est choisie dans le groupe constitué par : un haptène, une molécule biologiquement active, un antigène, un épitope, une protéine, un polypeptide, un peptide, un anticorps, un acide nucléique, un virus, une particule ressemblant à un virus (virus-like), un nucléotide, un ribonucléotide, un désoxyribonucléotide, un désoxyribonucléotide modifié, un hétéroduplex, une nanoparticule, un analogue synthétique de nucléotide, un analogue synthétique de ribonucléotide, un nucléotide modifié, un ribonucléotide modifié, un acide aminé, un analogue d'acide aminé, un acide aminé modifié, un analogue d'acide aminé modifié, un stéroïde, un protéoglycane, un lipide, un glucide, un colorant, une cible pertinente à diagnostiquer choisie de préférence dans le groupe constitué par : un antigène, un épitope, un peptide, un polypeptide, une protéine, un acide nucléique ou bien deux ou davantage ou une pluralité de n'importe lesquels de ceux mentionnés ci-dessus, ou des mélanges, des fusions, des combinaisons ou des couplages d'un ou plusieurs de ceux mentionnés ci-dessus.

6. Processus selon l'une quelconque des revendications précédentes, dans lequel l'entité détectable comprend l'une quelconque des protéines ou plusieurs parmi HER2, ER, PR, p16, Ki-67 et EGFR et les acides nucléiques les encodant.

7. Processus selon l'une quelconque des revendications précédentes, comprenant de plus la fourniture d'un second étalon de référence ou de sa coupe plane, l'obtention d'un second signal de référence indicateur de la quantité d'entité détectable dans un second étalon de référence, dans la coupe plane de celui-ci ou dans la zone définie ; et la comparaison du second signal obtenu en (c) par rapport au premier signal de référence et au second signal de référence.

8. Processus selon l'une quelconque des revendications précédentes, dans lequel la présence et/ou la quantité de l'entité détectable peut être révélée par un liant, de préférence un liant marqué, choisi de préférence dans le groupe constitué par : un anticorps, de préférence un anticorps susceptible d'une liaison spécifique avec l'entité détectable, un acide nucléique tel que l'ADN ou l'ARN, de préférence un acide nucléique susceptible d'une liaison spécifique avec l'entité détectable, un acide nucléique peptidique (PNA), un colorant, un colorant spécial, hématoxyline-éosine (H & E), colorant argent méthénamine Gomori (GMS), colorant acide périodique Schiff (PAS), trichrome bleu, trichrome de Masson, bleu de Prusse, Giemsa, Diff-quick, Reticulum, rouge Congo, bleu alcian, Steiner, BAAR (AFB), Papanicolaou (PAP), Gram, mucicarmin, Verhoeff-Van Gieson, colorant élastique, carbol-fuchsine et colorants de Golgi.

9. Processus selon l'une quelconque des revendications précédentes, dans lequel le signal détectable est choisi parmi le groupe constitué par : le rayonnement, la densité optique, la réflectance, la radioactivité, la fluorescence, l'activité enzymatique.

10. Processus selon l'une quelconque des revendications précédentes, dans lequel :
(a) l'étalon de référence a la forme d'une boîte rectangulaire et l'entité détectable a sensiblement la forme d'un bâtonnet droit disposé le long ou sensiblement de manière parallèle à l'axe longitudinal de l'étalon de référence ; ou
(b) la zone définie est présente dans au moins une autre coupe transversale de l'étalon de référence, comprenant de préférence une quantité similaire de l'entité détectable ; ou
(c) l'entité détectable est distribuée de manière sensiblement uniforme le long de la tige allongée ; ou
(d) l'entité détectable est présente avec sensiblement la même surface, quantité ou concentration dans toutes les coupes planes transversales de l'étalon de référence ;
ou une combinaison quelconque de ce qui précède.

11. Processus selon l'une quelconque des revendications précédentes, dans lequel le support comprend un milieu d'inclusion, dans lequel l'entité détectable est incluse, le milieu d'inclusion étant choisi de préférence dans le groupe constitué par : la glace, la cire, la paraffine, une résine acrylique, une résine méthacrylate, une résine époxyde, l'Epon, l'Araldite, le Lowicryl, le K4M, le LR White et le Durcupan

12. Processus selon l'une quelconque des revendications précédentes, dans lequel l'entité détectable est liée, de préférence couplée chimiquement, à une fibre allongée, choisie de préférence dans le groupe constitué par : une fibre de polyamide, une fibre de cellulose, une fibre de polyuréthane, une fibre de soie, une fibre de polyester, une fibre de Nylon, une fibre de rayonne et des mélanges de celles-ci.

13. Processus selon la revendication 12, dans lequel l'entité détectable est sensiblement uniforme à la fois sur les parties du coeur et de la surface de la fibre.

14. Processus selon la revendication 12, dans lequel l'entité détectable sur les portions de surface de la fibre est présente en quantité plus importante que sur les parties du coeur, de préférence dans lequel les parties du coeur de la fibre ne contiennent sensiblement pas d'entité détectable.

15. Processus selon l'une quelconque des revendications 1 à 11, dans lequel l'entité détectable est formée dans un canal allongé dans le milieu d'inclusion.

16. Processus selon l'une quelconque des revendications 12 à 15, dans lequel la fibre ou le canal a une surface de coupe transversale sensiblement uniforme d'un bout à l'autre de toute sa longueur, de préférence
dans lequel la fibre ou le canal a un diamètre compris entre 0,5 µm et 25 µm environ, de préférence entre 1,5 µm et 15 µm.

17. Processus selon l'une quelconque des revendications précédentes, dans lequel l'étalon de référence comprend :
(a) deux ou plus de deux canaux ou fibres linéaires avec des orientations sensiblement parallèles, de préférence formant un faisceau ; ou
(b) deux ou plus de deux entités détectables différentes, chacune d'elles étant disposée dans ou sur une fibre ou un canal individuel ; ou
(c) deux ou plus de deux fibres ou canaux chacun comprenant la même entité détectable, de préférence comprenant différentes quantités de l'entité détectable sur chacun ;
ou une combinaison quelconque de ce qui précède.

18. Processus selon l'une quelconque des revendications précédentes, dans lequel une coupe plane de l'étalon de référence comprend plusieurs zones sur lesquelles l'entité détectable est présente à différentes densités.

19. Processus selon l'une quelconque des revendications précédentes, dans lequel une coupe plane de l'étalon de référence comprend une première zone comprenant l'entité détectable sensiblement à une densité la rendant possible à diagnostiquer de façon significative, de préférence comprenant de plus un témoin comprenant une fibre ou un canal qui ne comprend sensiblement pas d'entité détectable.

20. Processus selon l'une quelconque des revendications précédentes pour obtenir une indication utile dans l'évaluation de la probabilité d'une maladie ou d'un état d'un individu, dans lequel la présence de l'entité détectable ou la présence d'une quantité prédéterminée de l'entité détectable, dans un échantillon biologique, est indicatrice de la probabilité d'une maladie ou d'un état dans l'échantillon biologique ou chez un individu sur lequel l'échantillon a été prélevé.

21. Processus selon l'une quelconque des revendications précédentes, dans lequel l'échantillon biologique comprend une cellule, un tissu ou un organe, de préférence une cellule, un tissu ou un organe d'un organisme soupçonné de souffrir d'une maladie ou d'un état.

22. Processus de fourniture d'une indication utile dans le diagnostic d'une maladie ou d'un état d'un individu, le processus comprenant la comparaison de la quantité d'une entité détectable dans un échantillon biologique provenant de l'individu ou dans un de ses composants à un étalon de référence au moyen d'un processus selon l'une quelconque des revendications précédentes, dans lequel on indique que l'individu est susceptible de souffrir de, ou d'être prédisposé à, une maladie ou un état si la quantité de l'entité détectable dans l'échantillon biologique ou dans le composant est semblable à, ou plus grande que, celle dans l'étalon de référence.

23. Processus d'évaluation de l'efficacité ou de la réussite d'un procédé, le processus comprenant les étapes de :
(a) fourniture d'un étalon de référence ou de la coupe plane de celui-ci, l'étalon de référence comprenant :
(i) un support ; et
(ii) une quantité d'une entité détectable portée par le support ;
dans lequel l'entité détectable a la forme d'une tige allongée dont la coupe transversale a une dimension comprise entre 0,5 µm et 100 µm ; dans lequel une quantité prédéterminée de l'entité détectable est présente dans une zone définie d'une coupe transversale de l'étalon de référence ; et
dans lequel une propriété détectable de l'entité détectable est modifiée à la suite du procédé ;
(b) la conduite du procédé sur l'étalon de référence ; et
(c) la détection d'une modification dans la propriété détectable de l'entité détectable.

24. Processus selon la revendication 23 pour l'évaluation de l'efficacité ou de la réussite d'un procédé effectué sur un échantillon, dans lequel le procédé est conduit sur l'étalon de référence ou sur la coupe plane de celui-ci sensiblement en parallèle avec l'échantillon.

25. Processus selon la revendication 23 ou 24, dans lequel une propriété détectable de l'entité détectable est modifiée suite à un procédé réussi, laquelle modification de la propriété détectable de l'entité détectable est détectée pour établir que le procédé a réussi.

26. Processus selon la revendication 23 ou 24, dans lequel une propriété détectable de l'entité détectable est modifiée suite à un procédé qui n'a pas réussi, laquelle modification de la propriété détectable de l'entité détectable est détectée pour établir que le procédé n'a pas réussi.

27. Processus selon l'une quelconque des revendications 23 à 26, dans lequel le procédé est choisi dans le groupe constitué par : un procédé d'hybridation *in situ,* un procédé immunohistochimique, le déparaffinage, le démasquage des antigènes, le blocage, le blocage biotine endogène, le blocage enzyme endogène, une étape de lavage, l'incubation avec un agent révélateur tel qu'un anticorps primaire, l'incubation avec des composants de visualisation secondaire, la coloration du chromogène, l'acquisition d'information et l'analyse par coloration.

28. Processus selon l'une quelconque des revendications 23 à 27, dans lequel le procédé est un procédé de démasquage des antigènes, et dans lequel la propriété détectable de l'entité détectable comprend le masquage et le démasquage d'un ou plusieurs épitopes.

29. Processus selon l'une quelconque des revendications 23 à 27, dans lequel l'entité détectable dans l'étalon de référence est modifiée pour masquer un ou plusieurs épitopes, dont certains ou tous sont démasqués dans un procédé de démasquage d'antigènes qui a réussi.

30. Processus selon l'une quelconque des revendications 23 à 27, dans lequel le procédé est un procédé de déparaffinage et dans lequel la propriété détectable de l'entité détectable comprend la présence ou une quantité de l'entité détectable dans l'étalon de référence suite au procédé de déparaffinage.

31. Processus selon l'une quelconque des revendications 23 à 27, dans lequel de l'entité détectable dans l'étalon de référence est soluble dans le milieu de déparaffinage, et dans lequel au moins une partie, de préférence la totalité, de l'entité détectable est enlevée suite à un procédé de déparaffinage réussi.

32. Processus selon l'une quelconque des revendications 23 à 27, dans lequel l'efficacité ou la réussite de deux ou plus de deux procédés est évaluée de préférence en parallèle.

33. Utilisation d'un étalon de référence pour valider le procédé, dans laquelle l'étalon de référence comprend :
(i) un support ; et
(ii) une quantité d'une entité détectable portée par le support ;
dans laquelle l'entité détectable a la forme d'une tige allongée dont la coupe transversale a une dimension comprise entre 0,5 µm et 100 µm ; dans laquelle une quantité prédéterminée de l'entité détectable est présente dans une zone définie de la coupe transversale de l'étalon de référence, et dans laquelle une propriété détectable de l'entité détectable est modifiée suite au procédé.

34. Utilisation selon la revendication 33, dans laquelle l'étalon de référence est utilisé comme étalon de validation de démasquage des antigènes, étalon de déparaffinage, étalon de validation de blocage, étalon de validation de lavage, étalon de validation d'anticorps primaire, étalon de validation d'anticorps secondaire, étalon de calibration ou étalon de diagnostic.

35. Ensemble comprenant un étalon de référence ou une coupe plane de celui-ci ainsi qu'une notice d'utilisation dans un processus selon l'une quelconque des revendications précédentes, dans lequel l'étalon de référence comprend :
(i) un support ; et
(ii) une quantité d'une entité détectable portée par le support ;
dans lequel l'entité détectable a la forme d'une tige allongée dont la coupe transversale a une dimension comprise entre 0,5 µm et 100 µm ; dans lequel une quantité prédéterminée de l'entité détectable est présente dans une zone définie de la coupe transversale de l'étalon de référence.

36. Ensemble selon la revendication 35, comprenant de plus un liant susceptible d'une liaison spécifique avec l'entité détectable, lequel liant produit un signal indicateur de la présence ou de la quantité de l'entité détectable dans l'étalon de référence, dans la coupe plane de celui-ci ou dans une zone définie.

37. Ensemble de diagnostic pour la détection de la présence ou d'une quantité pertinente à diagnostiquer d'une entité détectable dans un échantillon biologique, lequel ensemble de diagnostic comprend un ensemble selon la revendication 35 ou 36.

38. Ensemble ou ensemble de diagnostic selon l'une quelconque des revendications 35 à 37, accompagné d'un agent thérapeutique susceptible de soigner ou de soulager au moins un des symptômes d'une maladie ou d'un état d'un individu, de préférence comprenant un anticorps contre l'entité détectable.

39. Jeu de deux ou plus de deux coupes planes chacune obtenue à partir d'un étalon de référence, lequel étalon de référence comprend :
(i) un support ; et
(ii) une quantité d'une entité détectable portée par le support ;
dans lequel l'entité détectable a la forme d'une tige allongée dont la coupe transversale a une dimension comprise entre 0,5 µm et 100 µm ; dans lequel une quantité prédéterminée de l'entité détectable est présente dans une zone définie de la coupe transversale de l'étalon de référence, dans lequel au moins deux coupes planes du jeu comprennent une quantité différente de l'entité détectable.

40. Jeu de deux ou plus de deux coupes planes comprenant une zone définie comprenant une entité détectable, dans lequel la quantité de l'entité détectable dans la zone définie est différente entre au moins deux des coupes planes du jeu, chaque coupe plane étant obtenue à partir de l'étalon de référence comprenant :
(i) un support ; et
(ii) une quantité d'une entité détectable portée par le support ;
dans lequel l'entité détectable a la forme d'une tige allongée dont la coupe transversale a une dimension comprise entre 0,5 µm et 100 µm.
